(19) Europäisches Patentamt
European Patent Office
Office européen des brevets

(11) Veröffentlichungsnummer: **0 274 642 B1**

(12) **EUROPÄISCHE PATENTSCHRIFT**

(45) Veröffentlichungstag der Patentschrift: **11.08.93**

(21) Anmeldenummer: **87117732.5**

(22) Anmeldetag: **01.12.87**

Die Akte enthält technische Angaben, die nach dem Eingang der Anmeldung eingereicht wurden und die nicht in dieser Patentschrift enthalten sind.

(51) Int. Cl.5: **C07D 231/22**, C07D 231/24, C07D 231/26, C07D 231/32, C07D 231/52, C07D 401/04, C07D 405/04, C07D 405/06, C07D 409/04, C07D 409/06, C07D 417/04, //C07C243/22

# (54) Herbizide und fungizide Mittel auf Basis von substituierten Pyrazolin-5-on-Derivaten.

(30) Priorität: **17.12.86 DE 3643148**
**25.08.87 DE 3728278**

(43) Veröffentlichungstag der Anmeldung:
**20.07.88 Patentblatt 88/29**

(45) Bekanntmachung des Hinweises auf die Patenterteilung:
**11.08.93 Patentblatt 93/32**

(84) Benannte Vertragsstaaten:
**AT BE CH DE ES FR GB IT LI NL**

(56) Entgegenhaltungen:
**EP-A- 0 020 299**
**EP-A- 0 022 078**
**EP-A- 0 115 469**
**DD-A- 123 464**
**DE-A- 2 511 354**
**FR-A- 2 081 595**
**GB-A- 887 509**

(73) Patentinhaber: **BAYER AG**

**W-5090 Leverkusen 1 Bayerwerk(DE)**

(72) Erfinder: **Gehring, Reinhold, Dr.**
**Dasnöckel 49**
**W-5600 Wuppertal 11(DE)**
Erfinder: **Lindig, Markus, Dr.**
**Dahlienweg 16**
**W-4010 Hilden(DE)**
Erfinder: **Wroblowsky, Heinz-Jürgen, Dr.**
**Gladbacher Strasse 34**
**W-4018 Langenfeld(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ARCHIV DER PHARMAZIE, Band 318, Nr. 1, 1985, Seiten 89-91; A.KREUTZBERGER et al.: "Die Aminomethinylierung im System der 1-(Alkylphenyl)-2-pyrazolin-5-one"

JOURNAL OF THE AMERICAN CHEMICAL SOCIETY, Band 38, Nr. 8, August 1916, Seiten 1510-1517; F.B. DAINS et al.: "On the reactions of the formamidines. V. On some pyrazolone derivatives"

GAZETTA CHIMICA ITALIANA, Band 77, 1947, Seiten 3-12; M. RIDI: "Sintesi di derivati dell'aldeide antipirinica", & BEILSTEINS HANDBUCH DER ORGANISCHEN CHEMIE, E 3/4, Band 25, Teil 5, 1982, Seite 3732

ANNALI DI CHIMICA, Band 43, Nr. 12, November-Dezember 1953, Seiten 816-826; M. RIDI et al.: "Nuovi derivati antipirinici, isoantipirinici, pirazolidinici", & BEILSTEINS HANDBUCH DER ORGANISCHEN CHEMIE, E 3/4, Band 25, Teil 5, 1982, Seite 3733

CHEMICAL ABSTRACTS, Band 55, Nr. 2, 23. Januar 1961, Spalten 1583g-1584c, Columbus, Ohio, US; V.M. ZUBAROVSKII et al.: "Synthesis of thiazole derivatives. XV. Benzothiazolylpyrazolones", & ZHUR. OBSHCHEI KHIM. 1960, (30), 1585-1590

AUSTRALIAN JOURNAL OF CHEMISTRY, Band 34, 1981, Seiten 1117-1124; J.E. ROCKLEY et al.: "Structure of 5-methyl-4[(arylamino)-methylene]-2,4-dihydro-3H-pyrazol-3-ones"

JOURNAL OF HETEROCYCLIC CHEMISTRY, Band 23, Nr. 3, Mai-Juni 1986, Seiten 781-784; A. KREUTZBERGER et al.: "Antibakterielle Wirkstoffe. IX [1]. Die Aminomethinylierung im System des 3-Methyl-1-(4-nitrophenyl)-2-pyrazolin-5-ons"

ARCHIV DER PHARMAZIE, Band 319, Nr. 10, 1986, Seiten 865-871; A. KREUTZBERGER et al.: "1-(4-Chlorphenyl)-2-pyrazolin-5-on-Derivate"

ZURNAL ORGANICESKOG CHIMII, Band 13, Nr. 4, 1977, Seiten 863-868; L.V. ALAM et al.: "Complexes with organic ligands. II. Bromination of chelate compounds based on aminomethylene derivatives of some five-membered heterocycles" & CHEMICAL ABSTRACTS, Band 87, Nr. 2, 11. Juli 1977, Seite 648, Spalte 1, Zusammenfassung Nr. 15257j

CHEMICAL ABSTRACTS, Band 61, Nr. 12, 7. Dezember 1964, Spalten 14659e-14660a, Columbus, Ohio, US; B.A. PORAI-KOSHITS et al.: "Chemical transformations of N,N-disubstituted aminomethylene derivatives of pyrazolone and rhodanine" & ZH. OBSHCH. KHIM. 1964, 34(9), 2999-3005

INDIAN JOURNAL OF CHEMISTRY, Band 7, Nr. 10, 1969, Seiten 1006-1009; M.R. CHANDRAMOHAN et al.: "Studies on the application of Vilsmeier-Haack reaction to lactams: Part III. Reaction with lactams containing an additional hetero atom"

Erfinder: **Brandes, Wilhelm, Dr.**
**Eichendorffstrasse 3**
**W-5653 Leichlingen(DE)**
Erfinder: **Strang, Robert Harry, Dr.**
**Unterdorfstrasse 6A**
**W-4000 Düsseldorf 31(DE)**

**Beschreibung**

Die Erfindung betrifft die Verwendung von teilweise bekannten substituierten Pyrazolin-5-on Derivaten als Herbizide und Fungizide, neue substituierte Pyrazolin-5-on Derivate und Verfahren zu ihrer Herstellung.

Es ist bereits bekannt, daß substituierte Pyrazolin-5-one, wie beispielsweise 4-(Cyanmethyloximino)-3-methyl-1-phenyl-pyrazolin-5-on, fungizide Eigenschaften besitzen (vgl. EP-OS 0 166 171).

Es ist weiter bereits bekannt, daß substituierte Pyrazolin-5-one, wie beispielsweise [4-(2,4-Dichlorbenzoyl)-1,3-dimethylpyrazol-5-yl]-4-methylphenylsulfonat herbizide Eigenschaften besitzen (vgl. DE-OS 25 13 750).

Die Wirkung dieser Verbindungen ist jedoch insbesondere bei niedrigen Aufwandmengen- und konzentrationen nicht immer in allen Anwendungsbereichen völlig zufriedenstellend.

Weiterhin sind 1-(4-Chlorphenyl)-pyrazolin-5-on Derivate, wie z.B. 1-(4-Chlorphenyl)-3-methyl-4-piperidino-methylen-pyrazolin-5-on, 1-(4-Chlorphenyl)-3-methyl-4-morpholino-methylen-pyrazolin-5-on, 1-(4-Chlorphenyl)-4-[4-(fluorphenylamino)-methylen]-3-methyl-pyrazolin-5-on, 1-(4-Chlorphenyl)-3-methyl-4-aminomethylen-pyrazolin-5-on und 1-(2-Ethylphenyl)-3-methyl-4-aminomethylen-pyrazolin-5-on bekannt. Diese Verbindungen haben u.a. eine stark entzündungshemmende und fungizide Wirkung, aber auch eine herbizide Wirkung gegen dikotyle Unkräuter ist erwähnt (vgl. Kreuzberger u.a. Arch. Pharm. (Weinheim) 319, 865-871 (1986), 318, 89-91 (1985) und 319, 18 (1986)).

Ferner sind 4-Aminomethylen-pyrazolin-5-on Derivate bekannt, wie z.B. 1-(4-Bromphenyl)-3-methyl-4-methylaminomethylen-pyrazolin-5-on, die als Komplexbildner beschrieben sind (vgl. Alam u.a. Zh. Org. Khim. 1977, 13(4), 863-868 (Russ.)) oder 3-Methyl-4-chlorphenylaminomethylen-pyrazolin-5-one, mit denen Strukturuntersuchungen durchgeführt wurden (vgl. Jean E. Rockley u.a. Aust. J. Chem. 1981, 34(5), 1117-1124 (Engl.)).

Außerdem ist das 1-(4-Chlorphenyl)-3-methyl-4-anilino-methylen-pyrazolin-5-on als Ausgangsverbindung zur Herstellung von Nickelkomplexen von Azinen bekannt (vgl. EP 0 020 299).

Weiterhin ist das 1-Phenyl-3-(4-methoxyphenyl)-4-N,N-dimethylaminomethyliden-pyrazolin-5-on als Ausgangsverbindung zur Herstellung von pharmazeutischen Produkten bekannt (vgl. GB 887.509).

Es wurde gefunden, daß die teilweise bekannten substituierten Pyrazolin-5-on Derivate der Formel (I)

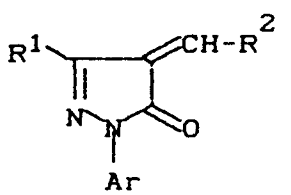

(I)

in welcher

R¹ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter Ar aufgeführten Arylsubstituenten in Frage kommen; $R^1$ weiterhin für Halogenalkenyl mit 2 bis 6 Kohlenstoffatomen und 1 bis 10 gleichen oder verschiedenen Halogenatomen, wie insbesondere Fluor undChlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegegebenenfalls durch Fluor, Chlor, Methyl und/oder Ethyl substituierten Heterocyclus, insbesondere Furanyl, Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter Ar aufgeführten Arylsubstituenten infrage

kommen, $R^1$ weiterhin für die Gruppierungen -NH-CO-$R^{10}$ oder -CO-O-$R^{11}$ steht, worin

$R^{10}$ und $R^{11}$ jeweils unabhängig voneinander für $C_1$-$C_4$-Alkyl oder Phenyl stehen,

$R^2$ für die Gruppierungen -$NHR^3$, -$NR^4R^5$ oder -$NHOR^6$ steht, worin

$R^3$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- und Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratome, Alkoxyalkyl mit jeweils 1 bis 8 Kohlenstoffatomen im Alkoxy- oder Alkylteil, für gegebenenfalls einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil, für gegebenenfalls einfach bis fünffach, gleich oder verschieden substituiertes Aryl mit 6 bis 10 Kohlenstoffatomen steht, wobei als Arylsubstituenten jeweils infrage kommen: Halogen, geradkettiges oder verzweigtes Alkyl mit 1 bis 4 Kohlenstoffatomen und Dialkylamino mit jeweils 1 bis 4 Kohlenstoffatomen im Alkylteil, $C_1$-$C_4$-Alkoxy und Halogen-$C_1$-$C_4$-alkyl,

$R^4$ für Alkyl mit 1 bis 6 Kohlenstoffatomen steht,

$R^5$ für Alkyl mit 1 bis 6 Kohlenstoffatomen steht oder

$R^4$ und $R^5$ gemeinsam mit dem Stickstoffatom, an welches sie gebunden sind, für einen heterocyclischen 5- oder 6-gliedrigen Ring, der Sauerstoff, Schwefel und/oder Stickstoff als weitere Heteroatome enthalten kann, stehen,

$R^6$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- oder Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratome, für gegebenenfalls einfach bis mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten Halogen, $C_1$-$C_4$-Alkyl, Halogen-$C_1$-$C_4$-alkyl und Nitro infrage kommen, und

Ar für gegebenenfalls einfach bis mehrfach, gleich oder verschieden substituiertes Aryl mit 6 bis 10 Kohlenstoffatomen, insbesondere Phenyl oder Naphthyl, steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy; oder $C_1$-$C_4$-Alkylthio; $C_3$-$C_6$-Alkinyloxy; Halogen-($C_1$-$C_4$)-alkyl, Halogen-($C_1$-$C_4$)-alkoxy oder Halogen($C_1$-$C_4$)alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen; und Di-($C_1$-$C_4$)-alkylamino für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei Ar aufgeführten Arylsubstituenten infrage kommen, oder für die Gruppe

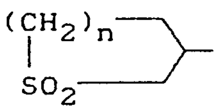

steht,

wobei

n für die Zahlen 1 oder 2 steht, und deren Salze,

ausgenommen die Verbindungen 1-(4-Chlorphenyl)-3-methyl-4-piperidino-methylen-pyrazolin-5-on, 1-(4-Chlorphenyl)-3-methyl-4-morpholino-methylen-pyrazolin-5-on, 1-(4-Chlorphenyl)-4-[4-(fluorphenylamino)-methylen]-3-methyl-pyrazolin-5-on, 1-(4-Chlorphenyl)-3-methyl-4-aminomethylen-pyrazolin-5-on (vgl. Kreutzberger, A. und Kolter, K., Arch. der Phar., 319, 10, 865-871, (1986)) und 4-Aminomethylen-3-ethoxycarbonyl-1-phenyl-2-pyrazolin-5-on,

starke herbizide und fungizide Eigenschaften aufweisen.

Die Verbindungen der Formel (I) können als geometrische Isomere (E/Z-Isomere) oder Isomerengemische unterschiedlicher Zusammensetzung vorliegen. Die Verwendung sowohl der reinen Isomeren als auch der Isomerengemische werden erfindungsgemäß beansprucht.

Außerdem können einige der Verbindungen der Formel (I) im tautomeren Gleichgewicht vorliegen:

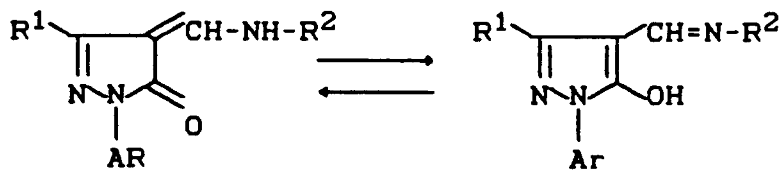

Im nachfolgenden wird der Einfachheit halber stets von der Verwendung von Verbindungen der Formel (I) gesprochen, obwohl sowohl die reinen Verbindungen als auch ihre Gemische mit unterschiedlichen Anteilen der tautomeren Verbindungen gemeint sind.

Überraschenderweise zeigen die teilweise bekannten substituierten Pyrazolin-5-on Derivate der Formel (I) bei entsprechenden Anwendungskonzentrationen bessere fungizide Eigenschaften, als das aus dem Stand der Technik bekannte 4-(Cyanmethyloximino)-3-methyl-1-phenyl-pyrazolin-5-on, welches ein konstitutionell ähnlicher Wirkstoff gleicher Wirkungsart ist. Außerdem zeigen die teilweise bekannten substituierten Pyrazolin-5-on Derivate der Formel (I) bei entsprechenden Anwendungskonzentrationen auch bessere herbizide Eigenschaften, als das aus dem Stand der Technik bekannte [4-(2,4-Dichlorbenzoyl)-1,3-dimethylpyrazol-5-yl]-4-methylphenylsulfonat, welches ein konstitutionell ähnlicher Wirkstoff gleicher Wirkungsart ist.

Die erfindungsgemäß verwendbaren substituierten Pyrazolin-5-on Derivate sind durch die Formel (I) allgemein definiert.

Bevorzugt werden die Verbindungen der Formel (I) verwendet, in welcher

$R^1$ für Wasserstoff, geradkettiges oder verzweigtes Alkyl mit 1 bis 4 Kohlenstoffatomen, Cycloalkyl mit 3 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach bis dreifach gleich oder verschieden substituiertes Phenyl genannt seien und wobei als Phenylsubstituenten die unter Ar aufgeführten Arylsubstituenten infrage kommen; $R^1$ weiterhin für Halogenalkenyl mit 3 oder 4 Kohlenstoffatomen und 1 bis 5 gleichen oder verschiedenen Halogenatomen wie insbesondere Fluor und Chlor, Alkoxy mit 1 bis 4 Kohlenstoffatomen, Alkoxyalkyl mit jeweils 1 bis 4 Kohlenstoffatomen in den einzelnen Alkylteilen, Alkylthioalkyl, Alkylsulfonylalkyl oder Alkylsulfinylalkyl mit jeweils 1 bis 4 Kohlenstoffatomen in den einzelnen Alkylteilen, Alkoxycarbonylalkyl mit 1 oder 2 Kohlenstoffatomen im Alkoxyteil und 1 oder 2 Kohlenstoffatomen im Alkylteil, einen 5-oder 6-gliedrigen, gegebenenfalls durch Fluor, Chlor, Methyl und/oder Ethyl substituierten Heterocyclus, insbesondere Furanyl, Thienyl; Furanylmethyl, Thienylmethyl oder

$R^1$ weiterhin für jeweils gegebenenfalls einfach bis fünffach, gleich oder verschieden substituiertes Phenyl, Naphthyl, Benzyl, Phenylethyl, Phenoxymethyl, Phenoxyethyl, Phenylthiomethyl oder Phenylthioethyl steht, wobei als Phenylsubstituenten jeweils die unter Ar aufgeführten Phenylsubstituenten infrage kommen; $R^1$ weiterhin für die Gruppierungen $-NH-CO-R^{10}$ oder $-CO-O-R^{11}$ steht, worin

$R^{10}$ und $R^{11}$ jeweils unabhängig voneinander für $C_1$-$C_4$-Alkyl oder Phenyl stehen,

$R^2$ für die Gruppierungen $-NHR^3$, $-NR^4R^5$ oder $-NHOR^6$ steht, worin

$R^3$ für Wasserstoff, geradkettiges oder verzweigtes Alkyl mit 1 bis 4 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, geradkettiges oder verzweigtes Alkenyl mit 2 bis 6 Kohlenstoffatomen, Halogenalkenyl mit 2 bis 4 Kohlenstoffatomen und 1 bis 7 gleichen oder verschiedenen Halogenatomen, wie insbesondere Fluor- und Chloratomen, Alkoxyalkyl mit jeweils 1 bis 4 Kohlenstoffatomen im Alkoxy- oder Alkylteil, für gegebenenfalls einfach bis fünffach, gleich oder verschieden substituiertes Phenylalkyl mit 1 bis 3 Kohlenstoffatomen im geradkettigen oder verzweigten Alkylteil, für gegebenenfalls einfach bis fünffach, gleich oder verschieden substituiertes Phenyl

steht, wobei als Phenylsubstituenten jeweils infrage kommen: Halogen, geradkettiges oder verzweigtes Alkyl mit 1 oder 2 Kohlenstoffatomen und Dialkylamino mit jeweils 1 oder 2 Kohlenstoffatomen im Alkylteil, $C_1$-$C_2$-Alkoxy, Halogen-$C_1$-$C_2$-alkyl,

$R^4$ für Alkyl mit 1 bis 4 Kohlenstoffatomen steht,

$R^5$ für Alkyl mit 1 bis 4 Kohlenstoffatomen steht oder

$R^4$ und $R^5$ gemeinsam mit dem Stickstoffatom, an welches sie gebunden sind, für einen heterocyclischen 5- oder 6-gliedrigen Ring, der Sauerstoff, Schwefel und/oder Stickstoff als weitere Heteroatome enthalten kann, stehen,

$R^6$ für Wasserstoff, geradkettiges oder verzweigtes Alkyl mit 1 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, geradkettiges oder verzweigtes Alkenyl mit 2 bis 6 Kohlenstoffatomen, Halogenalkenyl mit 2 oder 3 Kohlenstoffatomen und 1 bis 5 gleichen oder verschiedenen Halogenatomen, wie insbesondere Fluor-und Chloratomen, für gegebenenfalls einfach bis fünffach, gleich oder verschieden substituiertes Phenylalkyl mit 1 bis 3 Kohlenstoffatomen im Alkylteil steht, wobei als Phenylsubstituenten Halogen, $C_1$-$C_2$-Alkyl, Halogen-$C_1$-$C_2$-alkyl und Nitro infrage kommen, und

Ar für gegebenenfalls einfach bis fünffach, gleich oder verschieden substituiertes Phenyl steht, wobei als Phenylsubstituenten infrage kommen: Halogen; Nitro; Cyano; Carboxyl; Alkoxycarbonyl mit 1 bis 3 Kohlenstoffatomen; $C_1$-$C_3$-Alkyl, $C_1$-$C_3$-Alkoxy; $C_1$-$C_3$-Alkylthio, $C_3$-$C_4$-Alkinyloxy, Halogen-($C_1$-$C_3$)-alkyl, Halogen-($C_1$-$C_4$)-alkoxy oder Halogen-($C_1$-$C_4$)-alkylthio mit jeweils 1 bis 7 gleichen oder verschiedenen Halogenatomen; Phenyl; $C_1$-$C_3$-Alkylsulfonyl und Halogen-($C_1$-$C_3$)-alkylsulfonyl mit jeweils 1 bis 7 gleichen oder verschiedenen Halogenatomen; und Di-($C_1$-$C_3$)-alkylamino für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei Ar aufgeführten Phenylsubstituenten infrage kommen oder für die Gruppe

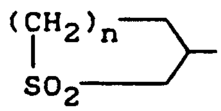

steht,
wobei

n für die Zahlen 1 oder 2 steht, und deren Salze ausgenommen die bereits bei der Formel (I) genannten Verbindungen.

Besonders bevorzugt werden erfindungsgemäß die Verbindungen der Formel (I) verwendet, in welcher

$R^1$ für Wasserstoff, Methyl, Ethyl, n-Propyl, i-Propyl, n-Butyl, i-Butyl, tert.-Butyl, Cyclopropyl, Cyclohexyl, Trifluormethyl, Vinyl, Allyl, Butenyl, Propargyl, 2-Phenylvinyl, Chlorallyl, Methoxy, Ethoxy, Methoxymethyl, Ethoxymethyl, Methylthiomethyl, Ethylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Furanyl, Furanylmethyl, Thienyl, Thienylmethyl, Pyridyl, Phenylthio, Ethoxycarbonylmethyl, Methoxycarbonylmethyl,

$R^1$ weiterhin für jeweils gegebenenfalls einfach bis dreifach, gleich oder verschieden substituiertes Phenyl, Naphthyl, Benzyl, Phenylethyl, Phenoxymethyl oder Phenylthiomethyl steht, wobei als Phenylsubstituenten jeweils die unter Ar aufgeführten Phenylsubstituenten infrage kommen; $R^1$ weiterhin für die Gruppierungen -NH-CO-$R^{10}$ oder -CO-O-$R^{11}$ steht, worin

$R^{10}$ und $R^{11}$ jeweils unabhängig voneinander für Methyl, Ethyl oder Phenyl stehen,

$R^2$ für die Gruppierungen -NH$R^3$, -N$R^4R^5$ oder -NHO$R^6$ steht, worin

$R^3$ für Wasserstoff, Methyl, Ethyl, n-Propyl, i-Propyl, i-Butyl, 2,2-Dimethylpropyl, n-Hexyl, Trifluormethyl, 2-Chlorethyl, 3-Chlorpropyl, Propenyl, Methoxymethyl, Methoxyethyl, Methoxypropyl, Ethoxymethyl, Ethoxyethyl, 3-Chlorallyl, α-Methylbenzyl, gegebenenfalls einfach bis fünffach, gleich oder verschieden substituiertes Phenyl steht, wobei als Phenylsubstituenten infrage kommen: Fluor, Chlor, Methyl, Methoxy, Ethoxy, Trifluorme-

thyl, Trifluorethyl, Dimethylamino,

$R^4$ für Methyl oder Ethyl steht,

$R^5$ für Methyl oder Ethyl steht, oder

$R^4$ und $R^5$ gemeinsam mit dem Stickstoffatom, an welches sie gebunden sind, für Piperidinyl, Piperazinyl, Morpholinyl oder Thiomorpholinyl stehen,

$R^6$ für Wasserstoff, Methyl, Ethyl, i-Propyl, Trifluormethyl, 2-Chlorethyl, 3-Chlorpropyl, Propenyl, 3-Chlorallyl, gegebenenfalls einfach bis fünffach, gleich oder verschieden substituiertes Benzyl steht, wobei als Phenylsubstituenten Fluor, Chlor, Methyl, Ethyl, Trifluormethyl und Nitro infrage kommen und

Ar für gegebenenfalls einfach bis fünffach, gleich oder verschieden substituiertes Phenyl steht, wobei als Phenylsubstituenten infrage kommen: Fluor, Chlor, Nitro, Cyano, Carboxyl, Methoxycarbonyl, Ethoxycarbonyl, Methyl, Ethyl, Methoxy, Ethoxy, Propargyloxy, Trifluormethyl, 2-Chlorethyl, 3-Chlorpropyl, Methylthio, Ethylthio, Trifluormethoxy, Trifluormethylthio, Phenyl, Methylsulfonyl, Trifluormethylsulfonyl; Dimethylamino, Diethylamino, ferner für jeweils einfach bis dreifach, gleich oder verschieden substituiertes Pyridyl, Benzthiazolyl oder Benzoxazolyl steht, wobei als Substituenten Nitro, Chlor, Cyano, Trifluormethyl, Methyl, Ethyl, Methoxy, Ethoxy und Trifluormethoxy genannt seien oder für die Gruppe

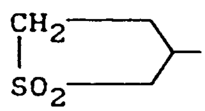

steht, und für deren Salze,

ausgenommen die bei der Formel (I) ausgeschlossenen Verbindungen.

Einige der erfindungsgemäß verwendbaren Verbindungen der Formel (I) sind nicht vorbeschrieben, so sind die substituierten Pyrazolin-5-on Derivate der Formel (Ia)

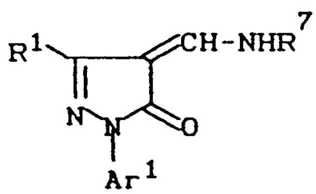

(Ia)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil

steht, wobei als Arylsubstituenten die unter $Ar^1$ aufgeführten Arylsubstituenten infrage kommen, $R^1$ weiterhin für die Gruppierungen $-NH-CO-R^{10}$ oder $-CO-O-R^{11}$ steht, worin

$R^{10}$ und $R^{11}$ jeweils unabhängig voneinander für $C_1$-$C_4$-Alkyl oder Phenyl stehen,

$R^7$ für geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- und Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratomen, Alkoxyalkyl mit jeweils 1 bis 8 Kohlenstoffatomen im Alkoxy- und Alkylteil, für gegebenenfalls einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil, für gegebenenfalls einfach bis fünffach, gleich oder verschieden substituiertes Aryl mit 6 bis 10 Kohlenstoffatomen steht, wobei als Arylsubstituenten jeweils infrage kommen: Halogen, geradkettiges oder verzweigtes Alkyl mit 1 bis 4 Kohlenstoffatomen und Dialkylamino mit jeweils 1 bis 4 Kohlenstoffatomen im Alkylteil, $C_1$-$C_4$-Alkoxy oder Halogen-$C_1$-$C_4$-alkyl,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen; und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

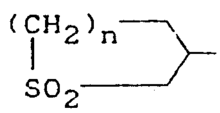

steht,

wobei

n für die Zahlen 1 oder 2 steht, und deren Salze,

ausgenommen die Verbindungen

1-(4-Chlorphenyl)-3-methyl-4-piperidinomethylen-2-pyrazolin-5-on [Arch. Pharm. 319, 865 (1986)]
1-(4-Chlorphenyl)-3-methyl-4-morpholinomethylen-2-pyrazolin-5-on [Arch. Pharm. 319, 865 (1986)]
1-(4-Chlorphenyl)-4-[4-(fluorphenylamino)methylen]-3-methyl-2-pyrazolin-5-on [Arch. Pharm. 319, 865 (1986)]
4-m-Toluido-methylen-1-p-tolyl-3-methyl-2-pyrazolin-5-on [J. Am. Chem. Soc. 38, 8, 1509 (1916)]
4-o-Toluido-methylen-1-p-tolyl-3-methyl-2-pyrazolin-5-on [J. Am. Chem. Soc. 38, 8, 1509 (1916)]
1-p-Tolyl-3-methyl-4-o-ethoxyanilinomethylen-2-pyrazolin-5-on [J. Am. Chem. Soc. 38, 8, 1509 (1916)]
1-p-Tolyl-3-methyl-4-p-bromoanilinomethylen-2-pyrazolin-5-on [J. Am. Chem. Soc, 38, 8, 1509 (1916)]
1-p-Tolyl-3-methyl-4-anilinomethylen-2-pyrazolin-5-on [J. Am. Chem. Soc. 38, 8, 1509 (1916)]
1-o-Tolyl-3-methyl-4-m-xylidomethylen-2-pyrazolin-5-on [J. Am. Chem. Soc. 38, 8, 1509 (1916)]
1-o-Tolyl-3-methyl-4-o-ethoxyanilinomethylen-2-pyrazolin-5-on [J, Am. Chem. Soc. 38, 8, 1509 (1916)]
1-o-Tolyl-3-phenyl-4-anilinomethylen-2-pyrazolin-5-on [J. Am. Chem. Soc, 38, 8, 1509 (1916)]
1-o-Tolyl-3-phenyl-4-m-xylidomethylen-2-pyrazolin-5-on [J. Am. Chem. Soc, 38, 8, 1509 (1916)]
1-o-Tolyl-3-phenyl-4-p-chloranilinomethylen-2-pyrazolin-5-on [J. Am. Chem. Soc. 38, 8, 1509 (1916)]
4-p-Bromanilinomethylen-3-phenyl-1-o-tolyl-2-pyrazolin-5-on [J. Am. Chem. Soc, 38, 8, 1509 (1916)]
4-m-Bromanilinomethylen-3-phenyl-1-o-tolyl-2-pyrazolin-5-on [J. Am. Chem. Soc. 38, 8, 1509 (1916)]
1-p-Bromphenyl-3-phenyl-4-anilinomethylen-2-pyrazolin-5-on (J. Am. Chem. Soc. 38, 8, 1509 (1916)]
1-(4-Ethoxyphenyl)-3-methyl-4-p-ethoxyanilinomethylen-2-pyrazolin-5-on [Gazetta chim. laliana 77, 3 (1947)-]
4-o-Aminoanilinomethylen-1-p-ethoxyphenyl-3-methyl-2-pyrazolin-5-on [Annali di Chimica 43, 12, 815

(1953)]
4-Anilinomethylen-1-p-ethoxyphenyl-3-methyl-2-pyrazolin-5-on [Beilstein E. 3/4, 25, 3731-3810 (1982)]
1-(2-Methyl-5-benzothiazolyl)-3-methyl-4-phenylaminomethylen-5-pyrazolon [Zh. Obshch. Khim. 30, 1585 (1960)]
1-p-Chlorphenyl-3-methyl-4-anilinomethylen-2-pyrazolin-5-on [EP-A-0 020 299]
1-m-Chlorphenyl-3-methyl-4-anilinomethylen-2-pyrazolin-5-on [EP-A-0 020 299]
1-m-Trifluormethylphenyl-3-methyl-4-anilinomethylen-2-pyrazolin-5-on [EP-A-0 020 299]
1-o,o-Dichlorphenyl-3-methyl-4-anilinomethylen-2-pyrazolin-5-on EP-A-0 020 299]
1-m-Sulfamoylphenyl-3-methyl-4-anilinomethylen-2-pyrazolin-5-on [EP-A-0 020 299]
4-Methylaminomethylen-1-p-bromphenyl-3-methyl-2-pyrazolin-5-on [Zh. Org. Khim. 13, 863 (1977)]
neu.

Es wurden weiter die neuen substituierten Pyrazolin-5-on Derivate der Formel (Ib) gefunden,

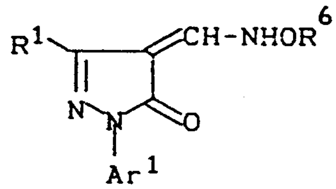

(Ib)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl oder Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen; $R^1$ weiterhin für die Gruppierungen -NH-CO-$R^0$ oder -CO-O-$R^{11}$ steht, worin

$R^{10}$ und $R^{11}$ jeweils unabhängig voneinander für $C_1$-$C_4$-Alkyl oder Phenyl stehen,

$R^6$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- und Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratomen, für gegebenenfalls einfach bis mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten Halogen, $C_1$-$C_4$-Alkyl, Halogen-$C_1$-$C_4$-alkyl und Nitro infrage kommen, und

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und

Halogen-($C_1$-$C_4$)-alkylsulfonyl mit jeweils 1 bis 9 gleichen oder verschiedenen Halogenatomen; und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

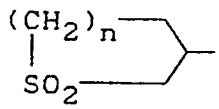

steht,

wobei

n für die Zahlen 1 oder 2 steht, und deren Salze.

Auch die substituierten Pyrazolin-5-on Derivate der Formel (Ic)

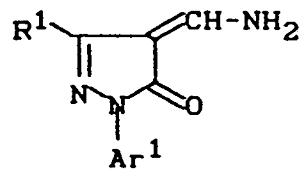

(Ic)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; Halogenalkenyl mit 2 bis 6 Kohlenstoffatomen und 1 bis 10 gleichen oder verschiedenen Halogenatommen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl, mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen, $R^1$ weiterhin für die Gruppierungen -NH-CO-$R^{10}$ oder -CO-O-$R^{11}$ steht, worin

$R^{10}$ und $R^{11}$ jeweils unabhängig voneinander für $C_1$-$C_4$-Alkyl oder Phenyl stehen,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthic mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

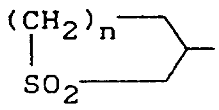

steht,
wobei

n für die Zahlen 1 oder 2 steht, und deren Salze,

ausgenommen die Verbindungen 4-Aminomethylen-1-(2-ethyl-phenyl)-3-methyl-2-pyrazolin-5-on, 4-Aminomethylen-1-(4-chlorphenyl)-3-methyl-2-pyrazolin-5-on, 4-Aminomethylen-3-methyl-1-(4-nitrophenyl)-2-pyrazolin-5-on, sind neu.

Nicht vorbeschrieben sind auch die substituierten Pyrazolin-5-on Derivate der Formel (Id)

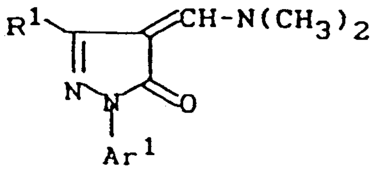

(Id)

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl, mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen, $R^1$ weiterhin für die Gruppierungen $-NH-CO-R^{10}$ oder $-CO-O-R^{11}$ steht, worin

$R^{10}$ und $R^{11}$ jeweils unabhängig voneinander für $C_1$-$C_4$-Alkyl oder Phenyl stehen,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen und Di-($C_1$-$C_4$)-alkylamino; ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

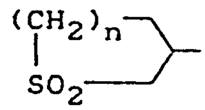

steht,

wobei

n für die Zahlen 1 oder 2 steht, und deren Salze,

ausgenommen die Verbindungen 1-(4-Nitrophenyl)-3-methyl-4-N,N-dimethylamino-methyliden-pyrazolin-5-on, 1-(4-(Chlorphenyl)-3-(2-nitrophenyl)-4-N,N-dimethylamino-methyliden-2-pyrazolin-5-on, 1-(3-Trifluormethylphenyl)-3-phenyl-4-N,N-dimethylaminomethyliden-2-pyrazolin-5-on, 1-p-Sulfophenyl-3-methyl-4-N,N-dimethylaminomethyliden-2-pyrazolin-5-on,

4-N,N-dimethylaminomethyliden-1-p-chlorphenyl-3-methyl-2-pyrazolin-5-on,

Nicht vorbeschrieben sind auch die substituierten Pyrazolin-5-on Derivate der Formel (If),

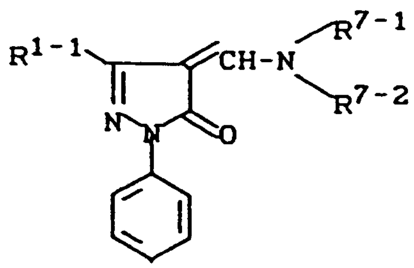

(If)

in welcher

$R^{1-1}$ für $C_1$-$C_8$-Alkoxy, Alkenyl mit 2 bis 6 Kohlenstoffatomen, gegebenenfalls substituiert durch unsubstituiertes Phenyl oder durch einfach bis dreifach, gleich oder verschieden substituiertes Phenyl, wobei als Phenylsubstituenten $C_1$-$C_4$-Alkyl, $C_1$-$C_4$-Alxoxy, Halogen-($C_1$-$C_4$)alkyl und Halogen-($C_1$-$C_4$)alkoxy genannt seien, $R^{1-1}$ weiterhin für jeweils einfach bis fünffach, gleich oder verschieden substituiertes Aryl mit 6 bis 10 Kohlenstoffatomen oder gegebenenfalls substituiertes Aralkyl mit 6 bis 10 Kohlenstoffatomen im Arylteil und 1 bis 4 Kohlenstoffatomen im Alkylteil, wobei als Arylsubstituenten jeweils Halogen, Nitro, Cyano, Carboxyl, $C_1$-$C_4$-Alkoxycarbonyl, $C_1$-$C_4$-Alkyl, $C_1$-$C_4$-Alkoxy, Halogen-($C_1$-$C_4$)-alkyl, Halogen-($C_1$-$C_4$)-alkoxy, Halogen-($C_1$-$C_4$)alkylthio, Phenyl, $C_1$-$C_4$-Alkylsulfonyl, Halogen-($C_1$-$C_4$)-alkylsulfonyl und Di-($C_1$-$C_4$)alkylamino genannt seien; $R^{1-1}$ ferner für einen 5- oder 6-gliedrigen gegebenenfalls durch Fluor, Chlor, Methyl und/oder Ethyl substituierten Heterocyclus, der ein oder zwei gleiche oder verschiedene Heteroatome, insbesondere Stickstoff, Sauerstoff und Schwefel, enthalten kann; für jeweils gegebenenfalls durch Fluor, Chlor, Methyl und/oder Ethyl substituiertes Furanyl-$C_1$-$C_4$-alkyl oder Thienyl-$C_1$-$C_4$-alkyl steht, oder

$R^{1-1}$ weiterhin für die Gruppe -NH-CO-$R^{10}$ steht, wobei

$R^{10}$ für $C_1$-$C_6$-Alkyl oder Phenyl steht,

$R^{7-1}$ für Wasserstoff, $C_1$-$C_8$-Alkyl, Halogen-$C_1$-$C_6$-alkyl, $C_2$-$C_6$-Alkenyl, Halogen-$C_2$-$C_6$-alkenyl, $C_1$-$C_8$-Alkoxy-$C_1$-$C_8$-alkyl, gegebenenfalls einfach bis fünffach, gleich oder verschieden substituiertes Aralkyl mit 6 bis 10 Kohlenstoffatomen im Arylteil und 1 bis 4 Kohlenstoffatomen im Alkylteil oder für gegebenenfalls einfach bis fünffach, gleich oder verschieden substituiertes Aryl mit 6 bis 10 Kohlenstoffatomen steht, wobei als Arylsubstituenten jeweils Halogen, $C_1$-$C_4$-Alkyl, $C_1$-$C_4$-Alkoxy, Halogen-$C_1$-$C_4$-alkyl und Di-($C_1$-$C_4$)-alkylamino genannt seien, und

$R^{7-2}$ für Wasserstoff oder Methyl steht,

ausgenommen die Verbindungen

1-Phenyl-3-(4-methoxyphenyl)-4-N,N-dimethylaminomethyliden-2-pyrazolin-5-on

4-N,N-dimethylaminomethyliden-3-(4-formamido-2-pyridyl)-1-phenyl-2-pyrazolin-5-on

1-m-Trifluormethylphenyl-1-phenyl-4-dimethylaminomethylen-2-pyrazolin-5-on

4-N,N-dimethylaminomethylen-1,3-diphenyl-2-pyrazolin-5-on

4-Methylaminomethylen-1,3-diphenyl-2-pyrazolin-5-on

4-[4-Bromanilinomethylen]-1,3-diphenyl-2-pyrazolin-5-on

4-p-Phenetidinomethylen-1,3-diphenyl-2-pyrazolin-5-on
4-Aminomethylen-1,3-diphenyl-2-pyrazolin-5-on
3-Methyl-1-phenyl-4-[4-phenylazoanilinomethylen]-2-pyrazolin-5-on
3-Methyl-4-p-phenetidinomethylen-1-phenyl-2-pyrazolin-5-on
4-Anilinomethylen-3-methyl-1-phenyl-2-pyrazolin-5-on
4-Aminomethylen-3-methyl-1-phenyl-2-pyrazolin-5-on.

Weiterhin wurde gefunden, daß die neuen substituierten Pyrazolin-5-on Derivate der Formel (Ia)

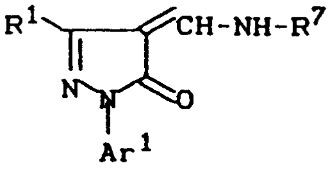

(Ia)

in welcher

$R^1$, $Ar^1$ und $R^7$ die oben angegebenen Bedeutungen haben, ausgenommen die bereits vorher unter der Formel (Ia) genannten Verbindungen,

erhalten werden, indem man Amine der Formel (II)

$H_2N\text{-}R^7$ (II)

in welcher

$R^7$ die oben angegebenen Bedeutungen hat,

α) mit den ebenfalls zur Erfindung gehörenden, neuen 4-(Dimethylamino-methyliden)-pyrazolin-5-on Derivaten der Formel (Id)

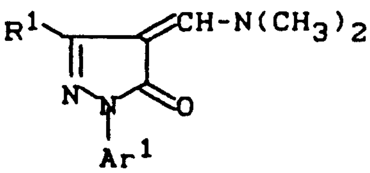

(Id)

in welcher

$R^1$ und $Ar^1$ die oben angegebenen Bedeutungen haben,

gegebenenfalls in Gegenwart von Verdünnungsmitteln oder

β) mit 4-Formyl-pyrazolin-5-on Derivaten der Formel (IV)

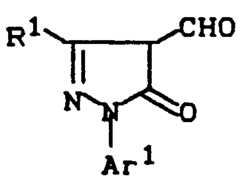

(IV)

in welcher

$R^1$ und $Ar^1$ die oben angegebenen Bedeutungen haben,

gegebenenfalls in Gegenwart von Verdünnungsmitteln umsetzt.

Weiter wurde gefunden, daß die neuen substituierten Pyrazolin-5-on Derivate der Formel (Ib)

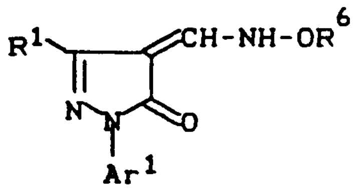

(Ib)

in welcher

$R^1$, $R^6$ und $Ar^1$ die oben angegebenen Bedeutungen haben,

erhalten werden, indem man die erfindunggemäßen 4-(Dimethylaminomethyliden)-pyrazolin-5-on Derivate der Formel (Id)

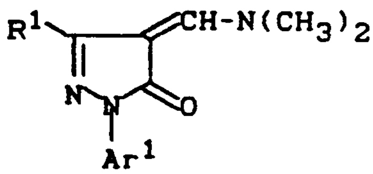

(Id)

in welcher

$R^1$ und $Ar^1$ die oben angegebene Bedeutung haben

mit Hydroxylaminen oder den entsprechenden Hydrochloriden der Formel (V)

$H_2N\text{-}OR^6$ (V)

in welcher

$R^6$ die oben angegebenen Bedeutungen hat,

gegebenenfalls in Gegenwart von Verdünnungsmitteln umsetzt.

Weiterhin wurde gefunden, daß die neuen substituierten Pyrazolin-5-on Derivate der Formel (Ic)

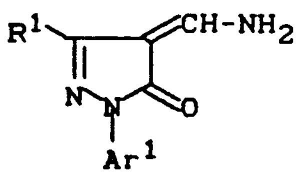

(Ic)

in welcher

$R^1$ und $Ar^1$ die oben angegebenen Bedeutungen haben, ausgenommen die bei der Formel (Ib) ausgeschlossenen Verbindungen

erhalten werden, indem man

α) die neuen, zur Erfindung gehörenden 4-(Dimethylaminomethyliden)-pyrazolin-5-on Derivate der Formel (Id)

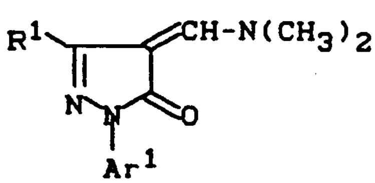

(Id)

in welcher

$R^1$ und $Ar^1$ die oben angegebenen Bedeutungen haben,

mit Ammoniak, gegebenenfalls in Gegenwart von Verdünnungsmitteln umsetzt,

oder

*β*) Pyrazolin-5-on der Formel (VI)

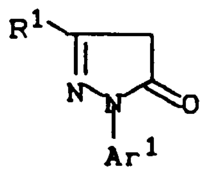

(VI)

in welcher

$R^1$ und $Ar^1$ die oben angegebenen Bedeutungen haben,

mit 1,3,5-Triazin der Formel (VIII)

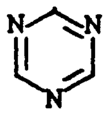

(VII)

gegebenenfalls in Gegenwart von Verdünnungsmitteln umsetzt.

Weiterhin wurde gefunden, daß die neuen substituierten Pyrazolin-5-on Derivate der Formel (Id)

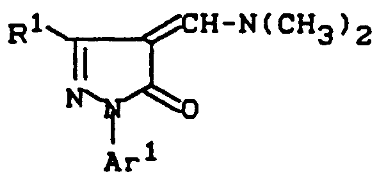

(Id)

in welcher

$R^1$ und $Ar^1$ die oben angegebenen Bedeutungen haben,

ausgenommen die Verbindungen 1-(4-Nitro-phenyl)-3-methyl-4-N,N-dimethylamino-methyliden-pyrazolin-5-on und 1-(4-Sulfophenyl)-3-methyl-4-N,N-dimethylaminomethyliden-pyrazolin-5-on erhalten werden, indem man Pyrazolin-5-on Derivate der Formel (VI)

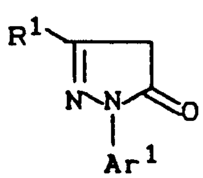

(VI)

in welcher

$R^1$ und $Ar^1$ die oben angegebenen Bedeutungen haben,

*α*) mit Dimethylformamid gegebenenfalls in Gegenwart von Verdünnungsmitteln bei Temperaturen von 10 °C bis 150 °C

oder

β) mit N,N-Dimethylformamiddimethylacetal der Formel (VIII)

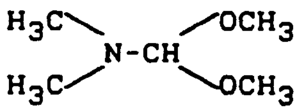

(VIII)

gegebenenfalls in Gegenwart von Verdünnungsmitteln, bei Temperaturen von 10 °C bis 150 °C umsetzt.

Weiterhin wurde gefunden, daß die neuen substituierten Pyrazolin-5-on Derivate der Formel (If)

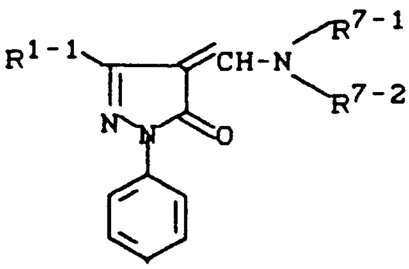

(If)

in welcher

$R^{1-1}$, $R^{7-1}$ und $R^{7-2}$ die oben angegebenen Bedeutung haben,

erhalten werden, indem man Amine der Formel (IIa)

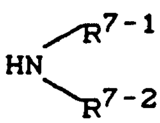

(IIa)

in welcher

$R^{7-1}$ und $R^{7-2}$ die oben angegebenen Bedeutungen haben,

α) mit Pyrazolin-5-on Derviaten der Formel (IIIa)

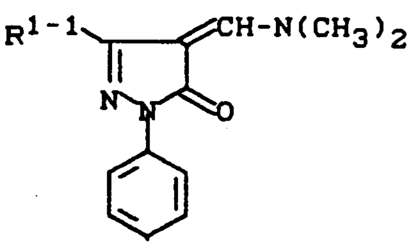

(IIIa)

in welcher

$R^{1-1}$ die oben angegebene Bedeutung hat,

gegebenenfalls in Gegenwart von Verdünnungsmitteln oder

$\beta$) mit 4-Formyl-pyrazolin-5-on Derviaten der Formel (IVb)

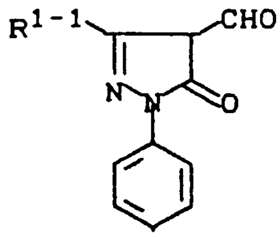

(IVb)

in welcher

$R^{1-1}$ die oben angegebene Bedeutung hat,

gegebenenfalls in Gegenwart von Verdünnungsmitteln umsetzt.

Weiterhin wurde gefunden, daß die substituierten Pyrazolin-5-on Derivate der Formel (I) bzw. (Ia), (Ib), (Ic) und (Id), in welcher $R^1$ für die Gruppe -NH-CO-$R^{10}$ steht mit $R^{10}$ = Alkyl oder Aryl, erhalten werden, indem man Arylhydrazine der Formel (X)

$Ar^1$-NH-$NH_2$ (X)

in welcher

$Ar^1$ die oben angegebene Bedeutung hat,

mit Verbindungen der Formel (XVI)

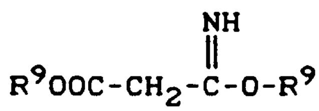

(XVI)

in welcher

$R^9$ für Methyl oder Ethyl steht,

in einer ersten Stufe gegebenenfalls in Gegenwart eines Verdünnungsmittels umsetzt zu den substituierten Arylhydrazinen der Formel (XVII)

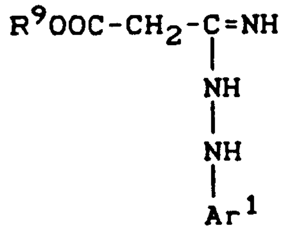

(XVII)

in welcher

$Ar^1$ und $R^9$ die oben angegebene Bedeutung haben,

und die Verbindungen (XVII) in einer zweiten Stufe [vgl. J. Am. Chem. Soc. 66,1851 (1944)] gegebenenfalls in Gegenwart eines Verdünnungsmittels und in Gegenwart einer starken Base umsetzt zu den 3-Aminopyrazolin-5-on Derivaten der Formel (XVIII)

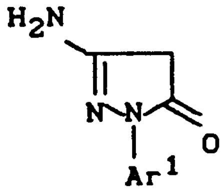

(XVIII)

in welcher

Ar[1] die oben angegebene Bedeutung hat,

und die Verbindungen (XVIII) anschließend mit Verbindungen der Formel (XIX)

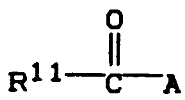

(XIX)

in welcher

$R^{11}$ die oben angegebene Bedeutung hat und

A für Halogen, insbesondere Chlor oder Brom, oder einen Rest $R^{11}$-CO-O- steht,

gegebenenfalls in Gegenwart eines Säurebindemittels und gegebenenfalls in Gegenwart eines Verdünnungsmittels acyliert zu den Verbindungen der Formel (VIa)

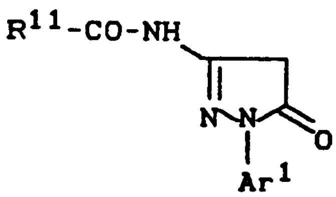

(VIa)

in welcher

$Ar^1$ und $R^{11}$ die oben angegebenen Bedeutungen haben,

welche dan gemäß Verfahrensvariante (Ic/$\beta$) mit 1,3,5-Triazin der Formel (VII) oder gemäß (Id/$\alpha$ und $\beta$) mit Dimethylformamid oder N,N-Diemthylformamiddimethylacetal der Formel (VIII) nach den dort beschriebenen Reaktionsbedingungen umsetzt.

Die so erhaltenen substituierten Pyrazolin-5-on-Derivate der Formel (Ie)

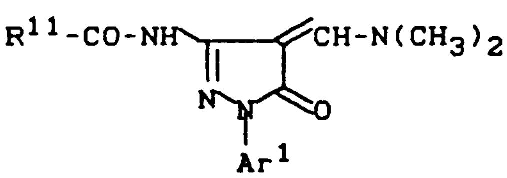

(Ie)

in welcher

$R^{11}$ und $Ar^1$ die oben angegebene Bedeutung haben,

können gegebenenfalls in Gegenwart eines Verdünnungsmittels und in Gegenwart einer Base entsprechend den Verfahrensbedingungen, die bei der Herstellung der Ausgangsstoffe der Formel (IV) beschrieben sind, hydrolisiert werden zu Verbindungen der Formel (IVb)

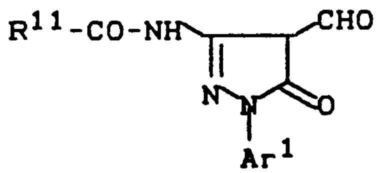

(IVb)

in welcher
$R^{11}$ und $Ar^1$ die oben angegebenen Bedeutungen haben,
welche dann gemäß Verfahrensvariante (Ia/β) weiter zu erfindungsgemäßen Pyrazolin-5-onen der Formel (I) umgesetzt werden können (vgl. Herstellungsbeispiele).

Analog zu dem oben beschriebenen Verfahren läßt sich die Gruppe $-NH-CO-R^{10}$ auch bei den Verbindungen der Formel (If) einführen.

Wie bei den Verbindungen der Formel (I) beschrieben, können die neuen Stoffe der Formeln (Ia), (Ib), (Ic), (Id) und (If) als geometrische Isomere (E/Z-Isomere) oder Isomerengemische unterschiedlicher Zusammensetzung vorliegen. Sowohl die reinen Isomeren als auch die Isomerengemische werden erfindungsgemäß beansprucht, ebenso wie die tautomeren Verbindungen wie unter der Formel (I) besprochen.

Die bekannten Verbindungen der Formel (I) lassen sich analog den obengenannten Verfahren zur Herstellung der neuen Verbindungen der Formeln (Ia), (Ib), (Ic), (Id) und (If) herstellen.

So können die Verbindungen der Formel (I)

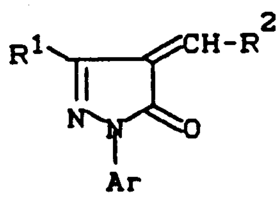

(I)

in welcher
$R^1$, $R^2$ und Ar die bereits genannten Bedeutungen haben, ausgenommen die bei der Formel (I) ausgeschlossenen Verbindungen
erhalten werden, indem man z.B. Verbindungen der Formel (III)

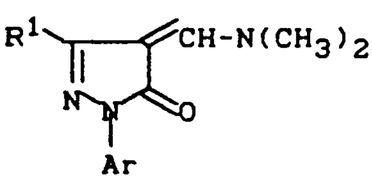

(III)

in welcher
$R^1$ und Ar die oben angegebenen Bedeutungen haben,
oder Verbindungen der Formel (IVa)

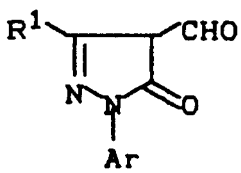

(IVa)

in welcher
$R^1$ und Ar die angegebenen Bedeutungen haben,

jeweils mit Aminen der Formel (II)

$H_2N-R^7$ (II)

in welcher

$R^7$ die angegebene Bedeutung hat,

umsetzt.

Im folgenden sind in bevorzugten, besonders bevorzugten und ganz besonders bevorzugten Bereichen der Verbindungen der Formeln (Ia), (Ic) und (Id) die jeweils bereits in der Hauptdefinition ausgeschlossenen Verbindungen ebenfalls ausgeschlossen.

Bevorzugt werden die neuen substituierten Pyrazolin-5-on-Derivate der Formel (Ia), in welcher

$R^1$ für Wasserstoff, geradkettiges oder verzweigtes Alkyl mit 1 bis 4 Kohlenstoffatomen, Cycloalkyl mit 3 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 3 oder 4 Kohlenstoffatomen und 1 bis 5 gleichen oder verschiedenen Halogenatomen, wie insbesondere Fluor und Chlor, Alkoxy mit 1 bis 4 Kohlenstoffatomen, Alkoxyalkyl mit jeweils 1 bis 4 Kohlenstoffatomen in den einzelnen Alkylteilen, Alkylthioalkyl, Alkylsulfonylalkyl oder Alkylsulfinylalkyl mit jeweils 1 bis 4 Kohlenstoffatomen in den einzelnen Alkylteilen, Alkoxycarbonylalkyl mit 1 oder 2 Kohlenstoffatomen im Alkoxyteil und 1 oder 2 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils gegebenenfalls einfach bis fünffach, gleich oder verschieden substituiertes Phenyl, Naphthyl, Benzyl, Phenylethyl, Phenoxymethyl, Phenoxyethyl, Phenylthiomethyl oder Phenylthioethyl steht, wobei als Phenylsubstituenten jeweils die unter $Ar^1$ aufgeführten Phenylsubstituenten infrage kommen; $R^1$ weiterhin für die Gruppierungen $-NH-CO-R^{10}$ oder $-CO-OR^{11}$ steht, worin

$R^{10}$ und $R^{11}$ jeweils unabhängig voneinander für $C_1$-$C_4$-Alkyl oder Phenyl stehen,

$R^7$ für geradkettiges oder verzweigtes Alkyl mit 1 bis 4 Kohlenstoffatomen; Halogenalkyl mit 1 bis 4 Kohlenstoffatomen und 1 bis 9 gleichen oder verschiedenen Halogenatomen, wie insbesondere Fluor- und Chloratomen; geradkettiges oder verzweigtes Alkenyl mit 2 bis 6 Kohlenstoffatomen; Halogenalkenyl mit 2 bis 4 Kohlenstoffatomen und 1 bis 7 gleichen oder verschiedenen Halogenatomen, wie insbesondere Fluor- und Chloratomen; Alkoxyalkyl mit jeweils 1 bis 4 Kohlenstoffatomen im Alkoxy- oder Alkylteil; für gegebenenfalls einfach bis fünffach, gleich oder verschieden substituiertes Phenylalkyl mit 1 bis 3 Kohlenstoffatomen im geradkettigen oder verzweigten Alkylteil oder für gegebenenfalls einfach bis fünffach, gleich oder verschieden substituiertes Phenyl steht, wobei als Phenylsubstituenten jeweils infrage kommen: Halogen, geradkettiges oder verzweigtes Alkyl mit 1 oder 2 Kohlenstoffatomen und Dialkylamino mit jeweils 1 oder 2 Kohlenstoffatomen im Alkylteil, $C_1$-$C_2$-Alkoxy oder Halogen-$C_1$-$C_2$-alkyl;

$Ar^1$ für einfach bis fünffach, gleich oder verschieden substituiertes Phenyl steht, wobei als Phenylsubstituenten infrage kommen: Halogen; Nitro; Cyano; Carboxyl; Alkoxycarbonyl mit 1 bis 3 Kohlenstoffatomen; $C_1$-$C_3$-Alkyl, $C_1$-$C_3$-Alkoxy; $C_1$-$C_3$-Alkylthio; $C_3$-$C_4$-Alkinoxy; Halogen-($C_1$-$C_3$)-alkyl oder Halogen-($C_1$-$C_4$)-alkoxy oder Halogen-($C_1$-$C_4$)-alkylthio mit jeweils 1 bis 7 gleichen oder verschiedenen Halogenatomen; Phenyl; $C_1$-$C_3$-Alkylsulfonyl und Halogen-($C_1$-$C_3$)-alkylsulfonyl mit jeweils 1 bis 7 gleichen oder verschiedenen Halogenatomen; und Di-($C_1$-$C_3$)-alkylamino; $Ar^1$ ferner für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei $Ar^1$ aufgeführten Phenylsubstituenten infrage kommen, oder für die Gruppe

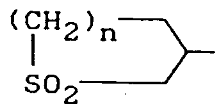

steht,

wobei

n für die Zahlen 1 oder 2 steht,

ausgenommen die vorne unter (Ia) ausgenommenen Verbindungen.

Besonders bevorzugt sind die neuen Verbindungen der Formel (Ia), in welcher

$R^1$ für Wasserstoff, Methyl, Ethyl, n-Propyl, i-Propyl, n-Butyl, i-Butyl, tert.-Butyl, Cyclopropyl, Cyclohexyl, Trifluormethyl, Vinyl, Allyl, Butenyl, Propargyl, 2-Phenylvinyl, Chlorallyl, Methoxy, Ethoxy, Methoxymethyl, Ethoxymethyl, Methylthiomethyl, Ethylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Furanyl, Pyridyl, Thienyl, Furanylmethyl, Thienylmethyl, Ethoxycarbonylmethyl, Methoxycarbonylmethyl,

$R^1$ weiterhin für jeweils gegebenenfalls einfach bis dreifach, gleich oder verschieden substituiertes Phenyl, Naphthyl, Benzyl, Phenylethyl, Phenoxymethyl, Phenylthiomethyl, wobei als Phenylsubstituenten jeweils die unter $Ar^1$ aufgeführten Phenylsubstituenten infrage kommen; $R^1$ weiterhin für die Gruppierungen -NH-CO-$R^{10}$ oder -CO-O-$R^{11}$ steht, worin

$R^{10}$ und $R^{11}$ jeweils unabhängig voneinander für Methyl, Ethyl oder Phenyl stehen,

$R^7$ für Methyl, Ethyl, n-Propyl, i-Propyl, i-Butyl, 2,2-Dimethylpropyl, n-Hexyl, Trifluormethyl, 2-Chlorethyl, 3-Chlorpropyl, Propenyl, Methoxymethyl, Methoxyethyl, Methoxypropyl, Ethoxymethyl, Ethoxyethyl, 3-Chlorallyl, $\alpha$-Methylbenzyl, einfach bis fünffach, gleich oder verschieden substituiertes Phenyl steht, wobei als Phenylsubstituenten infrage kommen: Fluor, Chlor, Methyl, Methoxy, Ethoxy, Trifluormethyl, Trifluorethyl und Dimethylamino,

$Ar^1$ für einfach bis fünffach, gleich oder verschieden substituiertes Phenyl steht, wobei als Phenylsubstituenten infrage kommen: Fluor, Chlor, Nitro, Cyano, Carboxyl, Methoxycarbonyl, Ethoxycarbonyl, Methyl, Ethyl, Methoxy, Ethoxy, Propargyloxy, Trifluormethyl, 2-Chlorethyl, 3-Chlorpropyl, Methylthio, Ethylthio, Trifluormethoxy, Trifluormethylthio, Phenyl, Methylsulfonyl, Trifluormethylsulfonyl; Dimethylamino, Diethylamino; $Ar^1$ weiterhin für jeweils einfach bis dreifach, gleich oder verschieden substituiertes Pyridyl, Benzthiasolyl und Benzoxazolyl steht, wobei als Substituenten Nitro, Chlor, Cyano, Methyl, Ethyl, Methoxy, Ethoxy, Trifluormethoxy und Trifluormethyl genannt seien oder für die Gruppe

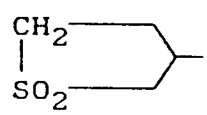

steht.

ausgenommen die vorne unter (Ia) ausgenommenen Verbindungen.

Im einzelnen seien außer den bei den Herstellungsbeispielen genannten Verbindungen die folgenden Pyrazolin-5-on Derivate der allgemeinen Formel (Ia) genannt:

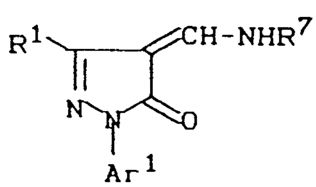

(Ia)

Tabelle 1

| $R^1$ | $R^7$ | $Ar^1$ |
|---|---|---|
| 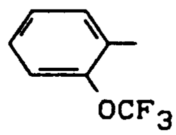 | $CH_3$ | 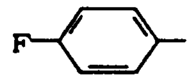 |
| 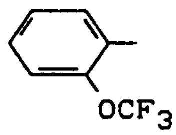 | $C_2H_5$ | 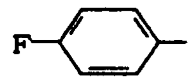 |
| 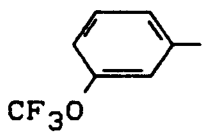 | $CH_3$ | 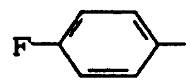 |
| 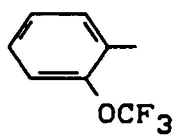 | $CH_3$ | 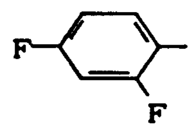 |
| 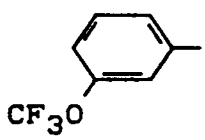 | $CH_3$ | 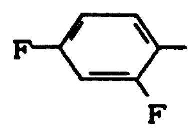 |
| 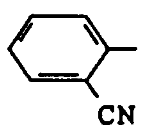 | $CH_3$ | 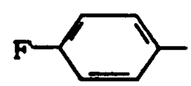 |
| 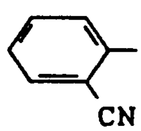 | $CH_3$ | 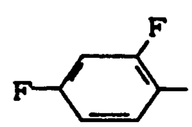 |

Tabelle 1 (Fortsetzung)

| $R^1$ | $R^7$ | $Ar^1$ |
|---|---|---|
| 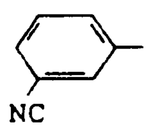 | $CH_3$ | 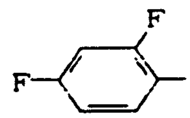 |
| 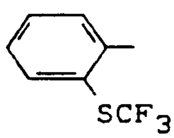 | $CH_3$ | 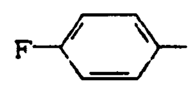 |
| 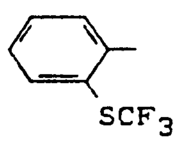 | $CH_3$ | 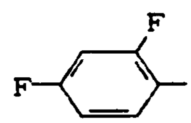 |
| 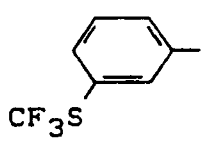 | $CH_3$ | 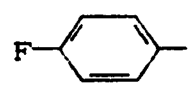 |
| 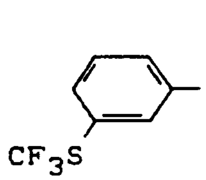 | $CH_3$ | 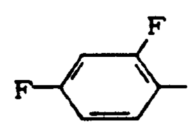 |
| 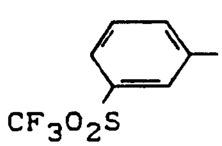 | $CH_3$ | 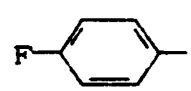 |

Tabelle 1 (Fortsetzung)

| $R^1$ | $R^7$ | $Ar^1$ |
|---|---|---|
| 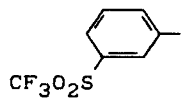 | $CH_3$ | 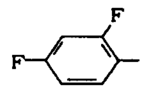 |
| 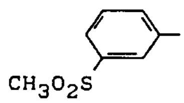 | $CH_3$ | 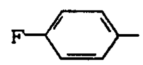 |
| 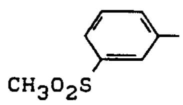 | $CH_3$ | 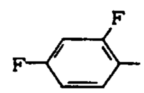 |
| 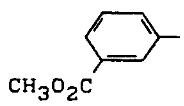 | $CH_3$ | 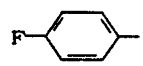 |
| 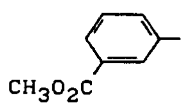 | $CH_3$ | 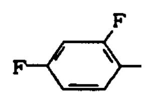 |
| 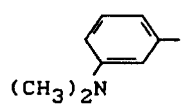 | $CH_3$ | 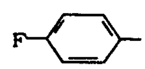 |

Tabelle 1 (Fortsetzung)

| $R^1$ | $R^7$ | $Ar^1$ |
|---|---|---|
| 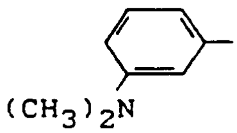 | $CH_3$ | 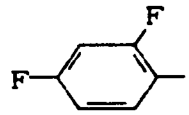 |
| 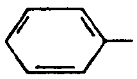 | $CH_3$ | 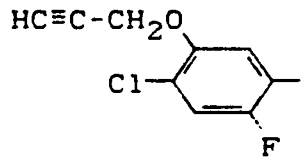 |
| 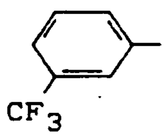 | $CH_3$ | 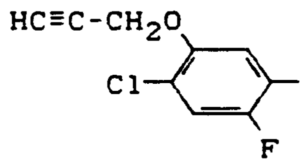 |
| 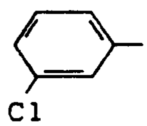 | $CH_3$ | 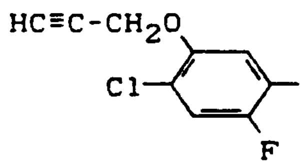 |
| 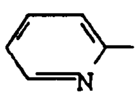 | $CH_3$ | 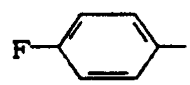 |
| 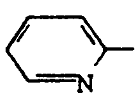 | $CH_3$ | 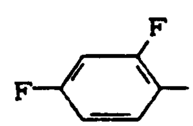 |
| 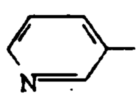 | $CH_3$ | 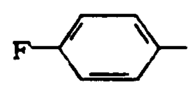 |

Tabelle 1 (Fortsetzung)

| $R^1$ | $R^7$ | $Ar^1$ |
|---|---|---|
| 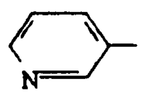 | $CH_3$ | 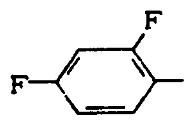 |
| 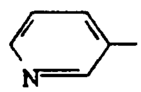 | $CH_3$ | 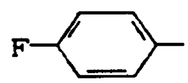 |
| 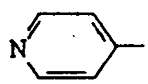 | $CH_3$ | 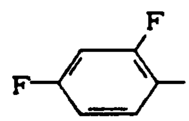 |
| 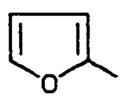 | $CH_3$ | 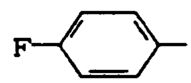 |
| 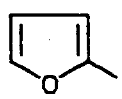 | $CH_3$ | 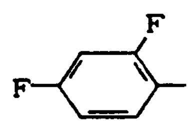 |
| 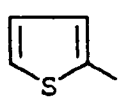 | $CH_3$ | 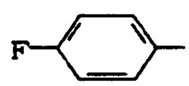 |
| 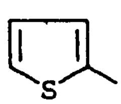 | $CH_3$ | 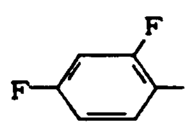 |

Tabelle 1 (Fortsetzung)

| $R^1$ | $R^7$ | $Ar^1$ |
|---|---|---|
| 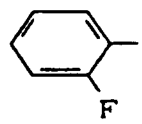 | $CH_3$ | 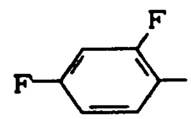 |
| 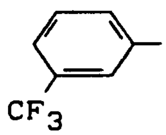 | $CH_3$ | 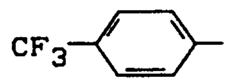 |
| 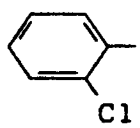 | $CH_3$ | 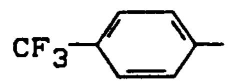 |
| 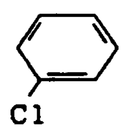 | $CH_3$ | 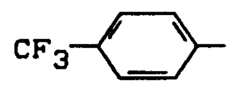 |
| 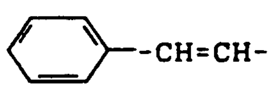 | $CH_3$ | 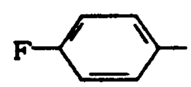 |
| 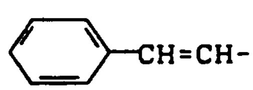 | $CH_3$ | 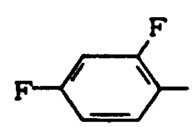 |
| 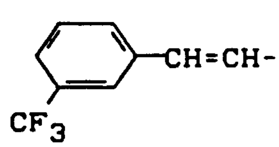 | $CH_3$ | 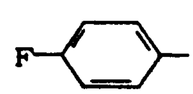 |

Tabelle 1 (Fortsetzung)

| $R^1$ | $R^7$ | $Ar^1$ |
| --- | --- | --- |
| 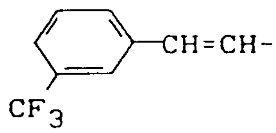 | $CH_3$ | 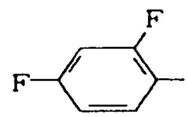 |
| 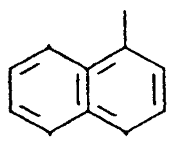 | $CH_3$ | 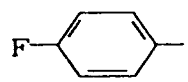 |
| 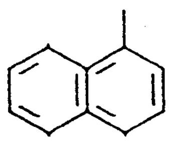 | $CH_3$ | 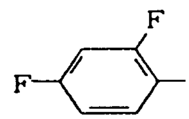 |

Bevorzugt werden die neuen Verbindungen der Formel (Ib), in welcher

$R^1$ für Wasserstoff, geradkettiges oder verzweigtes Alkyl mit 1 bis 4 Kohlenstoffatomen, Cycloalkyl mit 3 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 3 oder 4 Kohlenstoffatomen und 1 bis 5 gleichen oder verschiedenen Halogenatomen, wie insbesondere Fluor und Chlor, Alkoxy mit 1 bis 4 Kohlenstaffatomen, Alkoxyalkyl mit jeweils 1 bis 4 Kohlenstoffatomen in den einzelnen Alkylteilen, Alkylthioalkyl, Alkylsulfonylalkyl oder Alkylsulfinylalkyl mit jeweils 1 bis 4 Kohlenstoffatomen in den einzelnen Alkylteilen, Alkoxycarbonylalkyl mit 1 oder 2 Kohlenstoffatomen im Alkoxyteil und 1 oder 2 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils gegebenenfalls einfach bis fünffach, gleich oder verschieden substituiertes Phenyl, Naphthyl, Benzyl, Phenylethyl, Phenoxymethyl, Phenoxyethyl, Phenylthiomethyl oder Phenylthioethyl steht, wobei als Phenylsubstituenten jeweils die unter $Ar^1$ aufgeführten Phenylsubstituenten infrage kommen; $R^1$ weiterhin für die Gruppierungen -NH-CO-$R^{10}$ oder -CO-$OR^{11}$ steht, worin

$R^{10}$ und $R^{11}$ jeweils unabhängig voneinander für $C_1$-$C_4$-Alkyl oder Phenyl stehen,

$R^6$ für Wasserstoff, geradkettiges oder verzweigtes Alkyl mit 1 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, geradkettiges oder verzweigtes Alkenyl mit 2 bis 6 Kohlenstoffatomen, Halogenalkenyl mit 2 oder 3 Kohlenstoffatomen und 1 bis 5 gleichen oder verschiedenen Halogenatomen, wie insbesondere Fluor- und Chloratomen, für gegebenenfalls einfach bis fünffach, gleich oder verschieden substituiertes Phenylalkyl mit 1 bis 3 Kohlenstoffatomen im Alkylteil steht, wobei als Phenylsubstituenten Halogen, $C_1$-$C_2$-Alkyl, Halogen-$C_1$-$C_2$-alkyl und Nitro infrage kommen, und

$Ar^1$ für einfach bis fünffach, gleich oder verschieden substituiertes Phenyl steht, wobei als

Phenylsubstituenten infrage kommen: Halogen; Nitro; Cyano; Carboxyl; Alkoxycarbonyl mit 1 bis 3 Kohlenstoffatomen; $C_1$-$C_3$-Alkyl, $C_1$-$C_3$-Alkoxy oder $C_1$-$C_3$-Alkylthio; $C_3$-$C_4$-Alkinoxy; Halogen-($C_1$-$C_3$)-alkyl oder Halogen-($C_1$-$C_4$)-alkoxy oder Halogen-($C_1$-$C_4$)-alkylthio mit jeweils 1 bis 7 gleichen oder verschiedenen Halogenatomen; Phenyl; $C_1$-$C_3$-Alkylsulfonyl und Halogen-($C_1$-$C_3$)-alkylsulfonyl mit jeweils 1 bis 7 gleichen oder verschiedenen Halogenatomen; und Di-($C_1$-$C_3$)-alkylamino; ferner für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei $Ar^1$ aufgeführten Phenylsubstituenten infrage kommen, oder für die Gruppe

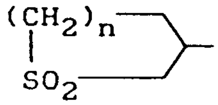

steht,
wobei
n für die Zahlen 1 oder 2 steht.

Besonders bevorzugt sind die neuen Verbindungen der Formel (Ib), in welcher

$R^1$ für Wasserstoff, Methyl, Ethyl, n-Propyl, i-Propyl, n-Butyl, i-Butyl, tert.-Butyl, Cyclopropyl, Cyclohexyl, Trifluormethyl, Vinyl, Allyl, Butenyl, Propargyl, 2-Phenylvinyl, Chlorallyl, Methoxy, Ethoxy, Methoxymethyl, Ethoxymethyl, Methylthiomethyl, Ethylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Furanyl, Pyridyl, Thienyl, Furanylmethyl, Thienylmethyl, Ethoxycarbonylmethyl, Methoxycarbonylmethyl,

$R^1$ weiterhin für jeweils gegebenenfalls einfach bis dreifach, gleich oder verschieden substituiertes Phenyl, Naphthyl, Benzyl, Phenylethyl, Phenoxymethyl, Phenylthiomethyl, wobei als Phenylsubstituenten jeweils die unter $Ar^1$ aufgeführten Phenylsubstituenten infrage kommen; $R^1$ weiterhin für die Gruppierungen -NH-CO-$R^{10}$ oder -CO-O-$R^{11}$ steht, worin

$R^{10}$ und $R^{11}$ jeweils unabhängig voneinander für Methyl, Ethyl oder Phenyl stehen,

$R^6$ für Wasserstoff, Methyl, Ethyl, i-Propyl, Trifluormethyl, 2-Chlorethyl, 3-Chlorpropyl, Propenyl, 3-Chlorallyl, gegebenenfalls einfach bis fünffach, gleich oder verschieden substituiertes Benzyl steht, wobei als Phenylsubstituenten Fluor, Chlor, Methyl, Ethyl, Trifluormethyl und Nitro infrage kommen und

$Ar^1$ für einfach bis fünffach, gleich oder verschieden substituiertes Phenyl steht, wobei als Phenylsubstituenten infrage kommen: Fluor, Chlor, Nitro, Cyano, Carboxyl, Methoxycarbonyl, Ethoxycarbonyl, Methyl, Ethyl, Methoxy, Ethoxy, Propargyloxy, Trifluormethyl, 2-Chlorethyl, 3-Chlorpropyl, Methylthio, Ethylthio, Trifluormethoxy, Trifluormethylthio, Phenyl, Methylsulfonyl, Trifluormethylsulfonyl; Dimethylamino, Diethylamino; $Ar^1$ weiterhin für jeweils einfach bis dreifach, gleich oder verschieden substituiertes Pyridyl, Benzthiazolyl und Benzoxazolyl steht, wobei als Substituenten Nitro, Chlor Cyano, Methyl, Ethyl, Methoxy, Ethoxy, Trifluormethoxy und Trifluormethyl genannt seien oder für die Gruppe

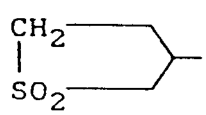

steht.

Bevorzugt sind die neuen substituierten Pyrazolin-5-on Derivate der Formel (Ic), in welcher

$R^1$ für Wasserstoff, geradkettiges oder verzweigtes Alkyl mit 1 bis 4 Kohlenstoffatomen, Cycloalkyl mit 3 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach

bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 3 oder 4 Kohlenstoffatomen und 1 bis 5 gleichen oder verschiedenen Halogenatomen, wie insbesondere Fluor und Chlor, Alkoxy mit 1 bis 4 Kohlenstoffatomen, Alkoxyalkyl mit jeweils 1 bis 4 Kohlenstoffatomen in den einzelnen Alkylteilen, Alkylthioalkyl, Alkylsulfonylalkyl oder Alkylsulfinylalkyl mit jeweils 1 bis 4 Kohlenstoffatomen in den einzelnen Alkylteilen, Alkoxycarbonylalkyl mit 1 oder 2 Kohlenstoffatomen im Alkoxyteil und 1 oder 2 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils gegebenenfalls einfach bis fünffach, gleich oder verschieden substituiertes Phenyl, Naphthyl, Benzyl, Phenylethyl, Phenoxymethyl, Phenoxyethyl, Phenylthiomethyl oder Phenylthioethyl steht, wobei als Phenylsubstituenten jeweils die unter $Ar^1$ aufgeführten Phenylsubstituenten infrage kommen; $R^1$ weiterhin für die Gruppierungen $-NH-CO-R^{10}$ oder $-CO-OR^{11}$ steht, worin

$R^{10}$ und $R^{11}$ jeweils unabhängig voneinander für $C_1$-$C_4$-Alkyl oder Phenyl stehen,

$Ar^1$ für einfach bis fünffach, gleich oder verschieden substituiertes Phenyl steht, wobei als Phenylsubstituenten infrage kommen: Halogen; Nitro; Cyano; Carboxyl; Alkoxycarbonyl mit 1 bis 3 Kohlenstoffatomen; $C_1$-$C_3$-Alkyl, $C_1$-$C_3$-Alkoxy; $C_1$-$C_3$-Alkylthio; $C_3$-$C_4$-Alkinoxy; Halogen-($C_1$-$C_3$)-alkyl, Halogen-($C_1$-$C_4$)-alkoxy oder Halogen-($C_1$-$C_4$)-alkylthio mit jeweils 1 bis 7 gleichen oder verschiedenen Halogenatomen; Phenyl; $C_1$-$C_3$-Alkylsulfonyl und Halogen-($C_1$-$C_3$)-alkylsulfonyl mit jeweils 1 bis 7 gleichen oder verschiedenen Halogenatomen; und Di-($C_1$-$C_3$)-alkylamino; $Ar^1$ ferner für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei $Ar^1$ aufgeführten Phenylsubstituenten infrage kommen, oder für die Gruppe

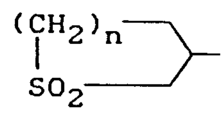

steht,
wobei

n für die Zahlen 1 oder 2 steht,

ausgenommen die vorne unter (Ic) ausgenommenen Verbindungen.

Besonders bevorzugt sind die neuen Verbindungen der Formel (Ic), in welcher

$R^1$ für Wasserstoff, Methyl, Ethyl, n-Propyl, i-Propyl, n-Butyl, i-Butyl, tert.-Butyl, Cyclopropyl, Cyclohexyl, Trifluormethyl, Vinyl, Alkyl, Butenyl, Propargyl, 2-Phenylvinyl, Chlorallyl, Methoxy, Ethoxy, Methoxymethyl, Ethoxymethyl, Methylthiomethyl, Ethylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Furanyl, Pyridyl, Thienyl, Furanylmethyl, Thienylmethyl, Ethoxycarbonylmethyl, Methoxycarbonylmethyl,

$R^1$ weiterhin für jeweils gegebenenfalls einfach bis dreifach, gleich oder verschieden substituiertes Phenyl, Naphthyl, Benzyl, Phenylethyl, Phenoxymethyl, Phenylthiomethyl, wobei als Phenylsubstituenten jeweils die unter $Ar^1$ aufgeführten Phenylsubstituenten infrage kommen; $R^1$ weiterhin für die Gruppierungen $-NH-CO-R^{10}$ oder $-CO-O-R^{11}$ steht, worin

$R^{10}$ und $R^{11}$ jeweils unabhängig voneinander für Methyl, Ethyl oder Phenyl stehen,

$Ar^1$ für einfach bis fünffach, gleich oder verschieden substituiertes Phenyl steht, wobei als Phenylsubstituenten infrage kommen: Fluor, Chlor, Nitro, Cyano, Carboxyl, Methoxycarbonyl, Ethoxycarbonyl, Methyl, Ethyl, Methoxy, Ethoxy, Propargyloxy, Trifluormethyl, 2-Chlorethyl, 3-Chlorpropyl, Methylthio, Ethylthio, Trifluormethoxy, Trifluormethylthio, Phenyl, Methylsulfonyl, Trifluormethylsulfonyl, Dimethylamino und Diethylamino; $Ar^1$ weiterhin für jeweils einfach bis dreifach, gleich oder verschieden substituiertes Pyridyl, Benzthiazolyl oder Benzoxazolyl steht, wobei als Substituenten Nitro, Chlor, Cyano, Methyl, Ethyl, Methoxy, Ethoxy, Trifluormethoxy und Trifluormethyl genannt seien oder

$Ar^1$ für die Gruppe

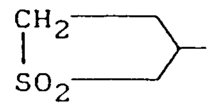

steht,

ausgenommen die Verbindungen, die vorne unter (Ic) ausgenommen sind.

Bevorzugt werden die neuen substituierten Pyrazolin-5-on-Derivate der Formel (Id), in welcher

$R^1$ für Wasserstoff, geradkettiges oder verzweigtes Alkyl mit 1 bis 4 Kohlenstoffatomen, Cycloalkyl mit 3 bis 6 Kohlenstoffatomen, Halogenalkyl mit 1 bis 3 Kohlenstoffatomen und 1 bis 7 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten jeweils unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 3 oder 4 Kohlenstoffatomen und 1 bis 5 gleichen oder verschiedenen Halogenatomen, wie insbesondere Fluor und Chlor, Alkoxy mit 1 bis 4 Kohlenstoffatomen, Alkoxyalkyl mit jeweils 1 bis 4 Kohlenstoffatomen in den einzelnen Alkylteilen, Alkylthioalkyl, Alkylsulfonylalkyl oder Alkylsulfinylalkyl mit jeweils 1 bis 4 Kohlenstoffatomen in den einzelnen Alkylteilen, Alkoxycarbonylalkyl mit 1 oder 2 Kohlenstoffatomen im Alkoxyteil und 1 oder 2 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils gegebenenfalls einfach bis fünffach, gleich oder verschieden substituiertes Phenyl, Naphthyl, Benzyl, Phenylethyl, Phenoxymethyl, Phenoxyethyl, Phenylthiomethyl oder Phenylthioethyl steht, wobei als Phenylsubstituenten jeweils die unter $Ar^1$ aufgeführten Phenylsubstituenten infrage kommen; $R^1$ weiterhin für die Gruppierungen -NH-CO-$R^{10}$ oder -CO-$OR^{11}$ steht, worin

$R^{10}$ und $R^{11}$ jeweils unabhängig voneinander für $C_1$-$C_4$-Alkyl oder Phenyl stehen,

$Ar^1$ für einfach bis fünffach, gleich oder verschieden substituiertes Phenyl steht, wobei als Phenylsubstituenten infrage kommen: Halogen; Nitro; Cyano; Carboxyl; Alkoxycarbonyl mit 1 bis 3 Kohlenstoffatomen; $C_1$-$C_3$-Alkyl, $C_1$-$C_3$-Alkoxy; $C_1$-$C_3$-Alkylthio; $C_3$-$C_4$-Alkinoxy; Halogen-($C_1$-$C_3$)-alkyl, Halogen-($C_1$-$C_4$)-alkoxy oder Halogen-($C_1$-$C_4$)-alkylthio mit jeweils 1 bis 7 gleichen oder verschiedenen Halogenatomen; Phenyl; $C_1$-$C_3$-Alkylsulfonyl und Halogen-($C_1$-$C_3$)-alkylsulfonyl mit jeweils 1 bis 7 gleichen oder verschiedenen Halogenatomen und Di-($C_1$-$C_3$)-alkylamino; $Ar^1$ ferner für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei $Ar^1$ aufgeführten Phenylsubstituenten infrage kommen, oder für die Gruppe

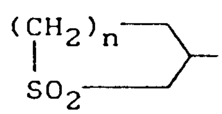

steht,

wobei

n für die Zahlen 1 oder 2 steht,

ausgenommen die vorne unter (Id) ausgenommenen Verbindungen.

Besonders bevorzugt sind die neuen Verbindungen der Formel (Id), in welcher

$R^1$ für Wasserstoff, Methyl, Ethyl, n-Propyl, i-Propyl, n-Butyl, i-Butyl, tert.-Butyl, Cyclopropyl, Cyclohexyl, Trifluormethyl, Vinyl, Allyl, Butenyl, Propargyl, 2-Phenylvinyl, Chlorallyl, Methoxy, Ethoxy, Methoxymethyl, Ethoxymethyl, Methylthiomethyl, Ethylthiomethyl, Methylsulfonylmethyl, Methylsulfonylethyl, Ethylsulfonylmethyl, Ethylsulfonylethyl, Methylsulfinylmethyl, Methylsulfinylethyl, Ethylsulfinylmethyl, Ethylsulfinylethyl, Furanyl, Pyridyl, Thienyl, Furanylmethyl, Thienylmethyl, Ethoxycarbonylmethyl, Methoxycarbonylmethyl,

$R^1$ weiterhin für jeweils gegebenenfalls einfach bis dreifach, gleich oder verschieden substituiertes Phenyl, Naphthyl, Benzyl, Phenylethyl, Phenoxymethyl, Phenylthiomethyl, wobei als Phenylsubstituenten jeweils die unter $Ar^1$ aufgeführten Phenylsubstituenten infrage kommen; $R^1$ weiterhin für die Gruppierungen $-NH-CO-R^{10}$ oder $-CO-O-R^{11}$ steht, worin

$R^{10}$ und $R^{11}$ jeweils unabhängig voneinander für Methyl, Ethyl oder Phenyl stehen,

$Ar^1$ für einfach bis fünffach, gleich oder verschieden substituiertes Phenyl steht, wobei als Phenylsubstituenten infrage kommen: Fluor, Chlor, Nitro, Cyano, Carboxyl, Methoxycarbonyl, Ethoxycarbonyl, Methyl, Ethyl, Methoxy, Ethoxy, Propargyloxy, Trifluormethyl, 2-Chlorethyl, 3-Chlorpropyl, Methylthio, Ethylthio, Trifluormethoxy, Trifluormethylthio, Phenyl, Methylsulfonyl, Trifluormethylsulfonyl; Dimethylamino, Diethylamino; für jeweils einfach bis dreifach, gleich oder verschieden substituiertes Pyridyl, Benzthiazolyl und Benzoxazolyl steht, wobei als Substituenten Nitro, Chlor, Cyano, Methyl, Ethyl, Methoxy, Ethoxy, Trifluormethoxy und Trifluormethyl genannt seien oder für die Gruppe

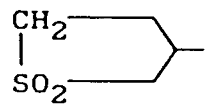

steht,

ausgenommen die vorne unter (Id) ausgenommenen Verbindungen.

Bevorzugt werden die Verbindungen der Formel (If), in welcher

$R^{1-1}$ für $C_1$-$C_6$-Alkoxy, Alkenyl mit 2 bis 4 Kohlenstoffatomen, gegebenenfalls substituiert durch Phenyl oder gegebenenfalls substituiert durch einfach bis dreifach, gleich oder verschieden substituiertes Phenyl, wobei als Phenylsubstituenten Fluor, Chlor, $C_1$-$C_3$-Alkyl, $C_1$-$C_3$-Alkoxy, Halogen-($C_1$-$C_3$)-alkyl und Halogen-($C_1$-$C_4$)alkoxy genannt seien; $R^{1-1}$ weiterhin für jeweils einfach bis fünffach, gleich oder verschieden substituiertes Phenyl oder Naphthyl oder jeweils gegebenenfalls einfach bis fünffach gleich oder verschieden substituiertes Benzyl oder Phenethyl, wobei als Phenylsubstituenten jeweils Fluor, Chlor, Brom, Nitro, Cyano, Carboxyl, Methoxycarbonyl, Ethoxycarbonyl, Methyl, Ethyl, Methoxy, Ethoxy, Halogen-($C_1$-$C_2$)-alkyl, Halogen-($C_1$-$C_2$)alkoxy, Halogen-($C_1$-$C_2$)-alkylthio mit jeweils 1 bis 5 gleichen oder verschiedenen Halogenatomen wie Fluor oder Chlor, Methylsulfonyl, Ethylsulfonyl, Trifluormethylsulfonyl, Dimethylamino und Diethylamino genannt seien; $R^{1-1}$ ferner für jeweils gegebenenfalls durch Fluor, Chlor, Methyl und/oder Ethyl substituiertes Furanylmethyl, Furanylethyl, Thienylmethyl oder Thienylethyl oder $R^{1-1}$ weiterhin die Gruppe $-NH-CO-R^{10}$ steht, wobei

$R^{10}$ für $C_1$-$C_4$-Alkyl oder Phenyl steht,

$R^{7-1}$ für Wasserstoff, $C_1$-$C_6$-Alkyl, Halogen-$C_1$-$C_4$-alkyl, $C_2$-$C_4$-Alkenyl, Halogen-$C_2$-$C_4$-alkenyl, $C_1$-$C_6$-Alkoxy-$C_1$-$C_6$-alkyl, jeweils gegebenenfalls einfach bis fünffach, gleich oder verschieden substituiertes Benzyl oder Phenethyl oder gegebenenfalls einfach bis fünffach, gleich oder verschieden substituiertes Phenyl steht, wobei als Phenylsubstituenten jeweils Fluor, Chlor, Brom, Methyl, Ethyl, Halogen-$C_1$-$C_2$-alkyl, insbesondere Trifluormethyl, Dimethylamino und Diethylamino genannt seien, und

$R^{7-2}$ für Wasserstoff oder Methyl steht,

ausgenommen die Verbindungen, die vorne unter (If) ausgenommen sind.

Besonders bevorzugt sind die Verbindungen der Formel (If), in welcher

$R^{1-1}$ für $C_1$-$C_4$-Alkoxy, Vinyl, Allyl, Butenyl, 2-Phenylvinyl, 2-(2-Trifluormethylphenyl)vinyl, jeweils einfach bis dreifach, gleich oder verschieden substituiertes Phenyl oder gegebenenfalls einfach bis dreifach gleich oder verschieden substituiertes Benzyl, wobei als Phenylsubstituenten jeweils Fluor, Chlor, Nitro, Cyano, Methoxycarbonyl, Ethoxycarbonyl, Methyl, Ethyl, Methoxy, Trifluormethyl, Trifluormethoxy, Trifluormethylthio, Difluormethylthio, Trifluormethylsulfonyl, Methylsulfonyl, Dimethylamino und Diethylamino genannt seien; $R^{1-1}$ weiterhin für jeweils gegebenenfalls durch Fluor, Chlor, Methyl und/oder Ethyl substituiertes Furanylmethyl oder Thienylmethyl oder für die Gruppe $-NH-CO-R^{10}$ steht, wobei

$R^{10}$ für Methyl, Ethyl oder Phenyl steht,

$R^{7-1}$ für Wasserstoff, Methyl, Ethyl, n- oder iso-Propyl, Halogen-$C_1$-$C_2$-alkyl, insbesondere Trifluormethyl, Allyl, Propargyl, Halogen-$C_2$-$C_3$-alkenyl, $C_1$-$C_2$-Alkoxy-$C_1$-$C_2$-alkyl, jeweils gegebenenfalls einfach bis dreifach, gleich oder verschieden substituiertes Benzyl oder Phenethyl oder einfach bis dreifach, gleich oder verschieden substituiertes Phenyl steht, wobei als Phenylsubstituenten jeweils Fluor, Chlor, Methyl, Trifluormethyl und Dimethylamino genannt seien, und

$R^{7-2}$ für Wasserstoff oder Methyl steht,

ausgenommen die Verbindungen, die vorne unter (If) ausgenommen sind.

Im einzelnen seien außer den bei den Herstellungsbeispielen genannten Verbindungen die folgenden Pyrazolin-5-on Derivate der Formel (If) genannt:

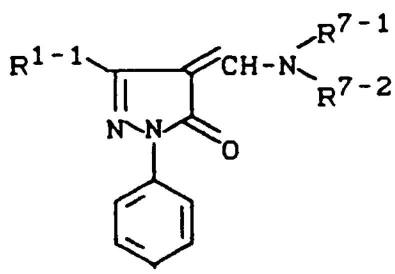

(If)

Tabelle 2

| $R^{1-1}$ | $R^{7-1}$ | $R^{7-2}$ |
|---|---|---|
| 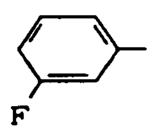 | $CH_3$ | $CH_3$ |
| 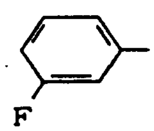 | H | $CH_3$ |
| 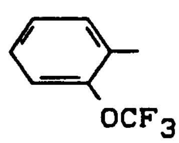 | $CH_3$ | $CH_3$ |
| 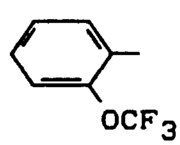 | H | $CH_3$ |

Tabelle 2 (Fortsetzung)

| $R^{1-1}$ | $R^{7-1}$ | $R^{7-2}$ |
|---|---|---|
| 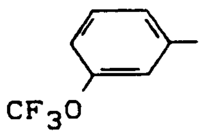 | $CH_3$ | $CH_3$ |
| 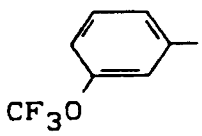 | H | $CH_3$ |
| 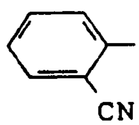 | $CH_3$ | $CH_3$ |
| 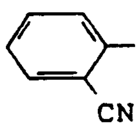 | H | $CH_3$ |
| 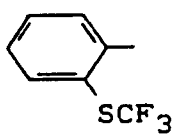 | $CH_3$ | $CH_3$ |
| 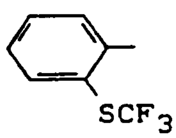 | H | $CH_3$ |
| 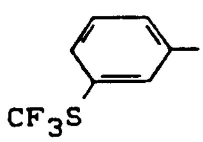 | $CH_3$ | $CH_3$ |

Tabelle 2 (Fortsetzung)

| $R^{1-1}$ | $R^{7-1}$ | $R^{7-2}$ |
|---|---|---|
| 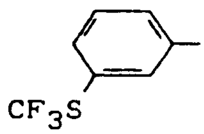 | H | $CH_3$ |
| 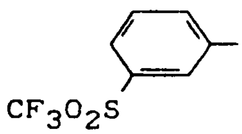 | $CH_3$ | $CH_3$ |
| 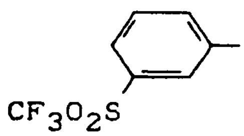 | $CH_3$ | $CH_3$ |
| 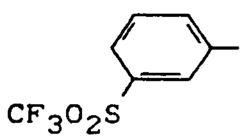 | H | $CH_3$ |
| 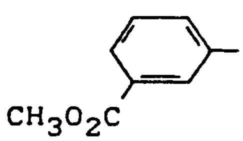 | $CH_3$ | $CH_3$ |
| 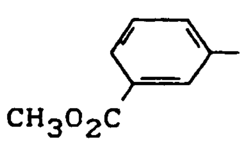 | H | $CH_3$ |
| 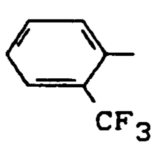 | $CH_3$ | $CH_3$ |

Tabelle 2 (Fortsetzung)

| $R^{1-1}$ | $R^{7-1}$ | $R^{7-2}$ |
|---|---|---|
| 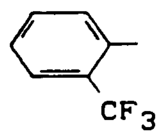 | H | $CH_3$ |
| 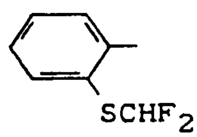 | $CH_3$ | $CH_3$ |
| 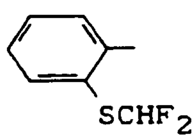 | H | $CH_3$ |
| 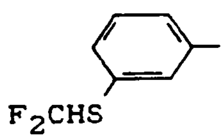 | $CH_3$ | $CH_3$ |
| 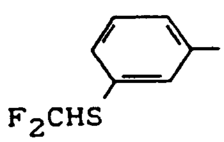 | H | $CH_3$ |
| 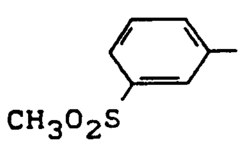 | $CH_3$ | $CH_3$ |
| 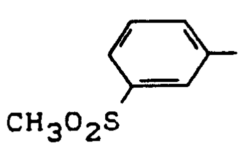 | H | $CH_3$ |

Tabelle 2 (Fortsetzung)

| $R^{1-1}$ | $R^{7-1}$ | $R^{7-2}$ |
|---|---|---|
| 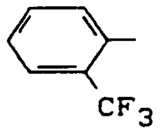 | $CH_3$ | $CH_3$ |
| 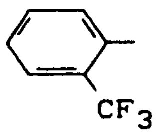 | H | $CH_3$ |
| 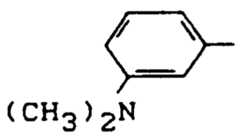 | $CH_3$ | $CH_3$ |
| 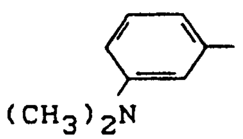 | H | $CH_3$ |
| 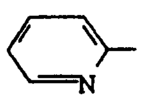 | $CH_3$ | $CH_3$ |
| 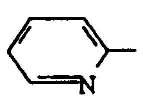 | H | $CH_3$ |
| 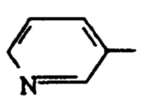 | $CH_3$ | $CH_3$ |
| 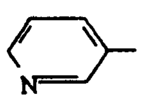 | H | $CH_3$ |

Tabelle 2 (Fortsetzung)

| $R^{1-1}$ | $R^{7-1}$ | $R^{7-2}$ |
|---|---|---|
| 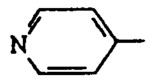 | $CH_3$ | $CH_3$ |
| 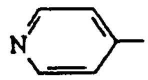 | H | $CH_3$ |
| 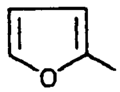 | $CH_3$ | $CH_3$ |
| 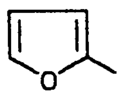 | H | $CH_3$ |
| 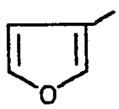 | $CH_3$ | $CH_3$ |
| 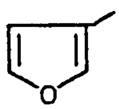 | H | $CH_3$ |
| 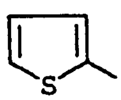 | $CH_3$ | $CH_3$ |
| 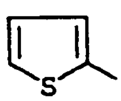 | H | $CH_3$ |
| 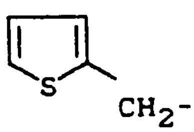 | $CH_3$ | $CH_3$ |

Tabelle 2 (Fortsetzung)

| $R^{1-1}$ | $R^{7-1}$ | $R^{7-2}$ |
|---|---|---|
| 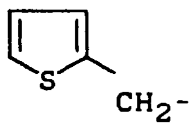 | H | $CH_3$ |
| 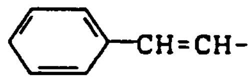 | $CH_3$ | $CH_3$ |
| 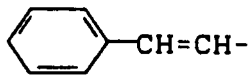 | H | $CH_3$ |
| 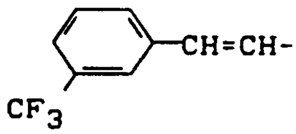 | $CH_3$ | $CH_3$ |
| 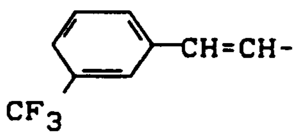 | H | $CH_3$ |
| 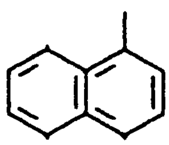 | $CH_3$ | $CH_3$ |
| 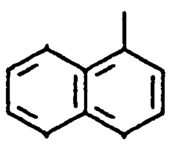 | H | $CH_3$ |

Verwendet man als Ausgangsstoffe beispielsweise 1-(2,6-Dichlor-4-trifluormethyl-phenyl)-4-(N,N-dimethylaminomethyliden)-3-methyl-pyrazolin-5-on und Methylamin, so läßt sich der Reaktionsablauf zur Herstellung der substituierten Pyrazolin-5-on Derivate der Formel (Ia) gemäß Verfahrensvariante (Ia/α) durch das folgende Formelschema beschreiben:

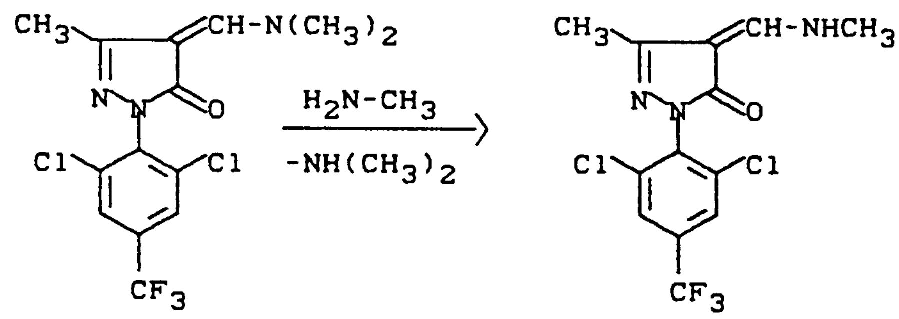


Verwendet man als Ausgangsstoffe beispielsweise 1-(2,6-Dichlor-4-trifluormethyl-phenyl)-4-formyl-3-methyl-pyrazolin-5-on und 2,4-Dichloranilin, so läßt sich der Reaktionsablauf zur Herstellung der substituierten Pyrazolin-5-on Derivate der Formel (Ia) gemäß Verfahrensvariante (Ia/β) durch das folgende Formelschema beschreiben:

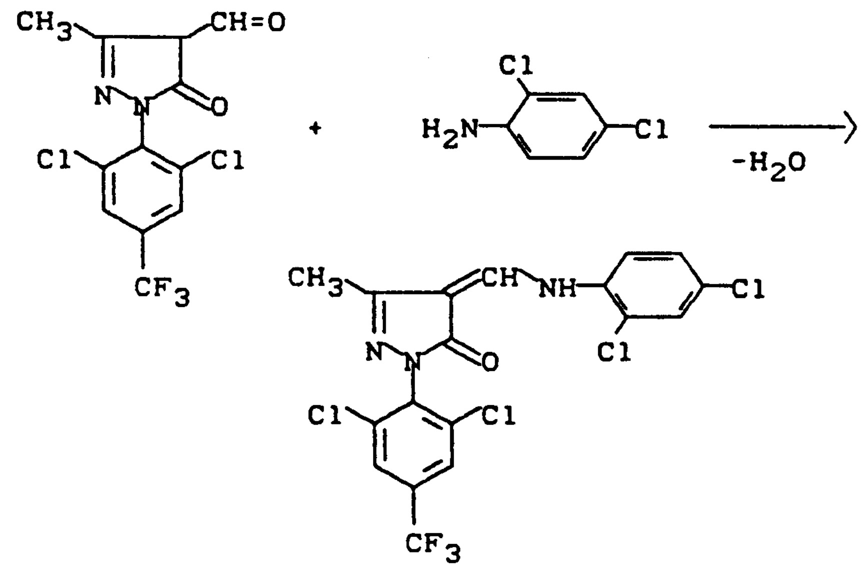


Verwendet man als Ausgangsstoffe beispielsweise 1-(2,6-Dichlor-4-trifluormethylphenyl)-4-(N,N-dimethylaminomethyliden)-pyrazolin-5-on und N-Methylhydroxylamin-Hydrochlorid, so läßt sich der Reaktionsablauf zur Herstellung der substituierten Pyrazolin-5-on Derivate der Formel (Ib) durch das folgende Formelschema beschreiben:

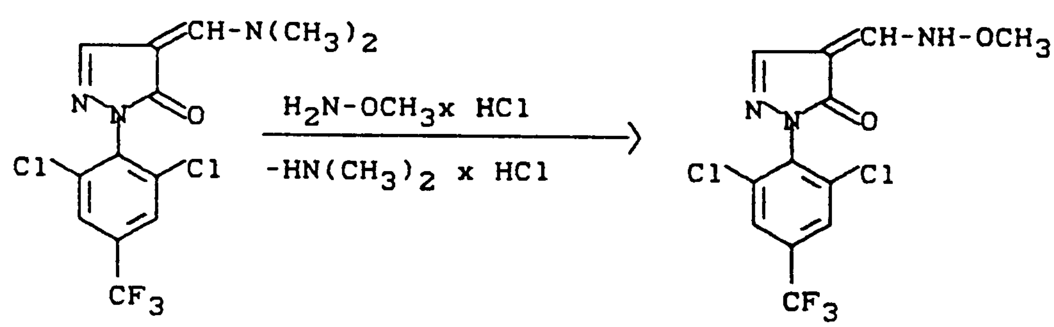


Verwendet man als Ausgangsstoffe beispielsweise 1-(2,6-Dichlor-4-trifluormethyl-phenyl)-4-(N,N-dimethylaminomethyliden)-3-methyl-pyrazolin-5-on und Ammoniak, so läßt sich der Reaktionsablauf zur Herstellung der substituierten Pyrazolin-5-on Derivate der Formel (Ic) gemäß Verfahrensvariante (Ic/α) durch das folgende Formelschema beschreiben:

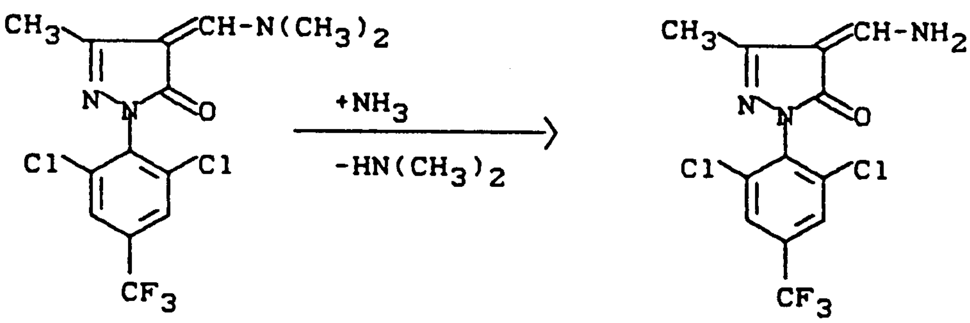


Verwendet man als Ausgangsstoffe beispielsweise 1-(2,6-Dichlor-4-trifluormethyl-phenyl)-3-methyl-pyrazolin-5-on und 1,3,5-Triazin, so läßt sich der Reaktionsablauf zur Herstellung der substituierten Pyrazolin-5-on Derivate der Formel (Ic) gemäß Verfahrensvariante (Ic/β) durch das folgende Formelschema beschreiben:

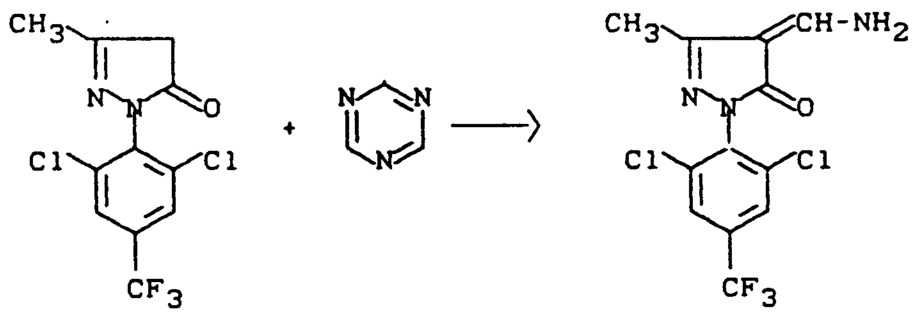

Verwendet man als Ausgangsstoffe beispielsweise 1-(2,6-Dichlor-4-trifluormethyl-phenyl)-3-methyl-pyrazolin-5-on und Dimethylformamid in Gegenwart eines Verdünnungsmittels, so läßt sich der Reaktionsablauf zur Herstellung der substituierten Pyrazolin-5-on Derivate der Formel (Id) gemäß Verfahrensvariante (Id/α) durch das folgende Formelschema beschreiben:

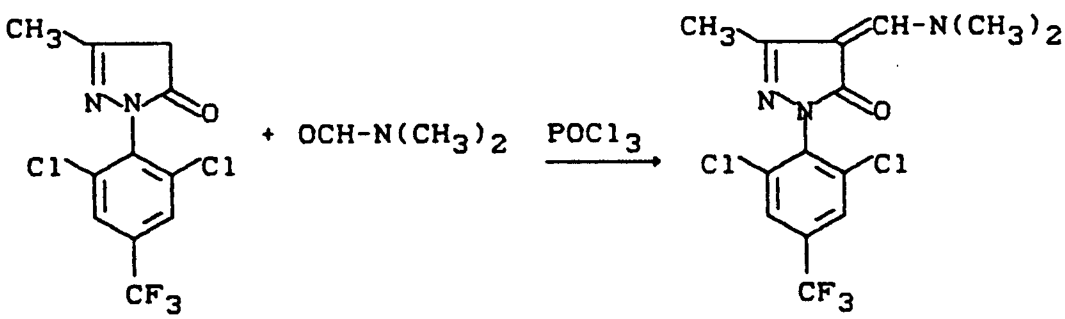


Verwendet man als Ausgangsstoffe beispielsweise 1-(2,6-Dichlor-4-trifluormethyl-phenyl)-3-methyl-pyrazolin-5-on und N,N-Dimethylformamiddimethylacetal, so läßt sich der Reaktionsablauf zur Herstellung der substituierten Pyrazolin-5-on Derivate der Formel (Id) gemäß Verfahrensvariante (Id/β) durch das folgende Formelschema beschreiben:

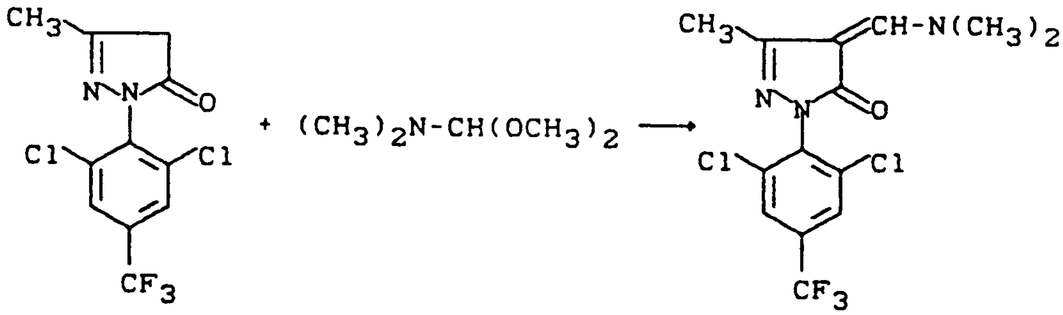

Die als Ausgangsstoffe zur Herstellung der substituierten Pyrazolin-5-on Derivate der Formel (I) zu verwendenden Pyrazolin-5-on Derivate der Formel (III)

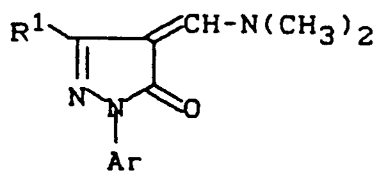

(III)

in welcher

R[1] und Ar für diejenigen Reste stehen, die bereits im Zusammenhang mit der Beschreibung der Stoffe der Formel (I) für diese Substituenten genannt wurden,

sind teilweise bekannt [vgl. Zh.Obs.Khimii, 32, (12) 4050 (1962)].

Die als Ausgangsstoffe zur Herstellung der substituierten Pyrazolin-5-on Derivate der Formeln (Ia), (Ib) und (Ic) zu verwendenden erfindungsgemäßen Pyrazolin-5-on Derivate der Formel (Id)

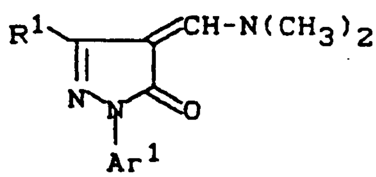

(Id)

in welcher

R[1] und Ar[1] die oben angegebenen Bedeutungen haben, ausgenommen die Verbindungen 1-(4-Nitrophenyl)-3-methyl-4-(N,N-dimethylamino-methyliden)-pyrazolin-5-on und 1-(4-Sulfophenyl)-3-methyl-4-(N,N-dimethylamino-methyliden)-pyrazolin-5-on,

sind neu, Teil der vorliegenden Erfindung und wie bereits beschrieben, herstellbar.

Die dazu benötigten Verbindungen der Formel (VIII) sind allgemein bekannte Verbindungen der organsichen Chemie.

Im einzelnen seien außer den bei den Herstellungsbeispielen genannten Ausgangsprodukten der Formel (III), die gleichzeitig zum Teil erfindungsgemäßen Endprodukte der Formel (Id) genannt:

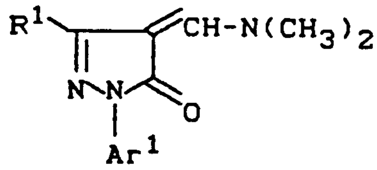

(Id)

Tabelle 3

| $R^1$ | $Ar^1$ |
|---|---|
| 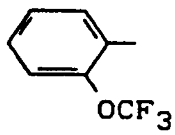 | 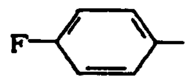 |

Tabelle 3 (Fortsetzung)

| $R^1$ | $Ar^1$ |
|---|---|
| 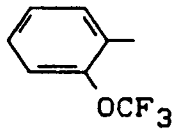 | 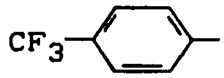 |
| 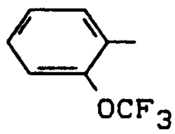 | 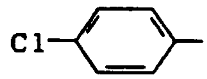 |
| 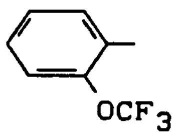 | 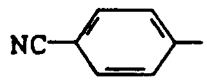 |
| 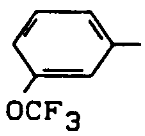 | 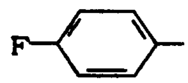 |
| 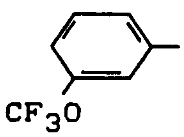 | 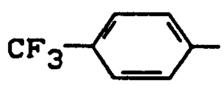 |
| 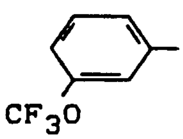 | 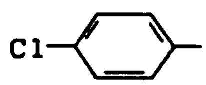 |
| 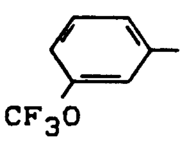 | 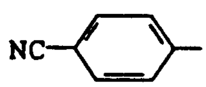 |

Tabelle 3 (Fortsetzung)

| $R^1$ | $Ar^1$ |
|---|---|
| 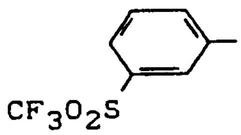 | 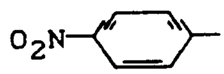 |
| 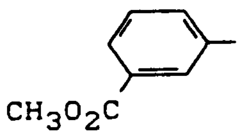 | 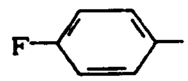 |
| 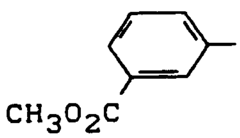 | 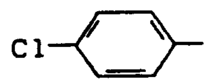 |
| 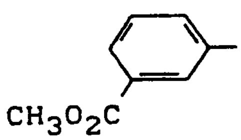 | 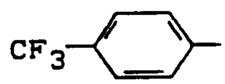 |
| 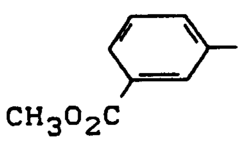 | 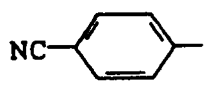 |
| 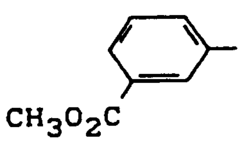 | 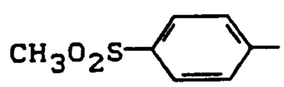 |
| 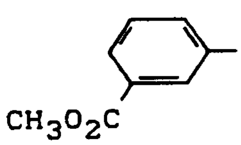 | 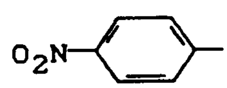 |

Tabelle 3 (Fortsetzung)

| $R^1$ | $Ar^1$ |
|---|---|
| 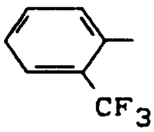 | 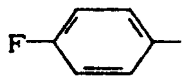 |
| 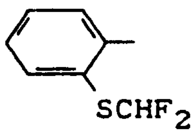 | 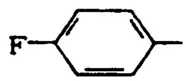 |
| 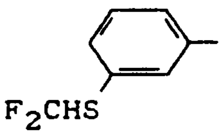 | 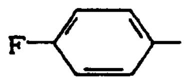 |
| 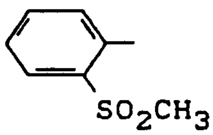 | 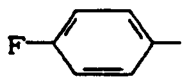 |
| 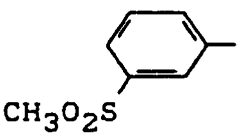 | 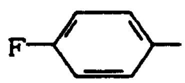 |
| 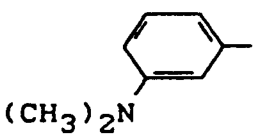 | 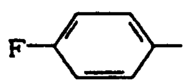 |
| 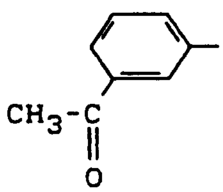 | 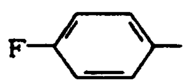 |

<u>Tabelle 3</u> (Fortsetzung)

| $R^1$ | $Ar^1$ |
|---|---|
| 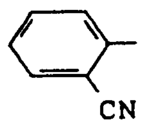 | 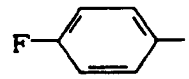 |
| 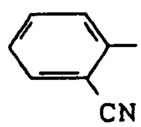 | 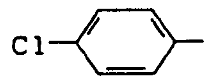 |
| 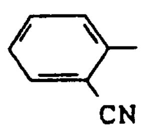 | 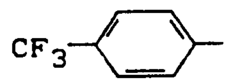 |
| 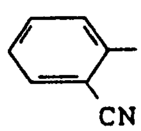 | 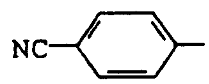 |
| 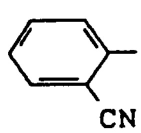 | 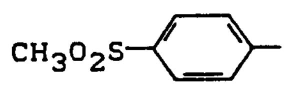 |
| 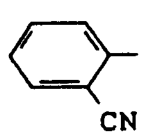 | 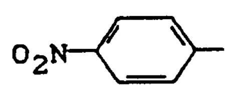 |
| 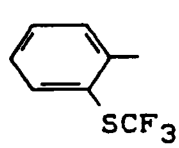 | 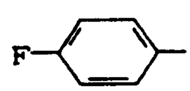 |

Tabelle 3 (Fortsetzung)

| $R^1$ | $Ar^1$ |
|---|---|
| 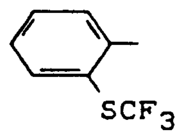 | 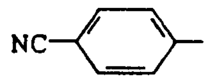 |
| 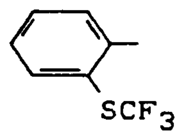 | 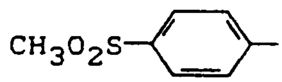 |
| 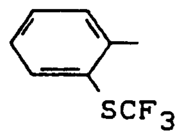 | 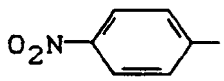 |
| 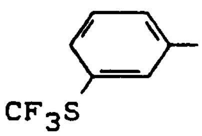 | 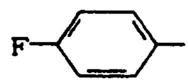 |
| 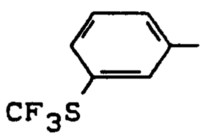 | 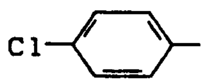 |
| 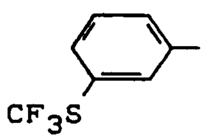 | 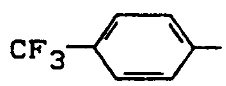 |
| 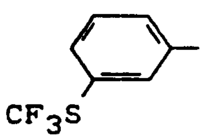 | 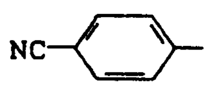 |

Tabelle 3 (Fortsetzung)

| $R^1$ | $Ar^1$ |
|---|---|
| 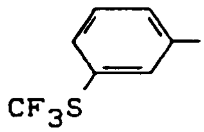 | 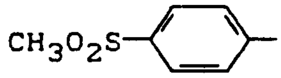 |
| 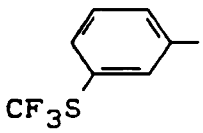 | 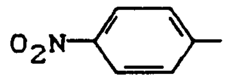 |
| 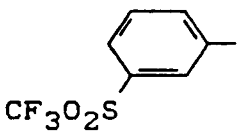 | 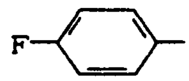 |
| 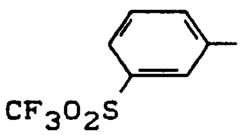 | 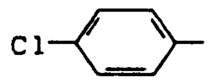 |
| 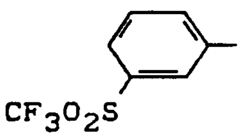 | 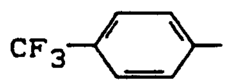 |
| 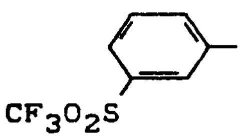 | 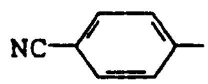 |
| 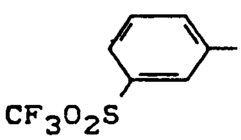 | 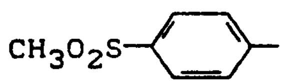 |

Tabelle 3 (Fortsetzung)

| $R^1$ | $Ar^1$ |
|---|---|
| 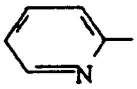 | 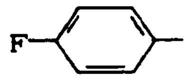 |
| 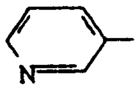 | 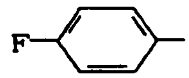 |
| 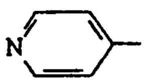 | 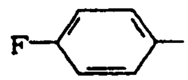 |
| 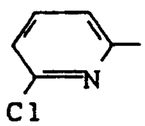 | 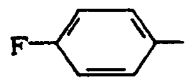 |
| 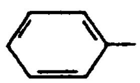 | 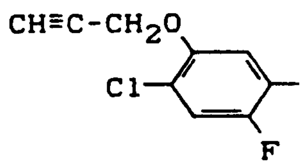 |
| 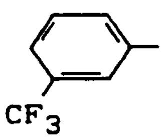 | 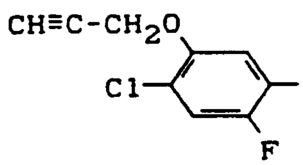 |
| 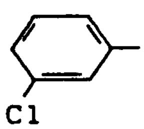 | 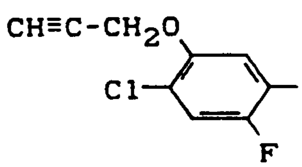 |

Tabelle 3 (Fortsetzung)

| $R^1$ | $Ar^1$ |
|---|---|
| 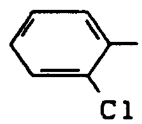 | 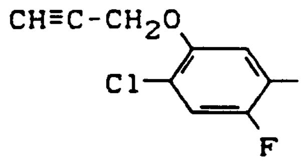 |
| 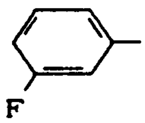 | 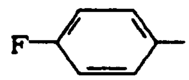 |
| 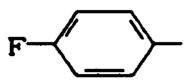 | 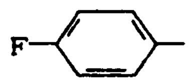 |
| 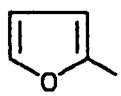 | 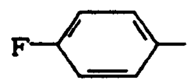 |
| 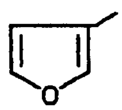 | 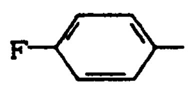 |
| 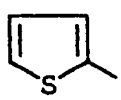 | 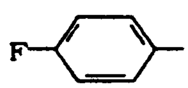 |
| 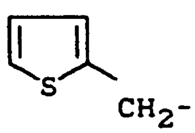 | 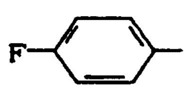 |

Tabelle 3 (Fortsetzung)

| $R^1$ | $Ar^1$ |
|---|---|
| 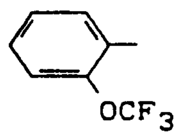 | 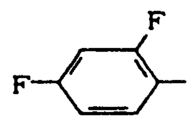 |
| 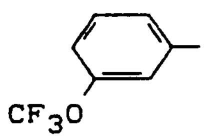 | 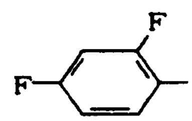 |
| 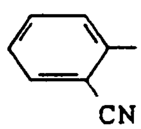 | 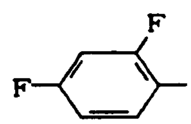 |
| 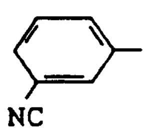 | 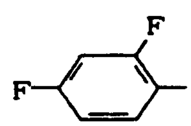 |
| 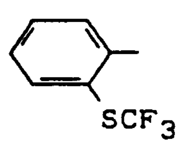 | 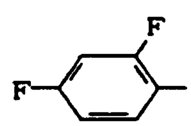 |
| 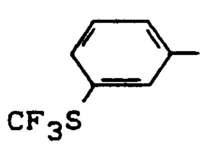 | 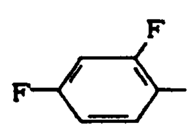 |

Tabelle 3 (Fortsetzung)

| $R^1$ | $Ar^1$ |
|---|---|
| 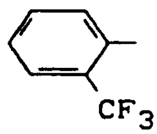 | 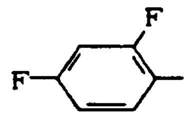 |
| 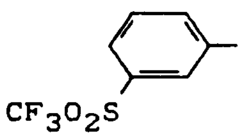 | 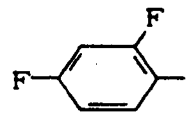 |
| 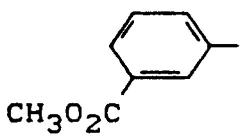 | 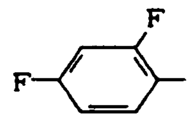 |
| 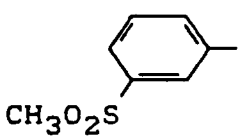 | 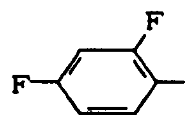 |
| 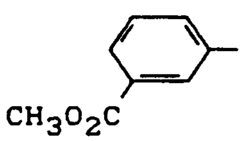 | 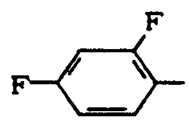 |
| 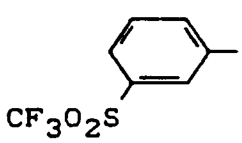 | 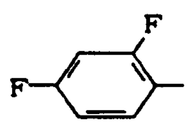 |

Tabelle 3 (Fortsetzung)

| $R^1$ | $Ar^1$ |
|---|---|
| 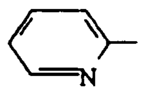 | 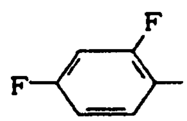 |
| 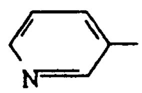 | 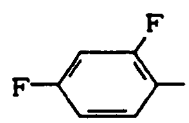 |
| 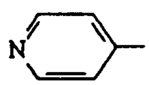 | 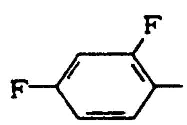 |
| 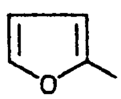 | 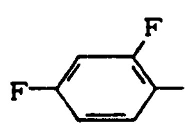 |
| 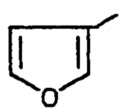 | 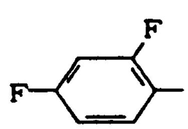 |
| 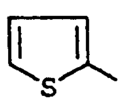 | 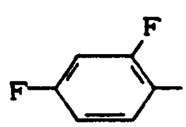 |
| 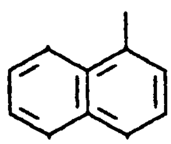 | 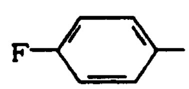 |

Tabelle 3 (Fortsetzung)

| $R^1$ | $Ar^1$ |
|---|---|
| 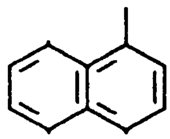 | 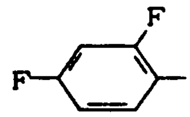 |
| 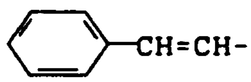 | 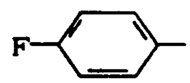 |
| 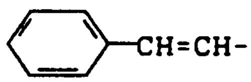 | 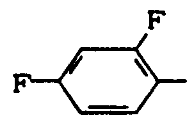 |
| 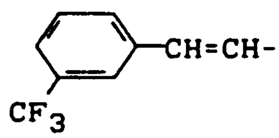 | 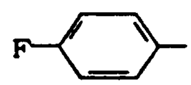 |
| 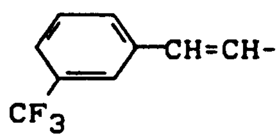 | 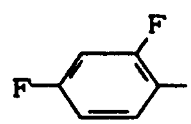 |

Die Pyrazolin-5-on Derivate der Formel (VI) sind teilweise bekannt und teilweise Gegenstand einer nicht zum veröffentlichten Stand der Technik gehörenden Patentanmeldung der Anmelderin (vgl. die Deutsche Anmeldung P 36 25 686).

Man erhält die neuen und bekannten Pyrazolin-5-on Derivate der Formel VI beispielsweise, indem man

1.) Alkoxymethylenmalonester der Formel (IX)

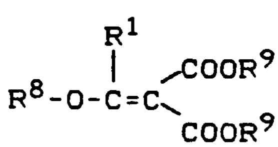

(IX)

in welcher

$R^1$ die oben angegebene Bedeutung hat und

$R^8$ und $R^9$ unabhängig voneinander für Methyl oder Ethyl stehen,

mit Arylhydrazinen der Formel (X)

$Ar^1$-NH-$NH_2$ (X)

in welcher

$Ar^1$ die oben angegebene Bedeutung hat,

zunächst in einer ersten Stufe gegebenenfalls in Gegenwart eines Verdünnnungsmittels, wie beispielsweise Methanol oder Ethanol, bei Temperaturen zwischen 10 ° C und 80 ° C umsetzt, wobei das intermediär auftretende Zwischenprodukt der Formel (XI)

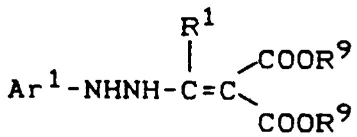

(XI)

in welcher

$R^1$, $R^9$ und $Ar^1$ die oben angegebene Bedeutung haben,

gegebenenfalls isoliert und in einer separaten Reaktionsstufe cyclisiert werden kann, und die so erhältlichen Pyrazolcarbonsäureester der Formel (XII)

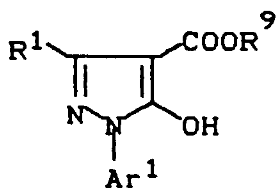

(XII)

in welcher

$R^1$,$R^9$ und $Ar^1$ die oben angegebenen Bedeutungen haben,

in einer zweiten Stufe gegebenenfalls in Gegenwart eines Verdünnungsmittels, wie beispielsweise Methanol, und gegebenenfalls in Gegenwart einer Base, wie beispielsweise Natriumhydroxid, bei Temperaturen zwischen 30 ° C und 70 ° C decarboxyliert werden.

Die Cyclisierung und anschließende Decarboxylierung kann gegebenenfalls auch in einer Reaktionsstufe als 'Eintopfverfahren' durchgeführt werden (vgl. z.B. Liebigs Ann. Chem. 373, 142 (1910) sowie die Herstellungsbeispiele).

Die Alkoxymethylenmalonester der Formel (IX) sind allgemein bekannte Verbindungen der organischen Chemie.

Die Arylhydrazine der Formel (X) sind bekannt bzw. können nach bekannten Verfahren erhalten werden (vgl. z.B. Houben-Weyl, Methoden der organischen Chemie, Band X, 2; S. 203, Thieme Verlag Stuttgart 1967)

oder man erhält die Verbindungen der Formel (VI),

2.) indem man β-Ketoester der Formel (XIII)

$R^1$-CO-$CH_2$-$COOR^9$ (XIII)

in welcher

$R^1$ und $R^9$ die oben angegebene Bedeutung haben,

mit Arylhydrazinen der Formel (X) gegebenenfalls in Gegenwart eines Verdünnungsmittels, wie beispielsweise Toluol, und gegebenenfalls in Gegenwart eines Katalysators, wie beispielsweise p-Toluolsulfonsäure, bei Temperaturen zwischen 0 ° C und 120 ° C umsetzt (vgl. beispielsweise J. Am. Chem. Soc. 64,2133 (1942),

oder

3.) indem man Propiolester der Formel (XIV)

CH≡C-$COOR^9$ (XIV)

in welcher

$R^9$ die oben angegebene Bedeutung hat,

mit Arylhydrazinen der Formel (X), gegebenenfalls in Gegenwart eines Verdünnungsmittels, wie beispielsweise Toluol, bei Temperaturen zwischen 0 ° C und 120 ° C umsetzt, und die so erhältliche Zwischenstufe der Formel (XV)

$Ar^1-NH-NH-CH=CH-COOR^9$ (XV)

in welcher

$Ar^1$ und $R^9$ die oben angegebene Bedeutung haben,

in Gegenwart einer starken Base, wie z.B. Natriummethylat und gegebenenfalls in Gegenwart eines Verdünnungsmittels, wie beispielsweise Methanol, bei Temperaturen zwischen 0 °C und 80 °C umsetzt, oder

4.) indem man Verbindungen der Formel (XIVa)

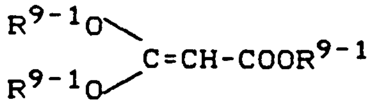

(XIVa)

in welcher

$R^{9-1}$ für $C_1$-$C_4$-Alkyl, insbesondere Methyl oder Ethyl, steht,

mit Arylhydrazinen der Formel (X) gegebenenfalls in Gegenwart eines Verdünnungsmittels wie beispielsweise Ethanol bei Temperaturen zwischen 0 °C und 120 °C umsetzt, und die so erhältliche Zwischenstufe der Formel (XVa)

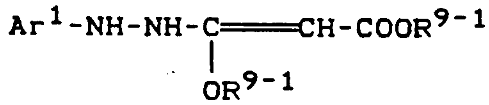

(XVa)

in welcher

$Ar^1$ und $R^{9-1}$ die oben angegebenen Bedeutungen haben,

in Gegenwart einer starken Base wie z. B. Natriummethylat und gegebenenfalls in Gegenwart eines Verdünnungsmittels wie beispielsweise Ethanol bei Temperaturen zwischen 50 °C und 150 °C umsetzt.

Die Propiolester der Formel (XIV) und die Verbindungen der Formel XIVa sind allgemein bekannte Verbindungen der organischen Chemie oder lassen sich nach bekannten Methoden herstellen (vgl. ).

Im einzelnen seien außer den bei den Herstellungsbeispielen genannten Verbindungen die folgenden Pyrazolin-5-on Derivate der Formel (VI) genannt:

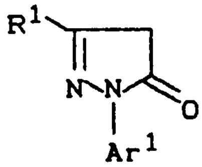

(VI)

Tabelle 4

| $R^1$ | $Ar^1$ |
|---|---|
| 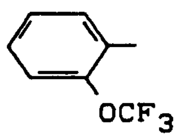 | 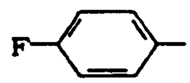 |
| 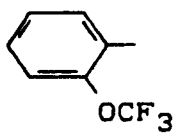 | 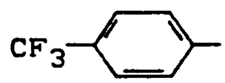 |
| 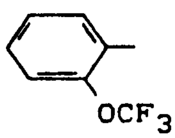 | 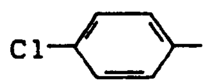 |

Tabelle 4 (Fortsetzung)

| $R^1$ | $Ar^1$ |
|---|---|
| 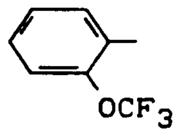 | 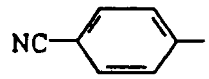 |
| 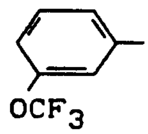 | 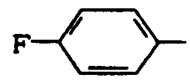 |
| 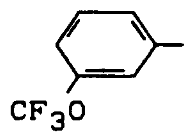 | 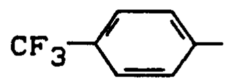 |
| 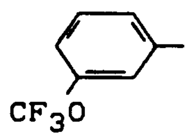 | 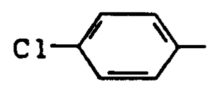 |
| 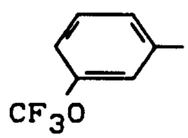 | 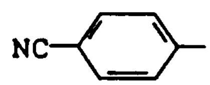 |
| 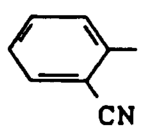 | 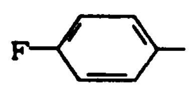 |
| 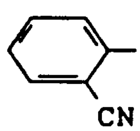 | 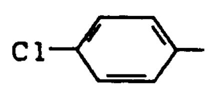 |

Tabelle 4 (Fortsetzung)

| $R^1$ | $Ar^1$ |
|---|---|
| 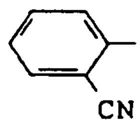 | 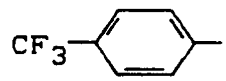 |
| 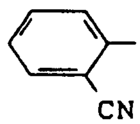 | 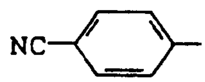 |
| 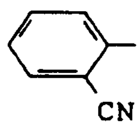 | 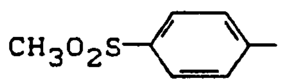 |
| 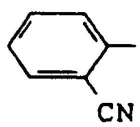 | 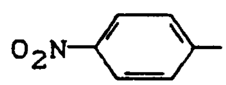 |
| 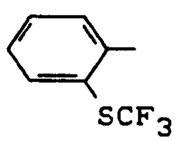 | 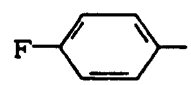 |
| 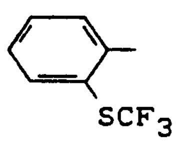 | 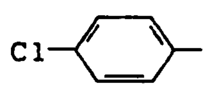 |
| 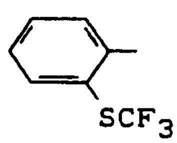 | 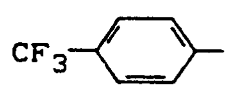 |

Tabelle 4 (Fortsetzung)

| $R^1$ | $Ar^1$ |
|---|---|
| 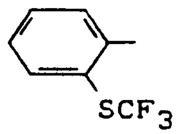 | 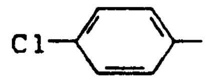 |
| 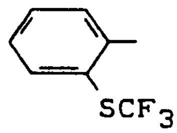 | 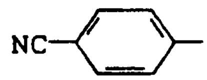 |
| 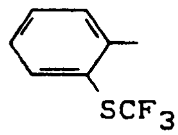 | 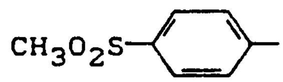 |
| 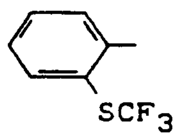 | 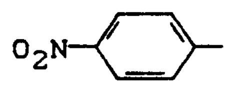 |
| 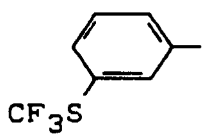 | 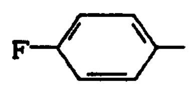 |
| 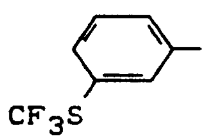 | 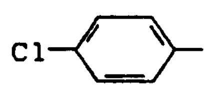 |
| 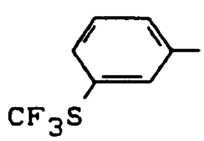 | 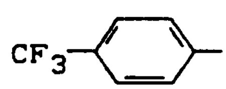 |

Tabelle 4 (Fortsetzung)

| $R^1$ | $Ar^1$ |
|---|---|
| 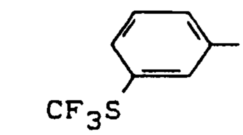 | 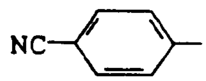 |
| 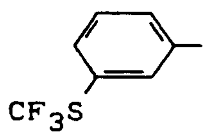 | 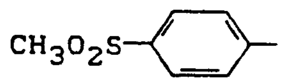 |
| 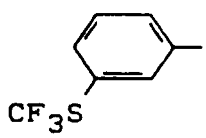 | 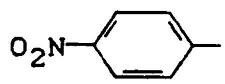 |
| 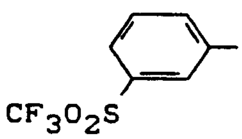 | 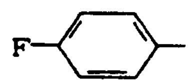 |
| 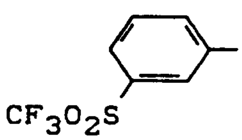 | 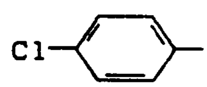 |
| 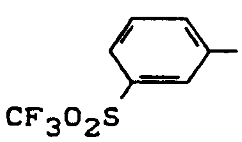 | 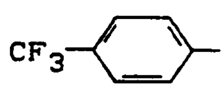 |
| 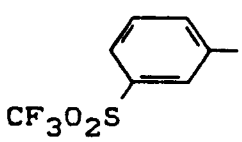 | 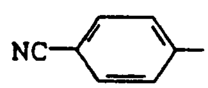 |

Tabelle 4 (Fortsetzung)

| $R^1$ | $Ar^1$ |
|---|---|
| 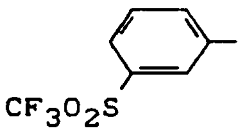 | 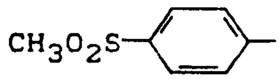 |
| 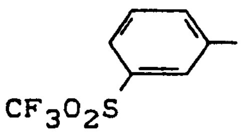 | 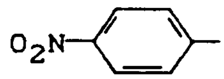 |
| 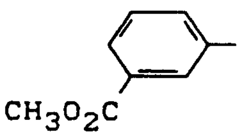 | 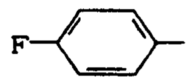 |
| 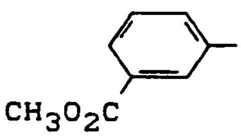 | 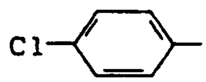 |
| 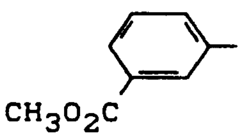 | 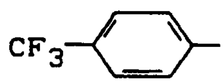 |
| 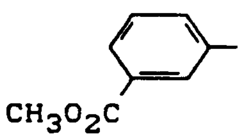 | 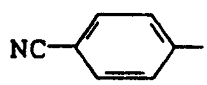 |
| 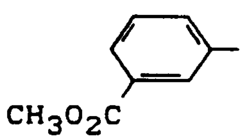 | 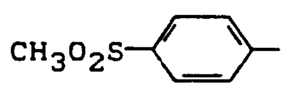 |

Tabelle 4 (Fortsetzung)

| $R^1$ | $Ar^1$ |
|---|---|
| 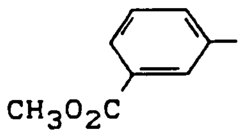 | 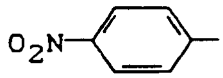 |
| 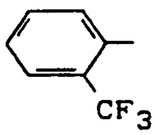 | 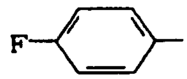 |
| 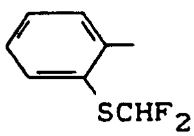 | 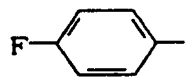 |
| 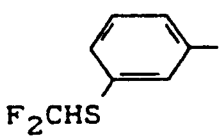 | 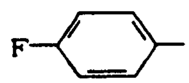 |
| 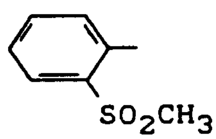 | 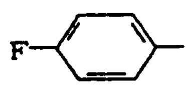 |
| 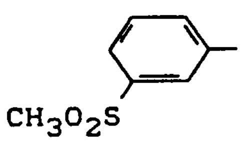 | 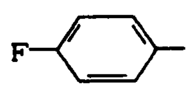 |
| 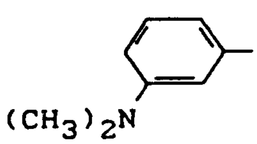 | 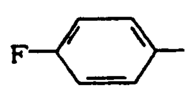 |

Tabelle 4 (Fortsetzung)

| $R^1$ | $Ar^1$ |
|---|---|
| 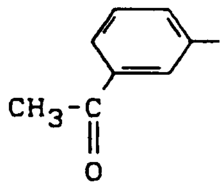 | 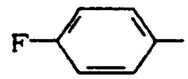 |
| 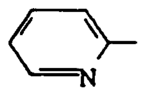 | 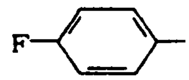 |
| 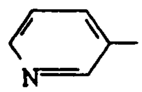 | 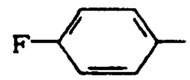 |
| 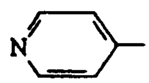 | 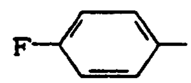 |
| 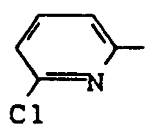 | 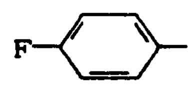 |
| 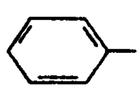 | 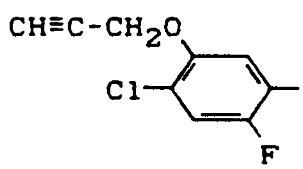 |
| 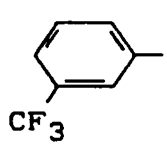 | 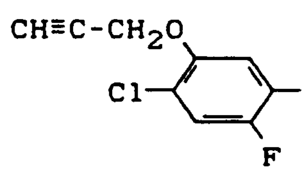 |

Tabelle 4 (Fortsetzung)

| $R^1$ | $Ar^1$ |
|---|---|
| 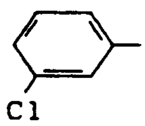 | 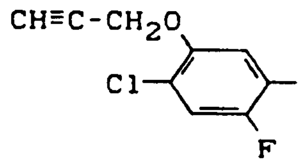 |
| 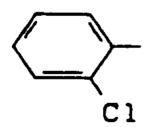 | 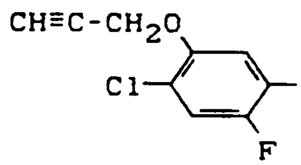 |
| 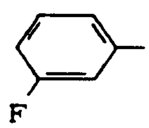 | 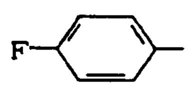 |
| 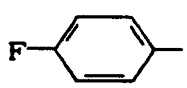 | 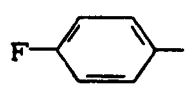 |
| 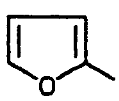 | 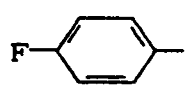 |
| 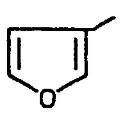 | 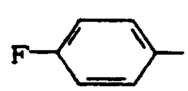 |
| 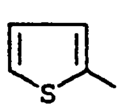 | 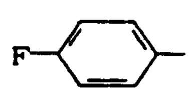 |

Tabelle 4 (Fortsetzung)

| $R^1$ | $Ar^1$ |
| --- | --- |
| 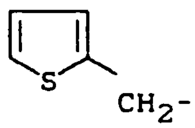 | 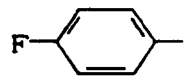 |
| 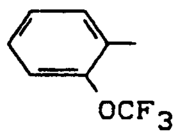 | 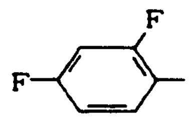 |
| 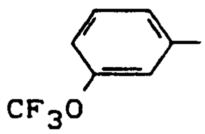 | 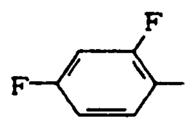 |
| 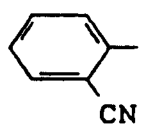 | 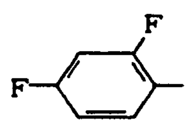 |
| 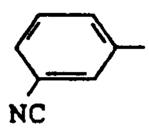 | 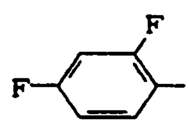 |
| 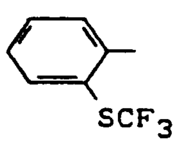 | 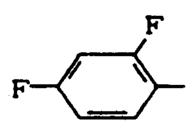 |

Tabelle 4 (Fortsetzung)

| $R^1$ | $Ar^1$ |
|---|---|
| 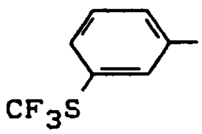 | 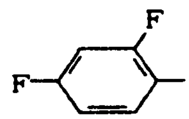 |
| 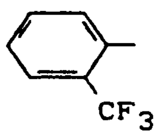 | 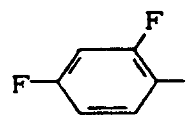 |
| 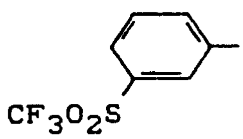 | 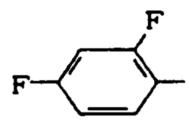 |
| 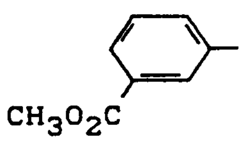 | 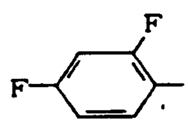 |
| 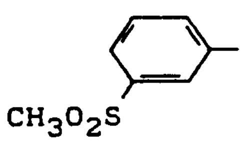 | 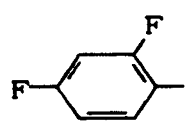 |
| 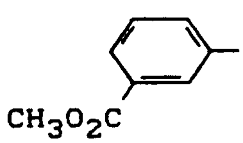 | 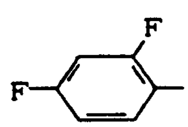 |

Tabelle 4 (Fortsetzung)

| $R^1$ | $Ar^1$ |
|---|---|
| 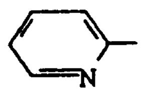 | 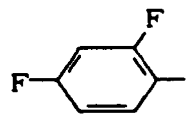 |
| 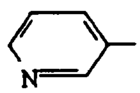 | 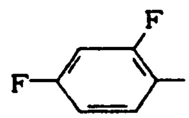 |
| 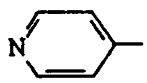 | 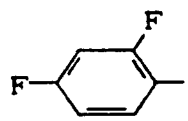 |
| 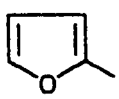 | 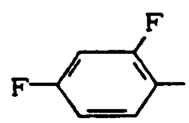 |
| 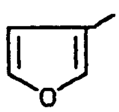 | 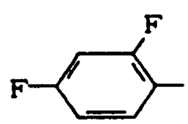 |
| 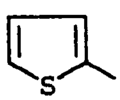 | 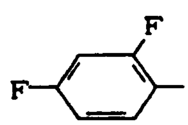 |
| 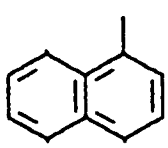 | 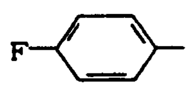 |

Tabelle 4 (Fortsetzung)

| $R^1$ | $Ar^1$ |
|---|---|
| 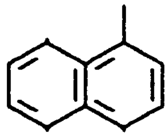 | 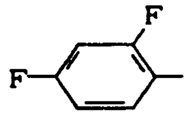 |
| 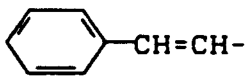 | 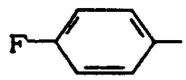 |
| 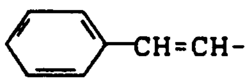 | 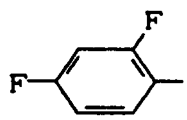 |
| 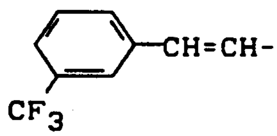 | 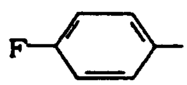 |
| 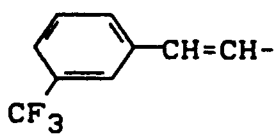 | 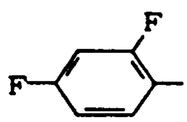 |

Die beim erfindungsgemäßen Verfahren (Ia/α) und (Ia/β) außerdem als Ausgangsstoffe zu verwendenden Amine sind durch die Formel (II) allgemein definiert. In dieser Formel (II) steht $R^7$ für diejenigen Reste, die bereits im Zusammenhang mit der Beschreibung der erfindungsgemäßen Stoffe der Formel (Ia) für diesen Substituenten genannt wurden.

Die Amine der Formel (II) sind allgemein bekannte Verbindungen der organischen Chemie.

Die als Ausgangsstoffe zur Herstellung der substituierten Pyrazolin-5-on Derivate der Formel (I) zu verwendenen 4-Formyl-pyrazolin-5-on Derivate der Formel (IVa)

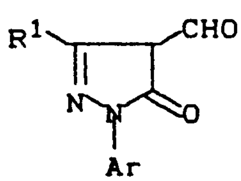

(IVa)

in welcher

$R^1$ und Ar für diejenigen Reste stehen, die bereits im Zusammenhang mit der Beschreibung der Stoffe der Formel (I) für diese Substituenten genannt wurden,

sind teilweise bekannt [Kurkorskaya, L.N., Zh. Org. Khim., 11 (8), 1734 (1975)].

Die beim erfindungsgemäßen Verfahren (Ia/β) außerdem als Ausgangsstoffe zu verwendenden 4-Formyl-pyrazolin-5-on Derivate sind durch die Formel (IV) allgemein definiert.

In dieser Formel (IV) stehen $R^1$ und $Ar^1$ für diejenigen Reste, die bereits im Zusammenhang mit der Beschreibung der erfindungsgemäßen Stoffe der Formel (Ia) für diese Substituenten genannt wurden.

Die Verbindungen der Formel (IV) sind neu und Teil der vorliegenden Erfindung.

Die Verbindungen der Formel (IV) können erhalten werden, indem man 4-(N, N-Dimethylamino-methyliden)-pyrazolin-5-on Derivate der Formel (Id)

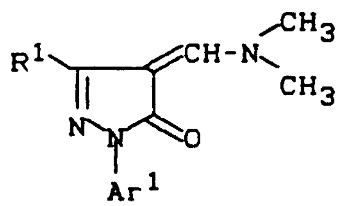

(Id)

in welcher

$R^1$ und $Ar^1$ die oben angegebene Bedeutung haben,

gegebenenfalls in Gegenwart eines Verdünnungsmittels und in Gegenwart einer Base hydrolisiert. Die Herstellung der Ausgangsstoffe der Formel (IV) erfolgt vorzugsweise unter Verwendung von Verdünnungsmitteln.

Als solche kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Basen kommen Alkalimetallhydroxide, wie z.B. Natrium-und Kaliumhydroxid, Erdalkalihydroxide, wie z.B. Calcium-hydroxid, Alkalicarbonate und -alkoholate wie Natrium- und Kaliumcarbonat, Natrium- und Kaliummethylat bzw. -ethylat, infrage, welche im Überschuß eingesetzt werden.

Die zur Herstellung der substituierten Pyrazolin-5-on Derivate der Formel (Ib) außerdem als Ausgangsstoffe zu verwendenden Hydroxylamine sind durch die Formel (V) allgemein definiert. In dieser Formel (V) steht $R^6$ vorzugsweise für diejenigen Reste, die bereits im Zusammenhang mit der Beschreibung der erfindungsgemäßen Stoffe der Formel (Ib) vorzugsweise für diesen Substituenten genannt wurden.

Die Hydroxylamine der Formel (V) sind allgemein bekannte Verbindungen der organischen Chemie.

Die zur Herstellung der substituierten Pyrazolin-5-on Derivate der Formeln (Ia), (Ib) und (Ic) außerdem als Ausgangsstoffe zu verwendenden 4-(N,N-Dimethylamino-methyliden)-pyrazolin-5-on Derivate, die Teil der Erfindung sind, sind durch die Formel (Id) allgemein definiert.

Das zur Herstellung der substituierten Pyrazolin-5-on Derivate der Formel (Ic) gemäß Verfahrensvariante (Ic/β) weiterhin benötigte 1,3,5-Triazin der Formel (VII) ist eine allgemein bekannte Verbindung der organischen Chemie.

Das zur Herstellung der substituierten Pyrazolin-5-on Derivate der Formel (Ic) gemäß Verfahrensvariante (Ic/β) außerdem als Ausgangsstoffe zu verwendenden Pyrazolin-5-one sind durch die Formel (VI) allgemein definiert. In dieser Formel (VI) stehen $R^1$ und $Ar^1$ vorzugsweise für diejenigen Reste, die bereits im Zusammenhang mit der Beschreibung der erfindungsgemäßen Stoffe der Formel (Ic) vorzugsweise für diese Substituenten genannt wurden.

Die Verbindungen der Formel (VI) wurden bereits oben bei der Beschreibung der Ausgangsstoffe zur Herstellung der neuen Verbindungen der Formel (Id) beschrieben. Die Verfahren zur Herstellung der neuen Pyrazolin-5-on Derivate der Formeln (Ia), (Ib), (Ic) und (Id) werden vorzugsweise unter Verwendung von Verdünnungsmitteln durchgeführt.

Als Verdünnungsmittel kommen dabei praktisch alle inerten organischen Lösungsmittel oder wäßrige Systeme infrage. Hierzu gehören vorzugsweise Alkohole wie Methanol, Ethanol, Methoxyethanol, Propanol oder t-Butanol, aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid, oder auch Wasser oder wäßrig-organische Zweiphasen-Gemische, wie Dichlormethan-Wasser oder Toluol-Wasser.

Die Reaktionstemperaturen können bei den Verfahren zur Herstellung der neuen Pyrazolin-5-on Derivate der Formeln (Ia), (Ib), (Ic) und (Id) in einem größeren Bereich variiert werden. Im allgemeinen arbeitet man bei Temperaturen zwischen 0 °C und 180 °C, vorzugsweise bei Temperaturen zwischen 10 °C und 150 °C.

Die Verfahren zur Herstellung der neuen Pyrazolin-5-on Derivate der Formeln (Ia) bis (Id) werden im allgemeinen bei Normaldruck durchgeführt. Unter bestimmten Voraussetzungen kann jedoch auch unter erhöhtem oder vermindertem Druck gearbeitet werden.

Zur Durchführung der Verfahren zur Herstellung der neuen Pyrazolin-5-on Derivate der Formeln (Ia), (Ib) (Ic) und (Id) werden die jeweils benötigten Ausgangsstoffe im allgemeinen in angenähert äquimolaren Mengen eingesetzt.

Es ist jedoch auch möglich, eine der beiden jeweils eingesetzten Komponenten in einem größeren Überschuß zu verwenden. Das Reaktionsgemisch wird mehrere Stunden bei der jeweils erforderlichen Temperatur gerührt. Die Aufarbeitung und Isolierung erfolgt nach üblichen Methoden.

Die erfindungsgemäßen Verbindungen der Formeln (Ia) bis (Id) bzw, die erfindungsgemäß zu verwendenden Verbindungen der Formel (I) können gegebenenfalls mit einer Säure oder Base in salzartige Verbindungen übergeführt werden.

Zur Herstellung von Säureadditionssalzen der Verbindungen der Formeln (I) bis (Id) kommen vorzugsweise folgende Säuren in Frage: Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren, wie z.B. p-Toluolsulfonsäure und 1,5-Naphthalindisulfonsäure.

Die Säureadditionssalze der Verbindungen der Formeln (I) bis (Id) können in einfacher Weise nach üblichen Salzbildungsmethoden, z.B. durch Lösen einer Verbindung der Formeln (I) bis (Id) in einem geeigneten organischen Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Basenadditionssalzen der Verbindungen der Formel (Ib) kommen vorzugsweise Amine in Frage: die Alkylamine, wie z.B. Methyl- und Dimethylamin; Cycloalkylamine, wie z.B. Cyclopentyl- und Cyclohexylamin; heterocyclische Amine, wie Piperidin, Pyrrolidin und Pyrazol. Weiterhin auch Alkali- und Erdalkalihydroxide, wie z.B. Natrium- und Kaliumhydroxid. Die Herstellung kann wie bei den Säureadditionssalzen beschrieben erfolgen.

Die Verfahrensbedingungen bei der Herstellung von Pyrazolin-5-on Derivaten der Formel (If) gemäß den Verfahrensvarianten (If/$\alpha$ und $\beta$) entsprechen den Reaktionsbedingungen, die bereits oben bei der Beschreibung der Verfahren (Ia/$\alpha$ und $\beta$) genannt wurden.

Die beim erfindungsgemäßen Verfahren (If/$\alpha$ und $\beta$) als Ausgangsstoffe benötigten Amine sind durch die Formel (IIa) allgemein definiert. In der Formel (IIa) steht $R^{7-1}$ für diejenigen Reste, die bereits im Zusammenhang mit der Beschreibung der erfindungsgemäßen Stoffe der Formel (If) für diesen Substituenten genannt wurden. Die Amine der Formel (IIa) sind bekannte Verbindungen der organischen Chemie.

Auf die weiterhin benötigten Verbindungen der Formel (IIIa) ist bereits oben bei der Beschreibung der Stoffe der Formel (III) näher eingegangen worden. In der Formel (IIIa) hat $R^{1-1}$ diejenigen Bedeutungen, die bereits bei der Beschreibung der erfindungsgemäßen Stoffe der Formel (If) für diesen Substituenten genannt wurden.

Die beim Verfahren (If/$\beta$) benötigten 4-Formyl-pyrazolin-5-on Derivate der Formel (IVb) fallen unter die oben beschriebene Formel (IV). $R^{1-1}$ in Formel (IVb) hat diejenigen Bedeutungen, die bei der Beschreibung der Stoffe der Formel (If) für diesen Rest genannt wurden.

Die bei dem Verfahren zur Herstellung von substituierten Pyrazolin-5-on Derivaten der Formel (I) bzw. (Ia), (Ib), (Ic) und (Id) mit $R^1$ = -NH-CO-$R^{10}$ benötigten Arylhydrazine der Formel (X), Verbindungen der Formel (XVI) und (XIX) sind bekannt oder lassen sich nach bekannten Methoden in einfacher Weise darstellen [vgl. z. B. Chem. Ber. 28, 478 (1895)].

In den Formeln (X), (XVII), (XVIII), (XIX) und (VIa) haben $Ar^1$ und $R^{11}$ diejenigen Bedeutungen, die bereits bei der Beschreibung der erfindungsgemäßen Stoffe der Formeln (Ia), (Ib), (Ic) bzw. (Id) für diese

Substituenten angegeben wurden.

Die erste und zweite Stufe des Verfahrens werden vorzugsweise in Gegenwart eines Verdünnungsmittels durchgeführt.

Als Verdünnungsmittel kommen dabei praktisch alle inerten organischen Lösungsmittel infrage. Hierzu gehören vorzugsweise Alkohole wie Methanol, Ethanol, Methoxy-ethanol, Propanol oder t-Butanol, aliphatische und aromatische, gegebenenfalls halogenierte Kohlenwasserstoffe wie Pentan, Hexan, Heptan, Cyclohexan, Petrolether, Benzin, Ligroin, Benzol, Tuluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl und Methyl-isobutylketon, Ester wie Essigsäuremethylester und -ethylester, Essigsäure, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid. Die zweite Stufe des Verfahrens wird in Gegenwart starker Basen durchgeführt. Vorzugsweise verwendet man Natriummethylat und Natriumethylat

Die Reaktionstemperaturen können sowohl bei der ersten, als auch der zweiten Stufe in einem größeren Bereich variiert werden. Im allgemeinen arbeitet man bei Temperaturen zwischen 0 °C und 180 °C, vorzugsweise bei Temperaturen zwischen 10 °C und 150 °C.

Zur Durchführung des Verfahrens der ersten und zweiten Stufe werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden.

Als Verdünnungsmittel zur Durchführung der Acylierung von Verbindungen der Formel (XVIII) kommen inerte organische Lösungsmittel infrage. Vorzugsweise verwendet man aliphatische oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff, Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid.

Verwendet man Verbindungen der Formel (XIX) in flüssiger Form, so ist es auch möglich, diese in entsprechendem Überschuß als Verdünnungsmittel einzusetzen.

Als Säurebindemittel kommen für die Acylierung alle üblicherweise verwendbaren anorganischen und organischen Basen infrage. Vorzugsweise verwendet man Alkalimetallhydroxide oder -carbonate, wie beispielsweise Natriumhydroxid, Kaliumhydroxid, Natriumcarbonat oder Kaliumcarbonat oder auch tertiäre, wie beispielsweise Triethylamin, N,N-Dimethylanilin, Pyridin, 4-(N,N-Dimethylamino)-pyridin, Diazabicyclooctan (DABCO), Diazobicyclononen (DBN) oder Diazabicycloundecen (DBU).

Die Reaktionstemperaturen können bei der Acylierung in einem größeren Bereich variiert werden. Im allgemeinen arbeitet man zwischen 0 °C und +180 °C, vorzugsweise zwischen 10 °C und +150 °C.

Zur Durchführung der Acylierung setzt man pro Mol 3-Amino-pyrazolin-5-on der Formel (XVIII) im allgemeinen 1 bis 20 Mol, vorzugsweise 1 bis 15 Mol an Acylierungsmittel der Formel (XIX) und im allgemeinen 1 bis 3 Mol, vorzugsweise 1 bis 2 Mol an Säurebindemittel ein. Die Reaktionsführung, Aufarbeitung und Isolierung der Verbindungen der Formel (VIa) erfolgt in üblicher Art und Weise.

Die erfindungsgemäß verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Bekämpfung mono- und dikotyler Unkräuter in mono- und dikotylen Kulturen im Vor- und Nachauflaufverfahren.

Die erfindungsgemäß verwendbaren Wirkstoffe weisen u.a. eine starke mikrobizide Wirkung auf und können zur Bekämpfung von unerwünschten Mikroorganismen praktisch eingesetzt werden. Die Wirkstoffe sind z.B. für den Gebrauch als Pflanzenschutzmittel geeignet, besonders als Fungizide.
Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%,

vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die erfindungsgemäß verwendbaren Wirkstoffe können mit besonders gutem Erfolg protektiv und systemisch gegen Phytophthora bei Tomaten eingesetzt werden.

Außerdem zeigen die erfindungsgemäßen Wirkstoffe auch eine fungizide Wirkung gegen Pyricularia an Reis.

Die erfindungsgemäß verwendbaren Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Tyluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwas-serstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid, als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z.B. N-(2-Benzthiazolyl)-N,N′-dimethylharnstoff, 3-(3-Chlor-4-methylphenyl)-1,1-dimethylharnstoff, 3-(4-Isopropylphenyl)-1,1-dimethylharnstoff, 3-(α,α,α-Trifluoro-m-tolyl)-1,1-dimethylharnstoff, 2-tert.-Butyl-amino-4-ethylamino-6-methylthio-s-triazin, 2-Chlor-N-{[4-methoxy-6-methyl-1,3,5-triazin-2-yl)-amino]-carbonyl}-benzolsulfonamid, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 2-(4-Chlor-2-methyl-phenoxy)-propionsäure, 3,5-Diiod-4-hydroxy-benzonitril, [(4-Amino-3,5-dichlor-6-fluor-2-pyridinyl)oxy]-essigsäure, 3-Isopropyl-2,1,3-benzothiadiazinon-(4)-2,2-dioxid, N-Methyl-2-(1,3-benzthiazol-2-yloxy)-acetanilid, α-Chlor-2′,6′-diethyl-N-(2-propoxyethyl)-acetanilid, Hexahydro-1H-azepin-1-carbamidsäurethiolethylester, N,N-Dimethyl-N′-(3,4-dichlorphenyl)-harnstoff, 2-Chlor-N-(2-ethyl-6-methylphenyl)-N-(2-methoxy-1-methylethyl)-acetamid, 2′-Chlor-2-(4-chlor-o-tolyloxy)-acetanilid, 5-(2,4-Dichlorphenoxy)-2-nitrobenzoesäuremethylester, 2-[[[[(4-Methoxy-6-methyl-1,3,5-triazin-2-yl)-amino]-carbonyl]-amino]-sulfonyl]-benzoesäuremethylester, 2-((((((4,6-Dimethoxy-2-pyrimidinyl)-amino)-carbonyl)-amino-sulfonyl)-methyl)-benzoesäuremethylester, N-(3,4-Dichlorphenyl)-propanamid. Einige Mischungen besitzen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Verwendung und die Herstellung der erfindungsgemäßen Wirkstoffe geht aus den nachfolgenden Beispielen hervor.

## Anwendungsbeispiele

In den folgenden Anwendungsbeispielen werden die nachstehend aufgeführten Verbindungen als Vergleichssubstanzen eingesetzt:

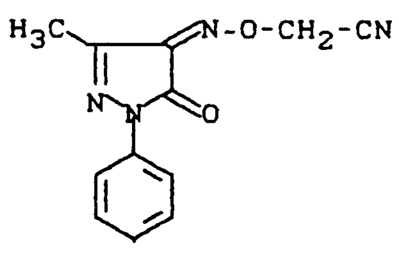

(A)

4-(Cyanmethyloximino)-3-methyl-1-phenyl-pyrazolin-5-on
(bekannt aus EP-OS 0 166 171) und

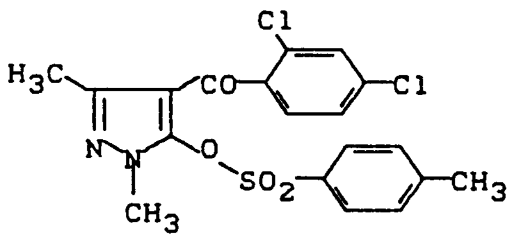

(B)

[4-(2,4-Dichlorbenzoyl)-1,3-dimethylpyrazol-5-yl]-4-methylphenylsulfonat
(bekannt aus DE-OS 25 13 750, Seite 43).

Beispiel A

Pre-emergence Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es beduuten:

0 % = keine Wirkung (wie unbehandelte Kontrolle)
100 % = totale Vernichtung

In diesem Test zeigen z.B. die Verbindungen der folgenden Tabelle A eine deutlich bessere herbizide Wirksamkeit als die Vergleichssubstanz (B).

**Tabelle A**

**Pre emergence Test / Gewächshaus**

| Wirkstoff | Aufwand-menge g/ha | Baum-wolle | Reis | Amaran-thus | Portu-lak | Sina-pis | Viola | Echino-chloa |
|---|---|---|---|---|---|---|---|---|
| B (bekannt) | 500 | 0 | 0 | 0 | 0 | 0 | 0 | 0 |
| Ia- 17 | 500 | 0 | 0 | 100 | 100 | 95 | 100 | 90 |
| Ia- 42 | 500 | 10 | 20 | 100 | 100 | 80 | 100 | 70 |
| Ia- 55 | 500 | 0 | 40 | 95 | 100 | 100 | 70 | 90 |
| Ia- 87 | 500 | 10 | 0 | 100 | 100 | 70 | 70 | 40 |
| Ia-106 | 500 | 10 | 0 | 100 | 100 | 70 | 95 | 60 |
| Ia-111 | 500 | 10 | 0 | 100 | 80 | 70 | 80 | 60 |
| Ia-133 | 500 | 0 | 0 | 100 | 90 | 50 | 90 | 20 |
| Ia-137 | 500 | 10 | 0 | 80 | 80 | 90 | 95 | 30 |
| Ia-146 | 500 | 0 | 0 | 100 | 100 | 90 | 70 | 70 |
| Ia-153 | 500 | 10 | 20 | 100 | 60 | 100 | 100 | 90 |
| Ia-154 | 500 | 10 | 10 | 100 | 100 | 100 | 70 | 90 |
| Ia-172 | 500 | 20 | 0 | 100 | 100 | 70 | 90 | 90 |
| Ia-188 | 500 | 0 | 0 | 95 | 80 | 50 | - | 60 |
| Ia-189 | 500 | 10 | 0 | 100 | 100 | 70 | - | 70 |
| Ia-190 | 500 | 10 | 20 | 100 | 100 | 95 | - | 80 |

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigen z.B. die in der folgenden Tabelle B aufgeführten Verbindungen insbesondere in Reis und Weizen eine sehr gute herbizide Wirkung.

Tabelle B

Post emergence Test / Gewächshaus

| Wirkstoff | Aufwandmenge g/ha | Reis | Weizen | Amaranthus | Galium | Viola | Setaria |
|---|---|---|---|---|---|---|---|
| Ia- 17 | 1000 | 10 | 0 | 100 | 70 | 80 | 80 |
| Ia- 42 | 1000 | 10 | 60 | 80 | 100 | 90 | 100 |
| Ia- 55 | 1000 | 20 | 20 | 90 | 20 | 80 | 95 |
| Ia- 87 | 500 | 10 | 0 | 80 | - | 70 | 40 |
| Ia-111 | 1000 | 60 | 40 | 100 | 60 | 90 | 90 |
| Ia-133 | 500 | 30 | 20 | 90 | 70 | 90 | 70 |
| Ia-136 | 500 | 10 | 0 | 95 | 70 | 80 | 80 |
| Ia-137 | 1000 | 0 | 0 | 95 | 50 | 95 | 70 |
| Ia-142 | 1000 | 20 | 20 | 90 | 30 | 70 | 50 |
| Ia-146 | 1000 | 10 | 10 | 95 | 80 | 50 | 60 |
| Ia-153 | 1000 | 10 | 10 | 100 | 100 | 95 | 100 |
| Ia-154 | 1000 | 20 | 10 | 60 | 30 | 70 | 60 |
| Ia-172 | 1000 | 0 | 10 | 100 | 60 | 90 | 100 |
| Ia-183 | 500 | 30 | 20 | 80 | 60 | 80 | 95 |
| Ia-187 | 500 | 60 | 20 | 70 | 60 | 90 | 70 |
| Ia-188 | 1000 | 30 | 0 | 80 | 10 | 80 | 40 |
| Ia-189 | 1000 | 30 | 0 | 100 | 95 | 95 | 50 |
| Ia-190 | 1000 | 30 | 0 | 90 | 50 | 100 | 70 |

Beispiel C

Phytophthora-Test (Tomate)/systemisch

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20 °C aufgestellt.

3 Tage nach der Inokulation erfolgt die Auswertung.

In diesem Test zeigen z.B. die erfindungsgemäßen Stoffe [(Ic)-1], [(Ic)-2], [(Ic)-5], [(Ic)-6], [(Ic)-12] und [-(Ic)-13] eine bessere Wirksamkeit als die Vergleichssubstanz (A).

## Tabelle C

Phytophthora-Test (Tomate)/systemisch

| Wirkstoff | Befall in % bei einer Wirkstoffkonzentration von 100 ppm |
|---|---|
| 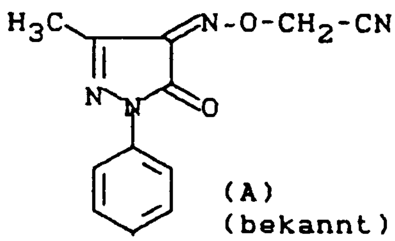 (A) (bekannt) | 70 |
| 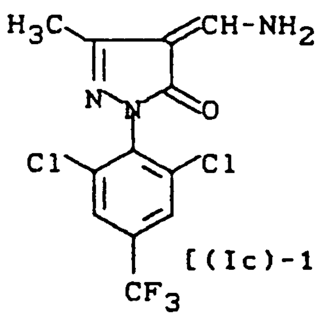 [(Ic)-1] | 10 |
| 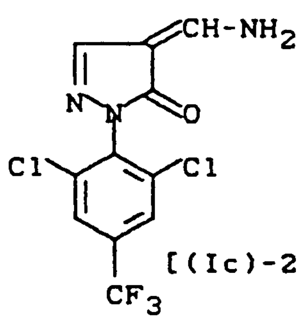 [(Ic)-2] | 24 |
| 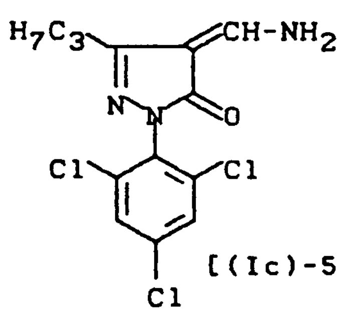 [(Ic)-5] | 5 |

Tabelle C (Fortsetzung)

Phytophthora-Test (Tomate)/systemisch

| Wirkstoff | Befall in % bei einer Wirkstoffkonzentration von 100ppm |
|---|---|
| 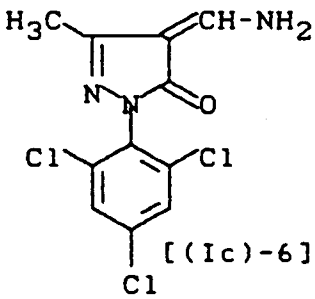 [(Ic)-6] | 2 |
| 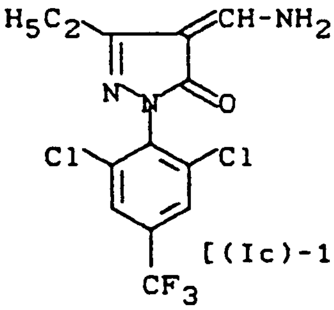 [(Ic)-12] | 29 |
| 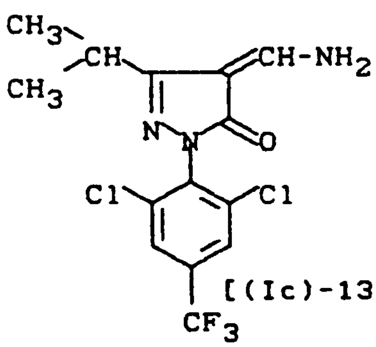 [(Ic)-13] | 15 |

Beispiel D

Phytophthora-Test (Tomate)/protektiv

Lösungsmittel: 4,7 Gewichtsteile Aceton
Emulgator: 0,3 Gewichtsteile Alkylarylpolyglylolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca 20 °C aufgestellt.

3 Tage nach der Inokulation erfolgt dei Auswertung.

In diesem Test zeigen z.B. die erfindungsgemäßen Stoffe [(Ic)-1], [(Ic)-2], [(Ic)-6], [(Ib)-11] und [(Ia)-11] eine bessere Wirksamkeit als die Vergleichssubstanz (A).

## Tabelle D

Phytophthora-Test (Tomate)/protektiv

| Wirkstoff | Befall in % bei einer Wirkstoffkonzentration von 10ppm |
|---|---|
| 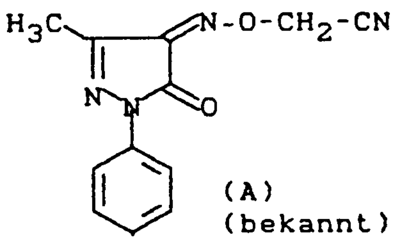 (A) (bekannt) | 63 |
| 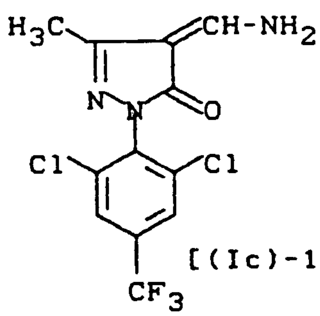 [(Ic)-1] | 7 |
| 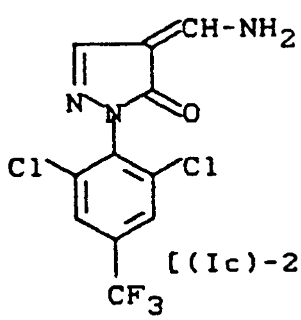 [(Ic)-2] | 10 |

Tabelle D (Fortsetzung)

Phytophthora-Test (Tomate)/protektiv

| Wirkstoff | Befall in % bei einer Wirkstoffkonzentration von 10ppm |
|---|---|
| 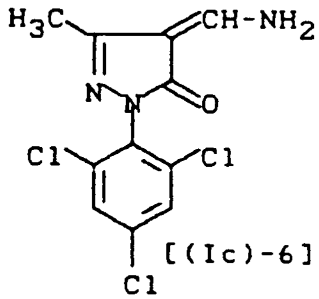 [(Ic)-6] | 10 |
| 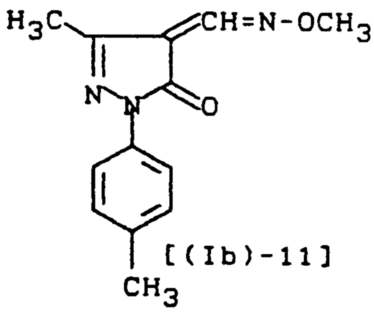 [(Ib)-11] | 30 |
| 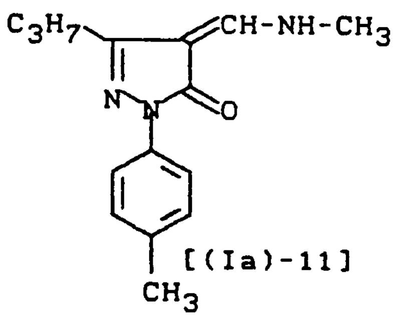 [(Ia)-11] | 30 |

Herstellungsbeispiele

Beispiel 1:

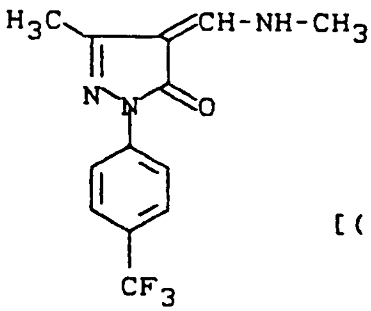

[(Ia)-1]

(Variante [(Ia)-α]

5 g (0,0168 Mol) 1-(4-Trifluormethyl-phenyl)-4-N,N-dimethylamino-methyliden-3-methyl-pyrazolin-5-on werden in 50 ml Methanol gelöst und nach Zugabe von 14 g 30%iger Methylaminlösung bis zum vollständigen Umsatz bei Raumtemperatur gerührt (chromatographische Kontrolle). Anschließend wird das Lösungsmittel abgezogen, der Rückstand in Petrolether verrührt, das Produkt abgesaugt und getrocknet.
Man erhält 3,8g (79,1 % der Theorie) 1-(4-Trifluormethyl-phenyl)-4-methylamino-methyliden-3-methyl-pyrazolin-5-on vom Schmelzpunkt 129-130 °C.

Herstellung der Ausgangsstoffe:

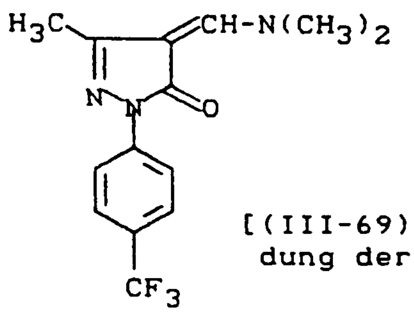

[(III-69) entspricht einer Verbindung der Formel (Id)]

28,6 g (0,12 Mol) 1-(4-Trifluormethyl-phenyl)-3-methyl-pyrazolin-5-onwerden in 150 ml Toluol aufgenommen und nach Zugabe von 10,9 g (0,129 Mol) N,N-Dimethylformamiddimethylacetal bis zum vollständigen Umsatz (chromatographische Kontrolle) bei Raumtemperatur gerührt. Anschließend wird das Lösungsmittel abgezogen, der Rückstand mit Petrolether verrührt, das Produkt abgesaugt und getrocknet.
Man erhält 29,4 g (82,6 % der Theorie) 1-(4-Trifluormethyl-phenyl)-4-N,N-dimethylamino-methyliden-3-methyl-pyrazolin-5-on vom Schmelzpunkt 230-233 °C.

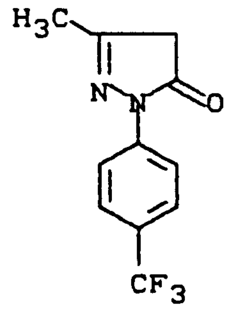

(VI-1)

(Verfahren 2)

22,2 g (0,17 Mol) Acetessigsäureethylester und 30 g (0,170 Mol) 4-Trifluormethyl-hydrazin werden nach Zugabe einer Spatelspitze p-Toluolsulfonsäure 24 Stunden in Toluol am Wasserabscheider erhitzt. Nach dem Abkühlen wird mit Wasser gewaschen, getrocknet und eingeengt. Der Rückstand wird mit Petrolether verrührt, das Produkt abgesaugt und getrocknet.

Man erhält 28,6 g (69,5 % der Theorie) 1-(4-Trifluormethyl-phenyl)-3-methyl-pyrazolin-5-on vom Schmelzpunkt 130 °C.

Beispiel 2

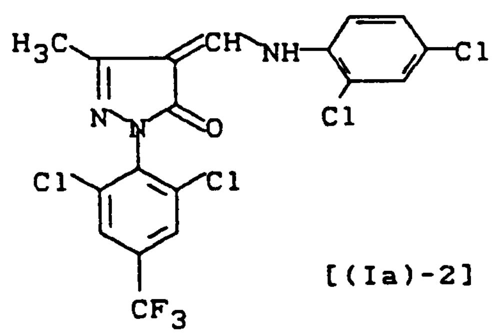

[(Ia)-2]

(Variante [(Ia)-β]

5 g (0,0148 Mol) 1-(2,6-Dichlor-4-trifluormethyl-phenyl)-4-formyl-3-methyl-pyrazolin-5-on werden in 50 ml Dioxan aufgenommen und nach Zugabe von 2,4 g (0,148 Mol) 2,4-Dichloranilin 30 Minuten auf 80 °C erwärmt. Anschließend wird das Lösungsmittel abgezogen, der Rückstand mit Petrolether verrührt, das Produkt abgesaugt und getrocknet.

Man erhält 5,0 g (70 % der Theorie) 4-[(2,4-Dichlorphenylamino)-methyliden]-1-(2,6-dichlor-4-trifluormethyl-phenyl)-3-methyl-pyrazolin-5-on vom Schmelzpunkt 230-232 °C.

Herstellung der Ausgangsstoffe:

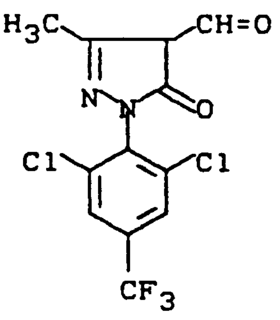

(IV-1)

7,32 g (0,02 Mol) 1-(2,6-Dichlor-4-trifluormethyl-phenyl)-3-methyl-4-N,N-dimethylamino-methyliden-pyrazolin-5-on werden in 70 ml Wasser suspendiert und nach Zugabe von 1,12 g (0,02 Mol) Kaliumhydroxid 3 Stunden bei 45 bis 50 °C gerührt. Dann wird auf 0 °C abgekühlt, mit 10%-iger Salzsäure angesäuert, der ausgefallene Feststoff abgesaugt und getrocknet.

Man erhält 6,15 g (90,4 % der Theorie) 1-(2,6-Dichlor-4-trifluormethyl-phenyl)-4-formyl-3-methyl-pyranzolin-5-on vom Schmelzpunkt 195-197 °C.

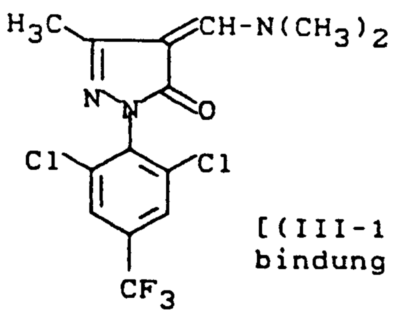

[(III-1) entspricht einer Verbindung der Formel (Id)]

18,7 g (0,06 Mol) 1-(4-Trifluormethyl-2,6-dichlorphenyl)-3-methyl-pyrazolin-5-on werden in 100 ml Toluol aufgenommen und bei Raumtemperatur 7,5 g (0,063 Mol) N,N-dimethylformamiddimethylacetal zugegeben. Anschließend wird der Reaktionsansatz bis zum vollständigen Umsatz (chromatographische Kontrolle) bei Raumtemperatur gerührt. Das Lösungsmittel wird abgezogen, der Rückstand mit Petrolether verrieben, abgesaugt und getrocknet.

Man erhält 21,8 g (99 % der Theorie) 1-(4-Trifluormethyl-2,6-dichlorphenyl)-3-methyl-4-N,N-dimethylamino-methyliden-pyrazolin-5-on vom Schmelzpunkt 195 °C.

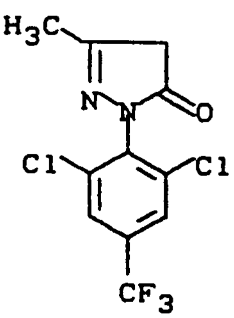

(VI-2)

(Verfahren 2)

116 g (1 Mol) Acetessigsäureethylester und 245 g (1 Mol) 2,6-Dichlor-4-trifluormethyl-phenylhydrazin werden in 1 l Toluol nach Zugabe einer katalytischen Menge p-Toluolsulfonsäure bis zum vollständigen Umsatz (chromatographische Kontrolle) am Wasserabscheider erhitzt. Anschließend wird im Eisbad abgekühlt, der ausgefallene Feststoff abgesaugt, mit Petrolether verrührt, abgesaugt und getrocknet.

Man erhält 218,5 g (70,2 % der Theorie) 1-(4-Trifluormethyl-2,6-dichlor-phenyl)-3-methyl-pyrazolin-5-on vom Schmelzpunkt 174-175 °C.

Analog den Herstellungsbeispielen [(Ia)-1] und [(Ia)-2] und entsprechend den angegebenen Verfahren können die folgenden Endprodukte der Formel (Ia) erhalten werden

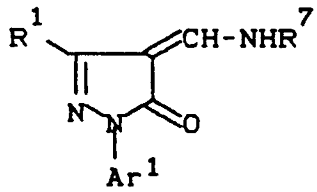

(Ia)

Tabelle 5

| Bsp.Nr. | $R^1$ | $R^7$ | $Ar^1$ | Schmelzpunkt (°C) |
|---|---|---|---|---|
| (Ia)-3 | $CH_3$ | $CH_3$ | 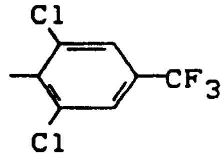 | 199 |
| (Ia)-4 | $CH_3$ | $C_2H_5$ | 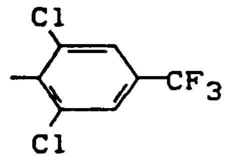 | 177 |
| (Ia)-5 | $CH_3$ | $-CH_2-CH=CH_2$ | 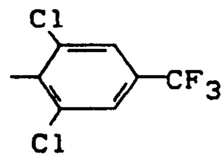 | 152 |
| (Ia)-6 | $CH_3$ | 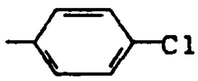 | 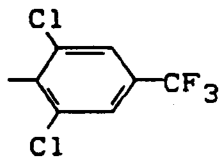 | 239 |
| (Ia)-7 | $CH_3$ | $-CH(CH_3)_2$ | 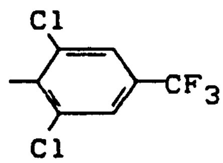 | 197 |
| (Ia)-8 | $CH_3$ | $CH_3$ | 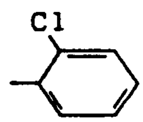 | 198 |
| (Ia)-9 | $CH_3$ | $CH_3$ | 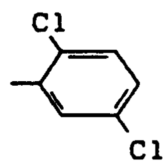 | 192 |
| (Ia)-10 | $CH_3$ | $CH_3$ | 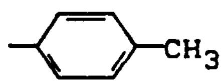 | 195 |
| (Ia)-11 | $n-C_3H_7$ | $CH_3$ | 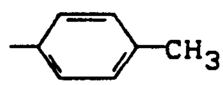 | 120 |

Tabelle 5 (Fortsetzung)

| Bsp.Nr. | $R^1$ | $R^7$ | $Ar^1$ | Schmelzpunkt (°C) |
|---|---|---|---|---|
| (Ia)-12 | $n\text{-}C_3H_7$ | $CH_3$ | 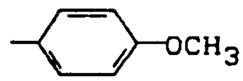 | 98 |
| (Ia)-13 | $C_2H_5$ | $CH_3$ | 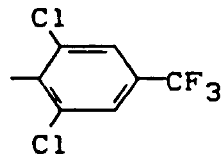 | 187-188 |
| (Ia)-14 | $-CH(CH_3)_2$ | $CH_3$ | 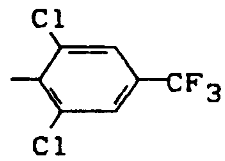 | 175 |
| (Ia)-15 | $-C(CH_3)_3$ | $CH_3$ | 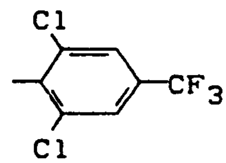 | 207 |
| (Ia)-16 | $CH_3$ | $CH_3$ | 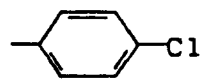 | 195 |
| (Ia)-17 | $n\text{-}C_3H_7$ | $CH_3$ | 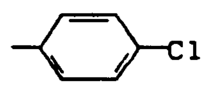 | 126 |
| (Ia)-18 | $CH_3$ | $CH_3$ | 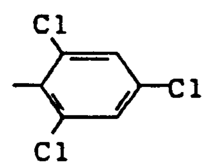 | 216 |
| (Ia)-19 | $n\text{-}C_3H_7$ | $CH_3$ | 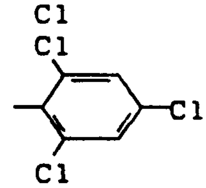 | 227 |
| (Ia)-20 | $CH_3$ | $CH_3$ | 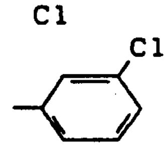 | 130-133 |

Tabelle 5 (Fortsetzung)

| Bsp.Nr. | $R^1$ | $R^7$ | $Ar^1$ | Schmelz-punkt(°C) |
|---|---|---|---|---|
| (Ia)-21 | H | $CH_3$ | 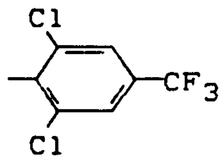 | 183 |
| (Ia)-22 | $CH_3$ | $CH_3$ | 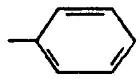 | 156 |
| (Ia)-23 | $n\text{-}C_3H_7$ | $CH_3$ | 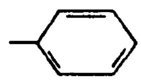 | 80 |
| (Ia)-24 | $CH_3$ | 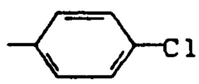 | 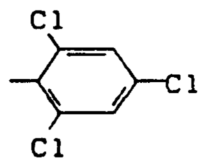 | 208-209 |
| (Ia)-25 | $CF_3$ | $CH_3$ | 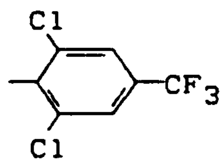 | 231-236 |
| (Ia)-26 | 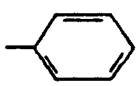 | $CH_3$ | 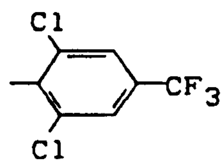 | 240-241 |
| (Ia)-27 | $n\text{-}C_3H_7$ | $CH_3$ | 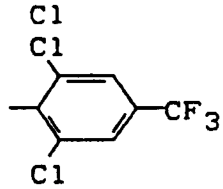 | 158 |
| (Ia)-28 | $CH_3$ | 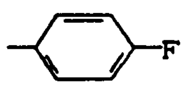 | 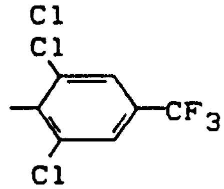 | 206 |
| (Ia)-29 | 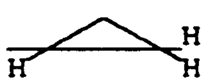 | $CH_3$ | 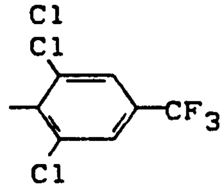 | 216-218 |

Tabelle 5 (Fortsetzung)

| Bsp.Nr. | $R^1$ | $R^7$ | $Ar^1$ | Schmelzpunkt (°C) |
|---|---|---|---|---|
| (Ia)-30 | $CH_3$ | $CH_3$ | 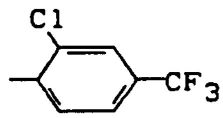 | 213 |
| (Ia)-31 | $CH_3$ | 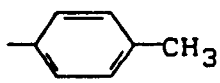 | 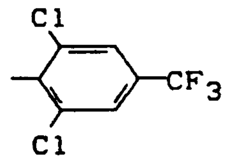 | 216 |
| (Ia)-32 | $CH_3$ | 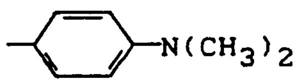 | 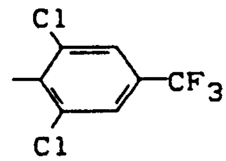 | 100(Zers.) |
| (Ia)-33 | $CH_3$ | 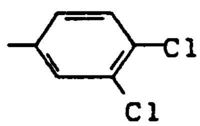 | 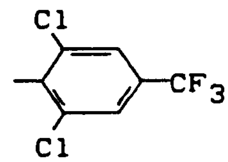 | 172-173 |
| (Ia)-34 | $CH_3$ | $CH_3$ | 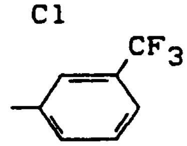 | 119-120 |
| (Ia)-35 | H | $CH_3$ | 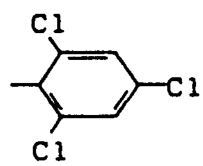 | 169-171 |
| (Ia)-36 | $n-C_3H_7$ | $-CH_2-C(CH_3)_3$ | 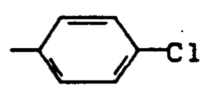 | 95 |
| (Ia)-37 | $n-C_3H_7$ | $-(CH_2)_5-CH_3$ | 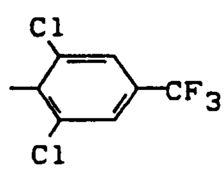 | 69-72 |

Tabelle 5 (Fortsetzung)

| Bsp.Nr. | $R^1$ | $R^7$ | $Ar^1$ | Schmelzpunkt (°C) |
|---|---|---|---|---|
| (Ia)-38 | $n-C_3H_7$ | $-CH(CH_3)_2$ | 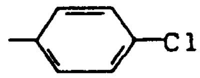 | 100 |
| (Ia)-39 | $n-C_3H_7$ | $-CH_2CH(CH_3)_2$ | 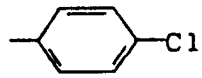 | 70-72 |
| (Ia)-40 | $CH_3$ | $CH_3$ | 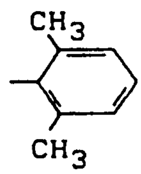 | 185 |
| (Ia)-41 | $-CH_2OCH_3$ | $CH_3$ | 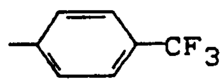 | 145-147 |
| (Ia)-42 | $n-C_3H_7$ | $CH_3$ | 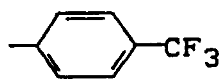 | 145-146 |
| (Ia)-43 | $n-C_3H_7$ | $C_2H_5$ | 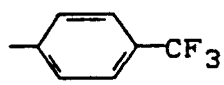 | 75-77 |
| (Ia)-44 | $-CH_2OCH_3$ | $C_2H_5$ | 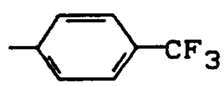 | 88-90 |
| (Ia)-45 | $CH_3$ | $C_2H_5$ | 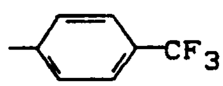 | 112-114 |
| (Ia)-46 | $n-C_3H_7$ | $C_2H_5$ | 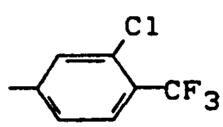 | 120-122 |
| (Ia)-47 | $n-C_3H_7$ | $n-C_3H_7$ | 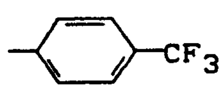 | 63-64 |

Tabelle 5 (Fortsetzung)

| Bsp.Nr. | $R^1$ | $R^7$ | $Ar^1$ | Schmelzpunkt (°C) |
|---|---|---|---|---|
| (Ia)-48 | $-CH_2OCH_3$ | $n-C_3H_7$ | 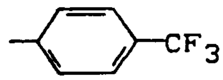 | 41-43 |
| (Ia)-49 | $CH_3$ | $n-C_3H_7$ | 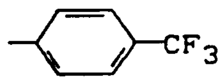 | 79 |
| (Ia)-50 | $n-C_3H_7$ | $n-C_3H_7$ | 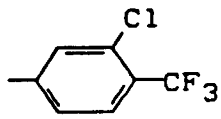 | 105-106 |
| (Ia)-51 | $n-C_3H_7$ | $-CH(CH_3)_2$ | 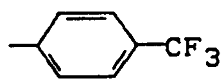 | 60-61 |
| (Ia)-52 | $CH_3$ | $-CH(CH_3)_2$ | 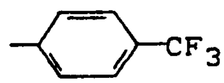 | 106 |
| (Ia)-53 | $-CH_2OCH_3$ | $-CH(CH_3)_2$ | 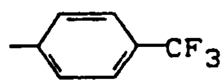 | 39-42 |
| (Ia)-54 | $n-C_3H_7$ | $-CH(CH_3)_2$ | 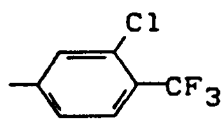 | 116-118 |
| (Ia)-55 | $n-C_3H_7$ | $CH_3$ | 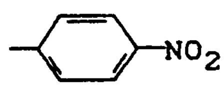 | 215-217 |
| (Ia)-56 | $n-C_3H_7$ | $C_2H_5$ | 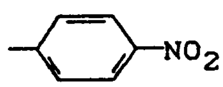 | 164-165 |
| (Ia)-57 | $n-C_3H_7$ | $n-C_3H_7$ | 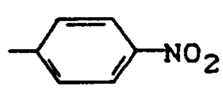 | 123-124 |
| (Ia)-58 | $n-C_3H_7$ | $-CH(CH_3)_2$ | 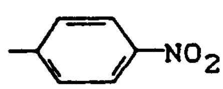 | 149-150 |

Tabelle 5 (Fortsetzung)

| Bsp.Nr. | $R^1$ | $R^7$ | $Ar^1$ | Schmelzpunkt (°C) |
|---|---|---|---|---|
| (Ia)-59 | $n-C_3H_7$ | 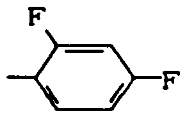 | 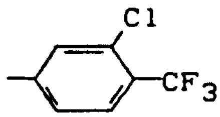 | 204 |
| (Ia)-60 | $-CH_2-CH(CH_3)_2$ | $CH_3$ | 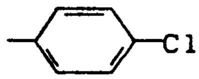 | 87-90 |
| (Ia)-61 | $-CH_2-CH(CH_3)_2$ | $C_2H_5$ | 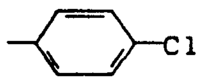 | 79-83 |
| (Ia)-62 | $-CH(CH_3)_2$ | $CH_3$ | 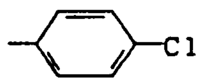 | 115-117 |
| (Ia)-63 | $-CH(CH_3)_2$ | $C_2H_5$ | 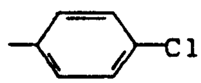 | 93-96 |
| (Ia)-64 | $n-C_4H_9$ | $CH_3$ | 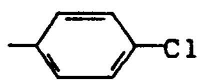 | 150-160 |
| (Ia)-65 | $n-C_4H_9$ | $C_2H_5$ | 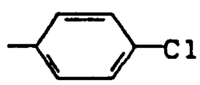 | 172 |
| (Ia)-66 | $-C(CH_3)_3$ | $CH_3$ | 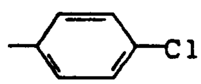 | 132-134 |
| (Ia)-67 | $-C(CH_3)_3$ | $C_2H_5$ | 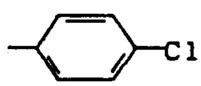 | 134-136 |
| (Ia)-68 | $C_2H_5$ | $CH_3$ | 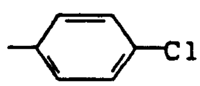 | 147-151 |

Tabelle 5 (Fortsetzung)

| Bsp.Nr. | $R^1$ | $R^7$ | $Ar^1$ | Schmelzpunkt (°C) |
|---|---|---|---|---|
| (Ia)-69 | $C_2H_5$ | $C_2H_5$ | 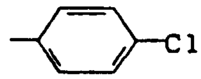 | 76-80 |
| (Ia)-70 | $C_2H_5$ | $n-C_3H_7$ | 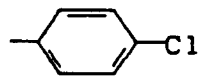 | 182 |
| (Ia)-71 | $C_2H_5$ | $-CH(CH_3)_2$ | 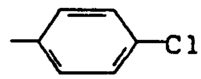 | 195-198 |
| (Ia)-72 | $-C(CH_3)_3$ | $-CH(CH_3)_2$ | 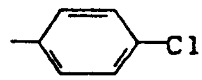 | 141-146 |
| (Ia)-73 | $n-C_3H_7$ | $n-C_3H_7$ | 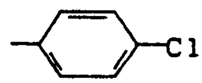 | 150-162 |
| (Ia)-74 | $-CH_2CH(CH_3)_2$ | $n-C_3H_7$ | 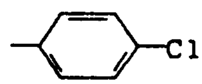 | 63-65 |
| (Ia)-75 | $-CH_2CH(CH_3)_2$ | $-CH(CH_3)_2$ | 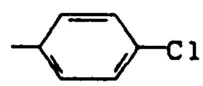 | 93-94 |
| (Ia)-76 | $-CH(CH_3)_2$ | $n-C_3H_7$ | 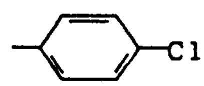 | 59-61 |
| (Ia)-77 | $-CH(CH_3)_2$ | $-CH(CH_3)_2$ | 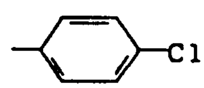 | 96-98 |
| (Ia)-78 | $n-C_4H_9$ | $-CH(CH_3)_2$ | 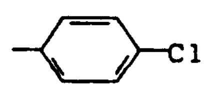 | 245-247 |
| (Ia)-79 | $n-C_3H_7$ | $CH_3$ | 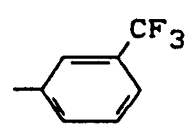 | 99 |

<u>Tabelle 5</u> (Fortsetzung)

| Bsp.Nr. | $R^1$ | $R^7$ | $Ar^1$ | Schmelzpunkt (°C) |
|---|---|---|---|---|
| (Ia)-80 | $n-C_3H_7$ | $C_2H_5$ | 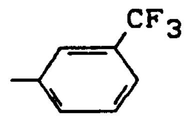 | 52-53 |
| (Ia)-81 | $n-C_3H_7$ | $n-C_3H_7$ | 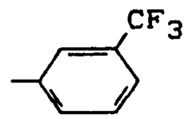 | 51 |
| (Ia)-82 | $n-C_3H_7$ | $-CH(CH_3)_2$ | 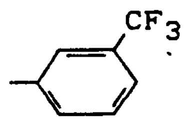 | 44 |
| (Ia)-83 | $n-C_3H_7$ | 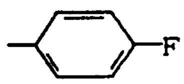 | 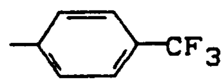 | 172-174 |
| (Ia)-84 | $n-C_3H_7$ | 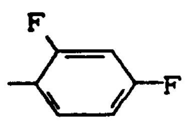 | 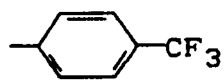 | 196-200 |
| (Ia)-85 | $n-C_3H_7$ | $CH_3$ | 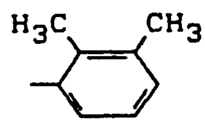 | 123 |
| (Ia)-86 | $CH_3$ | $CH_3$ | 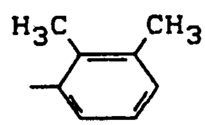 | 166 |
| (Ia)-87 | 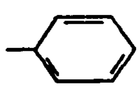 | $CH_3$ | 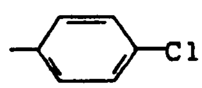 | 160 |
| (Ia)-88 | 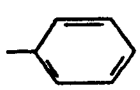 | $C_2H_5$ | 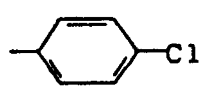 | 136 |

Tabelle 5 (Fortsetzung)

| Bsp.Nr. | $R^1$ | $R^7$ | $Ar^1$ | Schmelzpunkt (°C) |
|---|---|---|---|---|
| (Ia)-89 | $CH_3$ | 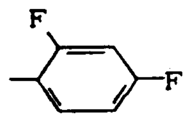 | 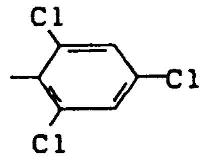 | 229-230 |
| (Ia)-90 | $CH_3$ | 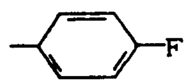 | 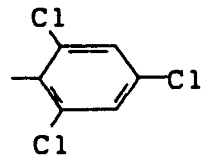 | 183 |
| (Ia)-91 | $CH_3$ | $CH_3$ | 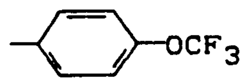 | 92-94 |
| (Ia)-92 | $C_2H_5$ | $CH_3$ | 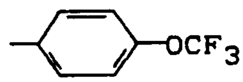 | 67-68 |
| (Ia)-93 | n-$C_3H_7$ | $CH_3$ | 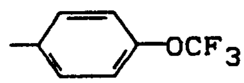 | 53-54 |
| (Ia)-94 | $CH_3$ | 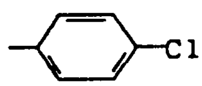 | 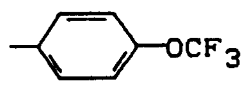 | 182-183 |
| (Ia)-95 | $C_2H_5$ | 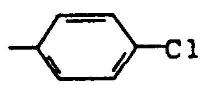 | 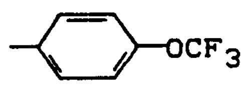 | 141-142 |
| (Ia)-96 | n-$C_3H_7$ | 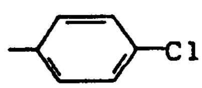 | 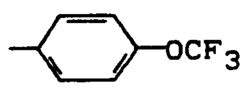 | 131-132 |
| (Ia)-97 | $CH_3$ | 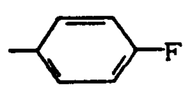 | 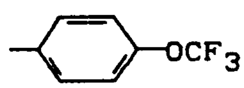 | 175-176 |
| (Ia)-98 | $C_2H_5$ | 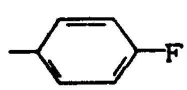 | 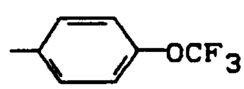 | 141-142 |

Tabelle 5 (Fortsetzung)

| Bsp.Nr. | $R^1$ | $R^7$ | $Ar^1$ | Schmelzpunkt(°C) |
|---|---|---|---|---|
| (Ia)-99 | $n-C_3H_7$ | 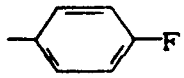 | 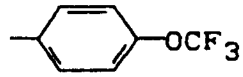 | 112-113 |
| (Ia)-100 | $n-C_3H_7$ | $CH_3$ | 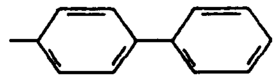 | 93 |
| (Ia)-101 | $C_2H_5$ | $CH_3$ | 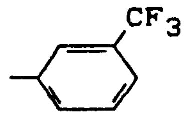 | 145 |
| (Ia)-102 | $-CH_2OCH_3$ | $CH_3$ | 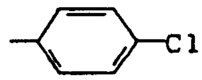 | 138-139 |
| (Ia)-103 | $CF_3$ | $CH_3$ | 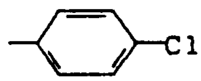 | 195 |
| (Ia)-104 | $CH_3$ | $CH_3$ | 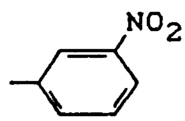 | 210-212 |
| (Ia)-105 | $CH_3$ | 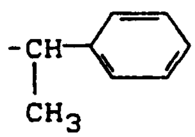 | 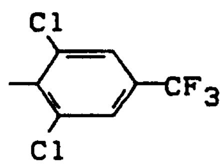 | 160 |
| (Ia)-106 | $n-C_3H_7$ | $CH_3$ | 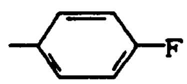 | 68 |
| (Ia)-107 | $n-C_3H_7$ | $CH_3$ | 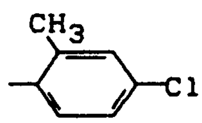 | 147-149 |
| (Ia)-108 | $-CH_2SC_2H_5$ | $CH_3$ | 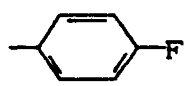 | 125 |
| (Ia)-109 | $CH_3$ | $CH_3$ | 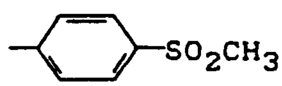 | 243 |
| (Ia)-110 | $CH_3$ | $CH_3$ | 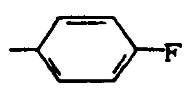 | 145 |

<u>Tabelle 5</u> (Fortsetzung)

| Bsp.Nr. | $R^1$ | $R^7$ | $Ar^1$ | Schmelzpunkt (°C) |
|---|---|---|---|---|
| (Ia)-111 | 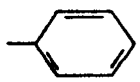 | $CH_3$ | 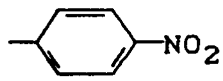 | 270 |
| (Ia)-112 | $CH_3$ | $CH_3$ | 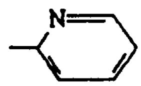 | 81 |
| (Ia)-113 | $-CH_2SC_2H_5$ | $CH_3$ | 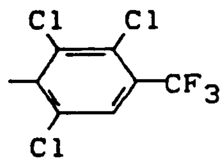 | 163-167 |
| (Ia)-114 | 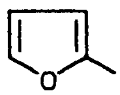 | $CH_3$ | 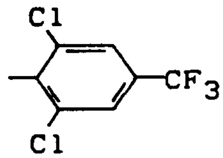 | 241 |
| (Ia)-115 | $-CH_2SCH_3$ | $CH_3$ | 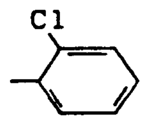 | 103 |
| (Ia)-116 | $-CH_2SCH_3$ | $CH_3$ | 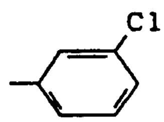 | 136 |
| (Ia)-117 | $-CH_2SCH_3$ | $CH_3$ | 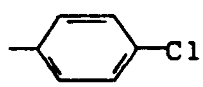 | 155 |
| (Ia)-118 | $-CH_2SCH_3$ | $CH_3$ | 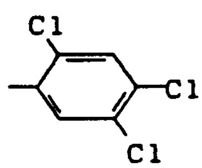 | 140 |
| (Ia)-119 | $-CH_2SCH_3$ | $CH_3$ | 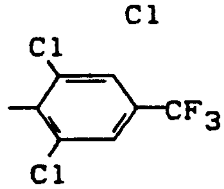 | 185 |
| (Ia)-120 | $-CH_2SCH_3$ | $CH_3$ | 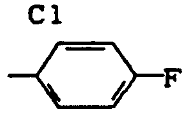 | 126 |
| (Ia)-121 | $-CH_2SCH_3$ | $CH_3$ | 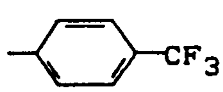 | 143 |

Tabelle 5 (Fortsetzung)

| Bsp.Nr. | $R^1$ | $R^7$ | $Ar^1$ | Schmelzpunkt (°C) |
|---|---|---|---|---|
| (Ia)-122 | $-CH_2SCH_3$ | $CH_3$ | 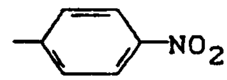 | 234 |
| (Ia)-123 | $-CH_2SCH_3$ | $CH_3$ | 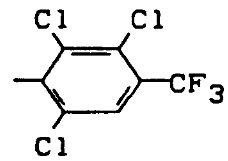 | 208 |
| (Ia)-124 | $-CH_2SCH_3$ | $-CH_2-CH=CH_2$ | 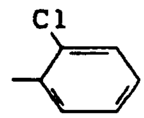 | 62 |
| (Ia)-125 | $-CH_2SCH_3$ | $-CH_2-CH=CH_2$ | 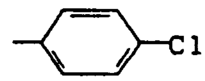 | 133 |
| (Ia)-126 | $-CH_2SCH_3$ | $-CH_2-CH=CH_2$ | 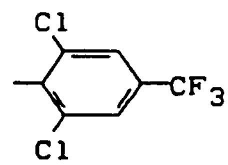 | 169 |
| (Ia)-127 | $-CH_2SCH_3$ | $-CH_2-CH=CH_2$ | 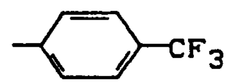 | 113 |
| (Ia)-128 | $-CH_2SCH_3$ | $-CH_2-CH=CH_2$ | 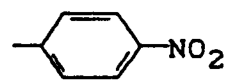 | 140 |
| (Ia)-129 | $-CH_2SCH_3$ | $-CH_2-CH=CH_2$ | 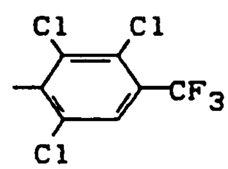 | 138 |
| (Ia)-130 | H | $CH_3$ | 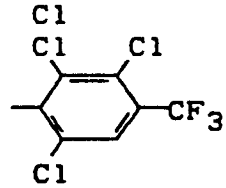 | 203-204 |
| (Ia)-131 | H | $CH_3$ | 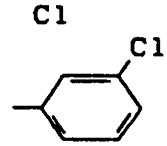 | 112-114 |
| (Ia)-132 | $n-C_3H_7$ | $CH_3$ | 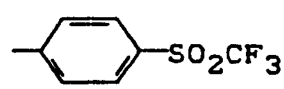 | 159 |

<u>Tabelle 5</u> (Fortsetzung)

| Bsp.Nr. | $R^1$ | $R^7$ | $Ar^1$ | Schmelzpunkt (°C) |
|---|---|---|---|---|
| (Ia)-133 | 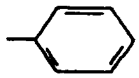 | $CH_3$ | 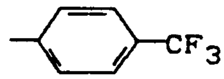 | 151 |
| (Ia)-134 | 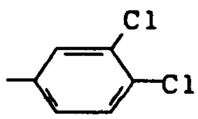 | $CH_3$ | 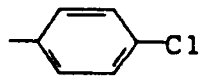 | 200-202 |
| (Ia)-135 | 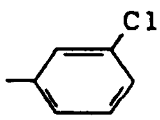 | $CH_3$ | 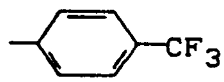 | 145-149 |
| (Ia)-136 | 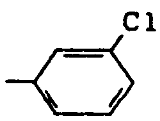 | $CH_3$ | 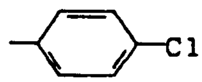 | 168-172 |
| (Ia)-137 | 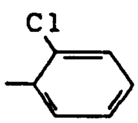 | $CH_3$ | 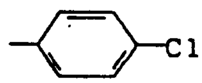 | 224 |
| (Ia)-138 | 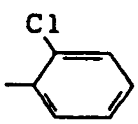 | $CH_3$ | 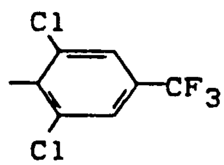 | 125-130 |
| (Ia)-139 | 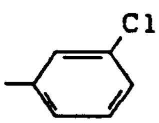 | $CH_3$ | 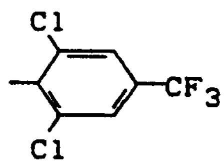 | 208-212 |
| (Ia)-140 | 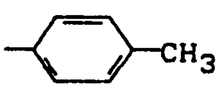 | $CH_3$ | 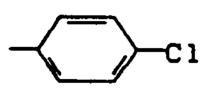 | 185 |
| (Ia)-141 | 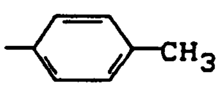 | $CH_3$ | 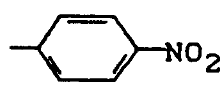 | 256-259 |
| (Ia-142) | 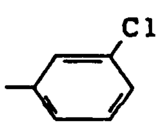 | $CH_3$ | 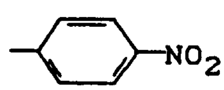 | 250 |

Tabelle 5 (Fortsetzung)

| Bsp.Nr. | $R^1$ | $R^7$ | $Ar^1$ | Schmelzpunkt (°C) |
|---|---|---|---|---|
| (Ia)-143 | 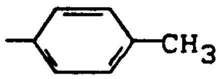 | $CH_3$ | 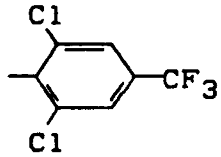 | 178 |
| (Ia)-144 | 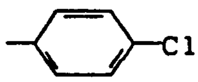 | $CH_3$ | 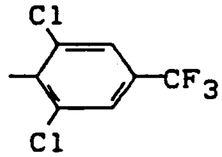 | 238 |
| (Ia)-145 | 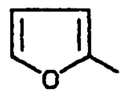 | $CH_3$ | 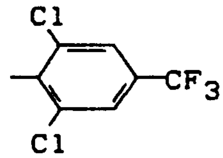 | 240 |
| (Ia)-146 | n-$C_3H_7$ | $CH_3$ | 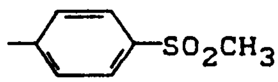 | 194 |
| (Ia)-147 | 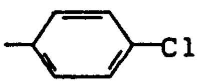 | $CH_3$ | 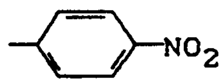 | 290 |
| (Ia)-148 | 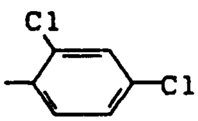 | $CH_3$ | 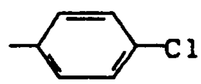 | 229 |
| (Ia)-149 | 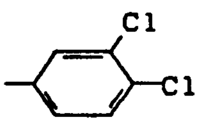 | $CH_3$ | 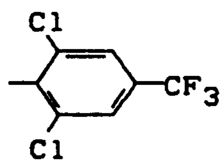 | 225-226 |
| (Ia)-150 | $CH_3$ | $CH_3$ | 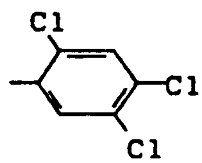 | 230-231 |
| (Ia)-151 | n-$C_3H_7$ | $CH_3$ | 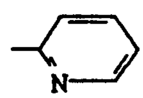 | 188-190 |
| (Ia)-152 | -$CH_2SC_2H_5$ | $CH_3$ | 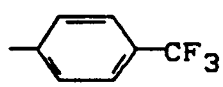 | 110 |

<u>Tabelle 5</u> (Fortsetzung)

| Bsp.Nr. | $R^1$ | $R^7$ | $Ar^1$ | Schmelzpunkt (°C) |
|---|---|---|---|---|
| (Ia)-153 | 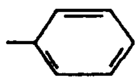 | $CH_3$ | 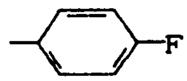 | 145 |
| (Ia)-154 | $n-C_3H_7$ | $CH_3$ | 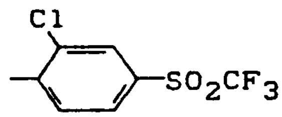 | 185 |
| (Ia)-155 | 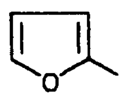 | $CH_3$ | 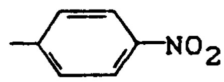 | 249 |
| (Ia)-156 | $-CH_2SC_2H_5$ | $CH_3$ | 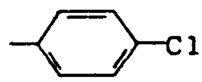 | 147 |
| (Ia)-157 | $CH_3$ | $CH_3$ | 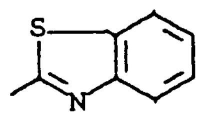 | 195(Zers.) |
| (Ia)-158 | $n-C_3H_7$ | $CH_3$ | 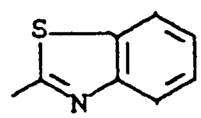 | 179-181 |
| (Ia)-159 | $CH_3$ | $CH_3$ | 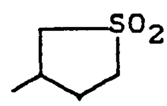 | 226 |
| (Ia)-160 | $CH_3$ | $n-C_3H_7$ | 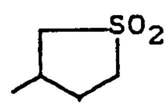 | 177 |
| (Ia)-161 | $-CH_2SC_2H_5$ | $CH_3$ | 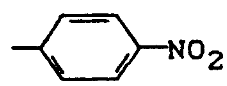 | 189-192 |
| (Ia)-162 | $CH_3$ | $-CH_2CH_2OCH_3$ | 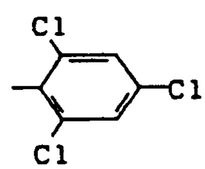 | 178-180 |

Tabelle 5 (Fortsetzung)

| Bsp.Nr. | $R^1$ | $R^7$ | $Ar^1$ | Schmelzpunkt (°C) |
|---|---|---|---|---|
| (Ia)-163 | $CH_3$ | $-CH_2CH_2CH_2OCH_3$ | 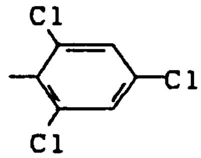 | 130-132 |
| (Ia)-164 | $CH_3$ | $-CH_2CH_2OCH_3$ | 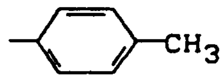 | 206 |
| (Ia)-165 | $CH_3$ | $-CH_2CH_2CH_2OCH_3$ | 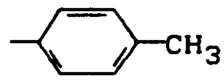 | 132 |
| (Ia)-166 | $CH_3$ | $-CH_2CH_2OCH_3$ | 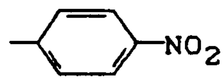 | 58 |
| (Ia)-167 | $CH_3$ | $-CH_2CH_2CH_2OCH_3$ | 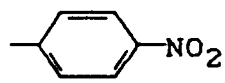 | 37 |
| (Ia)-168 | $n-C_3H_7$ | $-CH_2CH_2CH_2OCH_3$ | 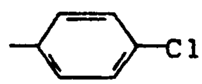 | 68-70 |
| (Ia)-169 | $CH_3$ | $-CH_2CH_2CH_2OCH_3$ | 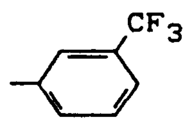 | |
| (Ia)-170 | 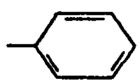 | $-CH_2CH_2OCH_3$ | 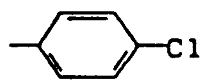 | 108 |
| (Ia)-171 | 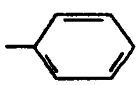 | $-CH_2CH_2CH_2OCH_3$ | 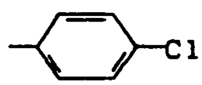 | 116 |
| (Ia)-172 | $n-C_3H_7$ | $CH_3$ | 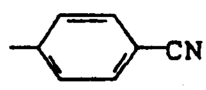 | 183 |
| (Ia)-173 | $n-C_3H_7$ | $-CH_2CH_2OCH_3$ | 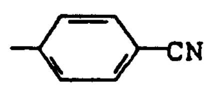 | 124 |

Tabelle 5 (Fortsetzung)

| Bsp.Nr. | $R^1$ | $R^7$ | $Ar^1$ | Schmelzpunkt (°C) |
|---|---|---|---|---|
| (Ia)-174 | $n-C_3H_7$ | $-CH_2CH_2OCH_3$ | 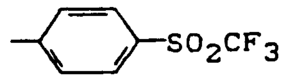 | 120 |
| (Ia)-175 | 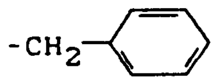 | $CH_3$ | 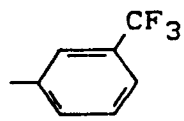 | 128 |
| (Ia)-176 | 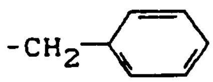 | $CH_3$ | 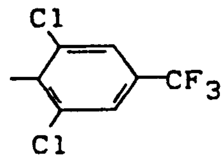 | 179 |
| (Ia)-177 | 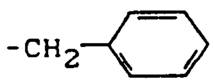 | $CH_3$ | 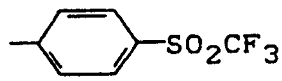 | 188 |
| (Ia)-178 | $-CH_2SCH_3$ | 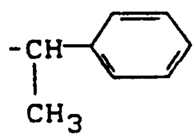 | 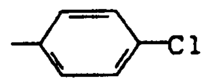 | 60 |
| (Ia)-179 | $-CH_2SCH_3$ | 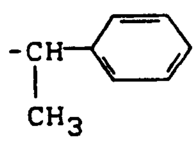 | 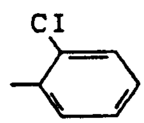 | Oel |
| (Ia)-180 | $-CH_2SCH_3$ | 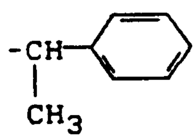 | 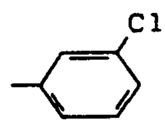 | Oel |
| (Ia)-181 | $-CH_2SCH_3$ | 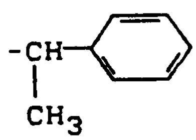 | 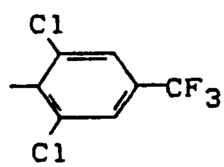 | Oel |
| (Ia)-182 | $nC_3H_7$ | $CH_3$ | 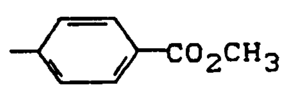 | 169 |
| (Ia)-183 | 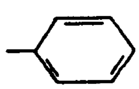 | $CH_3$ | 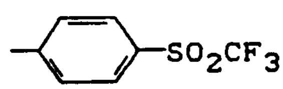 | 185-186 |

Tabelle 5 (Fortsetzung)

| Bsp.Nr. | $R^1$ | $R^7$ | $Ar^1$ | Schmelzpunkt (°C) |
|---|---|---|---|---|
| (Ia)-184 | 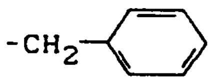 | $CH_3$ | 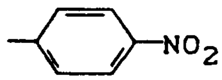 | 247 |
| (Ia)-185 | $-C_3H_7$ | $CH_3$ | 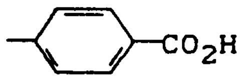 | 236 |
| (Ia)-186 | $-OC_2H_5$ | $CH_3$ | 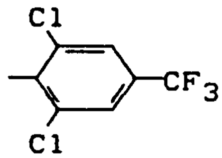 | 202 |
| (Ia)-187 | $-OC_2H_5$ | $CH_3$ | 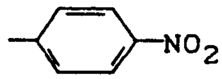 | 199 |
| (Ia)-188 | 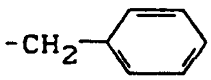 | $CH_3$ | 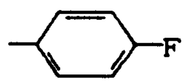 | 169 |
| (Ia)-189 | 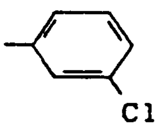 | $CH_3$ | 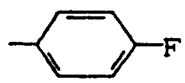 | 142 |
| (Ia)-190 | 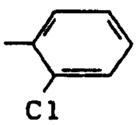 | $CH_3$ | 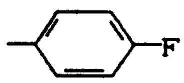 | 202-205 |
| (Ia)-191 | $-OC_2H_5$ | $CH_3$ | 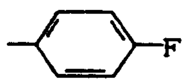 | 117 |
| (Ia)-192 | 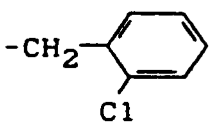 | $CH_3$ | 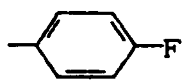 | 165-168 |
| (Ia)-193 | 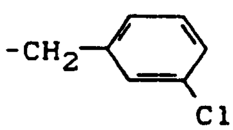 | $CH_3$ | 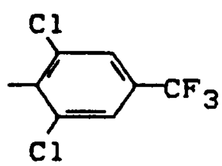 | 158 |
| (Ia)-194 | 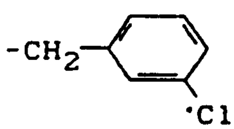 | $CH_3$ | 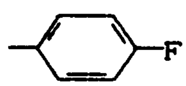 | 152 |

Tabelle 5 (Fortsetzung)

| Bsp.Nr. | $R^1$ | $R^7$ | $Ar^1$ | Schmelzpunkt (°C) |
|---|---|---|---|---|
| (Ia)-195 | $-CH_2$ 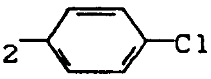 | $CH_3$ | 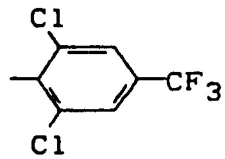 | 223-224 |
| (Ia)-196 | $-CH_2$ 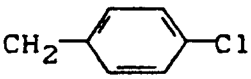 | $CH_3$ | 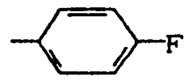 | 117-120 |
| (Ia)-197 | 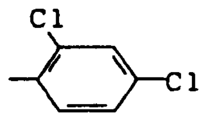 | $CH_3$ | 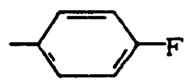 | 229 |
| (Ia)-198 | 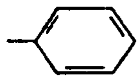 | $CH_3$ | 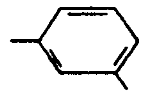 | 155-156 |
| (Ia)-200 | $CH_3$ | $CH_3$ | 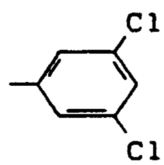 | 136 |
| (Ia)-201 | n-$C_3H_7$ | $CH_3$ | 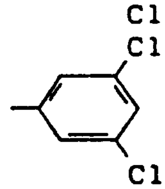 | 137 |
| (Ia)-202 | 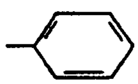 | $CH_3$ | 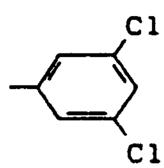 | 211-212 |
| (Ia)-203 | n-$C_3H_7$ | $CH_3$ | 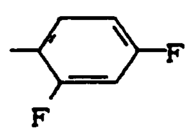 | 68 |
| (Ia)-204 | 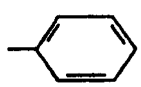 | $CH_3$ | 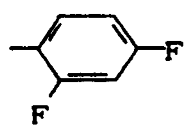 | 121 |

Tabelle 5 (Fortsetzung)

| Bsp.Nr. | $R^1$ | $R^7$ | $Ar^1$ | Schmelzpunkt($^\circ C$) |
|---|---|---|---|---|
| (Ia)-205 | 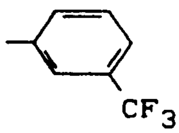 | $CH_3$ | 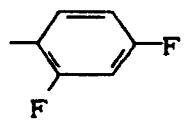 | 159-162 |
| (Ia)-206 | $CH_3$ | $CH_3$ | 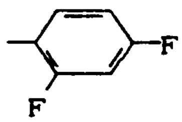 | 156 |
| (Ia)-207 | 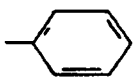 | $C_2H_5$ | 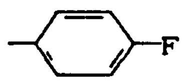 | 95 |
| (Ia)-208 | 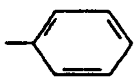 | $n-C_3H_7$ | 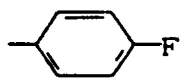 | 143 |
| (Ia)-209 | 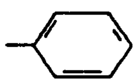 | $i-C_3H_7$ | 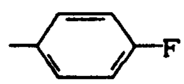 | 153 |
| (Ia)-210 | 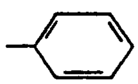 | tert.-$C_4H_9$ | 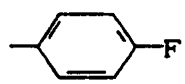 | 159 |
| (Ia)-211 | 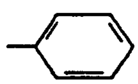 | $-CH_2-CH_2-OC_2H_5$ | 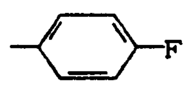 | 97 |
| (Ia)-212 | 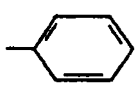 | $-CH_2-C(CH_3)_2-CH_3$ | 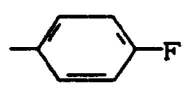 | 158-159 |
| (Ia)-213 | 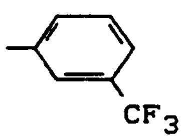 | $CH_3$ | 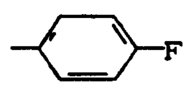 | 133 |
| (Ia)-214 | 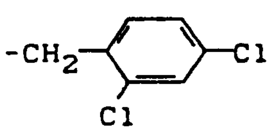 | $CH_3$ | 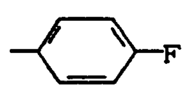 | 180 |
| (Ia)-215 | 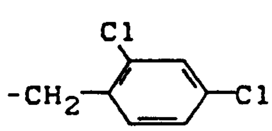 | $CH_3$ | 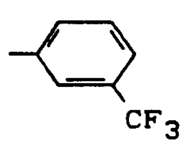 | 116 |

Tabelle 5 (Fortsetzung)

| Bsp.Nr. | $R^1$ | $R^7$ | $Ar^1$ | Schmelzpunkt (°C) |
|---|---|---|---|---|
| (Ia)-216 | 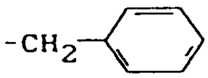 | $CH_3$ | 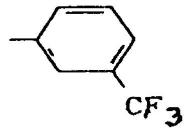 | 103-105 |
| (Ia)-218 | 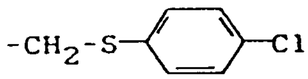 | $CH_3$ | 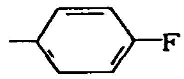 | 142-145 |
| (Ia)-219 | 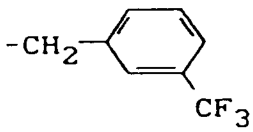 | $CH_3$ | 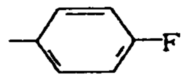 | 136 |
| (Ia)-220 | 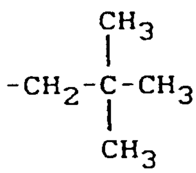 | $CH_3$ | 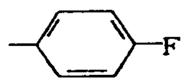 | 110-114 |
| (Ia)-222 | 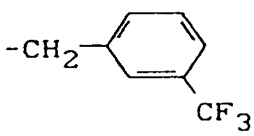 | $CH_3$ | 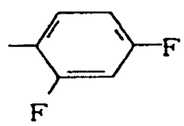 | 165 |
| (Ia)-223 | 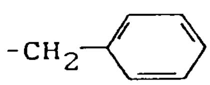 | $CH_3$ | 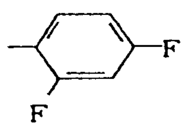 | 145 |
| (Ia)-224 | 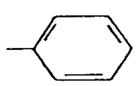 | $CH_3$ | 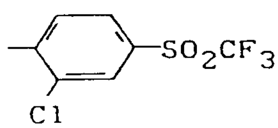 | 189 |

Tabelle 5 (Fortsetzung)

| Bsp.Nr. | $R^1$ | $R^7$ | $Ar^1$ | Schmelzpunkt (°C) |
|---|---|---|---|---|
| (Ia)-227 | $-COOCH_3$ | $CH_3$ | 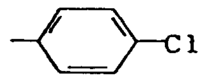 | 204 |
| (Ia)-228 | $-COOC_2H_5$ | $CH_3$ | 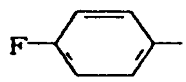 | 112-113 |
| (Ia)-229 | 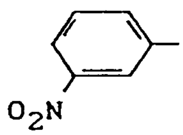 | $CH_3$ | 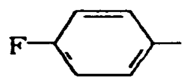 | 287-288 |
| (Ia)-230 | 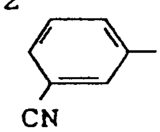 | $CH_3$ | 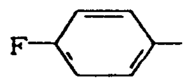 | 112-115 |
| (Ia)-231 | 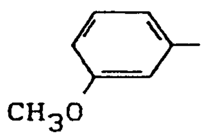 | $CH_3$ | 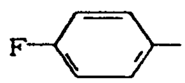 | 138-139 |
| (Ia)-232 | 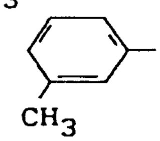 | $CH_3$ | 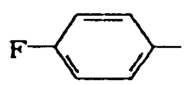 | 115 |
| (Ia)-233 | 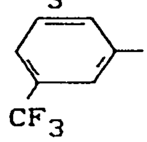 | $CH_3$ | 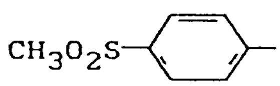 | 180 |
| (Ia)-234 | 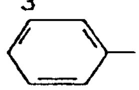 | $CH_3$ | 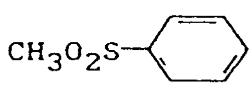 | 224 |
| (Ia)-235 | 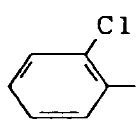 | $CH_3$ | 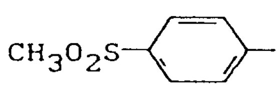 | 249 |
| (Ia)-236 | 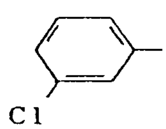 | $CH_3$ | 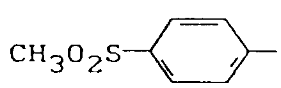 | 202 |

Tabelle 5 (Fortsetzung)

| Bsp.Nr. | $R^1$ | $R^7$ | $Ar^1$ | Schmelzpunkt(°C) |
|---|---|---|---|---|
| (Ia)-237 | 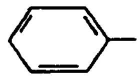 | $CH_3$ | 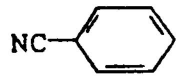 | 186 |
| (Ia)-238 | 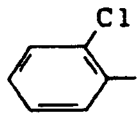 | $CH_3$ | 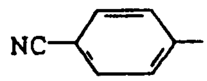 | 262 |
| (Ia)-239 | 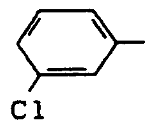 | $CH_3$ | 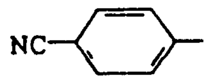 | 225 |
| (Ia)-240 | 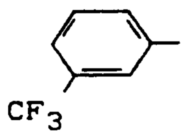 | $CH_3$ | 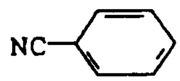 | 188 |
| (Ia)-241 | 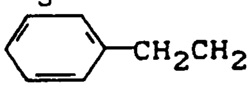 | $CH_3$ | 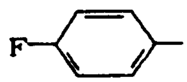 | 116 |
| (Ia)-242 | 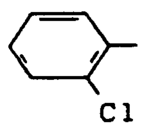 | $CH_3$ | 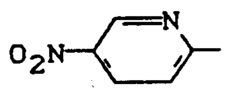 | 310 |
| (Ia)-243 | 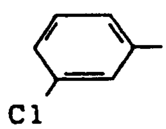 | $CH_3$ | 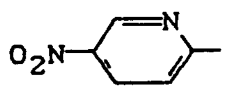 | 286 |
| (Ia)-244 | 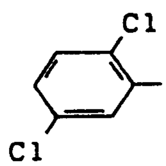 | $CH_3$ | 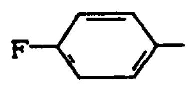 | 186 |

Tabelle 5 (Fortsetzung)

| Bsp.Nr. | $R^1$ | $R^7$ | $Ar^1$ | Schmelzpunkt(°C) |
|---|---|---|---|---|
| (Ia)-245 | 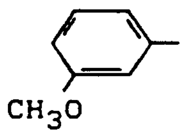 | $CH_3$ | 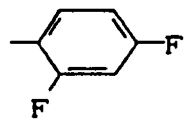 | 178-179 |
| (Ia)-246 | 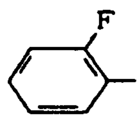 | $CH_3$ | 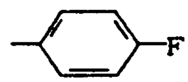 | 187-188 |
| (Ia)-247 | 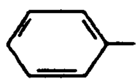 | $CH_3$ | 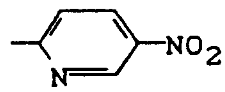 | 279-280 |
| (Ia)-248 | 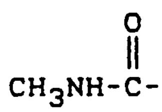 | $CH_3$ | 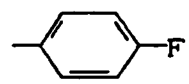 | 177-178 |
| (Ia)-249 | $CH_3$ | $CH_3$ | 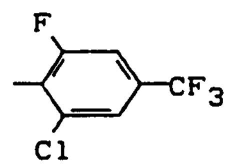 | 205 |
| (Ia)-250 | 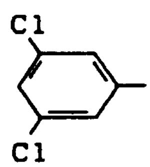 | $CH_3$ | 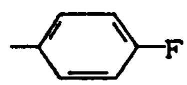 | 224-225 |
| (Ia)-251 | 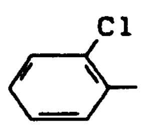 | $CH_3$ | 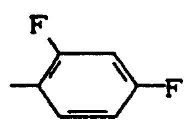 | 157 |
| (Ia)-252 | 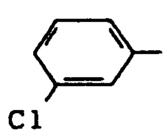 | $CH_3$ | 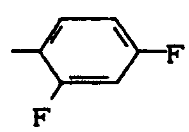 | 159 |

Tabelle 5 (Fortsetzung)

| Bsp.Nr. | $R^1$ | $R^7$ | $Ar^1$ | Schmelzpunkt (°C) |
|---|---|---|---|---|
| (Ia)-253 | 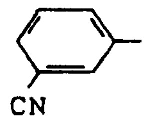 | $CH_3$ | 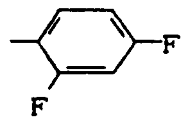 | 186 |
| (Ia)-254 | 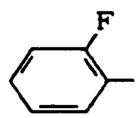 | $CH_3$ | 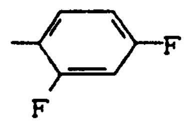 | 149 |
| (Ia)-255 | 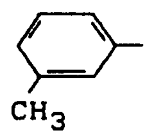 | $CH_3$ | 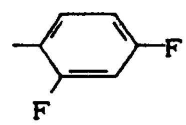 | 98 |
| (Ia)-256 | 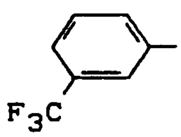 | $CH_3$ | 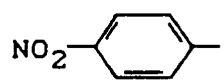 | 226 |
| (Ia)-257 | 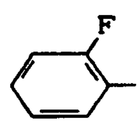 | $CH_3$ | 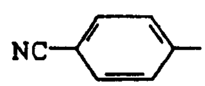 | 227 |
| (Ia)-258 | 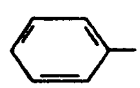 | $CH_3$ | 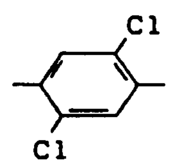 | 203 |
| (Ia)-259 | 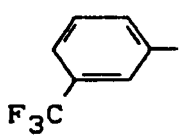 | $CH_3$ | 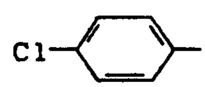 | 142 |
| (Ia)-260 | 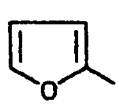 | $CH_3$ | 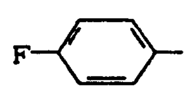 | 154 |
| (Ia)-261 | 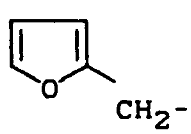 | $CH_3$ | 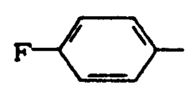 | 158-160 |

Tabelle 5 (Fortsetzung)

| Bsp.Nr. | $R^1$ | $R^7$ | $Ar^1$ | Schmelzpunkt(°C) |
|---|---|---|---|---|
| (Ia)-262 | 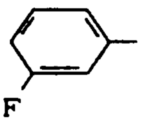 | $CH_3$ | 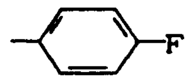 | 176 |
| (Ia)-263 | 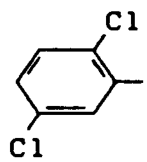 | $CH_3$ | 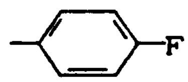 | 186 |
| (Ia)-264 | 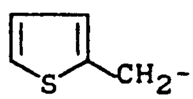 | $CH_3$ | 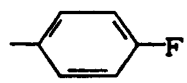 | 158-160 |
| (Ia)-265 | 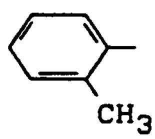 | $CH_3$ | 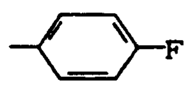 | 95-97 |
| (Ia)-266 | 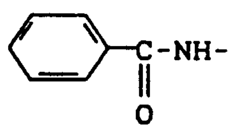 | $CH_3$ | 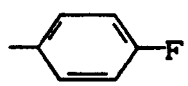 | 197-200 |
| (Ia)-267 | 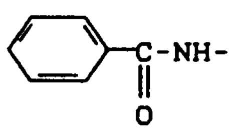 | $CH_3$ | 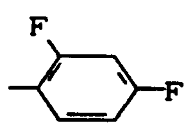 | 186 |
| (Ia)-268 | 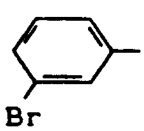 | $CH_3$ | 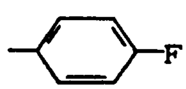 | 153 |

Beispiel 3

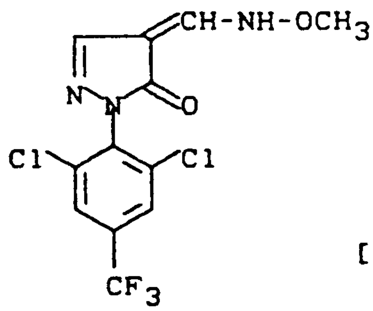

[(Ib)-1]

6 g (0,017 Mol) 1-(4-Trifluormethyl-2,6-dichlor-phenyl)-4-N,N-dimethylamino-methyliden-pyrazolin-5-on werden in 50 ml Ethanol gelöst und nach Zugabe von 1,4 g (0,017 Mol) O-Methylhydroxylamin-Hydrochlorid 2 Stunden bei Raumtemperatur gerührt. Danach wird einrotiert, mit Wasser aufgenommen und mit Methylenchlorid extrahiert. Die vereinigten organischen Phasen werden getrocknet und das Lösungsmittel abgezogen.

Man erhält 3,5 g (58,2 % der Theorie) 1-4-Trifluormethyl-2,6-dichlor-phenyl)-4-methyloximino-methyliden-pyrazolin-5-on vom Schmelzpunkt 154-160 ° C.

Herstellung der Ausgangsstoffe

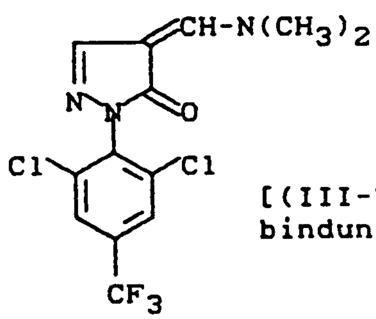

[(III-70) entspricht einer Verbindung der Formel (Id)]

5 g (0,0179 Mol) 5-Hydroxy-1-(2,6-dichlor-4-trifluormethyl-phenyl)-pyrazol werden in 50 ml Toluol aufgenommen und nach Zugabe von 2,4 g (0,02 Mol) N,N-Dimethylformamiddimethylacetal bis zum vollständigen Umsatz (chromatographische Kontrolle) bei Raumtemperatur gerührt. Der ausgefallene Feststoff wird abgesaugt, mit Petrolether verrührt, das Produkt abgesaugt und getrocknet.

Man erhält 2,55 g (40,5 % der Theorie) 1-(4-Trifluormethyl-2,6-dichlor-phenyl)-4-N,N-dimethylamino-methyliden-pyrazolin-5-on.

$^1$H-NMR ($CDCl_3$) δ [3,34 (s, 3H); 3,42 (s, 3H); 7,64 (s,1H; 7,70 (s, 2H); 7,85 (s,1H)

Vorprodukte zur Herstellung von Verbindungen der Formel (VI) nach Verfahren 1

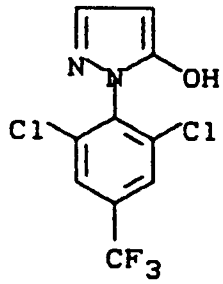

In eine Lösung aus 30 g (0,75 Mol) Natriumhydroxid in 1000 ml Wasser trägt man bei 80-85 °C portionsweise unter Rühren 105 g (0,253 Mol) fein gepulverten β-(2,6-Dichlor-4-trifluormethyl-phenyl)-hydrazinomethylenmalonsäurediethylester ein und rührt anschließend weitere 48 Stunden bei 97-98 °C. Die erkaltete Reaktionsmischung wird mit konzentrierter Salzsäure vorsichtig angesäuert auf pH 2 und der so erhaltene Niederschlag abgesaugt und auf Ton getrocknet.

Man erhält 100 g (67 % der Theorie) an 5-Hydroxy-1-(2,6-dichlor-4-trifluormethyl-phenyl)-pyrazol vom Schmelzpunkt 223-225 °C.

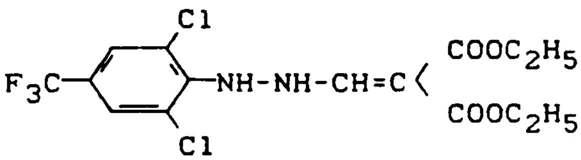

Zu einer Lösung von 122,5 g (0,5 Mol) 2,6-Dichlor-4-trifluormethyl-phenylhydrazin in 1000 ml Ethanol tropft man bei 70-75 °C innerhalb von 30 Minuten unter Rühren 115 g (0,53 Mol) Ethoxymethylenmalonsäurediethylester und rührt nach beendeter Zugabe weitere 5 Stunden bei 70 °C bis 75 °C. Zur Aufarbeitung entfernt man das Lösungsmittel im Vakuum, reibt den Rückstand mit Wasser an, saugt ab und trocknet auf Ton.

Man erhält 202 g (97 % der Theorie) an β-(2,6-Dichlor-4-trifluormethylphenyl)-hydrazinomethylenmalonsäurediethylester vom Schmelzpunkt 73 °C-83 °C.

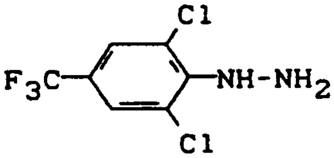

6,2 g (0,025 Mol) 3,4,5-Trichlor-trifluormethylbenzol und 6,25 g (0,125 Mol) Hydrazinhydrat werden in 12 ml Pyridin 48 Stunden bei 115-120 °C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten organischen Phasen werden über Magnesiumsulfat getrocknet, im Vakuum eingeengt und anschließend destilliert.

Man erhält 5,1 g (83 % der Theorie) an 2,6-Dichlor-4-trifluormethylphenylhydrazin vom Schmelzpunkt 56 bis 57 °C.

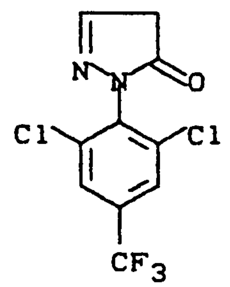

(VI-3)

(Verfahren 3)

240 g (0,73 Mol) 1-(2,6-Dichlor-4-trifluormethyl-phenylhydrazin)-acrylsäuremethylester werden in 730 ml Methanol gelöst und nach Zugabe von 151 g 30%iger Natriummethylatlösung 20 Stunden bei Raumtemperatur gerührt. Danach wird auf 3 l Wasser gegossen, mit 80 ml konzentrierter Salzsäure angesäuert, der Feststoff abgesaugt, mit Wasser gewaschen und getrocknet.

Man erhält 210 g (96,9 % der Theorie) 1-(2,6-Dichlor-4-trifluormethyl-phenyl)-pyrazolin-5-on vom Schmelzpunkt 228 ° C.

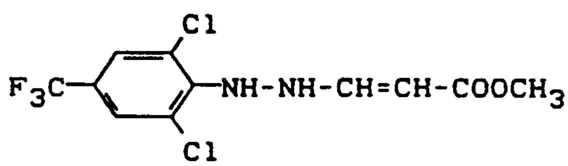

2,45 g (1,0 Mol) 2,6-Dichlor-4-trifluormethyl-phenylhydrazin und 100 g (1,2 Mol) Propiolsäuremethylester werden in 1000 ml Toluol gelöst und 24 Stunden bei 95-100 ° C gerührt. Anschließend wird das Lösungsmittel im Vakuum abgezogen, der Rückstand mit Petrolether verrührt und der ausgefallene Feststoff abgesaugt.

Man erhält 245 g (74,5 % der Theorie) 1-(2,6-Dichlor-4-trifluormethylphenylhydrazin)-acrylsäuremethylester vom Schmelzpunkt 70 ° C.

Analog Herstellungsbeispiel [(Ib)-1] und entsprechend den angegebenen Verfahren können die folgenden Endprodukte der Formel (Ib) erhalten werden:

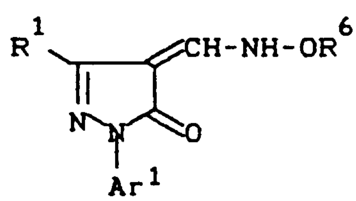

(Ib)

Tabelle 6

| Bsp.Nr. | $R^1$ | $R^6$ | $Ar^1$ | Schmelzpunkt (°C) |
|---|---|---|---|---|
| (Ib)-2 | $CH_3$ | $CH_3$ | 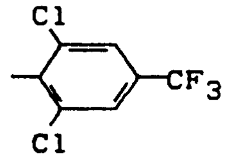 | 174 |
| (Ib)-3 | $CH_3$ | $-CH_2CH_3$ | 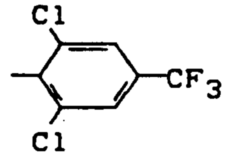 | 170 |
| (Ib)-4 | $CH_3$ | $-CH_2CH=CH_2$ | 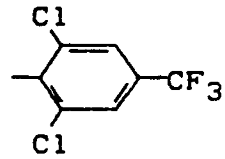 | 163 |
| (Ib)-5 | $CH_3$ | 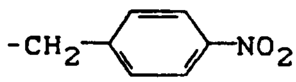 | 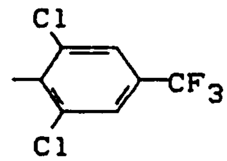 | 161-162 |
| (Ib)-6 | $CH_3$ | $-CH(CH_3)_2$ | 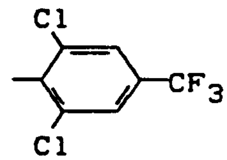 | 170 |
| (Ib)-7 | $CH_3$ | $-CH_3$ | 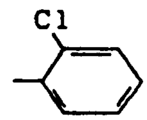 | 58 |
| (Ib)-8 | $CH_3$ | 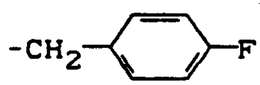 | 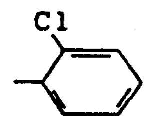 | 140* |
| (Ib)-9 | $CH_3$ | $-CH_3$ | 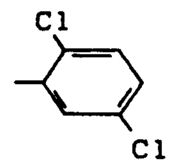 | 158-159 |
| (Ib)-10 | $CH_3$ | 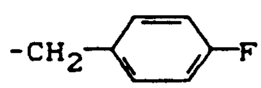 | 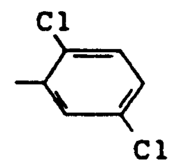 | 147* |

Tabelle 6 (Fortsetzung)

| Bsp.Nr. | $R^1$ | $R^6$ | $Ar^1$ | Schmelzpunkt (°C) |
|---|---|---|---|---|
| (Ib)-11 | $CH_3$ | $CH_3$ | 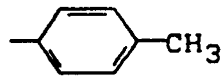 | 145 |
| (Ib)-12 | $CH_3$ | 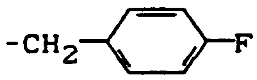 | 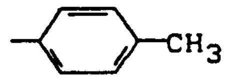 | 150-152* |
| (Ib)-13 | $n-C_3H_7$ | $CH_3$ | 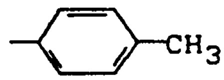 | 113 |
| (Ib)-14 | $n-C_3H_7$ | 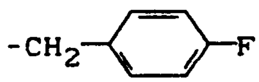 | 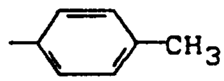 | 97* |
| (Ib)-15 | $n-C_3H_7$ | $CH_3$ | 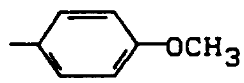 | 90 |
| (Ib)-16 | $n-C_3H_7$ | 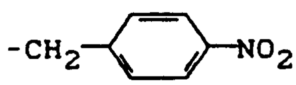 | 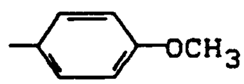 | 118-119* |
| (Ib)-17 | $-CH(CH_3)_2$ | $CH_3$ | 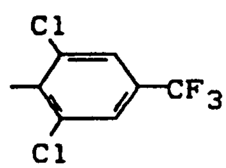 | 166 |
| (Ib)-18 | $-C(CH_3)_3$ | $CH_3$ | 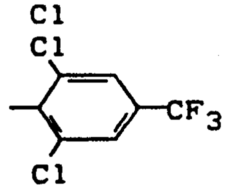 | 138 |
| (Ib)-19 | $-C(CH_3)_3$ | 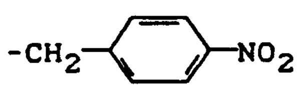 | 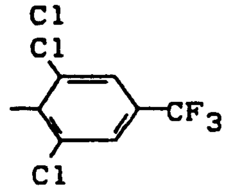 | 171-173* |

Tabelle 6 (Fortsetzung)

| Bsp.Nr. | $R^1$ | $R^6$ | $Ar^1$ | Schmelzpunkt (°C) |
|---|---|---|---|---|
| (Ib)-20 | $CH_3$ | $CH_3$ | 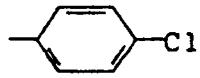 | 150 |
| (Ib)-21 | $CH_3$ | 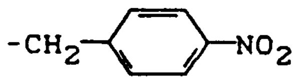 | 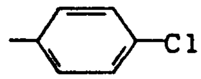 | 212* |
| (Ib)-22 | n-$C_3H_7$ | $CH_3$ | 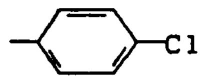 | 131 |
| (Ib)-23 | $CH_3$ | $CH_3$ | 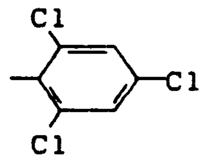 | 182 |
| (Ib)-24 | n-$C_3H_7$ | $CH_3$ | 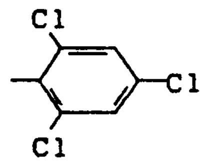 | 182 |
| (Ib)-25 | n-$C_3H_7$ | 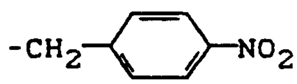 | 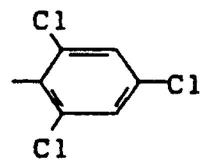 | 100-104* |
| (Ib)-26 | $CH_3$ | $CH_3$ | 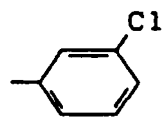 | 130 |
| (Ib)-27 | H | 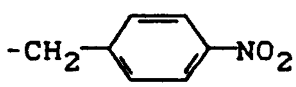 | 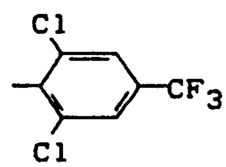 | 95-102* |
| (Ib)-28 | $-CF_3$ | $CH_3$ | 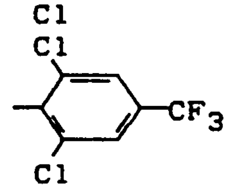 | 102 |
| (Ib)-29 | 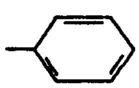 | $CH_3$ | 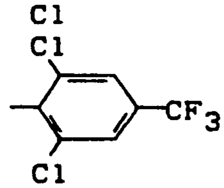 | 80-85 |

Tabelle 6 (Fortsetzung)

| Bsp.Nr. | $R^1$ | $R^6$ | $Ar^1$ | Schmelzpunkt (°C) |
|---|---|---|---|---|
| (Ib)-30 | n-$C_3H_7$ | $CH_3$ | 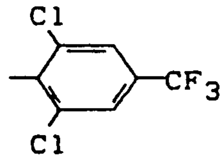 | 198 |
| (Ib)-31 | 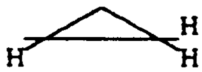 | $CH_3$ | 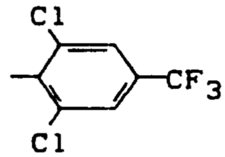 | 134 |
| (Ib)-32 | $CH_3$ | $CH_3$ | 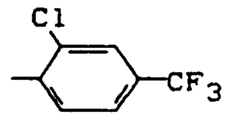 | 168 |
| (Ib)-33 | $CH_3$ | $CH_3$ | 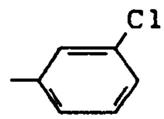 | 96 |
| (Ib)-34 | n-$C_3H_7$ | $-C_2H_5$ | 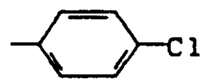 | 96 |
| (Ib)-35 | $CH_3$ | $CH_3$ | 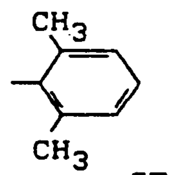 | 161 |
| (Ib)-36 | n-$C_3H_7$ | $-CH_2CH=CH_2$ | 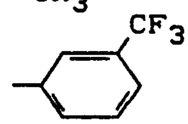 | 48 |
| (Ib)-37 | $-CH_2-CH(CH_3)_2$ | $-CH_2CH=CH_2$ | 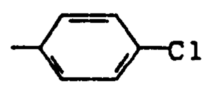 | 105 |
| (Ib)-38 | 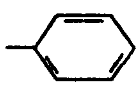 | $-CH_2CH=CH_2$ | 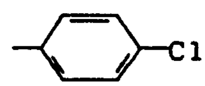 | 88 |
| (Ib)-39 | $CH_3$ | $-CH_2CH=CH_2$ | 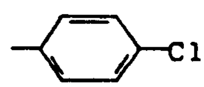 | 119 |

Tabelle 6 (Fortsetzung)

| Bsp.Nr. | $R^1$ | $R^6$ | $Ar^1$ | Schmelzpunkt (°C) |
|---|---|---|---|---|
| (Ib)-40 | $CH_3$ | $-CH_2CH=CH_2$ | 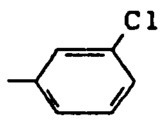 | 63 |
| (Ib)-41 | $CF_3$ | $-CH_2CH=CH_2$ | 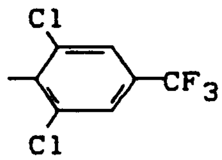 | $n_D^{23}$ =1,5192 |
| (Ib)-42 | $CH_3$ | $-CH_2CH=CH_2$ | 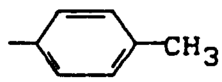 | 103 |
| (Ib)-43 | $CH_3$ | $-CH_2CH=CH_2$ | 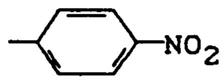 | 118 |
| (Ib)-44 | $-C(CH_3)_3$ | $-CH_2CH=CH_2$ | 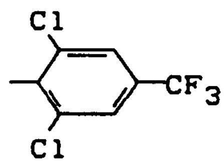 | 67 |
| (Ib)-45 | $n-C_3H_7$ | $-CH_2CH=CH_2$ | 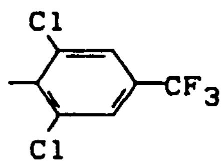 | 149 |
| (Ib)-46 | 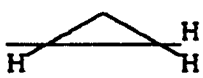 | $-CH_2CH=CH_2$ | 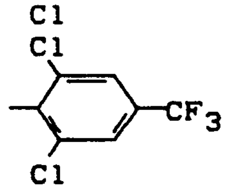 | 106 |
| (Ib)-47 | $CH_3$ | $-CH_2CH=CH_2$ | 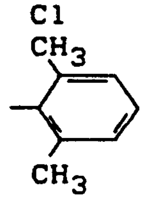 | 126-127 |
| (Ib)-48 | $-CH(CH_3)_2$ | $-CH_2CH=CH_2$ | 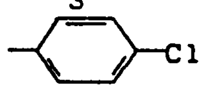 | 91 |
| (Ib)-49 | $n-C_3H_7$ | $-CH_2CH=CH_2$ | 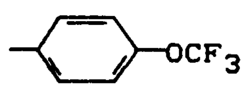 | 53 |
| (Ib)-50 | $CH_3$ | $-CH_2CH=CH_2$ | 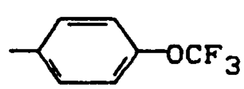 | 83 |

Tabelle 6 (Fortsetzung)

| Bsp.Nr. | $R^1$ | $R^6$ | $Ar^1$ | Schmelzpunkt (°C) |
|---|---|---|---|---|
| (Ib)-51 | $-C_2H_5$ | $-CH_2CH=CH_2$ | 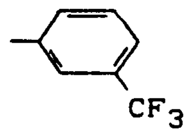 | 71-72 |
| (Ib)-52 | $-CF_3$ | $-CH_2CH=CH_2$ | 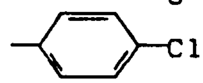 | 34 |
| (Ib)-53 | $CH_3$ | $-CH_2CH=CH_2$ | 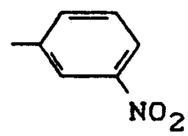 | 68-70 |
| (Ib)-54 | $n-C_3H_7$ | $-CH_2CH=CH_2$ | 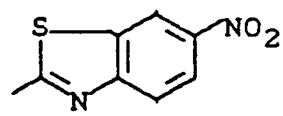 | 170(Zers.) |
| (Ib)-55 | $n-C_3H_7$ | $-CH_2CH=CH_2$ | 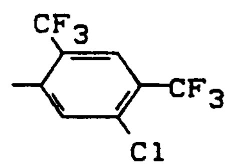 | 68 |
| (Ib)-56 | $CH_3$ | $-CH_2CH=CH_2$ | 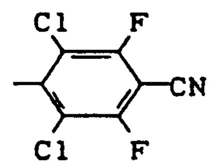 | 120 |
| (Ib)-57 | $n-C_3H_7$ | $CH_3$ | 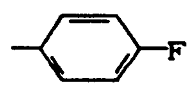 | 124 |
| (Ib)-58 | $n-C_3H_7$ | $CH_3$ | 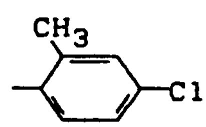 | 147-149 |
| (Ib)-59 | $CH_3$ | $CH_3$ | 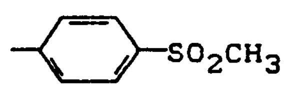 | 166(Zers.) |
| (Ib)-60 | $CH_3$ | $CH_3$ | 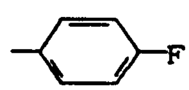 | 146 |
| (Ib)-61 | $-CH_2SCH_3$ | H | 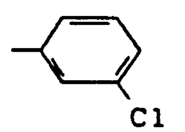 | 150 |

Tabelle 6 (Fortsetzung)

| Bsp.Nr. | $R^1$ | $R^6$ | $Ar^1$ | Schmelzpunkt (°C) |
|---|---|---|---|---|
| (Ib)-62 | $-CH_2SCH_3$ | H | 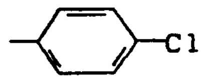 | 146 |
| (Ib)-63 | $-CH_2SCH_3$ | H | 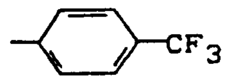 | 134 |
| (Ib)-64 | $-CH_2SCH_3$ | H | 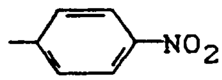 | 209 |
| (Ib)-65 | $-CH_2SCH_3$ | $CH_3$ | 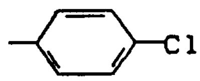 | 102 |
| (Ib)-66 | $-CH_2SCH_3$ | $CH_3$ | 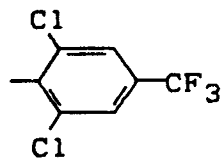 | 105 |
| (Ib)-67 | $-CH_2SCH_3$ | $CH_3$ | 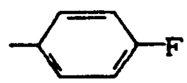 | 64 |
| (Ib)-68 | $-CH_2SCH_3$ | $CH_3$ | 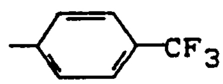 | 91 |
| (Ib)-69 | $-CH_2SCH_3$ | $-CH_2CH=CH_2$ | 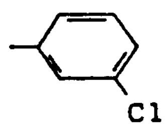 | Oel |
| (Ib)-70 | $-CH_2SCH_3$ | $-CH_2CH=CH_2$ | 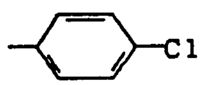 | 68 |
| (Ib)-71 | $-CH_2SCH_3$ | $-CH_2CH=CH_2$ | 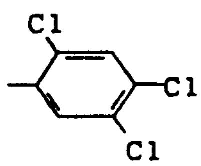 | 95 |
| (Ib)-72 | $-CH_2SCH_3$ | $-CH_2CH=CH_2$ | 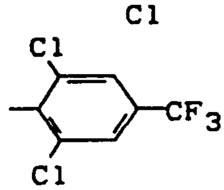 | Oel |

<u>Tabelle 6</u> (Fortsetzung)

| Bsp.Nr. | $R^1$ | $R^6$ | $Ar^1$ | Schmelzpunkt (°C) |
|---|---|---|---|---|
| (Ib)-73 | 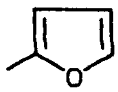 | $CH_3$ | 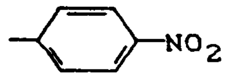 | 178-180 |
| (Ib)-74 | $CH_3$ | $CH_3$ | 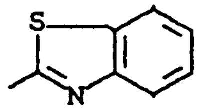 | 181-182 |
| (Ib)-75 | 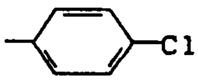 | $CH_3$ | 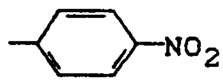 | 210-211 |
| (Ib)-76 | 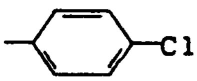 | $CH_3$ | 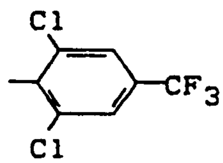 | 167-168 |
| (Ib)-77 | 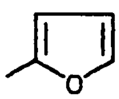 | $CH_3$ | 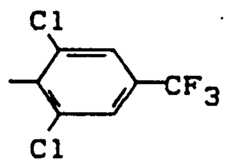 | 126 |
| (Ib)-78 | $n-C_3H_7$ | $CH_3$ | 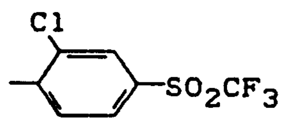 | 105-107 |
| (Ib)-79 | $n-C_3H_7$ | $CH_3$ | 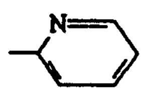 | 65 |
| (Ib)-80 | $-CH_2SC_2H_5$ | $CH_3$ | 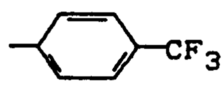 | 67 |
| (Ib)-81 | $-CH_2SC_2H_5$ | $CH_3$ | 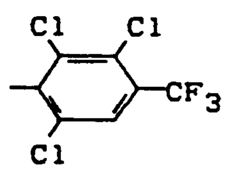 | 60-65 |
| (Ib)-82 | $CH_3$ | $CH_3$ | 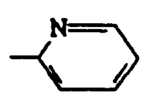 | 164 |

Tabelle 6 (Fortsetzung)

| Bsp.Nr. | $R^1$ | $R^6$ | $Ar^1$ | Schmelzpunkt (°C) |
|---|---|---|---|---|
| (Ib)-83 | 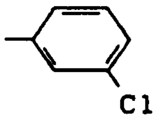 | $CH_3$ | 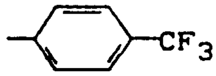 | 60 |
| (Ib)-84 | 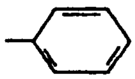 | $CH_3$ | 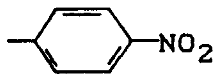 | 174-176 |
| (Ib)-85 | 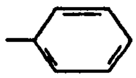 | $CH_3$ | 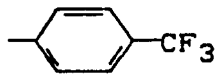 | 104 |
| (Ib)-86 | $CH_3$ | $CH_3$ | 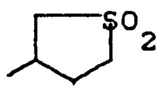 | 157 |
| (Ib)-87 | $n-C_3H_7$ | $CH_3$ | 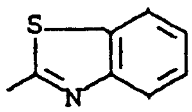 | 148 |
| (Ib)-88 | 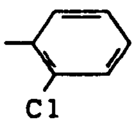 | $CH_3$ | 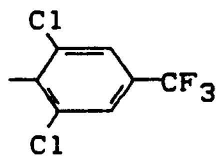 | 55-60 |
| (Ib)-89 | $-CH_2SC_2H_5$ | $CH_3$ | 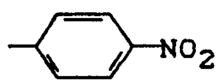 | 124-128 |
| (Ib)-90 | 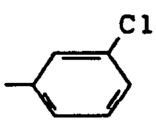 | $CH_3$ | 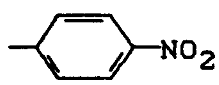 | 185-186 |
| (Ib)-91 | 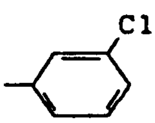 | $CH_3$ | 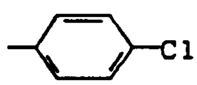 | 134-136 |
| (Ib)-92 | $n-C_3H_7$ | $C_2H_5$ | 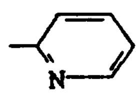 | 61 |
| (Ib)-93 | $n-C_3H_7$ | $-CH_2-CH=CH_2$ | 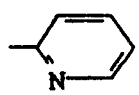 | 56 |

Die mit * gekennzeichneten Verbindungen liegen als Dimethylamin-Salz vor.

Beispiel 4

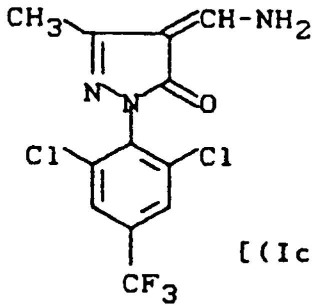

[(Ic)-1]

(Variante [(Ic)-α]

10 g (0,0273 Mol) 1-(4-Trifluormethyl-2,6-dichlorphenyl)-3-methyl-4-N,N-dimethylamino-methyliden-pyrazolin-5-on werden in 40 ml Toluol suspendiert und bei 80 °C Ammoniak bis zum vollständigen Umsatz (chromatographische Kontrolle) eingeleitet. Dann wird auf 0 °C abgekühlt, der Feststoff abgesaugt, mit Petrolether/Ether 2:1 nachgewaschen und getrocknet.

Man erhält 6,2 g (67,2 % der Theorie) 1-(4-Trifluormethyl-2,6-dichlorphenyl)-3-methyl-4-aminomethyliden-pyrazolin-5-on vom Schmelzpunkt 184-185 °C.

Beispiel 5

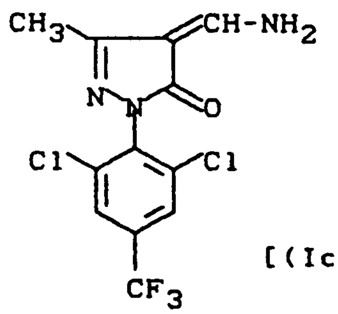

[(Ic)-1]

[Variante [(Ic)-β]

Zu einer Lösung von 5,67 g (0,07 Mol) s-Triazin in 100 ml absolutem Ethanol wird innerhalb von 8 Stunden die Lösung von 27,5 g (0,105 Mol) 1-(4-Trifluormethyl-2,6-dichlorphenyl)-3-methyl-pyrazolin-5-on in 500 ml absolutem Ethanol zugetropft. Anschließend wird über Nacht bei Raumtemperatur nachgerührt und bis zur Trockne eingeengt. Das zurückbleibende Öl wird durch Säulenchromatographie an Kieselgel (Laufmittel: Chloroform und 20 % Methanol) gereinigt.

Man erhält 17,9 g (50,4 % der Theorie) 1-(4-Trifluormethyl-2,6-dichlorphenyl)-3-methyl-4-aminomethyliden-pyrazolin-5-on vom Schmelzpunkt 184-185 °C.

Analog Herstellungsbeispiel [(Ic)-1] und entsprechend den angegebenen Verfahren können die folgenden Endprodukte der Formel (I-c) erhalten werden:

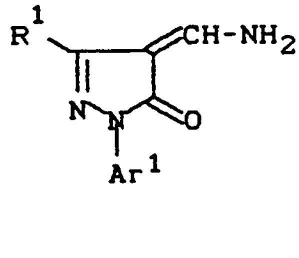

(Ic)

Tabelle 7

| Bsp.Nr. | $R^1$ | $Ar^1$ | Schmelz-punkt (°C) |
|---|---|---|---|
| (Ic)-2 | H | 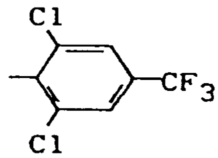 | 243 |
| (Ic)-5 | $n-C_3H_7$ | 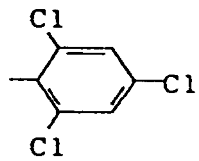 | 167 |
| (Ic)-6 | $CH_3$ | 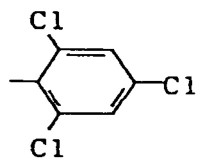 | 204 |
| (Ic)-7 | $n-C_3H_7$ | 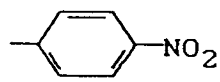 | 291-294 |
| (Ic)-8 | $CH_3$ | 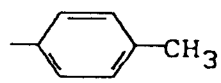 | 179 |
| (Ic)-9 | $CH_3$ | 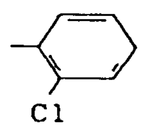 | 163-164 |
| (Ic)-10 | $CH_3$ | 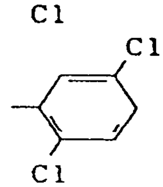 | 204 |
| (Ic)-11 | $n-C_3H_7$ | 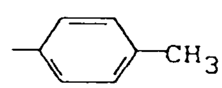 | 117 |
| (Ic)-12 | $-C_2H_5$ | 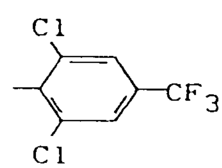 | 65 |

Tabelle 7 (Fortsetzung)

| Bsp.Nr. | $R^1$ | $Ar^1$ | Schmelzpunkt (°C) |
|---|---|---|---|
| (Ic)-13 | $-CH(CH_3)_2$ | 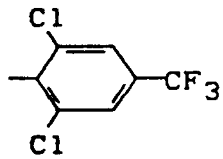 | 73 |
| (Ic)-14 | $-C(CH_3)_3$ | 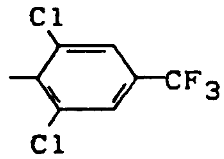 | 253 |
| (Ic)-15 | 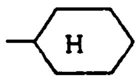 | 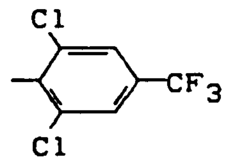 | 86-88 |
| (Ic)-16 | $-CF_3$ | 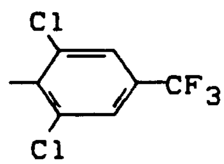 | 187 |
| (Ic)-17 | $CH_3$ | 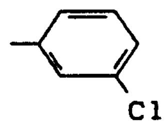 | 286 |
| (Ic)-18 | $CH_3$ | 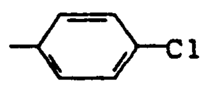 | 170 |
| (Ic)-19 | $n-C_3H_7$ | 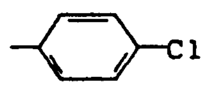 | 122 |
| (Ic)-20 | 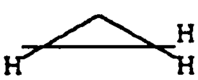 | 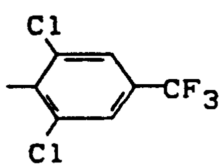 | 171 |
| (Ic)-21 | 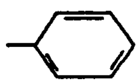 | 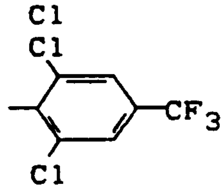 | 221 |

<u>Tabelle 7</u> (Fortsetzung)

| Bsp.Nr. | $R^1$ | $Ar^1$ | Schmelzpunkt (°C) |
|---|---|---|---|
| (Ic)-16 | n-$C_3H_7$ | 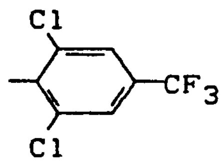 | 115-119 |
| (Ic)-23 | $CH_3$ | 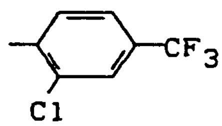 | 102-109 |
| (Ic)-24 | $CH_3$ | 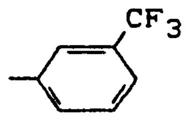 | 138 |
| (Ic)-25 | $CH_3$ | 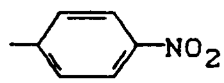 | >300 |
| (Ic)-26 | $CH_3$ | 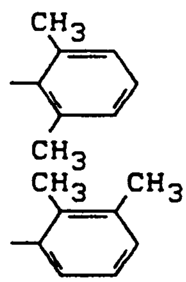 | 187 |
| (Ic)-27 | n-$C_3H_7$ | | 180 |
| (Ic)-28 | $CH_3$ | 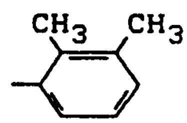 | 172-176 |
| (Ic)-29 | 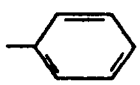 | 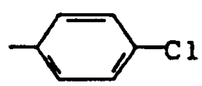 | 167-169 |
| (Ic)-30 | $CH_3$ | 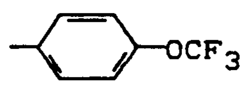 | 136 |
| (Ic)-31 | $C_2H_5$ | 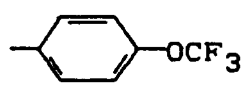 | 126-127 |
| (Ic)-32 | n-$C_3H_7$ | 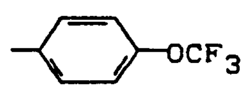 | 77-78 |

Tabelle 7 (Fortsetzung)

| Bsp.Nr. | $R^1$ | $Ar^1$ | Schmelzpunkt (°C) |
|---|---|---|---|
| (Ic)-33 | $CH_3$ | 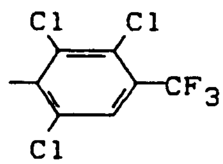 | 65 |
| (Ic)-34 | $n-C_3H_7$ | 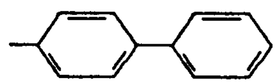 | 172 |
| (Ic)-35 | $C_2H_5$ | 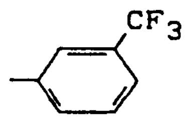 | 93 |
| (Ic)-36 | $-CH_2OCH_3$ | 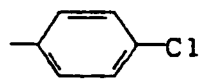 | 134-135 |
| (Ic)-37 | $-CF_3$ | 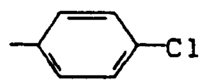 | 191-192 |
| (Ic)-38 | 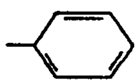 | 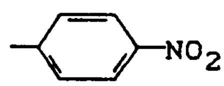 | >320 |
| (Ic)-39 | $n-C_3H_7$ | 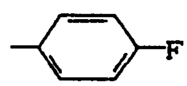 | 56-58 |
| (Ic)-40 | $-CH_2SCH_3$ | 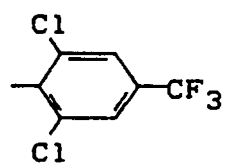 | 222 |
| (Ic)-41 | $-CH_2SCH_3$ | 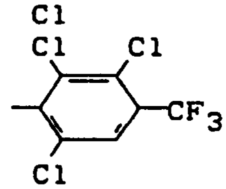 | 147 |
| (Ic)-42 | $-CH_2SCH_3$ | 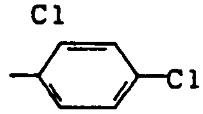 | 116 |
| (Ic)-43 | $-CH_2SCH_3$ | 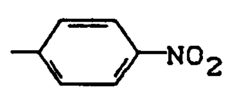 | >260 |

Tabelle 7 (Fortsetzung)

| Bsp.Nr. | $R^1$ | $Ar^1$ | Schmelzpunkt (°C) |
|---|---|---|---|
| (Ic)-44 | $-CH_2SCH_3$ | 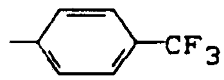 | 131 |
| (Ic)-45 | $-CH_2SCH_3$ | 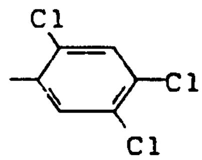 | 80 |
| (Ic)-46 | H | 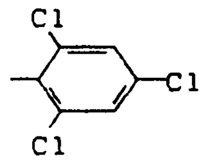 | 255-256 |
| (Ic)-47 | $CH_3$ | 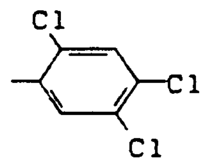 | 50-52 |
| (Ic)-48 | 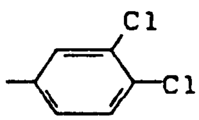 | 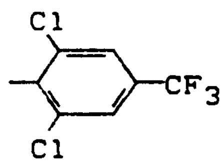 | 201-203 |
| (Ic)-49 | 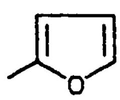 | 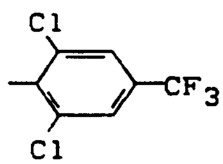 | 181-184 |
| (Ic)-50 | 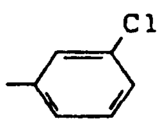 | 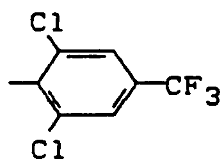 | 144 |
| (Ic)-51 | 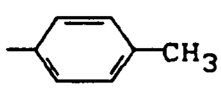 | 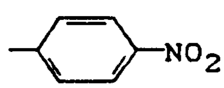 | $\rangle$320 |
| (Ic)-52 | 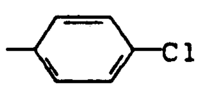 | 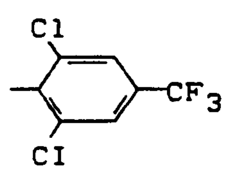 | 241 |

Tabelle 7 (Fortsetzung)

| Bsp.Nr. | $R^1$ | $Ar^1$ | Schmelzpunkt (°C) |
|---|---|---|---|
| (Ic)-53 | 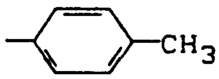 | 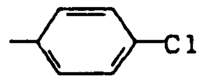 | 204 |
| (Ic)-54 | 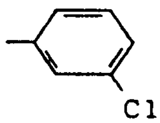 | 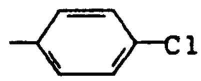 | 189 |
| (Ic)-55 | 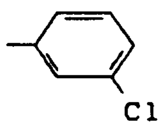 | 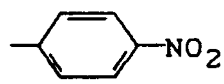 | >320 |
| (Ic)-56 | $-CH_2SC_2H_5$ | 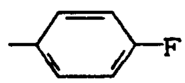 | 92 |
| (Ic)-57 | $n-C_3H_7$ | 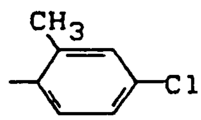 | 110-114 |
| (Ic)-58 | $-CH_2SC_2H_5$ | 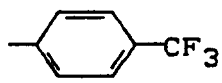 | 75-79 |
| (Ic)-59 | $CH_3$ | 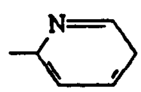 | 242 |
| (Ic)-60 | $n-C_3H_7$ | 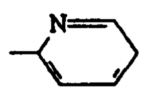 | 202 |
| (Ic)-61 | $-CH_2SC_2H_5$ | 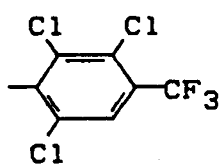 | 70 |
| (Ic)-62 | $n-C_3H_7$ | 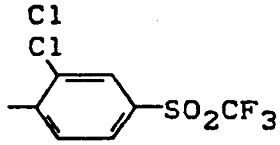 | 40-45 |
| (Ic)-63 | 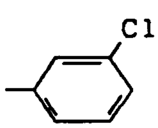 | 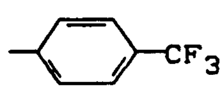 | 157 |
| (Ic)-64 | 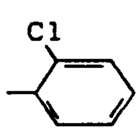 | 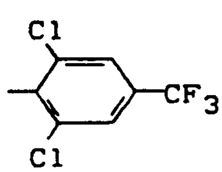 | 189-190 |

Tabelle 7 (Fortsetzung)

| Bsp.Nr. | $R^1$ | $Ar^1$ | Schmelzpunkt ($^\circ C$) |
|---|---|---|---|
| (Ic)-65 | 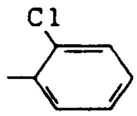 | 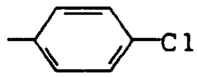 | 214-216 |
| (Ic)-66 | 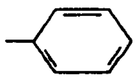 | 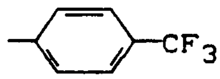 | 160-166 |
| (Ic)-67 | 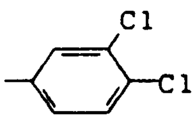 | 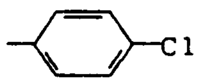 | 227 |
| (Ic)-68 | $-CH_2CO_2C_2H_5$ | 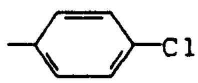 | 136 |
| (Ic)-69 | 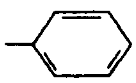 | 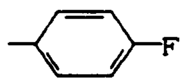 | 156 |
| (Ic)-70 | 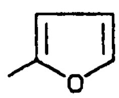 | 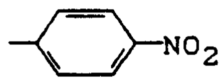 | 309 |
| (Ic)-71 | $-CH_2SC_2H_5$ | 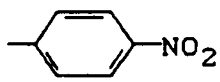 | 247-249 |
| (Ic)-72 | $n-C_3H_7$ | 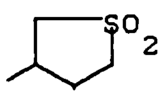 | 118-120 |
| (Ic)-73 | $n-C_3H_7$ | 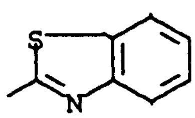 | 247-249 |
| (Ic)-74 | $CH_3$ | 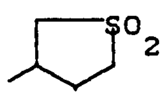 | 200-202 |
| (Ic)-75 | $CH_3$ | 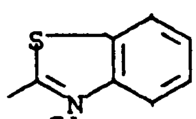 | 270-274 |
| (Ic)-76 | 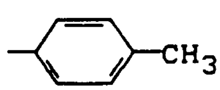 | 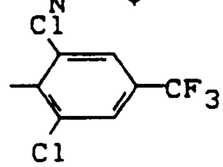 | 58 |

Tabelle 7 (Fortsetzung)

| Bsp.Nr. | $R^1$ | $Ar^1$ | Schmelzpunkt (°C) |
|---|---|---|---|
| (Ic)-77 | $CH_3$ | 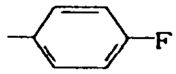 | 138-140 |
| (Ic)-78 | $n-C_3H_7$ | 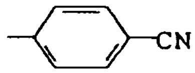 | 211 |
| (Ic)-79 | $-CH_2$ 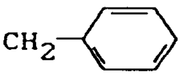 | 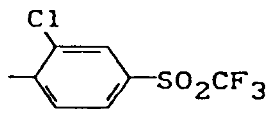 | 47 |
| (Ic)-80 | $-CH_2$ 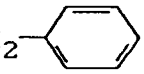 | 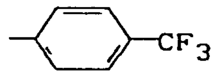 | 118 |
| (Ic)-81 | $-CH_2$ 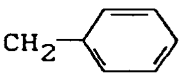 | 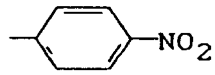 | 283 |
| (Ic)-82 | $-CH_2$ 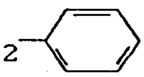 | 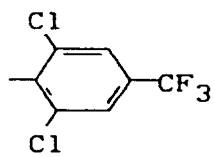 | 156 |
| (Ic)-83 | $n-C_3H_7$ | 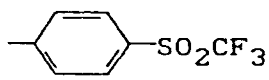 | 196 |
| (Ic)-84 | H | 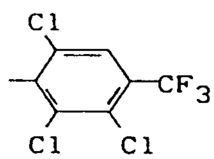 | 253-262 |
| (Ic)-85 | H | 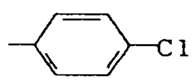 | 179 |
| (Ic)-87 | 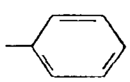 | 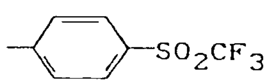 | 217 |
| (Ic)-88 | H | 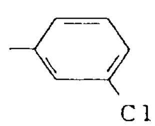 | 160 |

Tabelle 7 (Fortsetzung)

| Bsp.Nr. | $R^1$ | $Ar^1$ | Schmelzpunkt (°C) |
|---|---|---|---|
| (Ic)-89 | $C_3H_7$ | 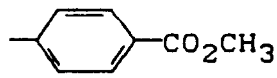 | 127 |
| (Ic)-90 | $-CH_2$ 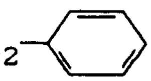 | 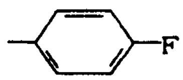 | 85 |
| (Ic)-91 | 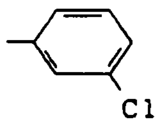 | 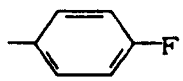 | 137-139 |
| (Ic)-92 | 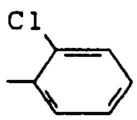 | 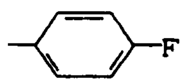 | 179 |
| (Ic)-93 | $-CH_2$ 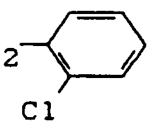 | 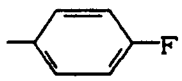 | 65-66 |
| (Ic)-94 | $-CH_2$ 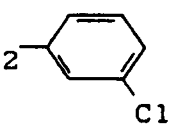 | 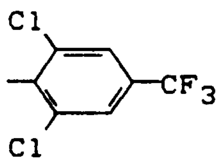 | 156 |
| (Ic)-95 | $-CH_2$ 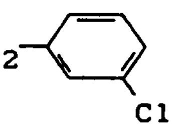 | 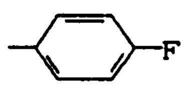 | 111 |
| (Ic)-96 | $-CH_2$ 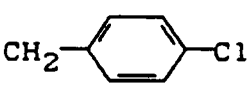 | 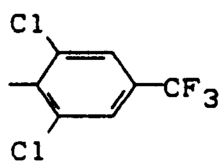 | 190-192 |
| (Ic)-97 | $-CH_2$ 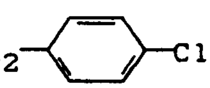 | 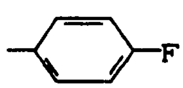 | 131-133 |
| (Ic)-98 | 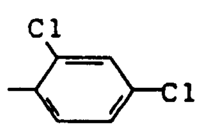 | 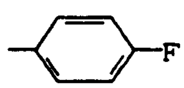 | 202 |
| (Ic)-99 | 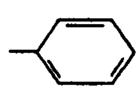 | 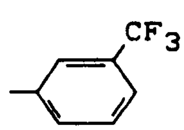 | 154 |

Tabelle 7 (Fortsetzung)

| Bsp.Nr. | $R^1$ | $Ar^1$ | Schmelzpunkt (°C) |
|---|---|---|---|
| (Ic)-100 | $CH_3$ | 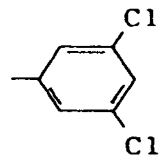 | 215 |
| (Ic)-101 | n-$C_3H_7$ | 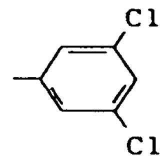 | 134 |
| (Ic)-102 | 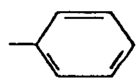 | 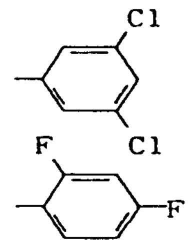 | 223 |
| (Ic)-103 | n-$C_3H_7$ | | 92 |
| (Ic)-104 | 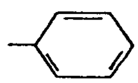 | 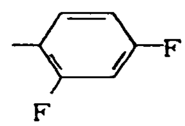 | 132 |
| (Ic)-105 | $CH_3$ | 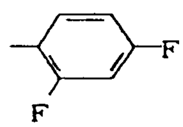 | 152 |
| (Ic)-106 | 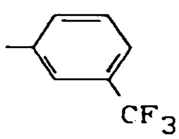 | 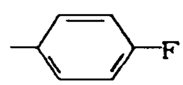 | 114 |
| (Ic)-108 | 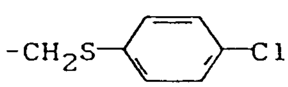 | 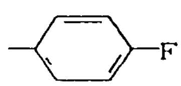 | 117 |
| (Ic)-109 | 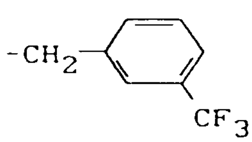 | 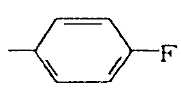 | 110 |

Tabelle 7 (Fortsetzung)

| Bsp.Nr. | $R^1$ | $Ar^1$ | Schmelzpunkt (°C) |
|---|---|---|---|
| (Ic)-110 | 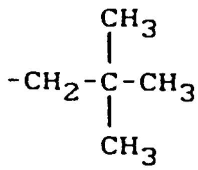 | 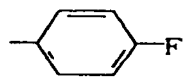 | 104 |
| (Ic)-111 | $-S-CH_2-$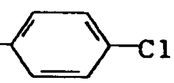 | 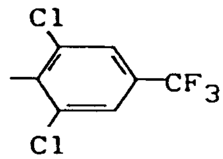 | 176 |
| (Ic)-113 | $-CH_2-$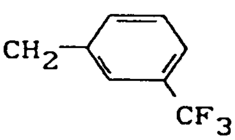 | 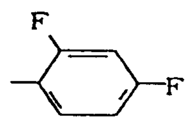 | 79-82 |
| (Ic)-114 | $-CH_2-$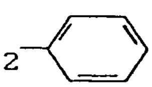 | 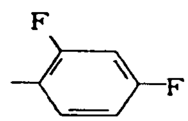 | 71 |
| (Ic)-115 | 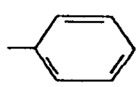 | 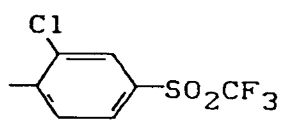 | 138 |
| (Ic)-116 | $-OC_2H_5$ | 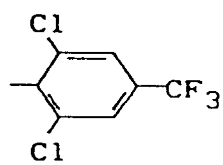 | 142 |
| (Ic)-117 | $-NH-CO-CH_3$ | 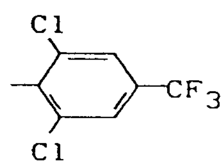 | 237 |
| (Ic)-118 | $-NH-CO-$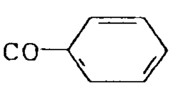 | 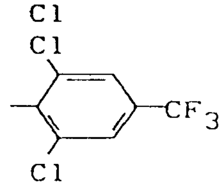 | 145-150 |

Tabelle 7 (Fortsetzung)

| Bsp.Nr. | $R^1$ | $Ar^1$ | Schmelzpunkt (°C) |
|---|---|---|---|
| (Ic)-120 | $CH_3$ | 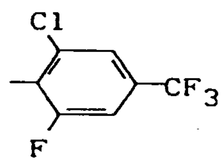 | 205 |

Analog den Herstellungsbeispielen (III-1), (III-69) und (III-70) werden die folgenden Ausgangsprodukte der Formel (III), die gleichzeitig zum Teil erfindungsgemäße Endprodukte der Formel (Id) sind, erhalten:

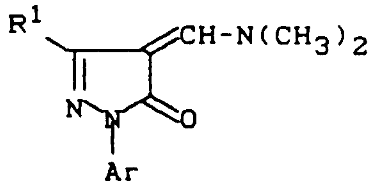

(III)

Tabelle 8

| Bsp.Nr. | $R^1$ | Ar | Brechungsindex ($n_D^{20}$); Schmelzpunkt (°C) |
|---|---|---|---|
| III-2 | $CH_3$ | 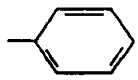 | 127 |
| III-3 | n-$C_3H_7$ | 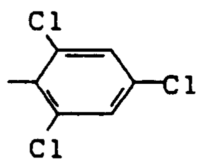 | 164 |
| III-4 | n-$C_3H_7$ | 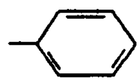 | 107 |
| III-5 | $CH_3$ | 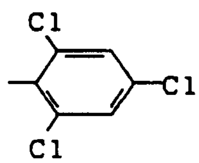 | 196 |
| III-6 | $C_2H_5$ | 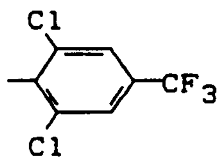 | 111-113 |
| III-7 | $CH_3$ | 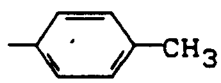 | 206 |
| III-8 | $CH_3$ | 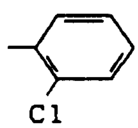 | 170 |

Tabelle 8 (Fortsetzung)

| Bsp.Nr. | $R^1$ | Ar | Brechungs-index ($n_D^{20}$); Schmelz-punkt(°C) |
|---|---|---|---|
| III-9 | $CH_3$ | 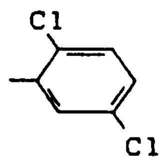 | 128 |
| III-10 | $-CH(CH_3)_2$ | 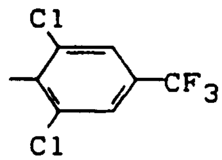 | 114 |
| III-11 | 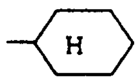 | 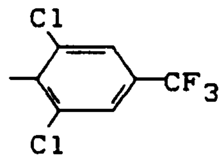 | 151-156 |
| III-12 | $-CF_3$ | 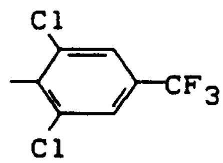 | 139 |
| III-13 | $CH_3$ | 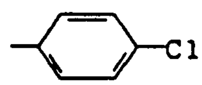 | 200 |
| III-14 | $CH_3$ | 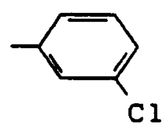 | 133 |
| III-15 | $n-C_3H_7$ | 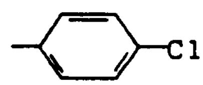 | 121 |
| III-16 | $n-C_3H_7$ | 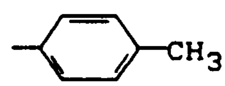 | 134 |
| III-17 | $n-C_3H_7$ | 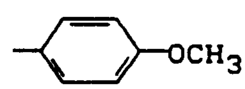 | 117 |
| III-18 | $-C(CH_3)_3$ | 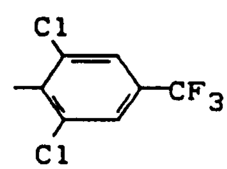 | 173 |

Tabelle 8 (Fortsetzung)

| Bsp.Nr. | $R^1$ | Ar | Brechungsindex ($n_D^{20}$); Schmelzpunkt (°C) |
|---|---|---|---|
| III-19 | 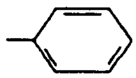 | 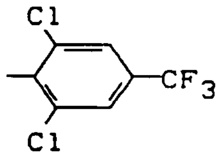 | 132-134 |
| III-20 | n-$C_3H_7$ | 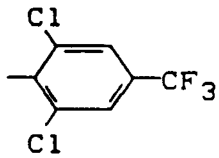 | 113-115 |
| III-21 | 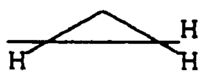 | 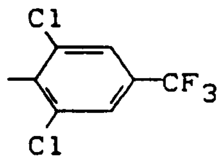 | 183-184 |
| III-22 | $CH_3$ | 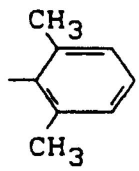 | 166 |
| III-23 | $C_2H_5$ | 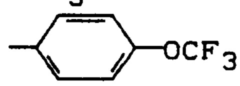 | 82-85 |
| III-24 | n-$C_3H_7$ | 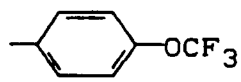 | 116-117 |
| III-25 | $C_2H_5$ | 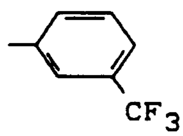 | 125 |
| III-26 | $CH_2OCH_3$ | 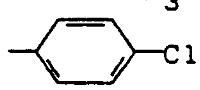 | 163 |
| III-27 | $CH_3$ | 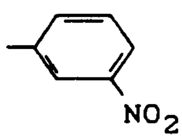 | 181-182 |
| III-28 | $CF_3$ | 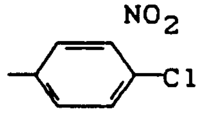 | 182 |

Tabelle 8 (Fortsetzung)

| Bsp.Nr. | $R^1$ | Ar | Brechungsindex ($n_D^{20}$); Schmelzpunkt (°C) |
|---|---|---|---|
| III-29 | $CH_3$ | 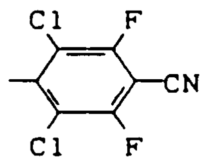 | 132 |
| III-30 | $-CH_2OCH_3$ | 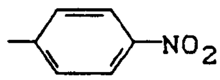 | 86 |
| III-31 | $n-C_3H_7$ | 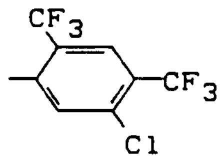 | 131-132 |
| III-32 | 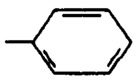 | 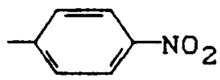 | 210-212 |
| III-33 | $n-C_3H_7$ | 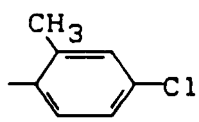 | 1,5983 |
| III-34 | $n-C_3H_7$ | 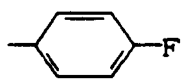 | 134-137 |
| III-35 | 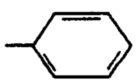 | 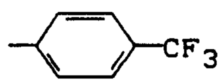 | 156-158 |
| III-36 | $n-C_3H_7$ | 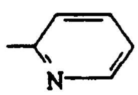 | 182 |
| III-37 | $CH_3$ | 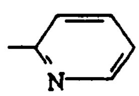 | 180 |
| III-38 | $n-C_3H_7$ | 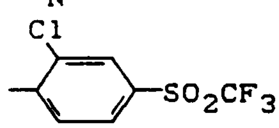 | 138-142 |
| III-39 | $CH_3$ | 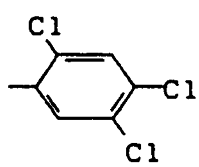 | 127 |

Tabelle 8 (Fortsetzung)

| Bsp.Nr. | $R^1$ | Ar | Brechungsindex ($n_D^{20}$); Schmelzpunkt (°C) |
|---|---|---|---|
| III-40 | n-$C_3H_7$ | 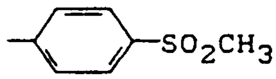 | 157 |
| III-41 | $CH_3$ | 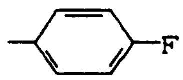 | 175 |
| III-42 | 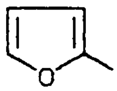 | 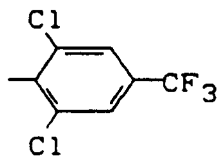 | 204-205 |
| III-43 | $-CH_2SC_2H_5$ | 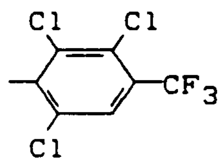 | 90-92 |
| III-44 | 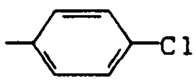 | 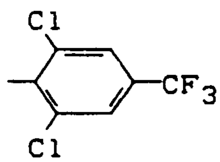 | 195-196 |
| III-45 | $-CH_2SC_2H_5$ | 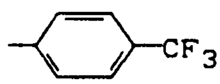 | 114-115 |
| III-46 | 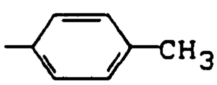 | 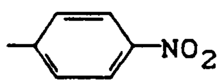 | 253-254 |
| III-47 | 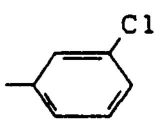 | 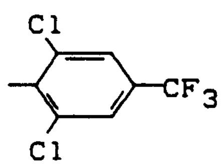 | 146 |
| III-48 | 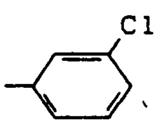 | 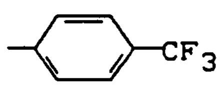 | 135-138 |
| III-49 | 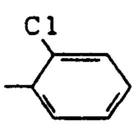 | 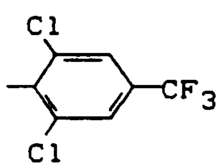 | 171-173 |

Tabelle 8 (Fortsetzung)

| Bsp.Nr. | $R^1$ | Ar | Brechungsindex ($n_D^{20}$); Schmelzpunkt ($^0$C) |
|---|---|---|---|
| III-50 | 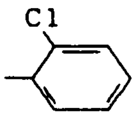 | 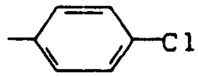 | 145 |
| III-51 | 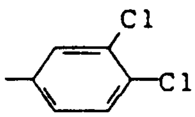 | 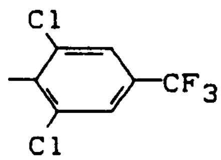 | 207 |
| III-52 | 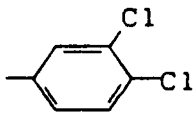 | 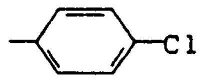 | 174 |
| III-53 | $-CH_2SCH_3$ | 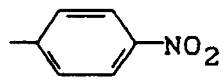 | 187 |
| III-54 | $-CH_2SCH_3$ | 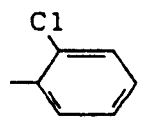 | 112 |
| III-55 | $-CH_2SCH_3$ | 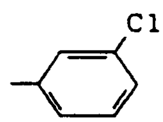 | Oel |
| III-56 | $-CH_2SCH_3$ | 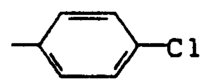 | 157 |
| III-57 | $-CH_2SCH_3$ | 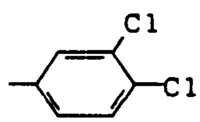 | 188 |
| III-58 | $-CH_2SCH_3$ | 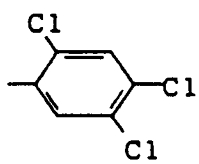 | 140 |
| III-59 | $-CH_2SCH_3$ | 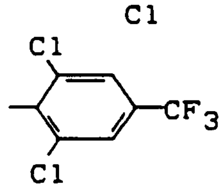 | 175 |

Tabelle 8 (Fortsetzung)

| Bsp.Nr. | $R^1$ | Ar | Brechungsindex ($n_D^{20}$); Schmelzpunkt (°C) |
|---|---|---|---|
| III-60 | $-CH_2SCH_3$ | 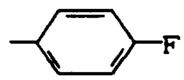 | 151 |
| III-61 | $-CH_2SCH_3$ | 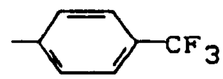 | 170 |
| III-62 | $-CH_2SCH_3$ | 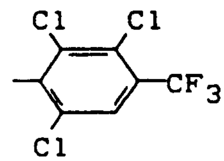 | 158 |
| III-63 | H | 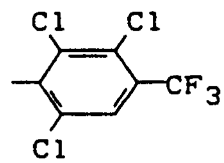 | 226 |
| III-64 | H | 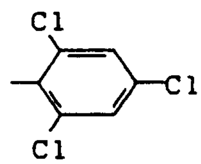 | >250 |
| III-65 | H | 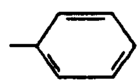 | 210 |
| III-66 | H | 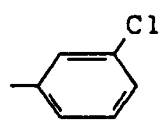 | 192-198 |
| III-67 | H | 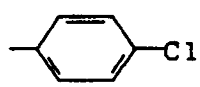 | 242-243 |
| III-68 | 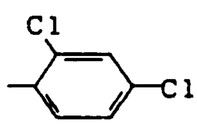 | 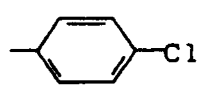 | 163 |
| III-69 | $CH_3$ | 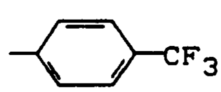 | 230-233 |
| III-70 | H | 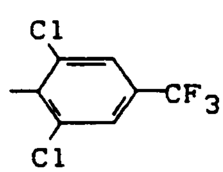 | 220 |

Tabelle 8 (Fortsetzung)

| Bsp.Nr. | $R^1$ | Ar | Brechungsindex ($n_D^{20}$); Schmelzpunkt ($^0$C) |
|---|---|---|---|
| III-71 | 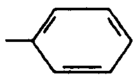 | 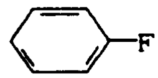 | 136 |
| III-72 | 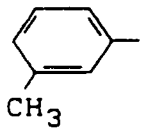 | 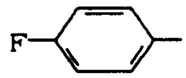 | 143-144 |
| III-73 | $CH_3$ | 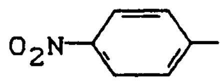 | ›300 |
| III-74 | $CH_3$ | 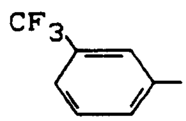 | 130 |
| III-75 | $CH_3$ | 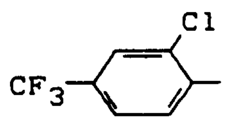 | 3.27/3.79 |
| III-76 | 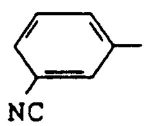 | 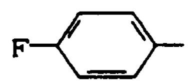 | 219 |
| III-77 | n-$C_3H_7$ | 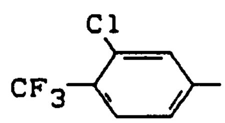 | 3.32/3.83 |
| III-78 | n-$C_3H_7$ | 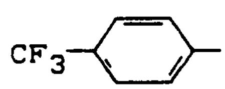 | 111-112 |

Tabelle 8 (Fortsetzung)

| Bsp.Nr. | $R^1$ | Ar | Schmelzpunkt (°C) bzw. $^1H$-NMR in $CDCl_3$ ($\delta$ $N(CH_3)_2$ |
|---|---|---|---|
| III-79 | 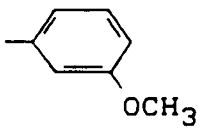 | 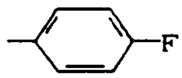 | 135-136 |
| III-80 | $n-C_3H_7$ | 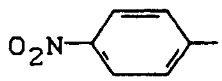 | 139 |
| III-81 | $n-C_3H_7$ | 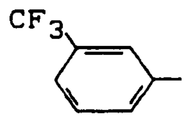 | 108-109 |
| III-82 | $(CH_3)_2CH-CH_2-$ | 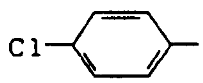 | 114 |
| III-83 | $(CH_3)_2CH-$ | 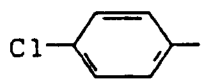 | 107 |
| III-84 | $n-C_4H_9$ | 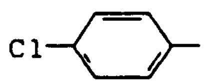 | 194 |
| III-85 | $(CH_3)_3C$ | 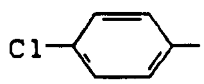 | 3.30/3.65 |
| III-86 | $n-C_3H_7$ | 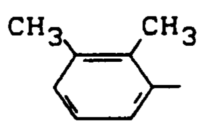 | 103-105 |
| III-87 | $CH_3$ | 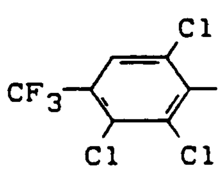 | 3.35/3.80 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-88 | $CH_3$ | 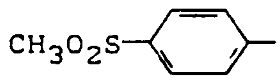 | 3.22/3.79 |
| III-89 | $C_2H_5SCH_2$- | 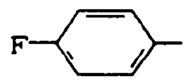 | 138 |
| III-90 | $C_2H_5SCH_2$- | 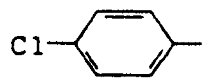 | 119 |
| III-91 | 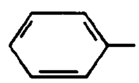 | 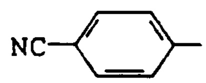 | 173 |
| III-92 | 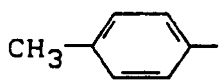 | 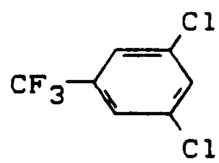 | 142 |
| III-93 | 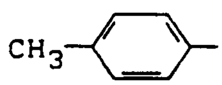 | 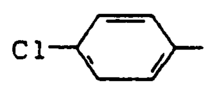 | 175 |
| III-94 | 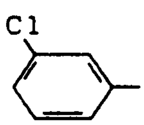 | 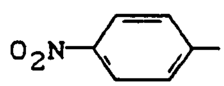 | 201 |
| III-95 | 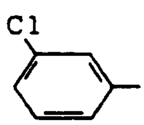 | 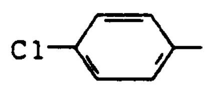 | 171 |
| III-96 | 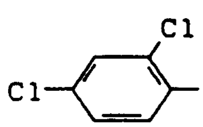 | 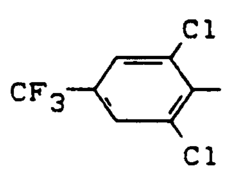 | 180-182 |
| III-97 | 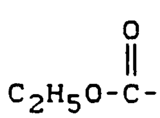 | 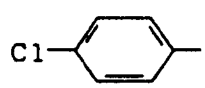 | 124-126 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-98 | 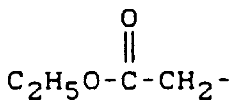 | 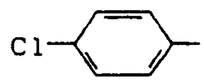 | 150 |
| III-99 | $n-C_3H_7$ | 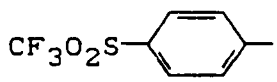 | 156 |
| III-100 | 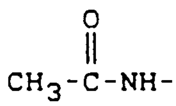 | 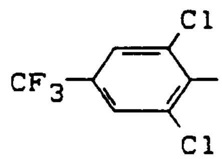 | 264 |
| III-101 | 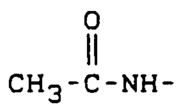 | 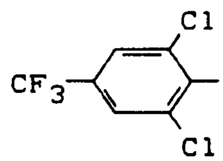 | 55-60 |
| III-102 | 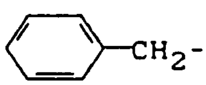 | 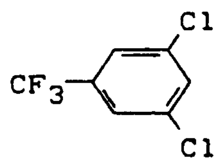 | 175-178 |
| III-103 | 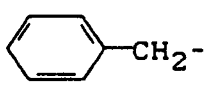 | 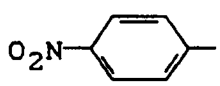 | 213 |
| III-104 | 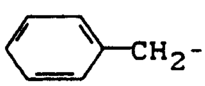 | 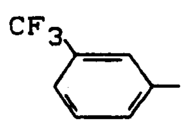 | 130 |
| III-105 | 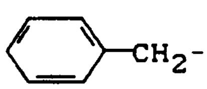 | 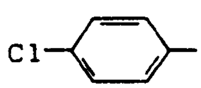 | 115 |
| III-106 | 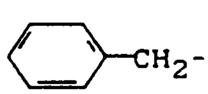 | 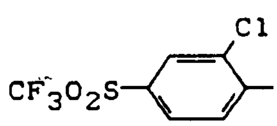 | 124 |
| III-107 | 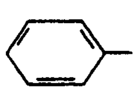 | 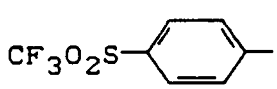 | 234 |

Tabelle 8 (Fortsetzung)

| Bsp.Nr. | $R^1$ | Ar | Schmelzpunkt (°C) bzw. $^1H$-NMR in $CDCl_3$ ($\delta$ $N(CH_3)_2$ |
|---|---|---|---|
| III-108 | $n-C_3H_7$ | 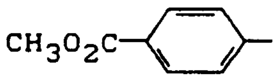 | 57 |
| III-109 | $C_2H_5O-$ | 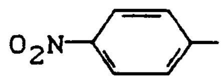 | 207 |
| III-110 | $C_2H_5O-$ | 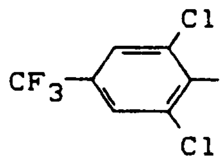 | 98 |
| III-111 | 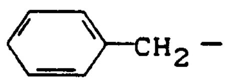 | 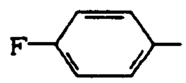 | 135 |
| III-112 | 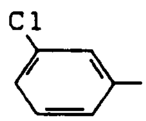 | 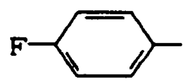 | 161 |
| III-113 | 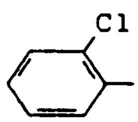 | 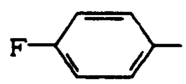 | 88-92 |
| III-114 | $C_2H_5O-$ | 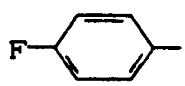 | 127 |
| III-115 | 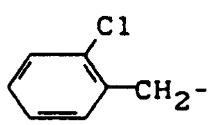 | 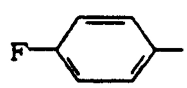 | 135 |
| III-116 | 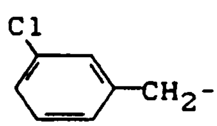 | 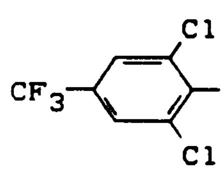 | 132 |
| III-117 | 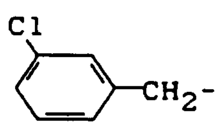 | 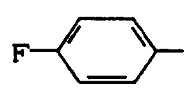 | 150 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-118 | 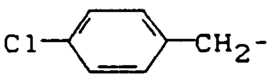 | 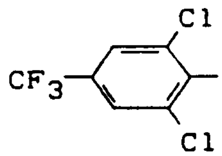 | 140 |
| III-119 | 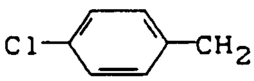 | 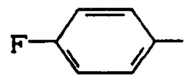 | 119 |
| III-120 | 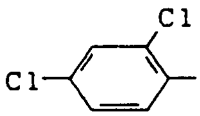 | 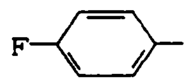 | 164 |
| III-121 | 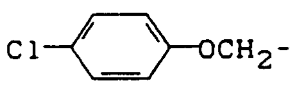 | 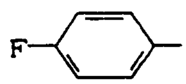 | 152-154 |
| III-122 | 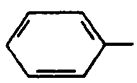 | 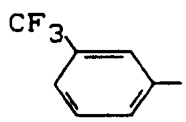 | 137-138 |
| III-123 | $CH_3$ | 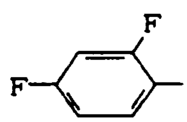 | 138 |
| III-124 | n-$C_3H_7$ | 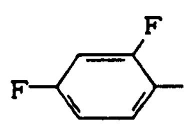 | 125 |
| III-125 | 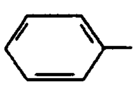 | 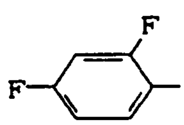 | 122 |
| III-126 | 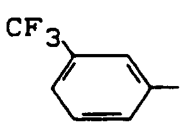 | 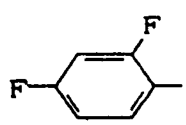 | 72 |
| III-127 | 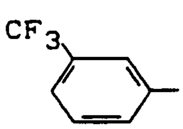 | 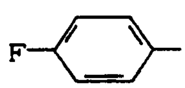 | 158 |

Tabelle 8 (Fortsetzung)

| Bsp.Nr. | $R^1$ | Ar | Schmelzpunkt (°C) bzw. $^1H$-NMR in $CDCl_3$ ($\delta$ $N(CH_3)_2$) |
|---|---|---|---|
| III-128 | 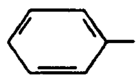 | 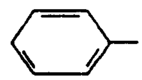 | 148-151 |
| III-129 | 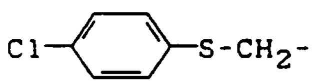 | 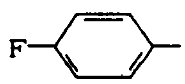 | 143-145 |
| III-130 | 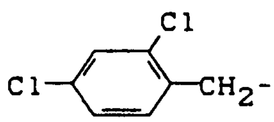 | 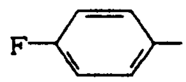 | 164-165 |
| III-131 | 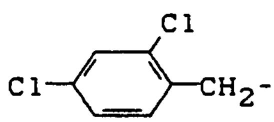 | 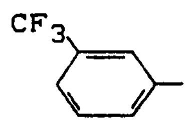 | 99-101 |
| III-132 | 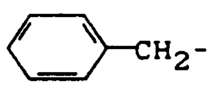 | 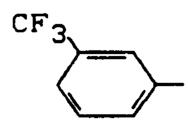 | 116-119 |
| III-133 | 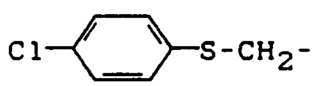 | 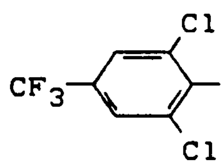 | 182-184 |
| III-134 | 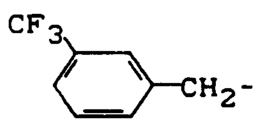 | 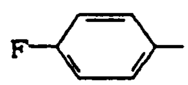 | 113-114 |
| III-135 | $(CH_3)_3C-CH_2-$ | 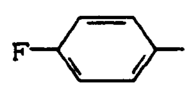 | 128 |
| III-136 | 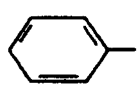 | 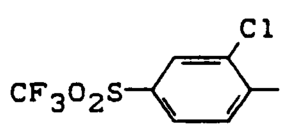 | 171 |
| III-137 | 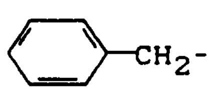 | 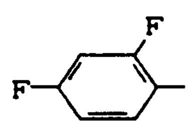 | 123-124 |
| III-138 | 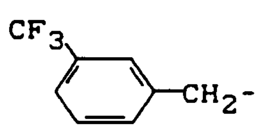 | 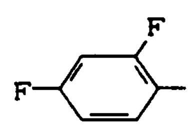 | 120-122 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-139 | 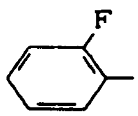 | 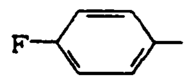 | 161-162 |
| III-140 | 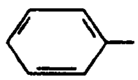 | 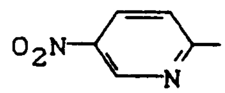 | 196-198 |
| III-141 | 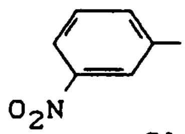 | 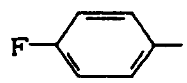 | 251-253 |
| III-142 | 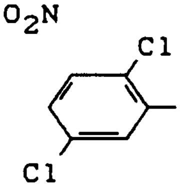 | 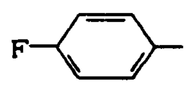 | 130 |
| III-143 | 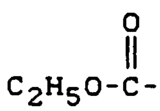 | 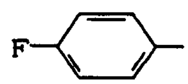 | 148-149 |
| III-144 | 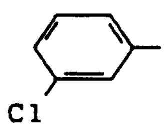 | 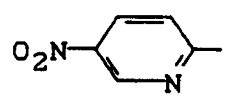 | 272 |
| III-145 | 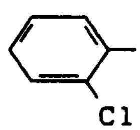 | 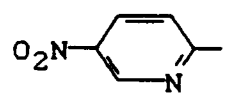 | 240 |
| III-146 | 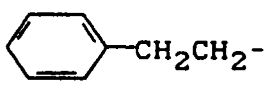 | 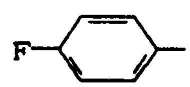 | 114 |
| III-147 | 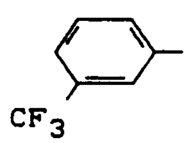 | 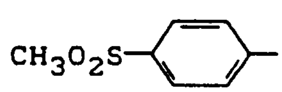 | 138 |
| III-148 | 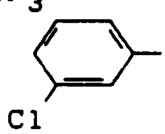 | 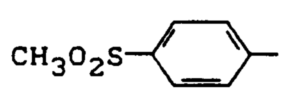 | 174 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-149 | 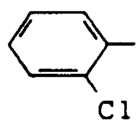 | 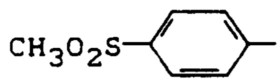 | 213 |
| III-150 | 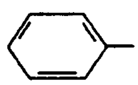 | 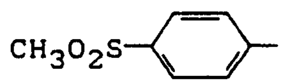 | 185-186 |
| III-151 | 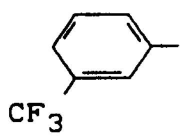 | 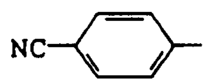 | 178 |
| III-152 | 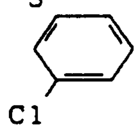 | 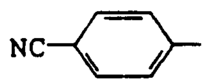 | 195-198 |
| III-153 | 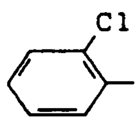 | 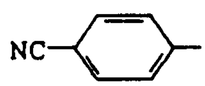 | 201 |
| III-154 | $CH_3$ | 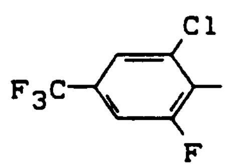 | 201 |
| III-155 | 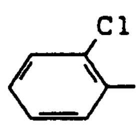 | 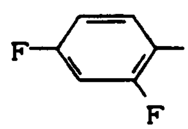 | 128 |
| III-156 | 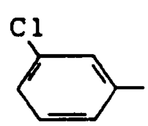 | 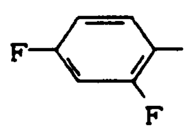 | 137 |

Tabelle 8 (Fortsetzung)

| Bsp.Nr. | $R^1$ | Ar | Schmelzpunkt (°C) bzw. $^1H$-NMR in $CDCl_3$ (δ $N(CH_3)_2$ |
|---|---|---|---|
| III-157 | 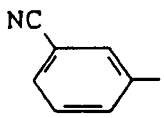 | 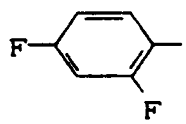 | 149 |
| III-158 | 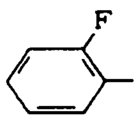 | 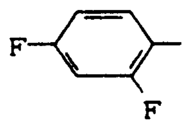 | 120 |
| III-159 | 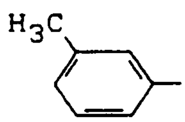 | 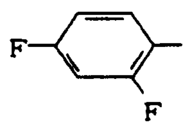 | 108 |
| III-160 | 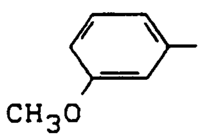 | 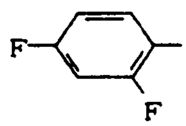 | 110 |
| III-161 | 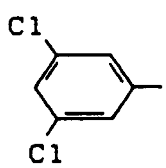 | 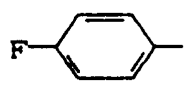 | 236 |
| III-162 | 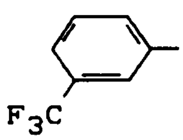 | 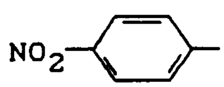 | 214 |

Tabelle 8 (Fortsetzung)

| Bsp.Nr. | $R^1$ | Ar | Schmelzpunkt ($^{o}C$) bzw. $^1H$-NMR in $CDCl_3$ ($\delta$ $N(CH_3)_2$ |
|---|---|---|---|
| III-163 | 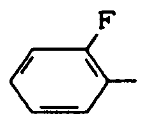 | 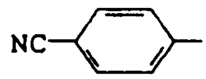 | 215 |
| III-164 | 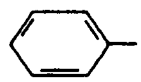 | 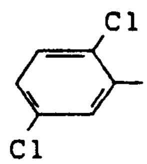 | 130 |
| III-165 | 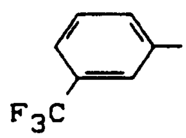 | 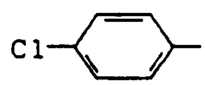 | 156 |
| III-166 | 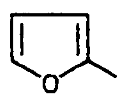 | 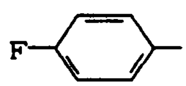 | 133 |
| III-167 | 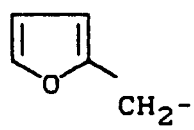 | 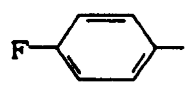 | 116-117 |

Die in der folgenden Tabelle 9 aufgeführten substituierten Pyrazolin-5-on Derivate der Formel (If)

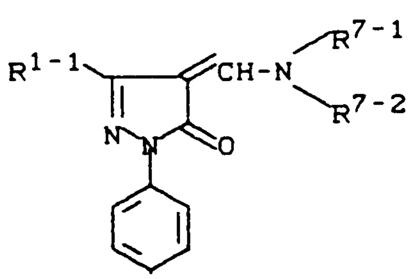

(If)

können entsprechend den angegebenen Verfahren, vgl. insbesondere die Reaktionsdurchführung gemäß den Herstellungsbeispielen [(Ia)-1] und [(Ia)-2], erhalten werden:

Tabelle 9

| Bsp.-Nr. | $R^{1-1}$ | $R^{7-1}$ | $R^{7-2}$ | Schmelz-punkt (°C) |
|---|---|---|---|---|
| If-1 | 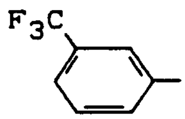 | $CH_3$ | $CH_3$ | 190 |
| If-2 | 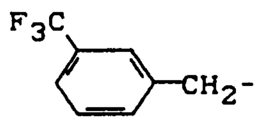 | $CH_3$ | $CH_3$ | 125 |
| If-3 | 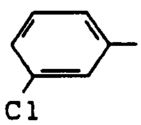 | $CH_3$ | $CH_3$ | 170 |
| If-4 | 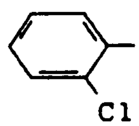 | $CH_3$ | $CH_3$ | 112-113 |

Tabelle 9 (Fortsetzung)

| Bsp.-Nr. | $R^{1-1}$ | $R^{7-1}$ | $R^{7-2}$ | Schmelzpunkt (°C) |
|---|---|---|---|---|
| If-5 | 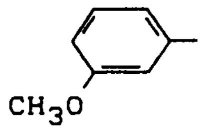 | $CH_3$ | $CH_3$ | 125 |
| If-6 | 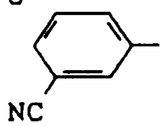 | $CH_3$ | $CH_3$ | 162-163 |
| If-7 | 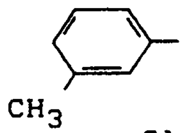 | $CH_3$ | $CH_3$ | 154 |
| If-8 | 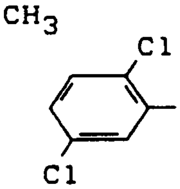 | $CH_3$ | $CH_3$ | |
| If-9 | 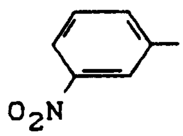 | $CH_3$ | $CH_3$ | |
| If-10 | 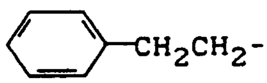 | $CH_3$ | $CH_3$ | 109 |
| If-11 | 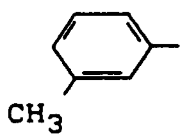 | H | $CH_3$ | 126 |
| If-12 | 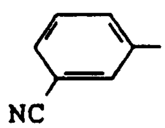 | H | $CH_3$ | 179 |
| If-13 | 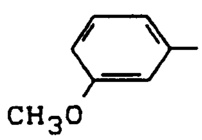 | H | $CH_3$ | 138-139 |

Tabelle 9 (Fortsetzung)

| Bsp.-Nr. | $R^{1-1}$ | $R^{7-1}$ | $R^{7-2}$ | Schmelzpunkt (°C) |
|---|---|---|---|---|
| If-14 | 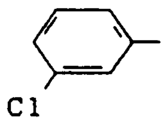 | H | $CH_3$ | 119-120 |
| If-15 | 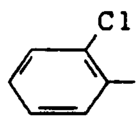 | H | $CH_3$ | 190-191 |
| If-16 | 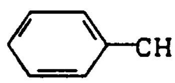 $CH_2$-$CH_2$- | H | $CH_3$ | 118 |
| If-17 | 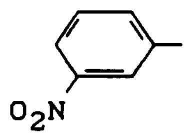 | H | $CH_3$ | 238 |
| If-18 | 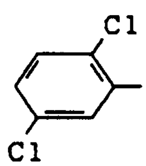 | H | $CH_3$ | 138 |
| If-19 | n-$C_3H_7$ | $CH_3$ | $CH_3$ | 136 |
| If-20 | 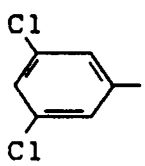 | H | $CH_3$ | 213-214 |
| If-21 | 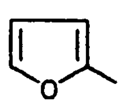 | $CH_3$ | $CH_3$ | 125 |

Tabelle 9 (Fortsetzung)

| Bsp.-Nr. | $R^{1-1}$ | $R^{7-1}$ | $R^{7-2}$ | Schmelzpunkt (°C) |
|---|---|---|---|---|
| If-22 | 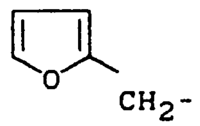 | $CH_3$ | $CH_3$ | 147 |
| If-23 | 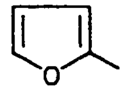 | H | $CH_3$ | 128 |
| If-24 | 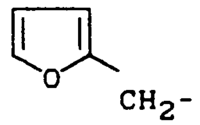 | H | $CH_3$ | 100-101 |
| If-25 | 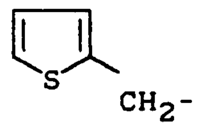 | H | $CH_3$ | 100-101 |
| If-26 | 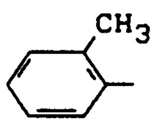 | H | $CH_3$ | 121 |
| If-27 | 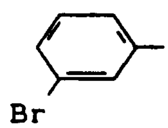 | H | $CH_3$ | 136 |

Analog dem Herstellungsbeispiel (IV-1) und entsprechend dem angegebenen verfahren können die Zwischenprodukte der Formel (IV), (IVa), (IVb) bzw. (IVc) erhalten werden, welche in der allgemeinen Formel (IVa) zusammengefaßt sind:

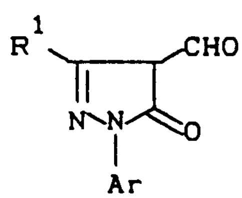

(IVa)

Tabelle 10

| Bsp.Nr. | $R^1$ | Ar | Schmelzpunkt (°C) |
|---|---|---|---|
| (IV-2) | $-CH_2SCH_3$ | 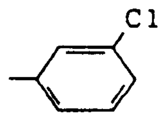 | Oel |
| (IV-3) | $-CH_2SCH_3$ | 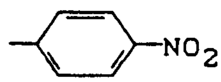 | 125 |
| (IV-4) | $-CH_2SCH_3$ | 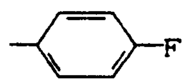 | 90 |
| (IV-5) | $-CH_2SCH_3$ | 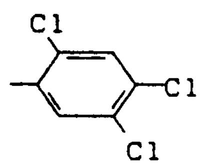 | 103 |
| (IV-6) | $-CH_2SCH_3$ | 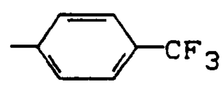 | 127 |
| (IV-7) | H | 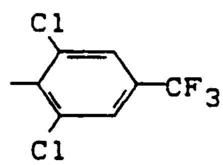 | 110 |
| (IV)-8 | H | 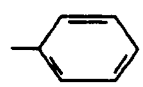 | 182 |
| (IV)-9 | H | 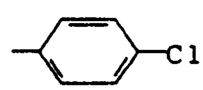 | 204 |
| (IV)-10 | 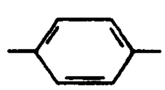 | 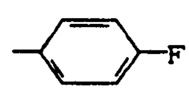 | 159-160 |

Analog den Herstellungsbeispielen (VI-1), (VI-2) und (VI-3) und entsprechend den angegebenen Verfahren können die in Tabelle 11 aufgeführten Zwischenprodukte der Formel (VI)

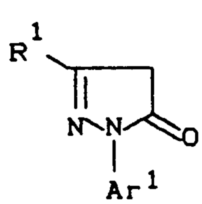

(VI)

bzw. der Formel (VIa)

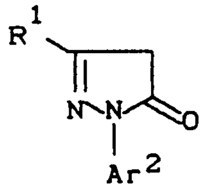

(VIa)

in welcher

$Ar^2$ für unsubstituiertes Phenyl steht,

erhalten werden. Verbindungen der Formel (VIa) dienen dabei als Zwischenprodukte zur Herstellung von bekannten Verbindungen der Formel (I).

**Tabelle 11**

| Bsp.Nr. | $R^1$ | $Ar^1$ | Schmelzpunkt (°C) |
|---|---|---|---|
| (VI-4) | $-CH_2SCH_3$ | 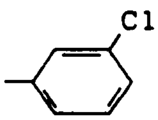 | 89 |

Tabelle 11 (Fortsetzung)

| Bsp.Nr. | $R^1$ | $Ar^1$ | Schmelzpunkt (°C) |
|---|---|---|---|
| (VI-5) | $-CH_2SCH_3$ | 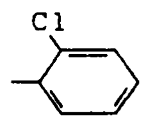 | 144 |
| (VI-6) | $-CH_2SCH_3$ | 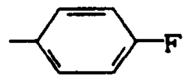 | 63 |
| (VI-7) | H | 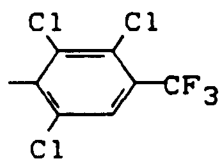 | 183-185 |
| (VI-8) | $-COOC_2H_5$ | 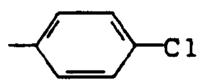 | 167-168 |
| (VI-9) | $CF_3$ | 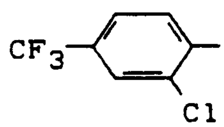 | 208-209 |
| (VI-10) | $CH_3$ | 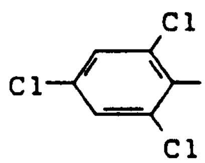 | 171 |
| (VI-11) | $n-C_3H_7$ | 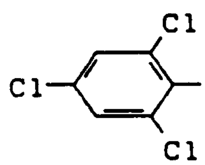 | 159 |
| (VI-12) | $CH_3$ | 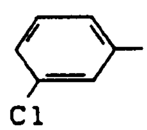 | 124 |
| (VI-13) | $n-C_3H_7$ | 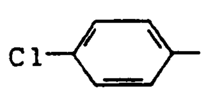 | 90-91 |

Tabelle 11 (Fortsetzung)

| Bsp.Nr. | $R^1$ | $Ar^1$ | Schmelzpunkt (°C) |
|---|---|---|---|
| (VI-14) | $CH_3$ | 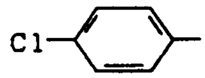 | 169 |
| (VI-15) | $(CH_3)_3C$ | 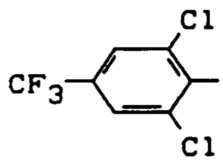 | 163 |
| (VI-16) | $C_2H_5$ | 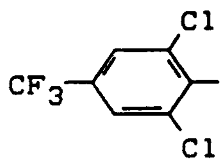 | 123 |
| (VI-17) | $n-C_3H_7$ | 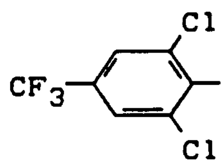 | 115 |
| (VI-18) | $(CH_3)_2CH-$ | 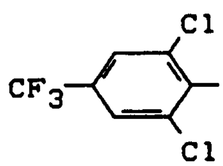 | 120 |
| (VI-19) | 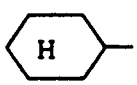 | 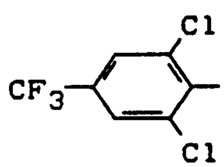 | 134 |
| (VI-20) | $n-C_3H_7$ | 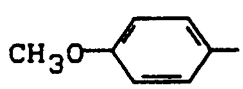 | 104-105 |
| (VI-21) | $CH_3$ | 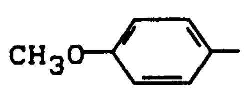 | 160 |

Tabelle 11 (Fortsetzung)

| Bsp.Nr. | $R^1$ | $Ar^1$ | Schmelzpunkt (°C) |
|---|---|---|---|
| (VI-22) | $n-C_3H_7$ | 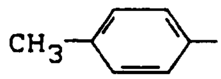 | 121 |
| (VI-23) | 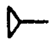 | 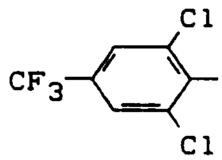 | 125-130 |
| (VI-24) | $CH_3$ | 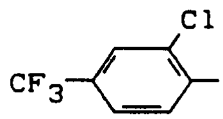 | 132-133 |
| (VI-25) | $CH_3$ | 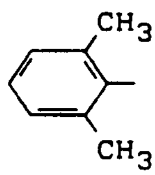 | 183-185 |
| (VI-26) | $n-C_3H_7$ | 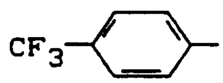 | 92-93 |
| (VI-27) | $CH_3$ | 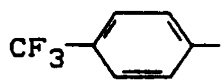 | 172 |
| (VI-28) | $CH_3OCH_2$- | 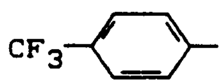 | 83 |
| (VI-29) | $n-C_3H_7$ | 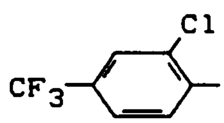 | 67 |
| (VI-30) | $n-C_3H_7$ | 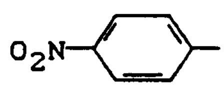 | 131-132 |

<u>Tabelle 11</u> (Fortsetzung)

| Bsp.Nr. | $R^1$ | $Ar^1$ | Schmelzpunkt(°C) |
|---|---|---|---|
| (VI-31) | $n-C_3H_7$ | 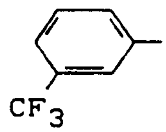 | 62-63 |
| (VI-32) | $(CH_3)_2CH-CH_2-$ | 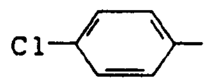 | 107-109 |
| (VI-33) | $(CH_3)_2CH-$ | 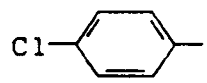 | 120 |
| (VI-34) | $n-C_4H_9$ | 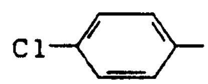 | 55 |
| (VI-35) | $(CH_3)_3C-$ | 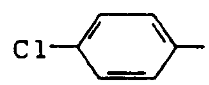 | 119-122 |
| (VI-36) | $C_2H_5$ | 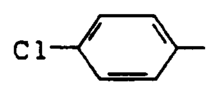 | 91-93 |
| (VI-37) | $n-C_3H_7$ | 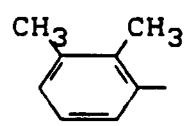 | 85 |
| (VI-38) | $CH_3$ | 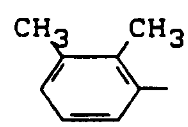 | 133-135 |
| (VI-39) | $CH_3OCH_2-$ | 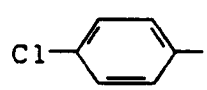 | 124 |
| (VI-40) | $CH_3$ | 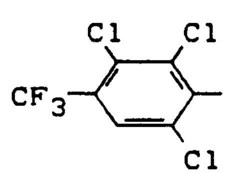 | 162-163 |

Tabelle 11 (Fortsetzung)

| Bsp.Nr. | $R^1$ | $Ar^1$ | Schmelzpunkt (°C) |
|---|---|---|---|
| (VI-41) | $C_2H_5$ | 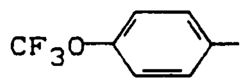 | 85-86 |
| (VI-42) | $n\text{-}C_3H_7$ | 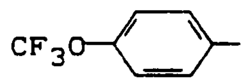 | 88-90 |
| (VI-43) | $CH_3$ | 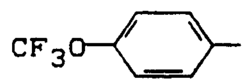 | 90-91 |
| (VI-44) | $CF_3$ | 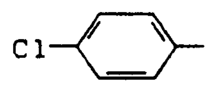 | 215 |
| (VI-45) | $C_2H_5\text{-}S\text{-}CH_2\text{-}$ | 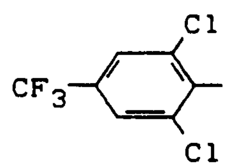 | 108-110 (Zers.) |
| (VI-46) | $C_2H_5\text{-}SCH_2\text{-}$ | 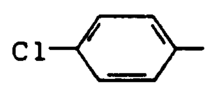 | 87 |
| (VI-47) | $n\text{-}C_3H_7$ | 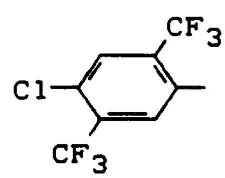 | 104-105 |
| (VI-48) | $n\text{-}C_3H_7$ | 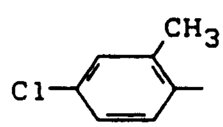 | 162-163 |
| (VI-49) | $n\text{-}C_3H_7$ | 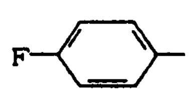 | 69-70 |

Tabelle 11 (Fortsetzung)

| Bsp.Nr. | $R^1$ | $Ar^1$ | Schmelzpunkt (°C) |
|---|---|---|---|
| (VI-50) | $CH_3$ | 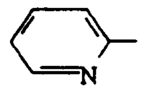 | 99-100 |
| (VI-51) | $n-C_3H_7$ | 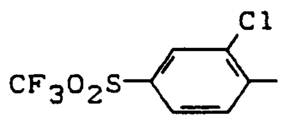 | 124-126 |
| (VI-52) | $n-C_3H_7$ | 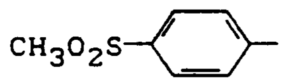 | 99-100 |
| (VI-53) | $n-C_3H_7$ | 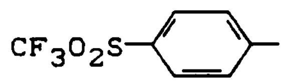 | 63-64 |
| (VI-54) | 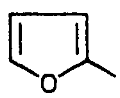 | 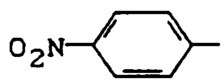 | 186-187 |
| (VI-55) | $C_2H_5-S-CH_2-$ | 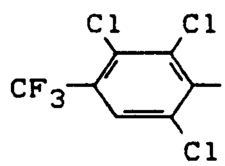 | 66-68 |
| (VI-56) | $C_2H_5-S-CH_2-$ | 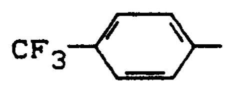 | 67 |
| (VI-57) | 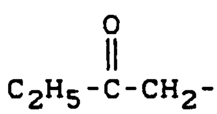 | 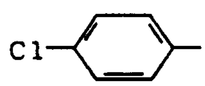 | 110 |
| (VI-58) | 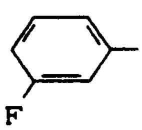 | 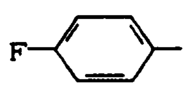 | 121-123 |

Tabelle 11 (Fortsetzung)

| Bsp.Nr. | $R^1$ | $Ar^1$ | Schmelzpunkt (° C) |
|---|---|---|---|
| (VI-59) | $CH_3-\overset{\overset{O}{\|}}{C}-NH-$ | 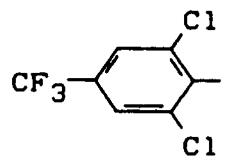 | 233 |
| (VI-60) | $CH_3$ | 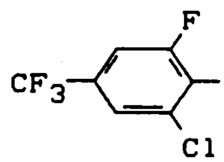 | 168-173 |
| (VI-61) | $n-C_3H_7$ | 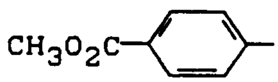 | 135-137 |
| (VI-62) | $C_2H_5O-$ | 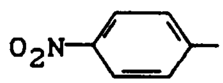 | 143 |
| (VI-63) | $C_2H_5O-$ | 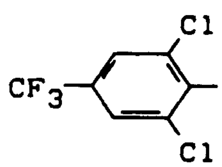 | 113 |
| (VI-64) | $C_2H_5O$ | 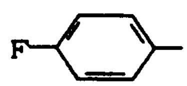 | 112 |
| (VI-65) | $CH_3$ | 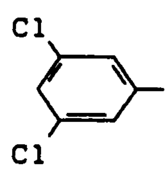 | 141-142 |
| (VI-66) | $n-C_3H_7$ | 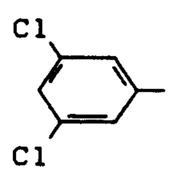 | 83-85 |

Tabelle 11 (Fortsetzung)

| Bsp.Nr. | $R^1$ | $Ar^1$ | Schmelz-punkt(°C) |
|---|---|---|---|
| (VI-67) | $CH_3$ | 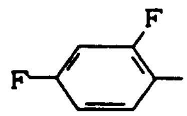 | 96-97 |
| (VI-68) | $n-C_3H_7$ | 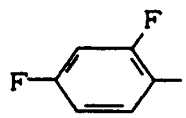 | 87-88 |
| (VI-69) | 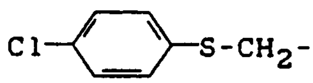 | 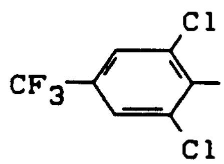 | 105-107 |
| (VI-70) | 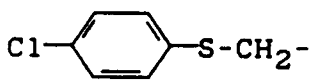 | 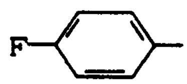 | 108-111 |
| (VI-71) | $(CH_3)_3C-CH_2$ | 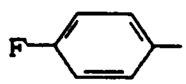 | 146-147 |
| (VI-72) | 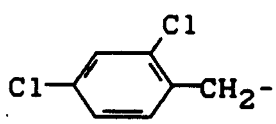 | 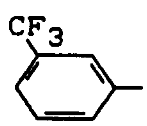 | 119 |
| (VI-73) | 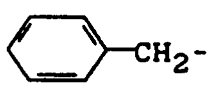 | 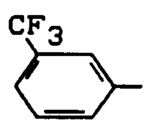 | 134 |
| (VI-74) | 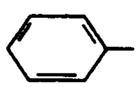 | 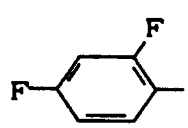 | 169-170 |
| (VI-75) | 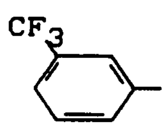 | 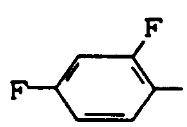 | 111 |

Tabelle 11 (Fortsetzung)

| Bsp.Nr. | $R^1$ | $Ar^1$ | Schmelzpunkt (°C) |
|---|---|---|---|
| (VI-76) | 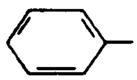 | 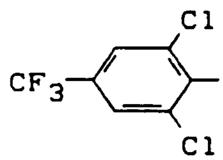 | 151-152 |
| (VI-77) | 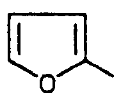 | 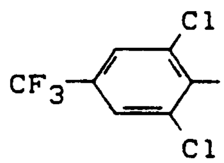 | 210-211 |
| (VI-78) | 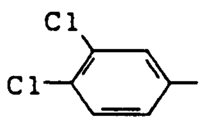 | 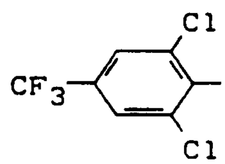 | 165-166 |
| (VI-79) | 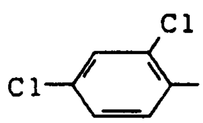 | 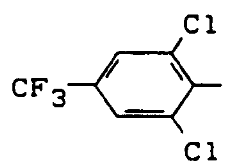 | 186-187 |
| (VI-80) | 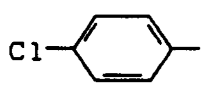 | 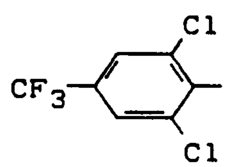 | 184-185 |
| (VI-81) | 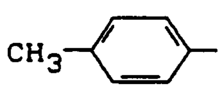 | 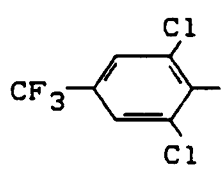 | 177-178 |
| (VI-82) | 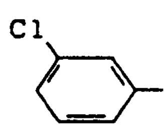 | 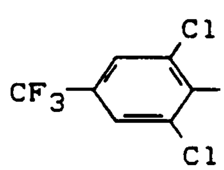 | 160-161 |

Tabelle 11 (Fortsetzung)

| Bsp.Nr. | $R^1$ | $Ar^1$ | Schmelzpunkt (°C) |
|---|---|---|---|
| (VI-83) | 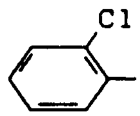 | 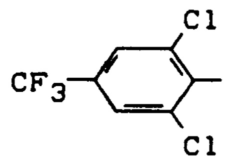 | 178-179 |
| (VI-84) | 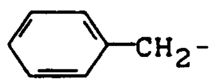 | 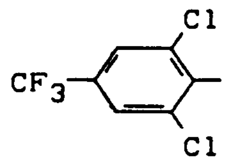 | 185 |
| (VI-85) | 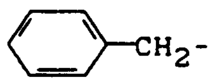 | 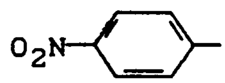 | 121 |
| (VI-86) | 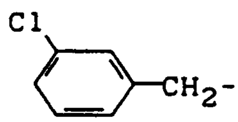 | 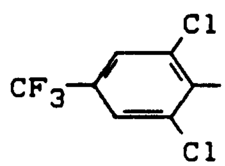 | 149-150 |
| (VI-87) | 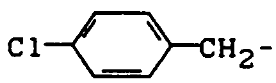 | 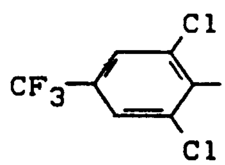 | 177 |
| (VI-88) | 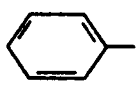 | 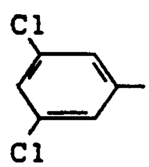 | 155-157 |
| (VI-89) | 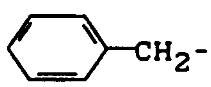 | 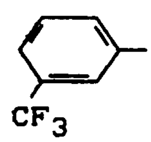 | 107 |
| (VI-90) | 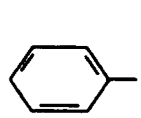 | 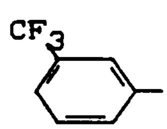 | 112-113 |

Tabelle 11 (Fortsetzung)

| Bsp.Nr. | $R^1$ | $Ar^1$ | Schmelzpunkt (°C) |
|---|---|---|---|
| (VI-91) | 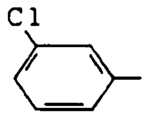 | 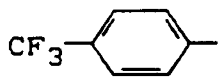 | 127-128 |
| (VI-92) | 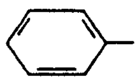 | 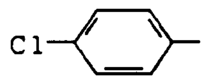 | 156-157 |
| (VI-93) | 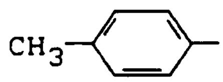 | 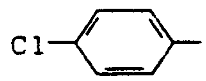 | 159-160 |
| (VI-94) | 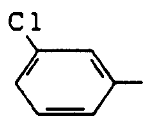 | 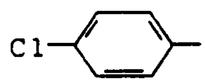 | 122-123 |
| (VI-95) | 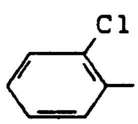 | 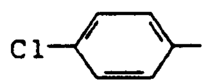 | 137-138 |
| (VI-96) | 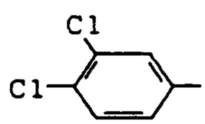 | 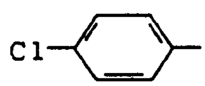 | 181-182 |
| (VI-97) | 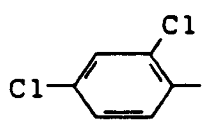 | 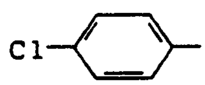 | 175-176 |
| (VI-98) | 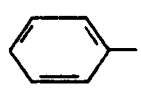 | 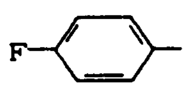 | 156-157 |
| (VI-99) | 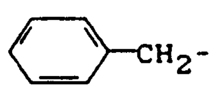 | 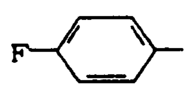 | 148-149 |

Tabelle 11 (Fortsetzung)

| Bsp.Nr. | $R^1$ | $Ar^1$ | Schmelzpunkt (°C) |
|---|---|---|---|
| (VI-100) | 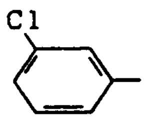 | 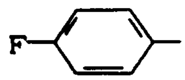 | 112 |
| (VI-101) | 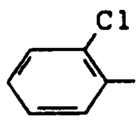 | 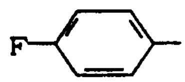 | 135-137 |
| (VI-102) | 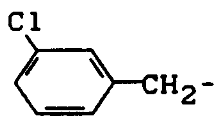 | 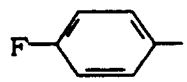 | 101 |
| (VI-103) | 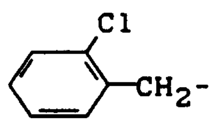 | 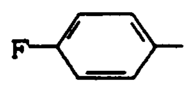 | 101 |
| (VI-104) | 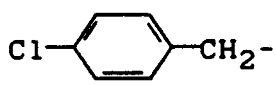 | 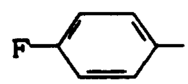 | 105 |
| (VI-105) | 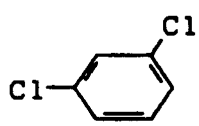 | 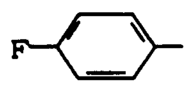 | 159 |
| (VI-106) | 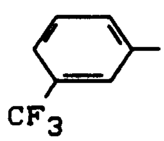 | 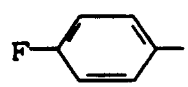 | 83 |
| (VI-107) | 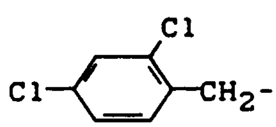 | 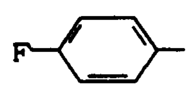 | 138 |

Tabelle 11 (Fortsetzung)

| Bsp.Nr. | $R^1$ | $Ar^1$ | Schmelz-punkt (°C) |
| --- | --- | --- | --- |
| (VI-108) | 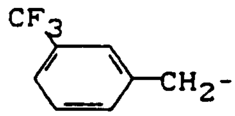 | 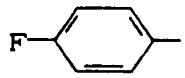 | 93-94 |
| (VI-109) | 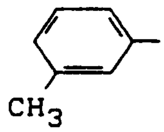 | 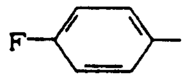 | 127 |
| (VI-110) | 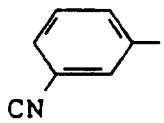 | 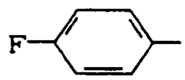 | 127 |
| (VI-111) | 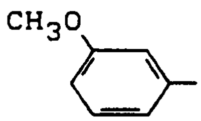 | 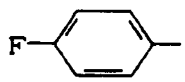 | 163-166 |
| (VI-112) | 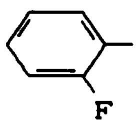 | 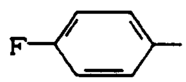 | 111-112 |
| (VI-113) | 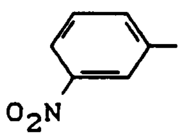 | 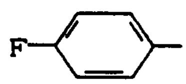 | 144-145 |
| (VI-114) | 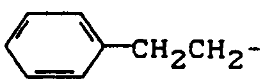 | 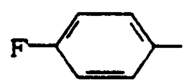 | 130 |
| (VI-115) | 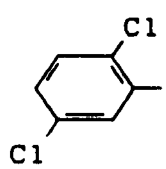 | 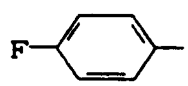 | 147 |
| (VI-116) | 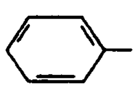 | 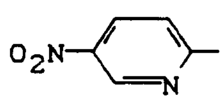 | 220-225 |

Tabelle 11 (Fortsetzung)

| Bsp.Nr. | $R^1$ | $Ar^1$ | Schmelzpunkt (°C) |
|---|---|---|---|
| (VI-117) | 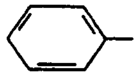 | 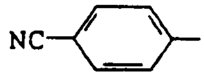 | 178 |
| (VI-118) | 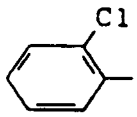 | 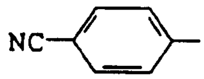 | 178 |
| (VI-119) | 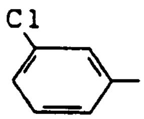 | 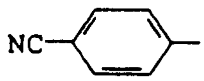 | 205 |
| (VI-120) | 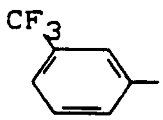 | 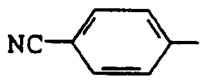 | 184 |
| (VI-121) | 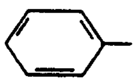 | 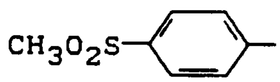 | 188 |
| (VI-122) | 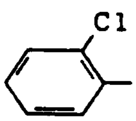 | 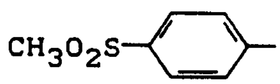 | 181 |
| (VI-123) | 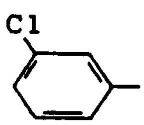 | 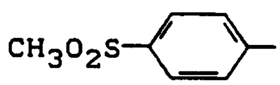 | 202 |
| (VI-124) | 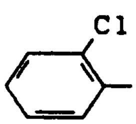 | 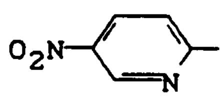 | 165 |
| (VI-125) | 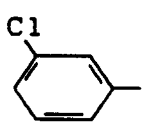 | 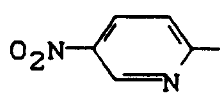 | 189 |

Tabelle 11 (Fortsetzung)

| Bsp.Nr. | $R^1$ | $Ar^1$ | Schmelzpunkt (°C) |
|---|---|---|---|
| (VI-126) | 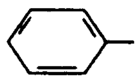 | 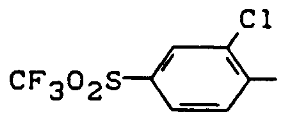 | 155-157 |
| (VI-127) | 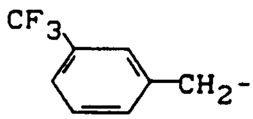 | 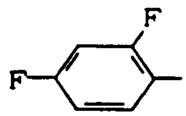 | 135 |
| (VI-128) | 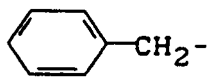 | 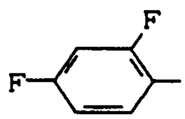 | 115 |
| (VI-129) | 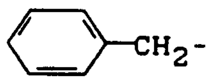 | 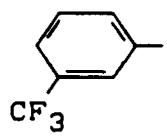 | 107 |
| (VI-130) | 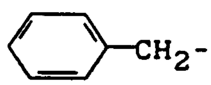 | 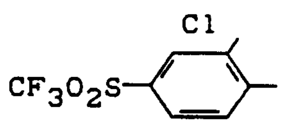 | 74 |
| (VI-131) | 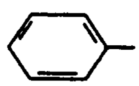 | 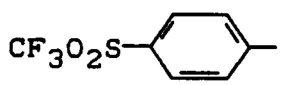 | 132 |
| (VI-132) | 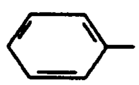 | 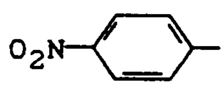 | 204-205 |
| (VI-133) | 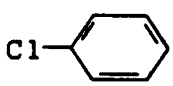 | 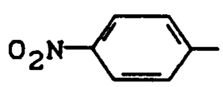 | 203-204 |
| (VI-134) | 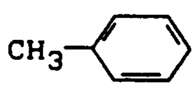 | 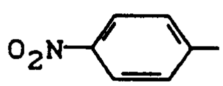 | 196-197 |

Tabelle 11 (Fortsetzung)

| Bsp.Nr. | $R^1$ | $Ar^1$ | Schmelzpunkt ($^\circ C$) |
|---|---|---|---|
| (VI-135) | 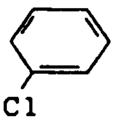 | 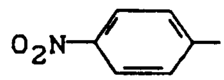 | 183-184 |
| (VI-136) | 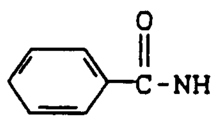 | 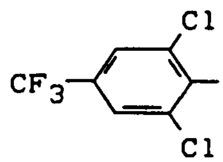 | 288 |
| (VI-137) | 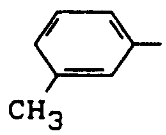 | 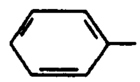 | 114 |
| (VI-138) | 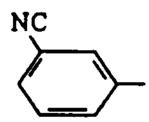 | 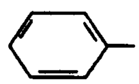 | 173-174 |
| (VI-139) | 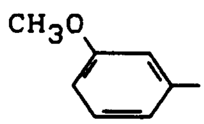 | 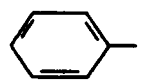 | 113 |
| (VI-140) | 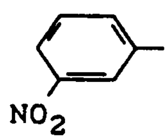 | 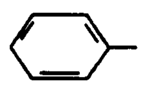 | 164 |
| (VI-141) | 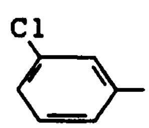 | 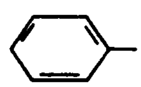 | 94-96 |
| (VI-142) | 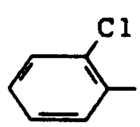 | 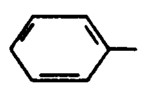 | 133-134 |

Tabelle 11 (Fortsetzung)

| Bsp.Nr. | $R^1$ | $Ar^1$ | Schmelzpunkt (°C) |
|---|---|---|---|
| (VI-143) | 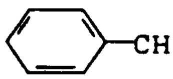 $CH_2CH_2$- | 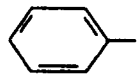 | 125 |
| (VI-144) | 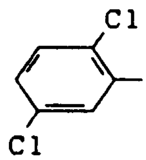 | 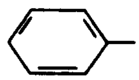 | 179 |
| (VI-145) | 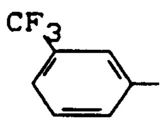 $CH_2$- | 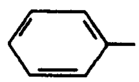 | 135 |
| (VI-146) | $CH_3$ | 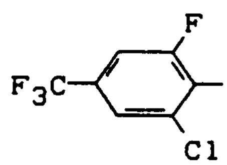 | |
| (VI-147) | 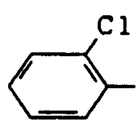 | 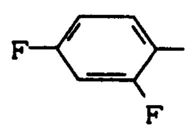 | 80 |
| (VI-148) | 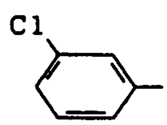 | 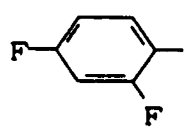 | 132 |
| (VI-149) | 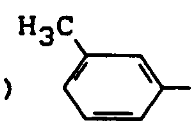 | 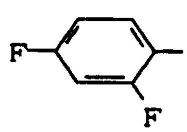 | 78 |
| (VI-150) | 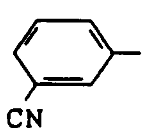 | 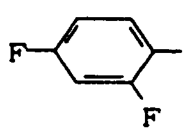 | 128 |

Tabelle 11 (Fortsetzung)

| Bsp.Nr. | $R^1$ | $Ar^1$ | Schmelzpunkt (°C) |
|---|---|---|---|
| (VI-151) | 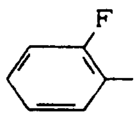 | 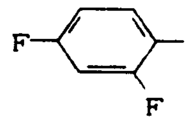 | 133 |
| (VI-152) | 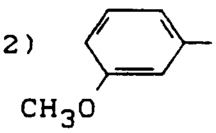 | 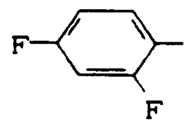 | 112 |
| (VI-153) | 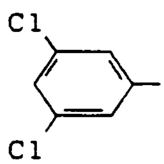 | 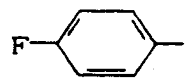 | 141-142 |
| (VI-154) | 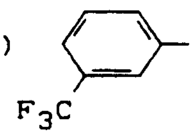 | 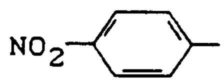 | 233 |
| (VI-155) | 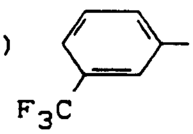 | 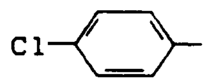 | 116 |
| (VI-156) | 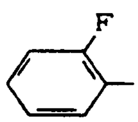 | 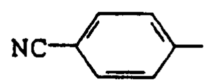 | 196 |
| (VI-157) | 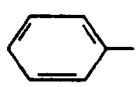 | 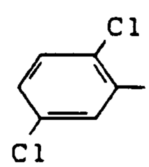 | 162 |
| (VI-158) | 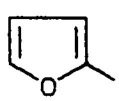 | 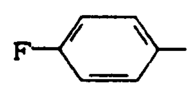 | 124 |

Tabelle 11 (Fortsetzung)

| Bsp.Nr. | $R^1$ | $Ar^1$ | Schmelzpunkt (°C) |
|---|---|---|---|
| (VI-159) | 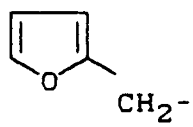 | 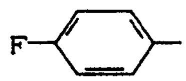 | 116 |
| (VI-160) | 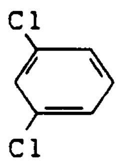 | 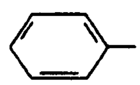 | 143-144 |
| (VI-161) | 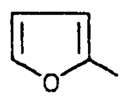 | 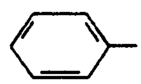 | 165 |
| (VI-162) | 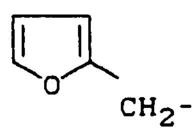 | 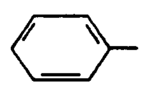 | 93-94 |

Beispiel VI-62

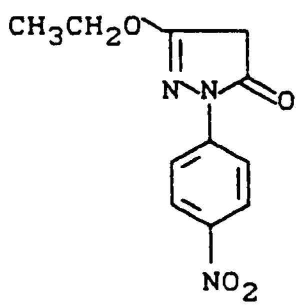

Verfahren 4

9,3 g (0,06 Mol) 4-Nitrophenylhydrazin und 11,4 g (0,06 Mol) Ethyl-β,β-diethyoxyacrylat werden in 50 ml absolutem Ethanol 30 Minuten unter Rückfluß erhitzt. Nach Zugabe von 1,4 g (0,06 Mol) Natrium in 40 ml absolutem Ethanol erhitzt man weitere 20 Minuten unter Rückfluß, kühlt die Reaktionsmischung ab und säuert mit verdünnter Essigsäure an. Anschließend wird der Feststoff abgesaugt, mit Wasser gewaschen und getrocknet (vgl. US-P 2,439,098).

Man erhält 11,0 g (73,6 % der Theorie) 3-Ethoxy-1-(4-nitrophenyl)-pyrazolin-5-on vom Schmelzpunkt 143 °C.

Beispiel 6

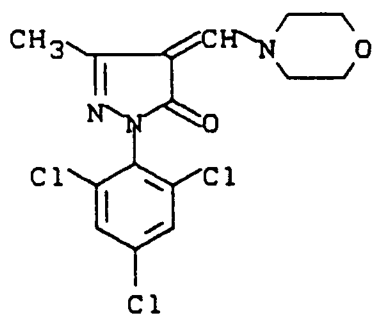

(I-1)

2,4 g (0,0079 Mol) 1-(2,4,6-Trichlorphenyl)-4-formyl-3-methyl-pyrazolin-5-on werden in 100 ml Dioxan gelöst und 0,7 g (0,0079 Mol) Morpholin zugegeben. Der Ansatz wird eine Stunde auf 100 °C erwärmt, danach das Lösungsmittel abgezogen und der Rückstand mit Petrolether verrührt. Das Produkt wird abgesaugt und getrocknet.

Man erhält 2,71 g (91,8 % der Theorie) 1-(2,4,6-Trichlorphenyl)-3-methyl-4-morpholinyl-methyliden-pyrazolin-5-on vom Schmelzpunkt 211-212 °C.

Analog dem Herstellungsbeispiel 6 = Verbindung (I-1) können die folgenden Verbindungen der Formel (I) erhalten werden:

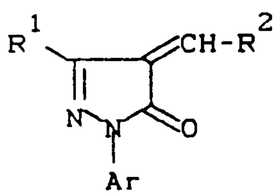

(I)

Tabelle 12

| Bsp.Nr. | $R^1$ | $R^2$ | Ar | Schmelzpunkt (°C) |
|---|---|---|---|---|
| (I-2) | $CH_3$ | 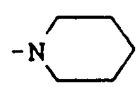 | 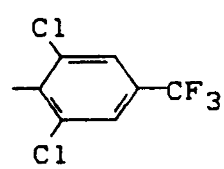 | 199 |

Beispiel VI-136

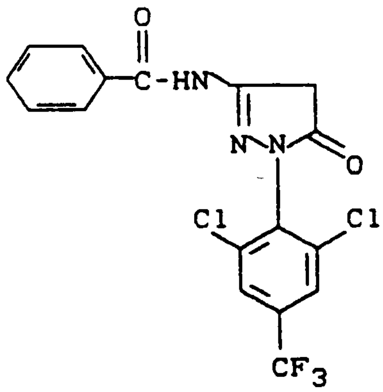

10 g (0,032 mol) 1-(2,6-Dichlor-4-trifluormethylphenyl)-3-amino-pyrazolin-5-on werden in 100 ml Dioxan aufgenommen und nach Zugabe von 4,5 g (0,032 mol) Benzoylchlorid 5 Stunden unter Rückfluß erhitzt. Anschließend wird die Reak tionsmischung eingeengt und mit Wasser verrührt. Der Fest stoff wird abgesaugt, mehrmals mit Wasser gewaschen und an der Luft getrocknet.

Man erhält 5,2 g (= 39,1 % d. Th.) 1-(2,6-Dichlor-4-trifluormethylphenyl)-3-Benzoylamino-pyrazolin-5-on vom Schmelzpunkt 288 °C.

Beispiel XVIII-1

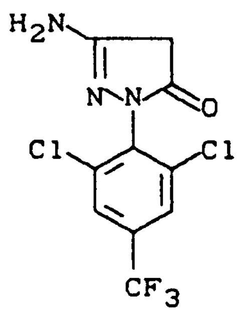

2,7 g (0,067 mol) Natrium werden in 50 ml Ethanol gelöst und anschließend werden 24 g (0,068 mol) β-(2,6-Dichlor-4-trifluormethylphenylhydrazino)-β-imino-propionsäureethylester zugegeben. Man erhitzt eine Stunde unter Rückfluß und engt dann die Reaktionsmischung im Vakuum ein. Nach dem Aufnehmen mit Wasser wird zweimal mit Ether extrahiert und die wässrige Phase mit verdünnter Salzsäure angesäuert. Der ausgefallene Feststoff wird abgesaugt und getrocknet.

Man erhält 9,6 g (45,9 % d. Th.) 1-(2,6-Dichlor-4-trifluormethylphenyl)-3-amino-pyrazolin-5-on vom Schmelzpunkt 206 - 207 °C.

Beispiel XVII-1

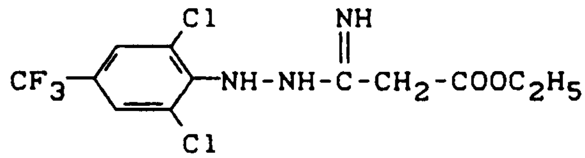

73,5 g (0,3 mol) 2,6-Dichlor-4-trifluormethylphenylhydrazin und 47,7 g (0,3 mol) β-Imino-β-ethoxy-propionsäureethylester werden in 300 ml Toluol 1 Stunde auf Rückfluß erhitzt. Nach dem Abkühlen gibt man Petrolether zu der Reaktionsmischung und saugt den ausgefallenen Feststoff ab.

Man erhält 60,3 g (56,2 % d. Th.) β-(2,6-Dichlor-4-trifluormethylphenylhydrazino)-β-imino-propionsäure-ethylester.

**Patentansprüche**

1. Verwendung von substituierten Pyrazolin-5-on Derivaten der Formel (I)

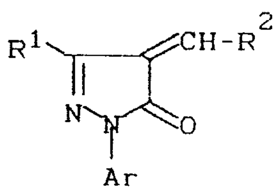

(I)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, jeweils gegebenenfalls substituiertes Alkenyl oder Alkinyl mit jeweils 2 bis 6 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter Ar aufgeführten Arylsubstituenten infrage kommen; $R^1$ weiterhin für Halogenalkenyl mit 2 bis 6 Kohlenstoffatomen und 1 bis 10 gleichen oder verschiedenen Halogenatomen, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl, Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter Ar aufgeführten Arylsubstituenten infrage kommen, $R^1$ weiterhin für die Gruppierungen -NH-CO-$R^{10}$ oder -CO-O-$R^{11}$ steht, worin

$R^{10}$ und $R^{11}$ jeweils unabhängig voneinander für $C_1$-$C_4$-Alkyl oder Phenyl stehen,

$R^2$ für die Gruppierungen -$NHR^3$, -$NR^4R^5$ oder -$NHOR^6$ steht, worin

$R^3$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, Alkoxyalkyl mit jeweils 1 bis 8 Kohlenstoffatomen im Alkoxy- oder Alkylteil, für gegebenenfalls einfach bis mehrfach, gleich oder

verschieden substituiertes Aralkyl mit 1 bis 4 Kohlenstoffatomen im geradkettigen oder verzweigten Alkylteil und 6 bis 10 Kohlenstoffatomen im Arylteil, für gegebenenfalls einfach bis fünffach, gleich oder verschieden substituiertes Aryl mit 6 bis 10 Kohlenstoffatomen steht, wobei als Arylsubstituenten jeweils infrage kommen: Halogen, geradkettiges oder verzweigtes Alkyl mit 1 bis 4 Kohlenstoffatomen und Dialkylamino mit jeweils 1 bis 4 Kohlenstoffatomen im Alkylteil, $C_1$-$C_4$-Alkoxy und Halogen-$C_1$-$C_4$-alkyl,

$R^4$ für Alkyl mit 1 bis 6 Kohlenstoffatomen steht,

$R^5$ für Alkyl mit 1 bis 6 Kohlenstoffatomen steht oder

$R^4$ und $R^5$ gemeinsam mit dem Stickstoffatom, an welches sie gebunden sind, für einen heterocyclischen 5- oder 6-gliedrigen Ring, der Sauerstoff, Schwefel und/oder Stickstoff als weitere Heteroatome enthalten kann, stehen,

$R^6$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, für gegebenenfalls einfach bis mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten Halogen, $C_1$-$C_4$-Alkyl, Halogen-$C_1$-$C_4$-alkyl und Nitro infrage kommen, und

Ar für gegebenenfalls einfach bis mehrfach, gleich oder verschieden substituiertes Aryl mit 6 bis 10 Kohlenstoffatomen steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy; oder $C_1$-$C_4$-Alkylthio; $C_3$-$C_6$-Alkinyloxy; Halogen-($C_1$-$C_4$)-alkyl, Halogen-($C_1$-$C_4$)-alkoxy oder Halogen($C_1$-$C_4$)alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen; und Di-($C_1$-$C_4$)-alkylamino für einen gegebenenfalls substituierten und/oder gegebenenfalls anellierten 6-gliedrigen, aromatischen Heterocyclus, welcher wenigstens ein Stickstoffatom enthält und wobei als Substituenten die oben bei Ar aufgeführten Arylsubstituenten infrage kommen, oder für die Gruppe

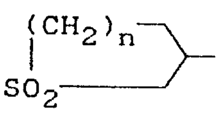

steht,
wobei

n für die Zahlen 1 oder 2 steht, und deren Salze,

zur Bekämpfung von Unkräutern oder Pilzen,
ausgenommen die Verbindungen 1-(4-Chlorphenyl)-3-methyl-4-piperidino-methylen-pyrazolin-5-on, 1-(4-Chlorphenyl)-3-methyl-4-morpholino-methylen-pyrazolin-5-on, 1-(4-Chlorphenyl)-4-[4-(fluorphenylamino)-methylen]-3-methyl-pyrazolin-5-on, 1-(4-Chlorphenyl)-3-methyl-4-aminomethylen-pyrazolin-5-on und 4-Aminomethylen-3-ethoxycarbonyl-1-phenyl-2-pyrazolin-5-on.

**2.** Verfahren zur Bekämpfung von Unkräutern oder Pilzen, dadurch gekennzeichnet, daß man substituierte Pyrazolin-5-on Derivate der Formel (I) gemäß dem Anspruch 1 auf Unkräuter oder Pilze oder ihren Lebensraum einwirken läßt.

**3.** Herbizide und fungizide Mittel von substituierten Pyrazolin-5-on Derivaten der Formel (I) gemäß dem Anspruch 1 zur Bekämpfung von Unkräutern oder Pilzen, ausgenommen die Verbindungen 1-(4-Chlorphenyl)-3-methyl-4-piperidino-methylen-pyrazolin-5-on, 1-(4-Chlorphenyl)-3-methyl-4-morpholinomethylen-pyrazolin-5-on, 1-(4-Chlorphenyl)-4-[4-(fluorphenylamino)-methylen]-3-methyl-pyrazolin-5-on, 1-(4-Chlorphenyl)-3-methyl-4-aminomethylenpyrazolin-5-on und 4-Aminomethylen-3-ethoxycarbonyl-1-phenyl-2-pyrazolin-5-on.

**4.** Substituierte Pyrazolin-5-on Derivate der Formel (Ia)

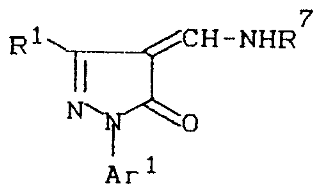

(Ia)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen, $R^1$ weiterhin für die Gruppierungen -NH-CO-$R^{10}$ oder -CO-O-$R^{11}$ steht, worin

$R^{10}$ und $R^{11}$ jeweils unabhängig voneinander für $C_1$-$C_4$-Alkyl oder Phenyl stehen,

$R^7$ für geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- und Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratomen, Alkoxyalkyl mit jeweils 1 bis 8 Kohlenstoffatomen im Alkoxy- und Alkylteil, für gegebenenfalls einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil, für gegebenenfalls einfach bis fünffach, gleich oder verschieden substituiertes Aryl mit 6 bis 10 Kohlenstoffatomen steht, wobei als Arylsubstituenten jeweils infrage kommen: Halogen, geradkettiges oder verzweigtes Alkyl mit 1 bis 4 Kohlenstoffatomen und Dialkylamino mit jeweils 1 bis 4 Kohlenstoffatomen im Alkylteil, $C_1$-$C_4$-Alkoxy oder Halogen-$C_1$-$C_4$-alkyl,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen; und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei

als Substituenten die oben bei $Ar^1$ aufgeführten Arylsubstituenten infrage kommen, oder für die Gruppe

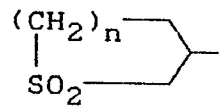

steht,
wobei
n für die Zahlen 1 oder 2 steht, und deren Salze,
ausgenommen die Verbindungen
1-(4-Chlorphenyl)-3-methyl-4-piperidinomethylen-2-pyrazolin-5-on
1-(4-Chlorphenyl)-3-methyl-4-morpholinomethylen-2-pyrazolin-5-on
1-(4-Chlorphenyl)-4-[4-(fluorphenylamino)methylen]-3-methyl-2-pyrazolin-5-on
4-m-Toluido-methylen-1-p-tolyl-3-methyl-2-pyrazolin-5-on
4-o-Toluido-methylen-1-p-tolyl-3-methyl-2-pyrazolin-5-on
1-p-Tolyl-3-methyl-4-o-ethoxyanilinomethylen-2-pyrazolin-5-on
1-p-Tolyl-3-methyl-4-p-bromoanilinomethylen-2-pyrazolin-5-on
1-p-Tolyl-3-methyl-4-anilinomethylen-2-pyrazolin-5-on
1-o-Tolyl-3-methyl-4-m-xylidomethylen-2-pyrazolin-5-on
1-o-Tolyl-3-methyl-4-o-ethoxyanilinomethylen-2-pyrazolin-5-on
1-o-Tolyl-3-phenyl-4-anilinomethylen-2-pyrazolin-5-on
1-o-Tolyl-3-phenyl-4-m-xylidomethylen-2-pyrazolin-5-on
1-o-Tolyl-3-phenyl-4-p-chloranilinomethylen-2-pyrazolin-5-on
4-p-Bromanilinomethylen-3-phenyl-1-o-tolyl-2-pyrazolin-5-on
4-m-Bromanilinomethylen-3-phenyl-1-o-tolyl-2-pyrazolin-5-on
1-p-Bromphenyl-3-phenyl-4-anilinomethylen-2-pyrazolin-5-on
1-(4-Ethoxyphenyl)-3-methyl-4-p-ethoxyanilinomethylen-2-pyrazolin-5-on
4-o-Aminoanilinomethylen-1-p-ethoxyphenyl-3-methyl-2-pyrazolin-5-on
4-Anilinomethylen-1-p-ethoxyphenyl-3-methyl-2-pyrazolin-5-on
1-(2-Methyl-5-benzothiazolyl)-3-methyl-4-phenylaminomethylen-5-pyrazolon
1-p-Chlorphenyl-3-methyl-4-anilinomethylen-2-pyrazolin-5-on
1-m-Chlorphenyl-3-methyl-4-anilinomethylen-2-pyrazolin-5-on
1-m-Trifluormethylphenyl-3-methyl-4-anilinomethylen-2-pyrazolin-5-on
1-o,o-Dichlorphenyl-3-methyl-4-anilinomethylen-2-pyrazolin-5-on
1-m-Sulfamoylphenyl-3-methyl-4-anilinomethylen-2-pyrazolin-5-on
4-Methylaminomethylen-1-p-bromphenyl-3-methyl-2-pyrazolin-5-on

**5.** Substituierte Pyrazolin-5-on Derivate der Formel (Ib)

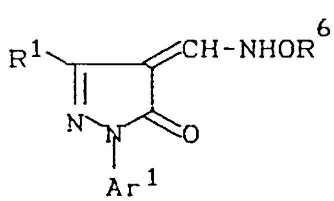

(Ib)

in welcher
$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; Halogenalkenyl mit 2 bis 6 Kohlenstoffatomen und 1

bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl oder Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen; $R^1$ weiterhin für die Gruppierungen -NH-CO-$R^0$ oder -CO-O-$R^{11}$ steht, worin

$R^{10}$ und $R^{11}$ jeweils unabhängig voneinander für $C_1$-$C_4$-Alkyl oder Phenyl stehen,

$R^6$ für Wasserstoff, geradkettiges oder verzweigtes Alkyl mit 1 bis 8 Kohlenstoffatomen, Halogenalkyl mit 1 bis 6 Kohlenstoffatomen und 1 bis 12 gleichen oder verschiedenen Halogenatomen, wie insbesondere Fluor- und Chloratomen, geradkettiges oder verzweigtes Alkenyl mit 2 bis 12 Kohlenstoffatomen, Halogenalkenyl mit 2 bis 6 Kohlenstoffatomen und 1 bis 10 gleichen oder verschiedenen Halogenatomen, wie insbesondere Fluor- und Chloratomen, für gegebenenfalls einfach bis mehrfach, gleich oder verschieden substituiertes Aralkyl mit 1 bis 4 Kohlenstoffatomen im Alkylteil und 6 bis 10 Kohlenstoffatomen im Arylteil steht, wobei als Arylsubstituenten Halogen, $C_1$-$C_4$-Alkyl, Halogen-$C_1$-$C_4$-alkyl und Nitro infrage kommen, und

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen; und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

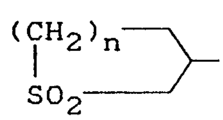

steht,

wobei

n für die Zahlen 1 oder 2 steht, und deren Salze.

**6.** Substituierte Pyrazolin-5-on Derivate der Formel (Ic)

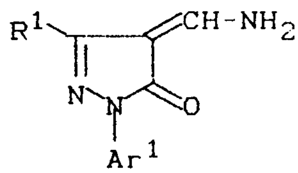

(Ic)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 6 Kohlenstoffatomen, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl, mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen, $R^1$ weiterhin für die Gruppierungen -NH-CO-$R^{10}$ oder -CO-O-$R^{11}$ steht, worin

$R^{10}$ und $R^{11}$ jeweils unabhängig voneinander für $C_1$-$C_4$-Alkyl oder Phenyl stehen,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen und Di-($C_1$-$C_4$)-alkylamino; $Ar^1$ ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

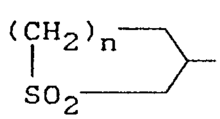

steht,
wobei

n für die Zahlen 1 oder 2 steht, und deren Salze,

ausgenommen die Verbindungen 4-Aminomethylen-1-(2-ethyl-phenyl)-3-methyl-2-pyrazolin-5-on,
4-Aminomethylen-1-(4-chlorphenyl)-3-methyl-2-pyrazolin-5-on,
4-Aminomethylen-3-methyl-1-(4-nitrophenyl)-2-pyrazolin-5-on,

**7.** Substituierte Pyrazolin-5-on Derivate der Formel (Id)

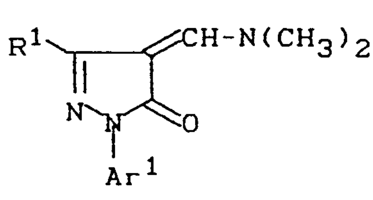

(Id)

in welcher

$R^1$ für Wasserstoff, geradkettiges oder verzweigtes Alkyl mit 1 bis 8 Kohlenstoffatomen, Cycloalkyl mit 3 bis 7 Kohlenstoffatomen, Halogenalkyl mit 1 bis 4 Kohlenstoffatomen und 1 bis 9 gleichen oder verschiedenen Halogenatomen, wie insbesondere Fluor- und Chloratomen, jeweils gegebenenfalls substituiertes Alkenyl oder Alkinyl mit jeweils 2 bis 4 Kohlenstoffatomen steht, wobei als Substituenten unsubstituiertes Phenyl oder einfach bis dreifach, gleich oder verschieden substituiertes Phenyl genannt seien und wobei als Phenylsubstituenten die unter $Ar^1$ aufgeführten Arylsubstituenten infrage kommen; $R^1$ weiterhin für Halogenalkenyl mit 2 bis 6 Kohlenstoffatomen und 1 bis 10 gleichen oder verschiedenen Halogenatomen, wie insbesondere Fluor und Chlor, Alkoxy mit 1 bis 8 Kohlenstoffatomen, Alkoxyalkyl mit jeweils 1 bis 8 Kohlenstoffatomen in den einzelnen Alkylteilen, Alkylthioalkyl, Alkylsulfonylalkyl oder Alkylsulfinylalkyl, mit jeweils 1 bis 8 Kohlenstoffatomen in den einzelnen Alkylteilen, Alkoxycarbonylalkyl mit 1 bis 4 Kohlenstoffatomen im Alkoxyteil und 1 bis 4 Kohlenstoffatomen im Alkylteil, einen 5- oder 6-gliedrigen, gegebenenfalls durch Fluor, Chlor, Methyl und/oder Ethyl substituierten Heterocyclus, insbesondere Furanyl oder Thienyl; Furanylmethyl, Thienylmethyl oder

$R^1$ weiterhin für jeweils im Arylteil gegebenenfalls einfach bis fünffach, gleich oder verschieden substituiertes Aryl, Arylalkyl, Aryloxyalkyl oder Arylthioalkyl mit jeweils 6 bis 10 Kohlenstoffatomen im Arylteil und gegebenenfalls 1 bis 4 Kohlenstoffatomen im Alkylteil steht, wobei als Arylsubstituenten die unter $Ar^1$ aufgeführten Arylsubstituenten infrage kommen, $R^1$ weiterhin für die Gruppierungen $-NH-CO-R^{10}$ oder $-CO-O-R^{11}$ steht, worin

$R^{10}$ und $R^{11}$ jeweils unabhängig voneinander für $C_1$-$C_4$-Alkyl oder Phenyl stehen,

$Ar^1$ für einfach bis mehrfach, gleich oder verschieden substituiertes Aryl mit 6 bis 10 Kohlenstoffatomen, insbesondere Phenyl oder Naphthyl steht, wobei als Arylsubstituenten infrage kommen: Halogen; Nitro; Cyano; Carboxyl; Alkoxycarbonyl mit 1 bis 4 Kohlenstoffatomen, $C_1$-$C_4$-Alkyl, $C_1$-$C_4$-Alkoxy oder $C_1$-$C_4$-Alkylthio; $C_3$-$C_6$-Alkinoxy; Halogen-($C_1$-$C_4$)-alkyl, Halogen-($C_1$-$C_4$)-alkoxy oder Halogen-($C_1$-$C_4$)-alkylthio mit jeweils 1 bis 9 gleichen oder verschiedenen Halogenatomen; Phenyl; $C_1$-$C_4$-Alkylsulfonyl und Halogen-($C_1$-$C_4$)-alkylsulfonyl mit jeweils 1 bis 9 gleichen oder verschiedenen Halogenatomen und Di-($C_1$-$C_4$)-alkylamino; ferner für einen gegebenenfalls substituierten und/oder gegebenenfalls anellierten 6-gliedrigen, aromatischen Heterocyclus steht, welcher wenigstens ein Stickstoffatom enthält und wobei als Substituenten die oben bei $Ar^1$ aufgeführten Arylsubstituenten infrage kommen, oder für die Gruppe

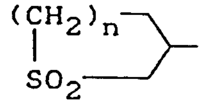

steht,

wobei

n für die Zahlen 1 oder 2 steht, und deren Salze,

ausgenommen die Verbindungen 1-(4-Nitrophenyl)-3-methyl-4-N,N-dimethylamino-methyliden-pyrazolin-5-on, 1-(4-(Chlorphenyl)-3-(2-nitrophenyl)-4-N,N-dimethylamino-methyliden-2-pyrazolin-5-on, 1-(3-Trifluormethylphenyl)-3-phenyl-4-N,N-dimethylaminomethyliden-2-pyrazolin-5-on, 1-p-Sulfophenyl-3-methyl-4-N,N-dimethylaminomethyliden-2-pyrazolin-5-on,

4-N,N-dimethylaminomethyliden-1-p-chlorphenyl-3-methyl-2-pyrazolin-5-on,

**8.** Substituierte Pyrazolin-5-on Derivate der Formel (If),

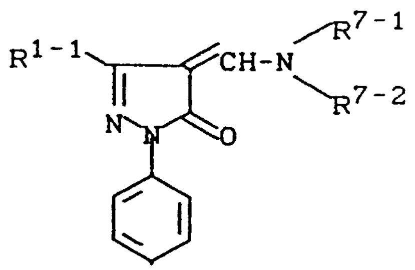

(If)

in welcher

$R^{1-1}$ für $C_1$-$C_8$-Alkoxy, Alkenyl mit 2 bis 6 Kohlenstoffatomen, gegebenenfalls substituiert durch unsubstituiertes Phenyl oder durch einfach bis dreifach, gleich oder verschieden substituiertes Phenyl, wobei als Phenylsubstituenten $C_1$-$C_4$-Alkyl, $C_1$-$C_4$-Alkoxy, Halogen-($C_1$-$C_4$)-alkyl und Halogen-($C_1$-$C_4$)alkoxy genannt seien, $R^{1-1}$ weiterhin für jeweils einfach bis fünffach, gleich oder verschieden substituiertes Aryl mit 6 bis 10 Kohlenstoffatomen oder gegebenenfalls substituiertes Aralkyl mit 6 bis 10 Kohlenstoffatomen im Arylteil und 1 bis 4 Kohlenstoffatomen im Alkylteil, wobei als Arylsubstituenten jeweils Halogen, Nitro, Cyano, Carboxyl, $C_1$-$C_4$-Alkoxycarbonyl, $C_1$-$C_4$-Alkyl, $C_1$-$C_4$-Alkoxy, Halogen-($C_1$-$C_4$)-alkyl, Halogen-($C_1$-$C_4$)-alkoxy, Halogen-($C_1$-$C_4$)alkylthio, Phenyl, $C_1$-$C_4$-Alkylsulfonyl, Halogen-($C_1$-$C_4$)-alkylsulfonyl und Di-($C_1$-$C_4$)alkylamino genannt seien; $R^{1-1}$ ferner für einen 5- oder 6-gliedrigen gegebenenfalls durch Fluor, Chlor, Methyl und/oder Ethyl substituierten Heterocyclus, der ein oder zwei gleiche oder verschiedene Heteroatome, insbesondere Stickstoff, Sauerstoff und Schwefel, enthalten kann; für jeweils gegebenenfalls durch Fluor, Chlor, Methyl und/oder Ethyl substituiertes Furanyl-$C_1$-$C_4$-alkyl oder Thienyl-$C_1$-$C_4$-alkyl steht, oder

$R^{1-1}$ weiterhin für die Gruppe -NH-CO-$R^{10}$ steht, wobei

$R^{10}$ für $C_1$-$C_6$-Alkyl oder Phenyl steht,

$R^{7-1}$ für Wasserstoff, $C_1$-$C_8$-Alkyl, Halogen-$C_1$-$C_6$-alkyl, $C_2$-$C_6$-Alkenyl, Halogen-$C_2$-$C_6$-alkeny, $C_1$-$C_8$-Alkoxy-$C_1$-$C_8$-alkyl, gegebenenfalls einfach bis fünffach, gleich oder verschieden substituiertes Aralkyl mit 6 bis 10 Kohlenstoffatomen im Arylteil und 1 bis 4 Kohlenstoffatomen im Alkylteil oder für gegebenenfalls einfach bis fünffach, gleich oder verschieden substituiertes Aryl mit 6 bis 10 Kohlenstoffatomen steht, wobei als Arylsubstituenten jeweils Halogen, $C_1$-$C_4$-Alkyl, $C_1$-$C_4$-Alkoxy, Halogen-$C_1$-$C_4$-alkyl und Di-($C_1$-$C_4$)-alkylamino genannt seien, und

$R^{7-2}$ für Wasserstoff oder Methyl steht,

ausgenommen die Verbindungen
1-Phenyl-3-(4-methoxyphenyl)-4-N,N-dimethylaminomethyliden-2-pyrazolin-5-on
4-N,N-dimethylaminomethyliden-3-(4-formamido-2-pyridyl)-1-phenyl-2-pyrazolin-5-on
1-m-Trifluormethylphenyl-1-phenyl-4-dimethylaminomethylen-2-pyrazolin-5-on
4-N,N-dimethylaminomethylen-1,3-diphenyl-2-pyrazolin-5-on
4-Methylaminomethylen-1,3-diphenyl-2-pyrazolin-5-on
4-[4-Bromanilinomethylen]-1,3-diphenyl-2-pyrazolin-5-on
4-p-Phenetidinomethylen-1,3-diphenyl-2-pyrazolin-5-on
4-Aminomethylen-1,3-diphenyl-2-pyrazolin-5-on
3-Methyl-1-phenyl-4-[4-phenylazoanilinomethylen]-2-pyrazolin-5-on
3-Methyl-4-p-phenetidinomethylen-1-phenyl-2-pyrazolin-5-on
4-Anilinomethylen-3-methyl-1-phenyl-2-pyrazolin-5-on
4-Aminomethylen-3-methyl-1-phenyl-2-pyrazolin-5-on.

**9.** Verbindungen der Formeln

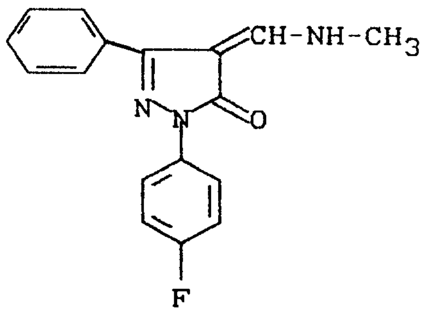

,

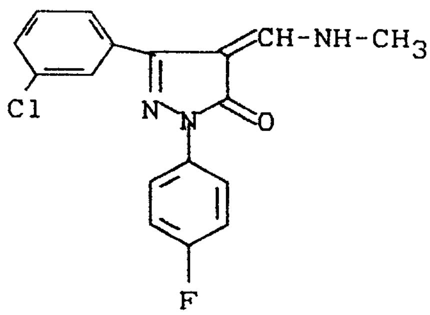

und

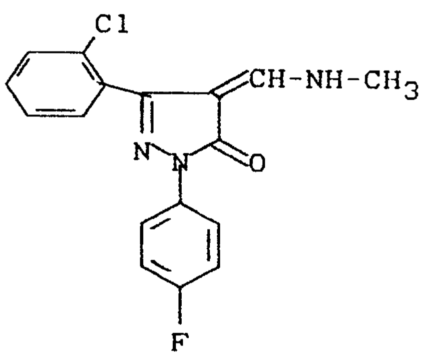

## Claims

**1.** Use of substituted pyrazolin-5-one derivatives of the formula (I)

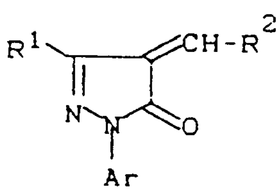

(I)

in which

$R^1$ represents hydrogen, a straight-chain or branched alkyl having 1 to 8 carbon atoms, cycloalkyl having 3 to 7 carbon atoms, halogenoalkyl having 1 to 4 carbon atoms and 1 to 9 identical or different halogen atoms, in each case optionally substituted

alkenyl or alkinyl, each of which has 2 to 6 carbon atoms, substituents which may be mentioned being unsubstituted phenyl or phenyl which is monosubstituted to trisubstituted by identical or different substituents and suitable phenyl substituents being the aryl substituents mentioned under Ar; $R^1$ furthermore represents halogenoalkenyl having 2 to 6 carbon atoms and 1 to 10 identical or different halogen atoms, alkoxy having 1 to 8 carbon atoms, alkoxyalkyl having 1 to 8 carbon atoms in each of the individual alkyl moieties, alkylthioalkyl, alkylsulphonylalkyl or alkylsulphinylalkyl, each of which has 1 to 8 carbon atoms in the individual alkyl moieties, alkoxy carbonylalkyl having 1 to 4 carbon atoms in the alkoxy moiety and 1 to 4 carbon atoms in the alkyl moiety, a 5- or 6-membered heterocycle which is optionally substituted by fluorine, chlorine, methyl and/or ethyl, in particular furanyl, thienyl; furanylmethyl, thienylmethyl or

$R^1$ furthermore represents aryl, arylalkyl, aryloxyalkyl or arylthioalkyl, each of which has 6 to 10 carbon atoms in the aryl moiety and, where appropriate, 1 to 4 carbon atoms in the alkyl moiety and each of which is optionally monosubstituted to pentasubstituted in the aryl moiety by identical or different substituents, suitable aryl substituents being the aryl substituents mentioned under Ar, $R^1$ furthermore represents the groups $-NH-CO-R^{10}$ or $-CO-O-R^{11}$ in which

$R^{10}$ and $R^{11}$ in each case independently of one another represent $C_1$-$C_4$-alkyl or phenyl,

$R^2$ represents the groups $-NHR^3$, $-NR^4R^5$ or $-NHOR^6$ in which

$R^3$ represents hydrogen, straight-chain or branched alkyl having 1 to 8 carbon atoms, halogenoalkyl having 1 to 6 carbon atoms and 1 to 12 identical or different halogen atoms, straight-chain or branched alkenyl having 2 to 12 carbon atoms, halogenoalkenyl having 2 to 6 carbon atoms and 1 to 10 identical or different halogen atoms, alkoxyalkyl having in each case 1 to 8 carbon atoms in the alkoxy or alkyl moiety, aralkyl which has 1 to 4 carbon atoms in the straight-chain or branched alkyl moiety and 6 to 10 carbon atoms in the aryl moiety and which is optionally monosubstituted to polysubstituted by identical or different substituents, aryl which has 6 to 10 carbon atoms and which is optionally monosubstituted to pentasubstituted by identical or different substituents, suitable aryl substituents in each case being: halogen, straight-chain or branched alkyl having 1 to 4 carbon atoms and dialkylamino having in each case 1 to 4 carbon atoms in the alkyl moiety, $C_1$-$C_4$-alkoxy and halogeno-$C_1$-$C_4$-alkyl,

$R^4$ represents alkyl having 1 to 6 carbon atoms,

$R^5$ represents alkyl having 1 to 6 carbon atoms or

$R^4$ and $R^5$ together with the nitrogen atom to which they are bonded represent a heterocyclic 5-or 6-membered ring which can contain oxygen, sulphur and/or nitrogen as further hetero atoms,

$R^6$ represents hydrogen, straight-chain or branched alkyl having 1 to 8 carbon atoms, halogenoalkyl having 1 to 6 carbon atoms and 1 to 12 identical or different halogen atoms, straight-chain or branched alkenyl having 2 to 12 carbon atoms, halogenoalkenyl having 2 to 6 carbon atoms and 1 to 10 identical or different halogen atoms, or represents aralkyl which has 1 to 4 carbon atoms in the alkyl moiety and 6 to 10 carbon atoms in the aryl moiety and which is optionally monosubstituted to polysubstituted by identical or different substituents, suitable aryl substituents being halogen, $C_1$-$C_4$-alkyl, halogeno-$C_1$-$C_4$-alkyl and nitro, and

Ar represents aryl which has 6 to 10 carbon atoms and which is optionally monosubstituted to polysubstituted by identical or different substituents, suitable aryl substituents being: halogen; nitro; cyano; carboxyl; alkoxycarbonyl having 1 to 4 carbon atoms, $C_1$-$C_4$-alkyl, $C_1$-$C_4$-alkoxy; or $C_1$-$C_4$-alkylthio; $C_3$-$C_6$-alkinyloxy; halogeno-($C_1$-$C_4$)-alkyl, halogeno-($C_1$-$C_4$)-alkoxy or halogeno($C_1$-$C_4$)alkylthio, each of which has 1 to 9 identical or different halogen atoms; phenyl; $C_1$-$C_4$-alkylsulphonyl and halogeno-($C_1$-$C_4$)-alkylsulphonyl having in each case 1 to 9 identical or different halogen atoms; and di-($C_1$-$C_4$)-alkylamino, or represents a 6-membered aromatic heterocycle which is optionally substituted and/or optionally fused and which contains at least one nitrogen atom and where suitable substituents are those aryl substituents mentioned above in the case of Ar, or represents the group

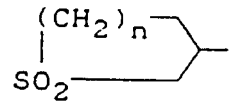

where

n represents the numbers 1 or 2, and their salts,

for combating weeds or fungi,

with the exception of the compounds 1-(4-chlorophenyl)-3-methyl-4-piperidino-methylene-pyrazolin-5-one, 1-(4-chlorophenyl)-3-methyl-4-morpholino-methylene-pyrazolin-5-one, 1-(4-chlorophenyl)-4-[4-(fluorophenylamino)-methylene]-3-methyl-pyrazolin-5-one, 1-(4-chlorophenyl)-3-methyl-4-aminomethylene-pyrazolin-5-one and 4-aminomethylene-3-ethoxycarbonyl-1-phenyl-2-pyrazolin-5-one.

**2.** Method of combating weeds or fungi, characterised in that substituted pyrazolin-5-one derivatives of the formula (I) according to Claim 1 are allowed to act on weeds or fungi or their environment.

**3.** Herbicidal and fungicidal agents of substituted pyrazolin-5-one derivatives of the formula (I) according to Claim 1 for combating weeds or fungi, with the exception of the compounds 1-(4-chlorophenyl)-3-methyl-4-piperidino-methylene-pyrazolin-5-one, 1-(4-chlorophenyl)-3-methyl-4-morpholinomethylene-pyrazolin-5-one, 1-(4-chlorophenyl)-4-[4-(fluorophenylamino)-methylene]-3-methyl-pyrazolin-5-one, 1-(4-chlorophenyl)-3-methyl-4-aminomethylene-pyrazolin-5-one and 4-aminomethylene-3-ethoxycarbonyl-1-phenyl-2-pyrazolin-5-one.

**4.** Substituted pyrazolin-5-one derivatives of the formula (Ia)

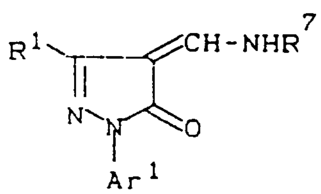

**(Ia)**

in which

$R^1$ represents hydrogen, straight-chain or branched alkyl having 1 to 8 carbon atoms, cycloalkyl having 3 to 7 carbon atoms, halogenoalkyl having 1 to 4 carbon atoms and 1 to 9 identical or different halogen atoms such as, in particular, fluorine and chlorine atoms, in each case optionally substituted alkenyl or alkinyl, each of which has 2 to 6 carbon atoms, substituents which may be mentioned being unsubstituted phenyl or phenyl which is monosubstituted to trisubstituted by identical or different substituents, suitable phenyl substituents being the aryl substituents mentioned under $Ar^1$; $R^1$ furthermore represents halogenoalkenyl having 2 to 6 carbon atoms and 1 to 10 identical or different halogen atoms such as, in particular, fluorine and chlorine, alkoxy having 1 to 8 carbon atoms, alkoxyalkyl having in each case 1 to 8 carbon atoms in the individual alkyl moieties, alkylthioalkyl, alkylsulphonylalkyl or alkylsulphinylalkyl, each of which has 1 to 8 carbon atoms in the individual alkyl moieties, alkoxycarbonylalkyl having 1 to 4 carbon atoms in the alkoxy moiety and 1 to 4 carbon atoms in the alkyl moiety, a 5- or 6-membered heterocycle which is optionally substituted by fluorine, chlorine, methyl and/or ethyl, in particular furanyl or thienyl; furanylmethyl, thienylmethyl or

$R^1$ furthermore represents aryl, arylalkyl, aryloxyalkyl or arylthioalkyl, each of which has 6 to 10 carbon atoms in the aryl moiety and, where appropriate, 1 to 4 carbon atoms in the alkyl moiety and each of which is optionally monosubstituted to pentasubstituted in the aryl moiety by identical or different substituents, suitable aryl substituents being the aryl substituents mentioned under $Ar^1$, $R^1$ furthermore represents the groups $-NH-CO-R^{10}$ or $-CO-O-R^{11}$ in which

$R^{10}$ and $R^{11}$ in each case independently of one another represent $C_1$-$C_4$-alkyl or phenyl,

$R^7$ represents a straight-chain or branched alkyl having 1 to 8 carbon atoms, halogenoalkyl having 1 to 6 carbon atoms and 1 to 12 identical or different halogen atoms such as, in particular, fluorine and chlorine atoms, straight-chain or branched alkenyl having 2 to 12 carbon atoms, halogenoalkenyl having 2 to 6 carbon atoms and 1 to 10 identical or different halogen atoms such as, in particular, fluorine and chlorine atoms, alkoxyalkyl having in each case 1 to 8 carbon atoms in the alkoxy and alkyl moiety, or represents aralkyl which has 1 to 4 carbon atoms in the straight-chain or branched alkyl moiety and 6 to 10 carbon atoms in the aryl moiety and which is optionally monosubstituted to polysubstituted by identical or different substituents, or represents aryl which has 6 to 10 carbon atoms and which is optionally monosubstituted to pentasubstituted by identical or different substituents, suitable aryl substituents in each case being: halogen, straight-chain or branched alkyl having 1 to 4 carbon atoms and dialkylamino having in each case 1 to 4 carbon atoms in the alkyl moiety, $C_1$-$C_4$-alkoxy or halogeno-$C_1$-$C_4$-alkyl,

$Ar^1$ represents aryl which has 6 to 10 carbon atoms, in particular phenyl or naphthyl, and which is monosubstituted to polysubstituted by identical or different substituents, suitable aryl substituents being: halogen; nitro; cyano; carboxyl; alkoxycarbonyl having 1 to 4 carbon atoms, $C_1$-$C_4$-alkyl, $C_1$-$C_4$-alkoxy or $C_1$-$C_4$-alkythio; $C_3$-$C_6$-alkinoxy; halogeno-($C_1$-$C_4$)-alkyl, halogeno-($C_1$-$C_4$)-alkoxy or halogeno-($C_1$-$C_4$)-alkylthio, each of which has 1 to 9 identical or different halogen atoms; phenyl; $C_1$-$C_4$-alkylsulphonyl and halogeno-($C_1$-$C_4$)-alkylsulphonyl having in each case 1 to 9 identical or different halogen atoms; and di-($C_1$-$C_4$)-alkylamino; $Ar^1$ furthermore represents a 6-membered aromatic heterocycle which is optionally substituted and/or optionally fused and which contains at least one nitrogen atom and where suitable substituents are the aryl substituents mentioned above in the case of $Ar^1$, or represents the group

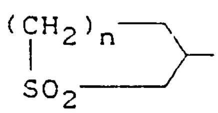

where

n represents the numbers 1 or 2, and their salts,

with the exception of compounds

1-(4-chlorophenyl)-3-methyl-4-piperidinomethylene-2-pyrazolin-5-one
1-(4-chlorophenyl)-3-methyl-4-morpholinomethylene-2-pyrazolin-5-one
1-(4-chlorophenyl)-4-[4-(fluorophenylamino)methylene]-3-methyl-2-pyrazolin-5-one
4-m-toluido-methylene-1-p-tolyl-3-methyl-2-pyrazolin-5-one
4-o-toluido-methylene-1-p-tolyl-3-methyl-2-pyrazolin-5-one
1-p-tolyl-3-methyl-4-o-ethoxyanilinomethylene-2-pyrazolin-5-one
1-p-tolyl-3-methyl-4-p-bromoanilinomethylene-2-pyrazolin-5-one
1-p-tolyl-3-methyl-4-anilinomethylene-2-pyrazolin-5-one
1-o-tolyl-3-methyl-4-m-xylidomethylene-2-pyrazolin-5-one
1-o-tolyl-3-methyl-4-o-ethoxyanilinomethylene-2-pyrazolin-5-one
1-o-tolyl-3-phenyl-4-anilinomethylene-2-pyrazolin-5-one
1-o-tolyl-3-phenyl-4-m-xylidomethylene-2-pyrazolin-5-one
1-o-tolyl-3-phenyl-4-p-chloroanilinomethylene-2-pyrazolin-5-one
4-p-bromanilinomethylene-3-phenyl-1-o-tolyl-2-pyrazolin-5-one
4-m-bromoanilinomethylene-3-phenyl-1-o-tolyl-2-pyrazolin-5-one
1-p-bromophenyl-3-phenyl-4-anilinomethylene-2-pyrazolin-5-one
1-(4-ethoxyphenyl)-3-methyl-4-p-ethoxyanilinomethylene-2-pyrazolin-5-one
4-o-aminoanilinomethylene-1-p-ethoxyphenyl-3-methyl-2-pyrazolin-5-one
4-anilinomethylene-1-p-ethoxyphenyl-3-methyl-2-pyrazolin-5-one
1-(2-methyl-5-benzothiazolyl)-3-methyl-4-phenylaminomethylene-5-pyrazolone
1-p-chlorophenyl-3-methyl-4-anilinomethylene-2-pyrazolin-5-one

1-m-chlorophenyl-3-methyl-4-anilinomethylene-2-pyrazolin-5-one
1-m-trifluoromethylphenyl-3-methyl-4-anilinomethylene-2-pyrazolin-5-one
1-o,o-dichlorophenyl-3-methyl-4-anilinomethylene-2-pyrazolin-5-one
1-m-sulphamoylphenyl-3-methyl-4-anilinomethylene-2-pyrazolin-5-one
4-methylaminomethylene-1-p-bromophenyl-3-methyl-2-pyrazolin-5-one

**5.** Substituted pyrazolin-5-one derivatives of the formula (Ib)

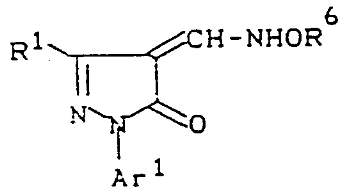

(Ib)

in which

$R^1$ represents hydrogen, straight-chain or branched alkyl having 1 to 8 carbon atoms, cycloalkyl having 3 to 7 carbon atoms, halogenoalkyl having 1 to 4 carbon atoms and 1 to 9 identical or different halogen atoms such as, in particular, fluorine and chlorine atoms, in each case optionally substituted alkenyl or alkinyl, each of which has 2 to 6 carbon atoms, substituents which may be mentioned being unsubstituted phenyl or phenyl which is monosubstituted to trisubstituted by identical or different substituents and suitable phenyl substituents being the aryl substituents mentioned under $Ar^1$; halogenoalkenyl having 2 to 6 carbon atoms and 1 to 10 identical or different halogen atoms such as, in particular, fluorine and chlorine, alkoxy having 1 to 8 carbon atoms, alkoxyalkyl having 1 to 8 carbon atoms in each of the individual alkyl moieties, alkylthioalkyl, alkylsulphonylalkyl or alkylsulphinylalkyl, each of which has 1 to 8 carbon atoms in the individual alkyl moieties, alkoxycarbonylalkyl having 1 to 4 carbon atoms in the alkoxy moiety and 1 to 4 carbon atoms in the alkyl moiety, a 5- or 6-membered heterocycle, which is optionally substituted by fluorine, chlorine, methyl and/or ethyl, in particular furanyl or thienyl; furanylmethyl or thienylmethyl or

$R^1$ furthermore represents aryl, arylalkyl, aryloxyalkyl or arylthioalkyl, each of which has 6 to 10 carbon atoms in the aryl moiety and, where appropriate, 1 to 4 carbon atoms in the alkyl moiety and each of which is optionally monosubstituted to pentasubstituted in the aryl moiety by identical or different substituents, suitable aryl substituents being the aryl substituents mentioned under $Ar^1$; $R^1$ furthermore represents the groups -NH-CO-$R^0$ or -CO-$OR^{11}$ in which

$R^{10}$ and $R^{11}$ in each case independently of one another represent $C_1$-$C_4$-alkyl or phenyl,

$R^6$ represents hydrogen, straight-chain or branched alkyl having 1 to 8 carbon atoms, halogenoalkyl having 1 to 6 carbon atoms and 1 to 12 identical or different halogen atoms such as, in particular, fluorine and chlorine atoms, straight-chain or branched alkenyl having 2 to 12 carbon atoms, halogenoalkenyl having 2 to 6 carbon atoms and 1 to 10 identical or different halogen atoms such as, in particular, fluorine and chlorine atoms, or represents aralkyl which has 1 to 4 carbon atoms in the alkyl moiety and 6 to 10 carbon atoms in the aryl moiety and which is optionally monosubstituted to polysubstituted by identical or different substituents, suitable aryl substituents being halogen, $C_1$-$C_4$-alkyl, halogeno-$C_1$-$C_4$-alkyl and nitro, and

$Ar^1$ represents aryl which has 6 to 10 carbon atoms, in particular phenyl or naphthyl, and which is monosubstituted to polysubstituted by identical or different substituents, suitable aryl substituents being: halogen; nitro; cyano; carboxyl; alkoxycarbonyl having 1 to 4 carbon atoms, $C_1$-$C_4$-alkyl, $C_1$-$C_4$-alkoxy or $C_1$-$C_4$-alkylthio; $C_3$-$C_6$-alkinoxy; halogeno-($C_1$-$C_4$)-alkyl, halogeno-($C_1$-$C_4$)-alkoxy or halogeno-($C_1$-$C_4$)-alkylthio, each of which has 1 to 9 identical or different halogen atoms; phenyl; $C_1$-$C_4$-alkylsulphonyl and halogeno-($C_1$-$C_4$)-alkylsulphonyl which has in each case 1 to 9 identical or different halogen atoms; and di-($C_1$-$C_4$)-alkylamino; $Ar^1$ furthermore

represents a 6-membered aromatic heterocycle which is optionally substituted and/or optionally fused and which contains at least one nitrogen atom and where suitable substituents are the aryl substitutents mentioned above in the case of $Ar^1$, or represents the group

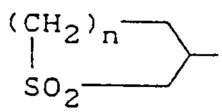

where

n represents the numbers 1 or 2, and their salts.

**6.** Substituted pyrazolin-5-one derivatives of the formula (Ic)

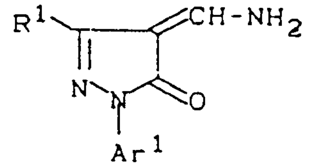

(Ic)

in which

$R^1$ represents hydrogen, straight-chain or branched alkyl having 1 to 8 carbon atoms, cycloalkyl having 3 to 7 carbon atoms, halogenoalkyl having 1 to 4 carbon atoms and 1 to 9 identical or different halogen atoms such as, in particular, fluorine and chlorine atoms, in each case optionally substituted alkenyl or alkinyl, each of which has 2 to 6 carbon atoms, substituents which may be mentioned being unsubstituted phenyl or phenyl which is monosubstituted to trisubstituted by identical or different substituents and suitable phenyl substituents being the aryl substituents mentioned under $Ar^1$; halogenoalkenyl having 2 to 6 carbon atoms and 1 to 10 identical or different halogen atoms such as, in particular, fluorine and chlorine, alkoxy having 1 to 8 carbon atoms, alkoxyalkyl having in each case 1 to 8 carbon atoms in the individual alkyl moieties, alkylthioalkyl, alkylsulphonylalkyl or alkylsulphinylalkyl, each of which has 1 to 8 carbon atoms in the individual alkyl moieties, alkoxycarbonylalkyl having 1 to 4 carbon atoms in the alkoxy moiety and 1 to 4 carbon atoms in the alkyl moiety, a 5- or 6-membered heterocycle which is optionally substituted by fluorine, chlorine, methyl and/or ethyl, in particular furanyl or thienyl; furanylmethyl, thienylmethyl or

$R^1$ furthermore represents aryl, arylalkyl, aryloxyalkyl or arylthioalkyl, each of which has 6 to 10 carbon atoms in the aryl moiety and, where appropriate, 1 to 4 carbon atoms in the alkyl moiety and each of which is optionally monosubstituted to pentasubstituted in the aryl moiety by identical or different substituents, suitable aryl substituents being the aryl substituents mentioned under $Ar^1$, $R^1$ furthermore represents the groups $-NH-CO-R^{10}$ or $-CO-O-R^{11}$ in which

$R^{10}$ and $R^{11}$ in each case independently of one another represent $C_1$-$C_4$-alkyl or phenyl,

$Ar^1$ represents aryl which has 6 to 10 carbon atoms and which is monosubstituted to polysubstituted by identical or different substituents, in particular phenyl or naphthyl, suitable aryl substituents being: halogen; nitro; cyano; carboxyl; alkoxycarbonyl having 1 to 4 carbon atoms, $C_1$-$C_4$-alkyl, $C_1$-$C_4$-alkoxy or $C_1$-$C_4$-alkylthio; $C_3$-$C_6$-alkinoxy; halogeno-($C_1$-$C_4$)-alkyl, halogeno-($C_1$-$C_4$)-alkoxy or halogeno-($C_1$-$C_4$)-alkylthio, each of which has 1 to 9 identical or different halogen atoms; phenyl; $C_1$-$C_4$-alkylsulphonyl and halogeno-($C_1$-$C_4$)-alkylsulphonyl having in each case 1 to 9 identical or different halogen atoms and di-($C_1$-$C_4$)-alkylamino; $Ar^1$ furthermore represents a 6-membered aromatic heterocycle which is optionally substituted and/or optionally fused and which contains at least one nitrogen atom and where

suitable substituents are the aryl substituents mentioned above in the case of $Ar^1$, or represents the group

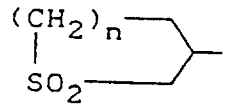

where

n represents the numbers 1 or 2, and their salts,

with the exception of the compounds 4-aminomethylene-1-(2-ethyl-phenyl)-3-methyl-2-pyrazolin-5-one, 4-aminomethylene-1-(4-chlorophenyl)-3-methyl-2-pyrazolin-5-one, 4-aminomethylene-3-methyl-1-(4-nitrophenyl)-2-pyrazolin-5-one.

**7.** Substituted pyrazolin-5-one derivatives of the formula (Id)

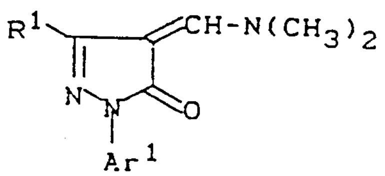

(Id)

in which

$R^1$ represents hydrogen, straight-chain or branched alkyl having 1 to 8 carbon atoms, cycloalkyl having 3 to 7 carbon atoms, halogenoalkyl having 1 to 4 carbon atoms and 1 to 9 identical or different halogen atoms such as, in particular, fluorine and chlorine atoms, in each case optionally substituted alkenyl or alkinyl, each of which has 2 to 6 carbon atoms, substituents which may be mentioned being unsubstituted phenyl or phenyl which is monosubstituted to trisubstituted by identical or different substituents and suitable phenyl substituents being the aryl substituents mentioned under $Ar^1$; $R^1$ furthermore represents halogenoalkenyl having 2 to 6 carbon atoms and 1 to 10 identical or different halogen atoms such as, in particular, fluorine and chlorine, alkoxy having 1 to 8 carbon atoms, alkoxyalkyl having in each case 1 to 8 carbon atoms in the individual alkyl moieties, alkylthioalkyl, alkylsulphonylalkyl or alkylsulphinylalkyl, each of which has 1 to 8 carbon atoms in the individual alkyl moieties, alkoxycarbonylalkyl having 1 to 4 carbon atoms in the alkoxy moiety and 1 to 4 carbon atoms in the alkyl moiety, a 5- or 6-membered heterocycle which is optionally substituted by fluorine, chlorine, methyl and/or ethyl, in particular furanyl or thienyl; furanylmethyl, thienylmethyl or

$R^1$ furthermore represents aryl, arylalkyl, aryloxyalkyl or arylthioalkyl, each of which has 6 to 10 carbon atoms in the aryl moiety and, where appropriate, 1 to 4 carbon atoms in the alkyl moiety and each of which is optionally monosubstituted to pentasubstituted in the aryl moiety by identical or different substituents, suitable aryl substituents being the aryl substituents mentioned under $Ar^1$, $R^1$ furthermore represents the groups -NH-CO-$R^{10}$ or -CO-O-$R^{11}$ in which

$R^{10}$ and $R^{11}$ in each case independently of one another represent $C_1$-$C_4$-alkyl or phenyl,

$Ar^1$ represents aryl which has 6 to 10 carbon atoms and which is monosubstituted to polysubstituted by identical or different substituents, in particular phenyl or naphthyl, suitable aryl substituents being: halogen; nitro; cyano; carboxyl; alkoxycarbonyl having 1 to 4 carbon atoms, $C_1$-$C_4$-alkyl, $C_1$-$C_4$-alkoxy or $C_1$-$C_4$-alkylthio; $C_3$-$C_6$-alkinoxy; halogeno-($C_1$-$C_4$)-alkyl, halogeno-($C_1$-$C_4$)-alkoxy or halogeno-($C_1$-$C_4$)-alkylthio, each of which has 1 to 9 identical or different halogen atoms; phenyl; $C_1$-$C_4$-alkylsulphonyl and halogeno-($C_1$-$C_4$)-alkylsulphonyl having in each case 1 to 9 identical or different halogen atoms and di-($C_1$-$C_4$)-alkylamino; $Ar^1$ furthermore represents a 6-membered aromatic heterocycle which is optionally substituted

and/or optionally fused and which contains at least one nitrogen atom and where suitable substituents are the aryl substituents mentioned above in the case of $Ar^1$, or represents the group

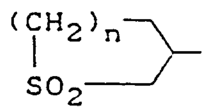

where

n represents the numbers 1 or 2, and their salts,

with the exception of the compounds 1-(4-nitrophenyl)-3-methyl-4-N,N-dimethylamino-methylidenepyrazolin-5-one, 1-(4-(chlorophenyl)-3-(2-nitrophenyl)-4-N,N-dimethylamino-methylidene-2-pyrazolin-5-one, 1-(3-trifluoromethylphenyl)-3-phenyl-4-N,N-dimethylaminomethylidene-2-pyrazolin-5-one, 1-p-sulphophenyl-3-methyl-4-N,N-dimethylaminomethylidene-2-pyrazolin-5-one, 4-N,N-dimethylaminomethylidene-1-p-chlorophenyl-3-methyl-2-pyrazolin-5-one.

**8.** Substituted pyrazolin-5-one derivatives of the formula (If)

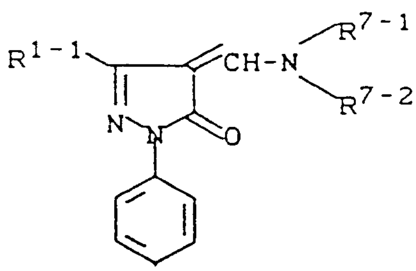

(If)

in which

$R^{1-1}$ represents $C_1$-$C_8$-alkoxy, alkenyl having 2 to 6 carbon atoms, optionally substituted by unsubstituted phenyl or by phenyl which is monosubstituted to trisubstituted by identical or different substituents, suitable phenyl substituents being $C_1$-$C_4$-alkyl, $C_1$-$C_4$-alkoxy, halogeno-($C_1$-$C_4$)alkyl and halogeno-($C_1$-$C_4$)alkoxy, $R^{1-1}$ furthermore represents aryl which has 6 to 10 carbon atoms and which is in each case monosubstituted to pentasubstituted by identical or different substituents or represents aralkyl which has 6 to 10 carbon atoms in the aryl moiety and 1 to 4 carbon atoms in the alkyl moiety and which is optionally substituted, aryl substituents which may be mentioned in each case being halogen, nitro, cyano, carboxyl, $C_1$-$C_4$-alkoxycarbonyl, $C_1$-$C_4$-alkyl, $C_1$-$C_4$-alkoxy, halogeno-($C_1$-$C_4$)-alkyl, halogeno-($C_1$-$C_4$)-alkoxy, halogeno-($C_1$-$C_4$)alkylthio, phenyl, $C_1$-$C_4$-alkylsulphonyl, halogeno-($C_1$-$C_4$)-alkylsulphonyl and di-($C_1$-$C_4$)alkylamino; $R^{1-1}$ furthermore represents a 5- or 6-membered heterocycle which is optionally substituted by fluorine, chlorine, methyl and/or ethyl and which can contain one or two identical or different hetero atoms, in particular nitrogen, oxygen and sulphur; or represents furanyl-$C_1$-$C_4$-alkyl or thienyl-$C_1$-$C_4$-alkyl, each of which is optionally substituted by fluorine, chlorine, methyl and/or ethyl, or

$R^{1-1}$ furthermore represents the group -NH-CO-$R^{10}$ where

$R^{10}$ represents $C_1$-$C_6$-alkyl or phenyl,

$R^{7-1}$ represents hydrogen, $C_1$-$C_8$-alkyl, halogeno-$C_1$-$C_6$-alkyl, $C_2$-$C_6$-alkenyl, halogeno-$C_2$-$C_6$-alkenyl, $C_1$-$C_8$-alkoxy-$C_1$-$C_8$-alkyl, aralkyl which has 6 to 10 carbon atoms in the aryl moiety and 1 to 4 carbon atoms in the alkyl moiety and which is optionally monosubstituted to pentasubstituted by identical or different substituents, or aryl which has 6 to 10 carbon atoms and which is optionally monosubstituted to pentasubstituted by identical or different substituents, aryl substituents which may be mentioned in each case being halogen, $C_1$-$C_4$-alkyl, $C_1$-$C_4$-alkoxy, halogeno-$C_1$-$C_4$-alkyl and di-($C_1$-$C_4$)-alkylamino, and

$R^{7-2}$ represents hydrogen or methyl,

with the exception of the compounds

1-phenyl-3-(4-methoxyphenyl)-4-N,N-dimethylaminomethylidene-2-pyrazolin-5-one
4-N,N-dimethylaminomethylidene-3-(4-formamido-2-pyridyl)-1-phenyl-2-pyrazolin-5-one
1-m-trifluoromethylphenyl-1-phenyl-4-dimethylaminomethylene-2-pyrazolin-5-one
4-N,N-dimethylaminomethylene-1,3-diphenyl-2-pyrazolin-5-one
4-methylaminomethylene-1,3-diphenyl-2-pyrazolin-5-one
4-[4-bromoanilinomethylene]-1,3-diphenyl-2-pyrazolin-5-one
4-p-phenetidinomethylene-1,3-diphenyl-2-pyrazolin-5-one
4-aminomethylene-1,3-diphenyl-2-pyrazolin-5-one
3-methyl-1-phenyl-4-[4-phenylazoanilinomethylene]-2-pyrazolin-5-one
3-methyl-4-p-phenetidinomethylene-1-phenyl-2-pyrazolin-5-one
4-anilinomethylene-3-methyl-1-phenyl-2-pyrazolin-5-one
4-aminomethylene-3-methyl-1-phenyl-2-pyrazolin-5-one.

**9.** Compounds of the formulae

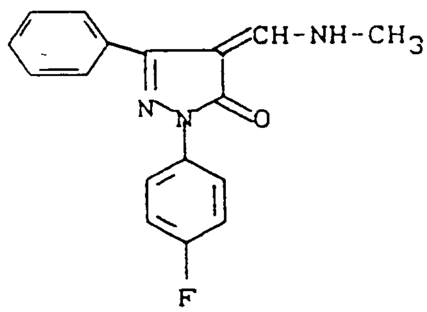

,

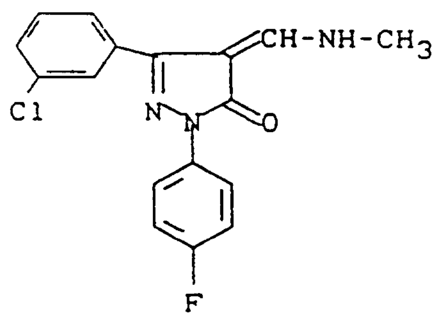

and

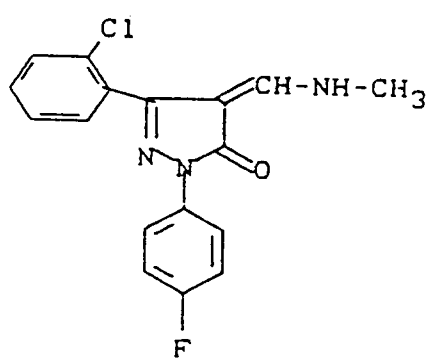

## Revendications

1. Utilisation de dérivés substitués de la pyrazoline-5-one répondant à la formule I

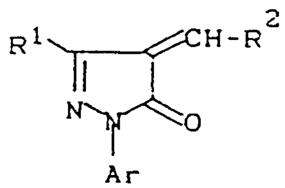

(I)

dans laquelle

$R^1$ représente l'hydrogène, un groupe alkyle à chaîne droite ou ramifiée en $C_1$-$C_8$, cycloalkyle en $C_3$-$C_7$, halogénoalkyle contenant 1 à 4 atomes de carbone et 1 à 9 atomes d'halogènes identiques ou différents, alcényle ou alcynyle contenant chacun 2 à 6 atomes de carbone et chacun d'eux éventuellement substitué, les substituants étant le groupe phényle non substitué ou le groupe phényle portant 1 à 3 substituants identiques ou différents, les substituants du groupe phényle étant les substituants des groupes aryle mentionnés en référence à Ar ; $R^1$ peut en outre représenter un groupe halogénoalcényle contenant 2 à 6 atomes de carbone et 1 à 10 atomes d'halogènes identiques ou différents, un groupe alcoxy en $C_1$-$C_8$, un groupe alcoxyalkyle contenant 1 à 8 atomes de carbone dans chacune des parties alkyle, un groupe alkylthioalkyle, alkylsulfonylalkyle ou alkylsulfinylalkyle contenant chacun 1 à 8 atomes de carbone dans les diverses parties alkyle, un groupe alcoxycarbonylalkyle contenant 1 à 4 atomes de carbone dans la partie alcoxy et 1 à 4 atomes de carbone dans la partie alkyle, un hétérocycle à 5 ou 6 chaînons éventuellement substitué par le fluor, le chlore, des groupes méthyle et/ou éthyle, en particulier un cycle furannyle, thiényle ; un groupe furannylméthyle ou thiénylméthyle, ou bien

$R^1$ peut en outre représenter un groupe aryle, arylalkyle, aryloxyalkyle ou alkythioalkyle contenant chacun 6 à 10 atomes de carbone dans la partie aryle et le cas échéant 1 à 4 atomes de carbone dans la partie alkyle, chacun d'eux portant éventuellement 1 à 5 substituants identiques ou différents dans la partie aryle, ces substituants étant les substituants des groupes aryle énumérés en référence à Ar.

$R^1$ peut en outre représenter les groupements -NH-CO-$R^{10}$ ou -CO-O-$R^{11}$ dans lesquels

$R^{10}$ et $R^{11}$ représentent chacun, indépendamment l'un de l'autre, un groupe alkyle en $C_1$-$C_4$ ou phényle,

$R^2$ représente les groupements -$NHR^3$, -$NR^4R^5$ ou -$NHOR^6$ dans lesquels

$R^3$ représente l'hydrogène, un groupe alkyle à chaîne droite ou ramifiée en $C_1$-$C_8$, halogénoalkyle contenant 1 à 6 atomes de carbone et 1 à 12 atomes d'halogènes identiques ou différents, alcényle à chaîne droite ou ramifiée en $C_2$-$C_{12}$, halogénoalcényle contenant 2 à 6 atomes de carbone et 1 à 10 atomes d'halogènes identiques ou différents, alcoxyalkyle contenant 1 à 8 atomes de carbone dans la partie alcoxy et dans la partie alkyle, aralkyle contenant 1 à 4 atomes de carbone dans la partie alkyle à chaîne droite ou ramifiée et 6 à 10 atomes de carbone dans la partie aryle et portant éventuellement un ou plusieurs substituants identiques ou différents, aryle en $C_6$-$C_{10}$ portant éventuellement 1 à 5 substituants identiques ou différents, les substituants du groupe aryle étant dans chaque cas : des halogènes, des groupes alkyle à chaîne droite ou ramifiée en $C_1$-$C_4$ et dialkylamino contenant 1 à 4 atomes de carbone dans chacune des parties alkyle, alcoxy en $C_1$-$C_4$ et halogénoalkyle en $C_1$-$C_4$,

$R^4$ représente un groupe alkyle en $C_1$-$C_6$,

$R^5$ représente un groupe alkyle en $C_1$-$C_6$,

ou bien

$R^4$ et $R^5$ forment ensemble et avec l'atome d'azote auquel ils sont reliés un hétérocycle à 5 ou 6 chaînons qui peut contenir de l'oxygène, du soufre et/ou de l'azote en tant qu'autres hétéroatomes,

$R^6$ représente l'hydrogène, un groupe alkyle à chaîne droite ou ramifiée en $C_1$-$C_8$,

halogénoalkyle contenant 1 à 10 atomes de carbone et 1 à 12 atomes d'halogènes identiques ou différents, alcényle à chaîne droite ou ramifiée en $C_2$-$C_{12}$, halogénoalcényle contenant 2 à 6 atomes de carbone et 1 à 10 atomes d'halogènes identiques ou différents, aralkyle contenant 1 à 4 atomes de carbone dans la partie alkyle et 6 à 10 atomes de carbone dans la partie aryle et portant le cas échéant un à plusieurs substituants identiques ou différents, les substituants de la partie aryle étant des halogènes, des groupes alkyle en $C_1$-$C_4$, halogénoalkyle en $C_1$-$C_4$ et nitro, et

Ar représente un groupe aryle en $C_6$-$C_{10}$, portant éventuellement un ou plusieurs substituants identiques ou différents, ces substituants étant : des halogènes ; des groupes nitro ; des groupes cyano ; des groupes carboxyle ; des groupes alcoxycarbonyle en $C_1$-$C_4$, alkyle en $C_1$-$C_4$, alcoxy en $C_1$-$C_4$; ou alkylthio en $C_1$-$C_4$ ; alcynyloxy en $C_3$-$C_6$ ; halogénoalkyle en $C_1$-$C_4$, halogénoalcoxy en $C_1$-$C_4$, halogénoalkylthio en $C_1$-$C_4$, avec chacun 1 à 9 atomes d'halogènes identiques ou différents ; phényle ; alkylsulfonyle en $C_1$-$C_4$ et halogénoalkylsulfonyle en $C_1$-$C_4$, avec chacun 1 à 9 atomes d'halogènes identiques ou différents ; et di-(alkyle en $C_1$-$C_4$)-amino ; un hétérocycle aromatique à 6 chaînons éventuellement substitué et/ou éventuellement condensé, qui contient au moins un atome d'azote, les substituants en question étant les substituants des groupes aryle mentionnés ci-dessus en référence à Ar, ou bien le groupe

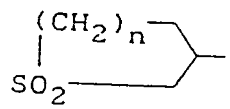

dans lequel

n est égal à 1 ou 2,

et leurs sels,

pour la lutte contre les végétaux adventices ou les mycètes,

à l'exception des composés 1-(4-chlorophényl)-3-méthyl-4-pipéridino-méthylène-pyrazoline-5-one, 1-(4-chlorophényl)-3-méthyl-4-morpholino-méthylène-pyrazoline-5-one, 1-(4-chlorophényl)-4-[4-(fluorophénylamino)-méthylène]-3-méthyl-pyrazoline-5-one, 1-(4-chlorophényl)-3-méthyl-4-aminométhylène-pyrazoline-5-one, et 4-aminométhylène-3-éthoxycarbonyl-1-phényl-2-pyrazoline-5-one.

2. Procédé pour combattre les végétaux adventices ou les mycètes, caractérisé en ce que l'on fait agir les dérivés substitués de la pyrazoline-5-one de formule I de la revendication 1 sur les végétaux adventices, les mycètes ou leur habitat.

3. Produits herbicides et fongicides à base de dérivés substitués de la pyrazoline-5-one de formule I de la revendication 1 pour la lutte contre les végétaux adventices ou les mycètes, à l'exception des composés 1-(4-chlorophényl)-3-méthyl-4-pipéridino-méthylène-pyrazoline-5-one, 1-(4-chlorophényl)-3-méthyl-4-morpholino-méthylène-pyrazoline-5-one, 1-(4-chlorophényl)-4-[4-(fluoro-phénylamino)-méthylène]-3-méthyl-pyrazoline-5-one, 1-(4-chlorophényl)-3-méthyl-4-aminométhylène-pyrazoline-5-one, et 4-aminométhylène-3-éthoxycarbonyl-1-phényl-2-pyrazoline-5-one,

4. Dérivés substitués de la pyrazoline-5-one de formule Ia

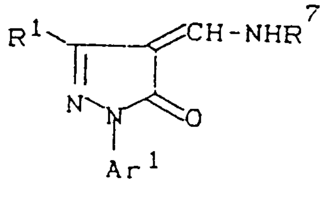

(Ia)

dans laquelle

$R^1$ représente l'hydrogène, un groupe alkyle à chaîne droite ou ramifiée en $C_1$-$C_8$,

cycloalkyle en $C_3$-$C_7$ halogénoalkyle contenant 1 à 4 atomes de carbone et 1 à 9 atomes d'halogènes identiques ou différents, en particulier des atomes de fluor et de chlore, alcényle ou alcynyle, contenant chacun 2 à 6 atomes de carbone et chacun d'eux éventuellement substitué, les substituants étant le groupe phényle non substitué ou le groupe phényle portant 1 à 3 substituants identiques ou différents, les substituants du groupe phényle étant les substituants des groupes aryle énumérés en référence à $Ar^1$ ; $R^1$ peut en outre représenter un groupe halogénoalcényle contenant 2 à 6 atomes de carbone et 1 à 10 atomes d'halogènes identiques ou différents, en particulier de fluor et de chlore, un groupe alcoxy en $C_1$-$C_8$,$C_1$-$C_8$, alcoxyalkyle contenant 1 à 8 atomes de carbone dans les diverses parties alkyle, alkylthioalkyle, alkylsulfonylalkyle ou alkylsulfinylalkyle contenant chacun 1 à 8 atomes de carbone dans les diverses parties alkyle, alcoxycarbonylalkyle contenant 1 ou 4 atomes de carbone dans la partie alcoxy et 1 à 4 atomes de carbone dans la partie alkyle, un hétérocycle à 5 ou 6 chaînons éventuellement substitué par le fluor, le chlore, des groupes méthyle et/ou éthyle, en particulier un cycle furannyle ou thiényle, un groupe furannylméthyle, thiénylméthyle, ou bien

$R^1$ peut en outre représenter un groupe aryle, arylalkyle, aryloxyalkyle ou arylthioalkyle contenant chacun 6 à 10 atomes de carbone dans la partie aryle et le cas échéant 1 à 4 atomes de carbone dans la partie alkyle, et portant chacun le cas échéant 1 à 5 substituants identiques ou différents dans la partie aryle, les substituants de la partie aryle étant les substituants des groupes aryle mentionnés en référence à $Ar^1$, $R^1$ peut en outre représenter les groupements -NH-CO-$R^{10}$ ou -CO-O-$R^{11}$ dans lesquels

$R^{10}$ et $R^{11}$ représentent chacun, indépendamment l'un de l'autre, un groupe alkyle en $C_1$-$C_4$ ou phényle,

$R^7$ représente un groupe alkyle à chaîne droite ou ramifiée en $C_1$-$C_8$, halogénoalkyle contenant 1 à 6 atomes de carbone et 1 à 12 atomes d'halogènes identiques ou différents, en particulier des atomes de fluor et de chlore, alcényle à chaîne droite ou ramifiée en $C_2$-$C_{12}$, halogénoalcényle contenant 2 à 6 atomes de carbone et 1 à 10 atomes d'halogènes identiques ou différents, en particulier des atomes de fluor et de chlore, alcoxyalkyle contenant 1 à 8 atomes de carbone dans la partie alkyle et dans la partie alcoxy, aralkyle contenant 1 à 4 atomes de carbone dans la partie alkyle à chaîne droite ou ramifiée et 6 à 10 atomes de carbone dans la partie aryle et portant le cas échéant un ou plusieurs substituants identiques ou différents, aryle en $C_6$-$C_{10}$ portant le cas échéant 1 à 5 substituants identiques ou différents, les substituants de la partie aryle étant dans chaque cas : des halogènes, des groupes alkyle à chaîne droite ou ramifiée en $C_1$-$C_4$ et dialkylamino contenant 1 à 4 atomes de carbone dans la partie alkyle, alcoxy en $C_1$-$C_4$ ou halogénoalkyle en $C_1$-$C_4$,

$Ar^1$ représente un groupe aryle en $C_6$-$C_{10}$, plus spécialement phényle ou naphtyle, portant 1 à 5 substituants identiques ou différents, les substituants des groupes aryle étant : des halogènes ; des groupes nitro ; des groupes cyano ; des groupes carboxyle ; des groupes alcoxycarbonyle en $C_1$-$C_4$, alkyle en $C_1$-$C_4$, alcoxy en $C_1$-$C_4$ ou alkythio en $C_1$-$C_4$ ; des groupes alcynoxy en $C_3$-$C_6$ ; halogénoalkyle en $C_1$-$C_4$, halogénoalcoxy en $C_1$-$C_4$ ou halogénoalkylthio en $C_1$-$C_4$ contenant chacun 1 à 9 atomes d'halogènes identiques ou différents ; phényle ; alkylsulfonyle en $C_1$-$C_4$ et halogénoalkylsulfonyle en $C_1$-$C_4$ avec 1 à 9 atomes d'halogènes identiques ou différents ; et des groupes di-(alkyle en $C_1$-$C_4$)-amino ; $Ar^1$ peut en outre représenter un hétérocycle aromatique à 6 chaînons éventuellement substitué et/ou éventuellement condensé, qui contient au moins un atome d'azote, les substituants en question étant les substituants des groupes aryle mentionnés ci-dessus en référence en $Ar^1$, ou bien le groupe

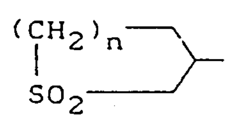

dans lequel

n est égal à 1 ou 2, et leurs sels,

à l'exception des composés suivants :
1-(4-chlorophényl)-3-méthyl-4-pipéridinométhylène-2-pyrazoline-5-one
1-(4-chlorophényl)-3-méthyl-4-morpholinométhylène-2-pyrazoline-5-one
1-(4-chlorophényl)-4-[4-(fluorophénylamino)-méthylène]-3-méthyl-2-pyrazoline-5-one
4-m-toluido-méthylène-1-p-tolyl-3-méthyl-2-pyrazoline-5-one
4-o-toluido-méthylène-1-p-tolyl-3-méthyl-2-pyrazoline-5-one1-p-tolyl-3-méthyl-4-o-éthoxyanilino-méthylène-2-pyrazoline-5-one
1-p-tolyl-3-méthyl-4-p-bromoanilinométhylène-2-pyrazoline-5-one
1-p-tolyl-3-méthyl-4-anilinométhylène-2-pyrazoline-5-one
1-o-tolyl-3-méthyl-4-m-xylidométhylène-2-pyrazoline-5-one
1-o-tolyl-3-méthyl-4-o-éthoxyanilinométhylène-2-pyrazoline-5-one
1-o-tolyl-3-phényl-4-anilinométhylène-2-pyrazoline-5-one
1-o-tolyl-3-phényl-4-m-xylidométhylène-2-pyrazoline-5-one
1-o-tolyl-3-phényl-4-p-chloranilinométhylène-2-pyrazoline-5-one
4-p-bromanilinométhylène-3-phényl-1-o--tolyl-2-pyrazoline-5-one
4-m-bromanilinométhylène-3-phényl-1-o-tolyl-2-pyrazoline-5-one
1-p-bromophényl-3-phényl-4-anilinométhylène-2-pyrazoline-5-one
1-(4-éthoxyphényl)-3-méthyl-4-p-éthoxyanilinométhylène-2--pyrazoline-5-one
4-o-aminoanilinométhylène-1-p-éthoxyphényl-3-méthyl-2-pyrazoline-5-one
4-anilinométhylène-1-p-éthoxyphényl-3-méthyl-2-pyrazoline-5-one
1-(2--méthyl-5-benzothiazolyl)-3-méthyl-4-phénylaminométhylène-5-pyrazolone
1-p-chlorophényl-3-méthyl-4-anilinométhylène-2-pyrazoline-5-one
1-m-chlorophényl-3-méthyl-4-anilinométhylène-2-pyrazoline-5-one
1-m-trifluorométhylphényl-3-méthyl-4-anilinométhylène-2-pyrazoline-5-one
1-o,o-dichorophényl-3-méthyl-4-anilinométhylène-2-pyrazoline-5-one
1-m-sulfamoylphényl-3-méthyl-4-anilinométhylène-2-pyrazoline-5-one
4-méthylaminométhylène-1-p-bromophényl-3-méthyl-2-pyrazoline-5-one

**5.** Dérivés substitués de la pyrazoline-5-one répondant à la formule Ib.

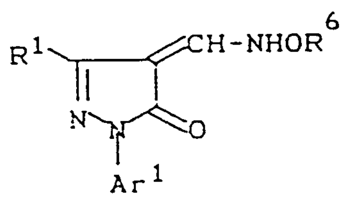

(Ib)

dans laquelle

$R^1$ représente l'hydrogène, un groupe alkyle à chaîne droite ou ramifiée en $C_1$-$C_8$, un groupe cycloalkyle en $C_3$-$C_7$, un groupe halogénoalkyle contenant 1 à 4 atomes de carbone et 1 à 9 atomes d'halogènes identiques ou différents, en particulier des atomes de fluor et de chlore, un groupe alcényle ou alcynyle contenant chacun 2 à 6 atomes de carbone et chacun d'eux éventuellement substitué, les substituants étant le groupe phényle non substitué ou le groupe phényle portant 1 à 3 substituants identiques ou différents, ces substituants étant les substituants des groupes aryles mentionnés en référence à $Ar^1$ ; un groupe halogénoalcényle contenant 2 à 6 atomes de carbone et 1 à 10 atomes d'halogènes identiques ou différents, en particulier des atomes de fluor et de chlore, un groupe alcoxy en $C_1$-$C_8$, un groupe alcoxyalkyle contenant 1 à 8 atomes de carbone dans les diverses parties alkyle, alkylthioalkyle, alkylsulfonylalkyle ou alkylsulfinylalkyle contenant chacun 1 à 8 atomes de carbone dans les diverses parties alkyle, alcoxycarbonylalkyle contenant 1 à 4 atomes de carbone dans la partie alcoxy et 1 à 4 atomes de carbone dans la partie alkyle, un hétérocycle à 5 ou 6 chaînons éventuellement substitué par le fluor, le chlore, les groupes méthyle et/ou éthyle, en particulier un cycle furannyle ou thiényle ; un groupe furannylméthyle ou thiénylméthyle, ou bien

$R^1$ peut en outre représenter un groupe aryle, arylalkyle, aryloxyalkyle ou alkythioalkyle contenant chacun 6 à 10 atomes de carbone dans la partie aryle et le cas échéant 1 à 4 atomes de carbone dans la partie alkyle, portant le cas échéant dans la partie aryle, 1 à 5 substituants identiques ou différents ces substituants étant les substituants des groupes aryle mentionnés en référence à $Ar^1$ ; $R^1$ peut en outre représenter les groupements $-NH-CO-R^{10}$ ou $-CO-O-R^{11}$ dans lesquels

$R^{10}$ et $R^{11}$ représentent chacun, indépendamment l'un de l'autre, un groupe alkyle en $C_1$-$C_4$ ou phényle,

$R^6$ représente l'hydrogène, un groupe alyle à chaîne droite ou ramifiée en $C_1$-$C_8$, un groupe halogénoalkyle contenant 1 à 6 atomes de carbone et 1 à 12 atomes d'halogènes identiques ou différents, en particulier des atomes de fluor et de chlore, un groupe alcényle à chaîne droite ou ramifiée en $C_2$-$C_{12}$, un groupe halogénoalcény-de contenant 2 à 6 atomes de carbone et 1 à 10 atomes d'halogènes identiques ou différents, en particulier des atomes de fluor et de chlore, un groupe aralkyle contenant 1 à 4 atomes de carbone dans la partie alkyle et 6 à 10 atomes de carbone dans la partie aryle, et portant le cas échéant un ou plusieurs substituants identiques ou différents, les substituants des groupes aryle étant des halogènes, des groupes alkyle en $C_1$-$C_4$, halogénoalkyle en $C_1$-$C_4$ et des groupes nitro, et

$Ar^1$ représente un groupe aryle en $C_6$-$C_{10}$, en particulier phényle ou naphtyle, portant un ou plusieurs substituants identiques ou différents, les substituants des groupes aryle étant : des halogènes ; des groupes nitro ; des groupes cyano ; des groupes carboxyle ; des groupes alcoxycarbonyle en $C_1$-$C_4$, alkyle en $C_1$-$C_4$, alcoxy en $C_1$-$C_4$ ou alkylhio en $C_1$-$C_4$ ; alcynoxy en $C_3$-$C_6$ ; halogénoalkyle en $C_1$-$C_4$, halogénoalcoxy en $C_1$-$C_4$ ou halogénoalkylthio en $C_1$-$C_4$ avec chacun 1 à 9 atomes d'halogènes identiques ou différents ; phényle ; alkylsulfonyle en $C_1$-$C_4$ et halogénoalkylsulfonyle en $C_1$-$C_4$ avec 1 à 9 atomes d'halogènes identiques ou différents ; et di-(alkyle en $C_1$-$C_4$)-amino ; $Ar^1$ peut en outre représenter un hétérocycle aromatique à aromatique à 6 chaînons éventuellement substitué et/ou éventuellement condensé, qui contient au moins un atome d'azote, les substituants en question étant les substituants des groupes aryle mentionnés ci-dessus en référence en $Ar^1$, ou bien le groupe

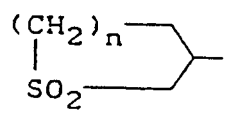

dans lequel

n est égal à 1 ou 2, et leurs sels.

**6.** Dérivés substitués de la pyrazoline-5-one répondant à la formule Ic

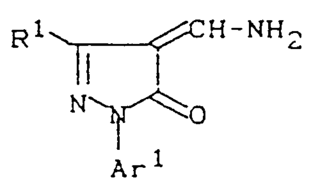

(Ic)

dans laquelle

$R^1$ représente l'hydrogène, un groupe alkyle à chaîne droite ou ramifiée en $C_1$-$C_8$, un groupe cycloalkyle en $C_3$-$C_7$, un groupe halogénoalkyle contenant 1 à 4 atomes de carbone et 1 à 9 atomes d'halogènes identiques ou différents, en particulier des atomes de fluor et de chlore, un groupe alcényle ou alcynyle contenant chacun 2 à 6 atomes de carbone et chacun d'eux éventuellement substitué, les substituants étant le groupe phényle non substitué ou le groupe phényle portant 1 à 3 substituants identiques ou différents, ces substituants étant les substituants du groupe aryle

mentionnés en référence à $Ar^1$ ; un groupe halogénoalcényle contenant 2 à 6 atomes de carbone et 1 à 10 atomes d'halogènes identiques ou différents, en particulier des atomes de fluor et de chlore, un groupe alcoxy en $C_1$-$C_8$, un groupe alcoxyalkyle contenant 1 à 8 atomes de carbone dans les diverses parties alkyle, alkylthioalkyle, alkylsulfonylalkyle ou alkylsulfinylalkyle contenant chacun 1 à 8 atomes de carbone dans les diverses parties alkyle, alcoxycarbonylalkyle contenant 1 à 4 atomes de carbone dans la partie alcoxy et 1 à 4 atomes de carbone dans la partie alkyle, un hétérocycle à 5 ou 6 chaînons éventuellement substitué par le fluor, le chlore, des groupes méthyle et/ou éthyle, en particulier un cycle furannyle ou thiényle ; un groupe furannylméthyle, thiénylméthyle, ou bien

$R^1$ peut en outre représenter un groupe aryle, arylalkyle, aryloxyalkyle et arylthioalkyle contenant chacun 6 à 10 atomes de carbone dans la partie aryle et 1 à 4 atomes de carbone dans la partie alkyle, et portant éventuellement 1 à 5 substituants identiques ou différents, ces substituants étant les substituants des groupes aryle mentionnés en référence à $Ar^1$ ; $R^1$ peut en outre représenter les groupements -NH-CO-$R^{10}$ ou -CO-O-$R^{11}$ dans lesquels

$R^{10}$ et $R^{11}$ représentent chacun, indépendamment l'un de l'autre, un groupe alkyle en $C_1$-$C_4$ ou phényle,

$Ar^1$ représente un groupe aryle en $C_6$-$C_{10}$, en particulier phényle ou naphtyle, portant un ou plusieurs substituants identiques ou différents, les substituants étant : des halogènes ; des groupes nitro ; des groupes cyano ; des groupes carboxyle ; des groupes alcoxycarbonyle en $C_1$-$C_4$, alkyle en $C_1$-$C_4$, alcoxy en $C_1$-$C_4$ ou alkylhio en $C_1$-$C_4$ ; alcynoxy en $C_3$-$C_6$ ; halogénoalkyle en $C_1$-$C_4$, halogénoalcoxy en $C_1$-$C_4$ ou halogénoalkylthio en $C_1$-$C_4$ avec dans chaque cas 1 à 9 atomes d'halogènes identiques ou différents ; phényle ; alkylsulfonyle en $C_1$-$C_4$ et halogénoalkylsulfonyle en $C_1$-$C_4$ avec 1 à 9 atomes d'halogènes identiques ou différents et di-(alkyle en $C_1$-$C_4$)-amino ; $Ar^1$ peut en outre représenter un hétérocycle aromatique à 6 chaînons éventuellement substitué et/ou éventuellement condensé, qui contient au moins un atome d'azote, les substituants étant les substituants des groupes aryle mentionnés ci-dessus en référence en $Ar^1$, ou bien le groupe

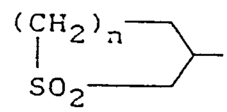

dans lequel

n est égal à 1 ou 2, et leurs sels,

à l'exception des composés : 4-aminométhylène-1-(2-éthylphényl)-3-méthyl-2-pyrazoline-5-one,
4-aminométhylène-1-(4-chlorophényl)-3-méthyl-2-pyrazoline-5-one,
4-aminométhylène-3-méthyl-1-(4-nitrophényl)-2-pyrazoline-5-one,

**7.** Dérivés substitués de la pyrazoline-5-one de formule Id,

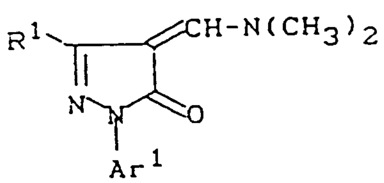

(Id)

dans laquelle

$R^1$ représente l'hydrogène, un groupe alkyle à chaîne droite ou ramifiée en $C_1$-$C_8$, cycloalkyle en $C_3$-$C_7$, halogénoalkyle contenant 1 à 4 atomes de carbone et 1 à 9 atomes d'halogènes identiques ou différents, en particulier des atomes de fluor et de chlore, alcényle ou alcynyle contenant chacun 2 à 4 atomes de carbone et chacun

d'eux éventuellement substitué, les substituants étant le groupe phényle non substitué ou le groupe phényle portant 1 à 3 substituants identiques ou différents, ces substituants étant les substituants des groupes aryle mentionnés en référence à $Ar^1$ ; $R^1$ peut en outre représenter un groupe halogénoalcényle contenant 2 à 6 atomes de carbone et 1 à 10 atomes d'halogènes identiques ou différents, en particulier de fluor et de chlore, un groupe alcoxy en $C_1$-$C_8$, alcoxyalkyle contenant 1 à 8 atomes de carbone dans chacune des parties alkyle, alkylthioalkyle, alkylsulfonylalkyle ou alkylsulfinylalkyle contenant chacun 1 à 8 atomes de carbone dans les diverses parties alkyle, alcoxycarbonylalkyle contenant 1 à 4 atomes de carbone dans la partie alcoxy et 1 à 4 atomes de carbone dans la partie alkyle, un hétérocycle à 5 ou 6 chaînons éventuellement substitué par le fluor, le chlore, des groupes méthyle et/ou éthyle, en particulier un cycle furannyle ou thiényle ; un groupe furannylméthyle ou thiénylméthyle, ou bien

$R^1$ peut en outre représenter un groupe aryle, arylalkyle, aryloxyalkyle ou arylthioalkyle contenant chacun 6 à 10 atomes de carbone dans la partie aryle et le cas échéant 1 à 4 atomes de carbone dans la partie alkyle, et portant chacun le cas échéant dans la partie aryle 1 à 5 substituants identiques ou différents, ces substituants étant les substituants des groupes aryle mentionnés en référence à $Ar^1$, $R^1$ peut en outre représenter les groupements -NH-CO-$R^{10}$ ou -CO-O-$R^{11}$ dans lesquels

$R^{10}$ et $R^{11}$ représentent chacun, indépendamment l'un de l'autre, un groupe alkyle en $C_1$-$C_4$ ou phényle,

$Ar^1$ représente un groupe aryle en $C_6$-$C_{10}$, en particulier phényle ou naphtyle, portant un ou plusieurs substituants identiques ou différents, les substituants du groupe aryle étant : des halogènes ; des groupes nitro, des groupes cyano ; des groupes carboxyle ; des groupes alcoxycarbonyle en $C_1$-$C_4$, alkyle en $C_1$-$C_4$, alcoxy en $C_1$-$C_4$ ou alkylthio en $C_1$-$C_4$ ; alcynoxy en $C_3$-$C_6$ ; halogénoalkyle en $C_1$-$C_4$, halogénoalcoxy en $C_1$-$C_4$ ou halogénoalkylthio en $C_1$-$C_4$, avec dans chaque cas 1 à 9 atomes d'halogènes identiques ou différents ; phényle ; alkylsulfonyle en $C_1$-$C_4$ et halogénoalkylsulfonyle en $C_1$-$C_4$ avec dans chaque cas 1 à 9 atomes d'halogènes identiques ou différents, et di-(alkyle en $C_1$-$C_4$), amino ; ou encore un hétérocycle aromatique à 6 chaînons éventuellement substitué et/ou éventuellement condensé, qui contient au moins un atome d'azote, les substituants étant les substituants des goupes aryle mentionnés en référence à $Ar^1$, ou bien le groupe

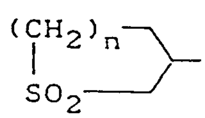

dans lequel

n est égal à 1 ou 2, et leurs sels,

à l'exception des composés : 1--(4--nitrophényl)--3--méthyl--4--N,N-diméthylamino-méthylidène-pyrazoline-5-one, 1--(4--chlorophényl)--3-(2-nitrophényl)--4-N,N-diméthylamino-méthylidène-2-pyrazoline-5-one, 1-(3-trifluorométhyl-phényl)--3--phényl--4--N,N-diméthylaminométhylidène-2-pyrazoline--5--one, 1--p-sulfophényl--3--méthyl-4-N,N--diméthylamino-méthylidène-2--pyrazoline--5-one, 4--N,N--diméthylaminométhylidène-1-p-chlorophényl-3-méthyl-2-pyrazoline-5-one.

**8.** Dérivés substitués de la pyrazoline-5-one de formule If :

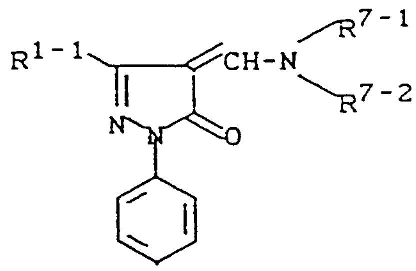

(If)

dans laquelle

$R^{1-1}$ représente un groupe alcoxy en $C_1$-$C_8$, alcényle en $C_2$-$C_6$, éventuellement substitué par un groupe phényle non substitué ou par un groupe phényle portant 1 à 3 substituants identiques ou différents, les substituants du groupe phényle étant des groupes alkyle en $C_1$-$C_4$, alcoxy en $C_1$-$C_4$, halogénoalkyle en $C_1$-$C_4$ et halogénoalcoxy en $C_1$-$C_4$, $R^{1-1}$ peut en outre représenter un groupe aryle en $C_6$-$C_{10}$ portant 1 à 5 substituants identiques ou différents ou un groupe aralkyle éventuellement substitué contenant 6 à 10 atomes de carbone dans la partie aryle et 1 à 4 atomes de carbone dans la partie alkyle, les substituants des parties aryle étant dans chaque cas des halogènes, des groupes nitro, cyano, carboxyle, alcoxcarbonyle en $C_1$-$C_4$, alkyle en $C_1$-$C_4$, alcoxy en $C_1$-$C_4$, halogénoalkyle en $C_1$-$C_4$, halogénoalcoxy en $C_1$-$C_4$, halogénoalkythio en $C_1$-$C_4$, phényle, alkylsulfonyle en $C_1$-$C_4$, halogénoalkylsulfonyle en $C_1$-$C_4$ et di-(alkyle en $C_1$-$C_4$)-amino ; $R^{1-1}$ peut en outre représenter un hétérocycle à 5 ou 6 chaînons éventuellement substitué par le fluor, le chlore, des groupes méthyle et/ou éthyle, et qui peut contenir 1 ou 2 hétéroatomes identiques ou différents, en particulier d'azote, d'oxygène et de soufre ; un groupe furannylalkyle en $C_1$-$C_4$ ou thiényl-alkyle en $C_1$-$C_4$, chacun d'eux éventuellement substitué par le fluor, le chlore, des groupes méthyle et/ou éthyle, ou bien

$R^{1-1}$ peut en outre représenter le groupement -NH-CO-$R^{10}$ dans lequel

$R^{10}$ représente un groupe alkyle en $C_1$-$C_6$ ou phényle,

$R^{7-1}$ représente l'hydrogène, un groupe alkyle en $C_1$-$C_8$, halogénoalkyle en $C_1$-$C_6$, alcényle en $C_2$-$C_6$, halogénoalcényle en $C_2$-$C_6$ (alcoxy en $C_1$-$C_8$)alkyle en $C_1$-$C_8$, aralkyle contenant 6 à 10 atomes de carbone dans la partie aryle et 1 à 4 atomes de carbone dans la partie alkyle et portant éventuellement 1 à 5 substituants identiques ou différents, ou bien un groupe aryle en $C_6$-$C_{10}$ portant éventuellement 1 à 5 substituants identiques ou différents, les substituants des groupes aryles étant dans chaque cas des halogènes, des groupes alkyle en $C_1$-$C_4$, alcoxy en $C_1$-$C_4$, halogénoalkyle en $C_1$-$C_4$ et di-(alkyle en $C_1$-$C_4$)-amino, et

$R^{7-2}$ représente l'hydrogène ou un groupe méthyle,

à l'exception des composés suivants :

1-phényl-3-(4-méthoxyphényl)-4-N,N,-diméthylaminométhylidène-2-pyrazoline-5-one
4-N,N-diméthylaminométhylidène-3-(4-formamido-2-pyridyl)-1-phényl-2-pyrazoline-5-one
1-m-trifluorométhylphényl-1-phényl-4-diméthylaminométhylène-2-pyrazoline-5-one
4-N,N-diméthylaminométhylène-1,3-diphényl-2-pyrazoline-5-one
4-méthylaminométhylène-1,3-diphényl-2-pyrazoline-5-one
4-[4-bromanilinométhylène]-1,3-diphényl-2-pyrazoline-5-one
4-p-phénétidinométhylène-1,3-diphényl-2-pyrazoline-5-one
4-aminométhylène-1,3-diphényl-2-pyrazoline-5-one
3-méthyl-1-phényl-4-[4-phénylazoanilinométhylène]-2-pyrazoline-5-one
3-méthyl-4-p-phénétidinométhylène-1-phényl-2-pyrazoline-5-one
4-anilinométhylène-3-méthyl-1-phényl-2-pyrazoline-5-one
4-aminométhylène-3-méthyl-1-phényl-2-pyrazoline-5-one

**9.** Composés de formules

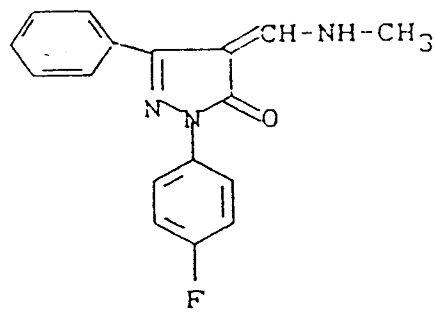

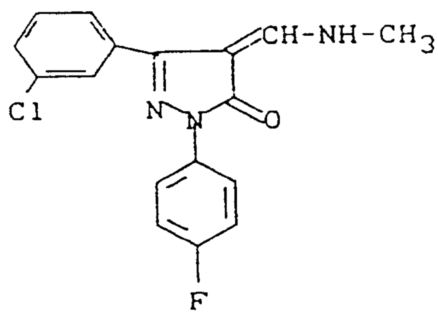

et